# EUROPEAN PATENT SPECIFICATION

(11) **EP 1 914 319 B1**
(45) Date of publication and mention of the grant of the patent: **12.11.2014**
(21) Application number: 07018218.3
(22) Date of filing: 30.09.1997
(51) Int. Cl.: C12Q 1/68, C07H 21/04, C12N 15/63

(54) **Polymorphisms in the region of the human hemochromatosis gene**
Polymorphismen im Bereich menschlicher Hämochromatose-Gene
Polymorphismes dans la région du gène de l'hémochromatose humaine

(30) Priority: 01.10.1996 US 724394; 07.05.1997 US 852495
(43) Date of publication of application: 23.04.2008
(62) Divisional of application: 97910741.4
(73) Proprietor: Bio-Rad Laboratories, Inc., Hercules, CA 94547 (US)
(72) Inventor: Feder, John, N., Belle Mead, NJ 08502 (US); Kronmal, Gregory, S., Livermore, CA 94550 (US); Lauer, Peter, M., San Francisco CA 94131 (US); Ruddy, David, A., San Francisco CA 94133 (US); Thomas, Winston, J., San Mateo CA 94402 (US); Tsuchihashi, Zenta, Skillman, NJ 08558 (US); Wolff, Roger, K., Mill Valley CA 94941 (US)
(74) Representative: Weber, Martin

(56) References cited:
- WO-A-97/38137
- FEDER ET AL: "A NOVEL MHC CLASS I-LIKE GENE IS MUTATED IN PATIENTS WITH HEREDITARY HAEMOCHROMATOSIS" NATURE GENETICS, NEW YORK, NY, US, vol. 13, August 1996 (1996-08), pages 399-408, XP002113585 ISSN: 1061-4036

### SEQUENCE LISTING

<110> Progenitor, Inc.
<120> POLYMORPHISMS AND NEW GENES IN THE
   REGION OF THE HUMAN HEMOCHROMATOSIS GENE
<130> 8907-0057-227
<140> EP 97910741.4
   <141> 1997-09-30
<150> PCT/US97/17658
   <151> 1997-09-30
<150> US 08/852,495
   <151> 1997-05-07
<150> US 08/724,394
   <151> 1997-10-01
<160> 49
<170> FastSEQ for Windows Version 3.0
<210> 1
   <211> 526
   <212> PRT
   <213> Homo sapien
<400> 1
   Met Ala Val Phe Pro Ser Ser Gly Leu Pro Arg Cys Leu Leu Thr Leu
   1               5                   10                  15
   Ile Leu Leu Gln Leu Pro Lys Leu Asp Ser Ala Pro Phe Asp Val Ile
               20                  25                  30
   Gly Pro Pro Glu Pro Ile Leu Ala Val Val Gly Glu Asp Ala Glu Leu
           35                  40                  45
   Pro Cys Arg Leu Ser Pro Asn Ala Ser Ala Glu His Leu Glu Leu Arg
       50                  55                  60
   Trp Phe Arg Lys Lys Val Ser Pro Ala Val Leu Val His Arg Asp Gly
   65                  70                  75                  80
   Arg Glu Gln Glu Ala Glu Gln Met Pro Glu Tyr Arg Gly Arg Ala Thr
                   85                  90                  95
   Leu Val Gln Asp Gly Ile Ala Lys Gly Arg Val Ala Leu Arg Ile Arg
               100                 105                 110
   Gly Val Arg Val Ser Asp Asp Gly Glu Tyr Thr Cys Phe Phe Arg Glu
           115                 120                 125
   Asp Gly Ser Tyr Glu Glu Ala Leu Val His Leu Lys Val Ala Ala Leu
       130                 135                 140
   Gly Ser Asp Pro His Ile Ser Met Gln Val Gln Glu Asn Gly Glu Ile
   145                 150                 155                 160
   Cys Leu Glu Cys Thr Ser Val Gly Trp Tyr Pro Glu Pro Gln Val Gln
                   165                 170                 175
   Trp Arg Thr Ser Lys Gly Glu Lys Phe Pro Ser Thr Ser Glu Ser Arg
               180                 185                 190
   Asn Pro Asp Glu Glu Gly Leu Phe Thr Val Ala Ala Ser Val Ile Ile
           195                 200                 205
   Arg Asp Thr Ser Thr Lys Asn Val Ser Cys Tyr Ile Gln Asn Leu Leu
       210                 215                 220
   Leu Gly Gln Glu Lys Lys Val Glu Ile Ser Ile Pro Ala Ser Ser Leu
   225                 230                 235                 240
   Pro Arg Leu Thr Pro Trp Ile Val Ala Val Ala Val Ile Leu Met Val
                   245                 250                 255
   Leu Gly Leu Leu Thr Ile Gly Ser Ile Phe Phe Thr Trp Arg Leu Tyr
               260                 265                 270
   Asn Glu Arg Pro Arg Glu Arg Arg Asn Glu Phe Ser Ser Lys Glu Arg
           275                 280                 285
   Leu Leu Glu Glu Leu Lys Trp Lys Lys Ala Thr Leu His Ala Val Asp
       290                 295                 300
   Val Thr Leu Asp Pro Asp Thr Ala His Pro His Leu Phe Leu Tyr Glu
   305                 310                 315                 320
   Asp Ser Lys Ser Val Arg Leu Glu Asp Ser Arg Gln Lys Leu Pro Glu
                   325                 330                 335
   Lys Thr Glu Arg Phe Asp Ser Trp Pro Cys Val Leu Gly Arg Glu Thr
               340                 345                 350
   Phe Thr Ser Gly Arg His Tyr Trp Glu Val Glu Val Gly Asp Arg Thr
           355                 360                 365
   Asp Trp Ala Ile Gly Val Cys Arg Glu Asn Val Met Lys Lys Gly Phe
       370                 375                 380
   Asp Pro Met Thr Pro Glu Asn Gly Phe Trp Ala Val Glu Leu Tyr Gly
   385                 390                 395                 400
   Asn Gly Tyr Trp Ala Leu Thr Pro Leu Arg Thr Pro Leu Pro Leu Ala
                   405                 410                 415
   Gly Pro Pro Arg Arg Val Gly Ile Phe Leu Asp Tyr Glu Ser Gly Asp
               420                 425                 430
   Ile Ser Phe Tyr Asn Met Asn Asp Gly Ser Asp Ile Tyr Thr Phe Ser
           435                 440                 445
   Asn Val Thr Phe Ser Gly Pro Leu Arg Phe Phe Phe Cys Leu Trp Ser
       450                 455                 460
   Ser Gly Lys Lys Pro Leu Thr Ile Cys Pro Ile Ala Asp Gly Pro Glu
   465                 470                 475                 480
   Arg Val Thr Val Ile Ala Asn Ala Gln Asp Leu Ser Lys Glu Ile Pro
                   485                 490                 495
   Leu Ser Pro Met Gly Glu Glu Ser Ala Pro Arg Asp Ala Asp Thr Leu
               500                 505                 510
   His Ser Lys Leu Ile Pro Thr Gln Pro Ser Gln Gly Ala Pro
           515                 520                 525
<210> 2
   <211> 527
   <212> PRT
   <213> Homo sapien
<400> 2
   Met Glu Ser Ala Ala Ala Leu His Phe Ser Arg Pro Ala Ser Leu Leu
    1               5                  10                  15
   Leu Leu Leu Leu Ser Leu Cys Ala Leu Val Ser Ala Gln Phe Ile Val
               20                  25                  30
   Val Gly Pro Thr Asp Pro Ile Leu Ala Thr Val Gly Glu Asn Thr Thr
           35                  40                  45
   Leu Arg Cys His Leu Ser Pro Glu Lys Asn Ala Glu Asp Met Glu Val
       50                  55                  60
   Arg Trp Phe Arg Ser Gln Phe Ser Pro Ala Val Phe Val Tyr Lys Gly
   65                  70                  75                  80
   Gly Arg Glu Arg Thr Glu Glu Gln Met Glu Glu Tyr Arg Gly Arg Thr
                   85                  90                  95
   Thr Phe Val Ser Lys Asp Ile Ser Arg Gly Ser Val Ala Leu Val Ile
               100                 105                 110
   His Asn Ile Thr Ala Gln Glu Asn Gly Thr Tyr Arg Cys Tyr Phe Gln
           115                 120                 125
   Glu Gly Arg Ser Tyr Asp Glu Ala Ile Leu His Leu Val Val Ala Gly
       130                 135                 140
   Leu Gly Ser Lys Pro Leu Ile Ser Met Arg Gly His Glu Asp Gly Gly
   145                 150                 155                 160
   Ile Arg Leu Glu Cys Ile Ser Arg Gly Trp Tyr Pro Lys Pro Leu Thr
                   165                 170                 175
   Val Trp Arg Asp Pro Tyr Gly Gly Val Ala Pro Ala Leu Lys Glu Val
               180                 185                 190
   Ser Met Pro Asp Ala Asp Gly Leu Phe Met Val Thr Thr Ala Val Ile
           195                 200                 205
   Ile Arg Asp Lys Ser Val Arg Asn Met Ser Cys Ser Ile Asn Asn Thr
       210                 215                 220
   Leu Leu Gly Gln Lys Lys Glu Ser Val Ile Phe Ile Pro Glu Ser Phe
   225                 230                 235                 240
   Met Pro Ser Val Ser Pro Cys Ala Val Ala Leu Pro Ile Ile Val Val
                   245                 250                 255
   Ile Leu Met Ile Pro Ile Ala Val Cys Ile Tyr Trp Ile Asn Lys Leu
               260                 265                 270
   Gln Lys Glu Lys Lys Ile Leu Ser Gly Glu Lys Glu Phe Glu Arg Glu
           275                 280                 285
   Thr Arg Glu Ile Ala Leu Lys Glu Leu Glu Lys Glu Arg Val Gln Lys
       290                 295                 300
   Glu Glu Glu Leu Gln Val Lys Glu Lys Leu Gln Glu Glu Leu Arg Trp
   305                 310                 315                 320
   Arg Arg Thr Phe Leu His Ala Val Asp Val Val Leu Asp Pro Asp Thr
                   325                 330                 335
   Ala His Pro Asp Leu Phe Leu Ser Glu Asp Arg Arg Ser Val Arg Arg
               340                 345                 350
   Cys Pro Phe Arg His Leu Gly Glu Ser Val Pro Asp Asn Pro Glu Arg
           355                 360                 365
   Phe Asp Ser Gln Pro Cys Val Leu Gly Arg Glu Ser Phe Ala Ser Gly
       370                 375                 380
   Lys His Tyr Trp Glu Val Glu Val Glu Asn Val Ile Glu Trp Thr Val
   385                 390                 395                 400
   Gly Val Cys Arg Asp Ser Val Glu Arg Lys Gly Glu Val Leu Leu Ile
                   405                 410                 415
   Pro Gln Asn Gly Phe Trp Thr Leu Glu Met His Lys Gly Gln Tyr Arg
               420                 425                 430
   Ala Val Ser Ser Pro Asp Arg Ile Leu Pro Leu Lys Glu Ser Leu Cys
           435                 440                 445
   Arg Val Gly Val Phe Leu Asp Tyr Glu Ala Gly Asp Val Ser Phe Tyr
       450                 455                 460
   Asn Met Arg Asp Arg Ser His Ile Tyr Thr Cys Pro Arg Ser Ala Phe
   465                 470                 475                 480
   Ser Val Pro Val Arg Phe Phe Phe Arg Leu Gly Cys Glu Asp Ser Pro
                   485                 490                 495
   Ile Phe Ile Cys Pro Ala Leu Thr Gly Ala Asn Gly Val Thr Val Pro
               500                 505                 510
   Glu Glu Gly Leu Thr Leu His Arg Val Gly Thr His Gln Ser Leu
           515                 520                 525
<210> 3
   <211> 523
   <212> PRT
   <213> Homo sapien
<400> 3
   Met Glu Pro Ala Ala Ala Leu His Phe Ser Leu Pro Ala Ser Leu Leu
    1               5                  10                  15
   Leu Leu Leu Leu Leu Leu Leu Leu Ser Leu Cys Ala Leu Val Ser Ala
               20                  25                  30
   Gln Phe Thr Val Val Gly Pro Ala Asn Pro Ile Leu Ala Met Val Gly
           35                  40                  45
   Glu Asn Thr Thr Leu Arg Cys His Leu Ser Pro Glu Lys Asn Ala Glu
       50                  55                  60
   Asp Met Glu Val Arg Trp Phe Arg Ser Gln Phe Ser Pro Ala Val Phe
   65                  70                  75                  80
   Val Tyr Lys Gly Gly Arg Glu Arg Thr Glu Glu Gln Met Glu Glu Tyr
                   85                  90                  95
   Arg Gly Arg Ile Thr Phe Val Ser Lys Asp Ile Asn Arg Gly Ser Val
               100                 105                 110
   Ala Leu Val Ile His Asn Val Thr Ala Gln Glu Asn Gly Ile Tyr Arg
           115                 120                 125
   Cys Tyr Phe Gln Glu Gly Arg Ser Tyr Asp Glu Ala Ile Leu Arg Leu
       130                 135                 140
   Val Val Ala Gly Leu Gly Ser Lys Pro Leu Ile Glu Ile Lys Ala Gln
   145                 150                 155                 160
   Glu Asp Gly Ser Ile Trp Leu Glu Cys Ile Ser Gly Gly Trp Tyr Pro
                   165                 170                 175
   Glu Pro Leu Thr Val Trp Arg Asp Pro Tyr Gly Glu Val Val Pro Ala
               180                 185                 190
   Leu Lys Glu Val Ser Ile Ala Asp Ala Asp Gly Leu Phe Met Val Thr
           195                 200                 205
   Thr Ala Val Ile Ile Arg Asp Lys Tyr Val Arg Asn Val Ser Cys Ser
       210                 215                 220
   Val Asn Asn Thr Leu Leu Gly Gln Glu Lys Glu Thr Val Ile Phe Ile
   225                 230                 235                 240
   Pro Glu Ser Phe Met Pro Ser Ala Ser Pro Trp Met Val Ala Leu Ala
                   245                 250                 255
   Val Ile Leu Thr Ala Ser Pro Trp Met Val Ser Met Thr Val Ile Leu
               260                 265                 270
   Ala Val Phe Ile Ile Phe Met Ala Val Ser Ile Cys Cys Ile Lys Lys
           275                 280                 285
   Leu Gln Arg Glu Lys Lys Ile Leu Ser Gly Glu Lys Lys Val Glu Gln
       290                 295                 300
   Glu Glu Lys Glu Ile Ala Gln Gln Leu Gln Glu Glu Leu Arg Trp Arg
   305                 310                 315                 320
   Arg Thr Phe Leu His Ala Ala Asp Val Val Leu Asp Pro Asp Thr Ala
                   325                 330                 335
   His Pro Glu Leu Phe Leu Ser Glu Asp Arg Arg Ser Val Arg Arg Gly
               340                 345                 350
   Pro Tyr Arg Gln Arg Val Pro Asp Asn Pro Glu Arg Phe Asp Ser Gln
           355                 360                 365
   Pro Cys Val Leu Gly Trp Glu Ser Phe Ala Ser Gly Lys His Tyr Trp
       370                 375                 380
   Glu Val Glu Val Glu Asn Val Met Val Trp Thr Val Gly Val Cys Arg
   385                 390                 395                 400
   His Ser Val Glu Arg Lys Gly Glu Val Leu Leu Ile Pro Gln Asn Gly
                   405                 410                 415
   Phe Trp Thr Leu Glu Met Phe Gly Asn Gln Tyr Arg Ala Leu Ser Ser
               420                 425                 430
   Pro Glu Arg Ile Leu Pro Leu Lys Glu Ser Leu Cys Arg Val Gly Val
           435                 440                 445
   Phe Leu Asp Tyr Glu Ala Gly Asp Val Ser Phe Tyr Asn Met Arg Asp
       450                 455                 460
   Arg Ser His Ile Tyr Thr Cys Pro Arg Ser Ala Phe Thr Val Pro Val
   465                 470                 475                 480
   Arg Phe Phe Phe Arg Leu Gly Ser Asp Asp Ser Pro Ile Phe Ile Cys
                   485                 490                 495
   Pro Ala Leu Thr Gly Ala Ser Gly Val Met Val Pro Glu Glu Gly Leu
               500                 505                 510
   Lys Leu His Arg Val Gly Thr His Gln Ser Leu
           515                 520
<210> 4
   <211> 513
   <212> PRT
   <213> Homo sapien
<400> 4
   Met Lys Met Ala Ser Phe Leu Ala Phe Leu Leu Leu Asn Phe Arg Val
   1               5                   10                  15
   Cys Leu Leu Leu Leu Gln Leu Leu Met Pro His Ser Ala Gln Phe Ser
               20                  25                  30
   Val Leu Gly Pro Ser Gly Pro Ile Leu Ala Met Val Gly Glu Asp Ala
           35                  40                  45
   Asp Leu Pro Cys His Leu Phe Pro Thr Met Ser Ala Glu Thr Met Glu
       50                  55                  60
   Leu Lys Trp Val Ser Ser Ser Leu Arg Gln Val Val Asn Val Tyr Ala
   65                  70                  75                  80
   Asp Gly Lys Glu Val Glu Asp Arg Gln Ser Ala Pro Tyr Arg Gly Arg
                   85                  90                  95
   Thr Ser Ile Leu Arg Asp Gly Ile Thr Ala Gly Lys Ala Ala Leu Arg
               100                 105                 110
   Ile His Asn Val Thr Ala Ser Asp Ser Gly Lys Tyr Leu Cys Tyr Phe
           115                 120                 125
   Gln Asp Gly Asp Phe Tyr Glu Lys Ala Leu Val Glu Leu Lys Val Ala
       130                 135                 140
   Ala Leu Gly Ser Asp Leu His Val Asp Val Lys Gly Tyr Lys Asp Gly
   145                 150                 155                 160
   Gly Ile His Leu Glu Cys Arg Ser Thr Gly Trp Tyr Pro Gln Pro Gln
                   165                 170                 175
   Ile Gln Trp Ser Asn Asn Lys Gly Glu Asn Ile Pro Thr Val Glu Ala
               180                 185                 190
   Pro Val Val Ala Asp Gly Val Gly Leu Tyr Ala Val Ala Ala Ser Val
           195                 200                 205
   Ile Met Arg Gly Ser Ser Gly Glu Gly Val Ser Cys Thr Ile Arg Ser
       210                 215                 220
   Ser Leu Leu Gly Leu Glu Lys Thr Ala Ser Ile Ser Ile Ala Asp Pro
   225                 230                 235                 240
   Phe Phe Arg Ser Ala Gln Arg Trp Ile Ala Ala Leu Ala Arg Thr Leu
                   245                 250                 255
   Pro Val Leu Leu Leu Leu Leu Gly Gly Ala Gly Tyr Phe Leu Trp Gln
               260                 265                 270
   Gln Gln Glu Glu Lys Lys Thr Gln Phe Arg Lys Lys Lys Arg Glu Gln
           275                 280                 285
   Glu Leu Arg Glu Met Ala Trp Ser Thr Met Lys Gln Glu Gln Ser Thr
       290                 295                 300
   Arg Val Lys Leu Leu Glu Glu Leu Arg Trp Arg Ser Ile Gln Tyr Ala
   305                 310                 315                 320
   Ser Arg Gly Glu Arg His Ser Ala Tyr Asn Glu Trp Lys Lys Ala Leu
                   325                 330                 335
   Phe Lys Pro Ala Asp Val Ile Leu Asp Pro Lys Thr Ala Asn Pro Ile
               340                 345                 350
   Leu Leu Val Ser Glu Asp Gln Arg Ser Val Gln Arg Ala Lys Glu Pro
           355                 360                 365
   Gln Asp Leu Pro Asp Asn Pro Glu Arg Phe Asn Trp His Tyr Cys Val
       370                 375                 380
   Leu Gly Cys Glu Ser Phe Ile Ser Gly Arg His Tyr Trp Glu Val Glu
   385                 390                 395                 400
   Val Gly Asp Arg Lys Glu Trp His Ile Gly Val Cys Ser Lys Asn Val
                   405                 410                 415
   Gln Arg Lys Gly Trp Val Lys Met Thr Pro Glu Asn Gly Phe Trp Thr
               420                 425                 430
   Met Gly Leu Thr Asp Gly Asn Lys Tyr Arg Thr Leu Thr Glu Pro Arg
           435                 440                 445
   Thr Asn Leu Lys Leu Pro Lys Pro Pro Lys Lys Val Gly Val Phe Leu
       450                 455                 460
   Asp Tyr Glu Thr Gly Asp Ile Ser Phe Tyr Asn Ala Val Asp Gly Ser
   465                 470                 475                 480
   His Ile His Thr Phe Leu Asp Val Ser Phe Ser Glu Ala Leu Tyr Phe
                   485                 490                 495
   Val Phe Arg Ile Leu Thr Leu Glu Pro Thr Ala Leu Ser Ile Cys Pro
                   500                 505                 510
   Ala
<210> 5
   <211> 584
   <212> PRT
   <213> Homo sapien
<400> 5
   Met Lys Met Ala Ser Ser Leu Ala Phe Leu Leu Leu Asn Phe His Val
    1               5                  10                  15
   Ser Leu Phe Leu Val Gln Leu Leu Thr Pro Cys Ser Ala Gln Phe Ser
               20                  25                  30
   Val Leu Gly Pro Ser Gly Pro Ile Leu Ala Met Val Gly Glu Asp Ala
           35                  40                  45
   Asp Leu Pro Cys His Leu Phe Pro Thr Met Ser Ala Glu Thr Met Glu
       50                  55                  60
   Leu Arg Trp Val Ser Ser Ser Leu Arg Gln Val Val Asn Val Tyr Ala
   65                  70                  75                  80
   Asp Gly Lys Glu Val Glu Asp Arg Gln Ser Ala Pro Tyr Arg Gly Arg
                   85                  90                  95
   Thr Ser Ile Leu Arg Asp Gly Ile Thr Ala Gly Lys Ala Ala Leu Arg
               100                 105                 110
   Ile His Asn Val Thr Ala Ser Asp Ser Gly Lys Tyr Leu Cys Tyr Phe
           115                 120                 125
   Gln Asp Gly Asp Phe Tyr Glu Lys Ala Leu Val Glu Leu Lys Val Ala
       130                 135                 140
   Ala Leu Gly Ser Asp Leu His Ile Glu Val Lys Gly Tyr Glu Asp Gly
   145                 150                 155                 160
   Gly Ile His Leu Glu Cys Arg Ser Thr Gly Trp Tyr Pro Gln Pro Gln
                   165                 170                 175
   Ile Lys Trp Ser Asp Thr Lys Gly Glu Asn Ile Pro Ala Val Glu Ala
               180                 185                 190
   Pro Val Val Ala Asp Gly Val Gly Leu Tyr Ala Val Ala Ala Ser Val
           195                 200                 205
   Ile Met Arg Gly Ser Ser Gly Gly Gly Val Ser Cys Ile Ile Arg Asn
       210                 215                 220
   Ser Leu Leu Gly Leu Glu Lys Thr Ala Ser Ile Ser Ile Ala Asp Pro
   225                 230                 235                 240
   Phe Phe Arg Ser Ala Gln Pro Trp Ile Ala Ala Leu Ala Gly Thr Leu
                   245                 250                 255
   Pro Ile Ser Leu Leu Leu Leu Ala Gly Ala Ser Tyr Phe Leu Trp Arg
               260                 265                 270
   Gln Gln Lys Glu Lys Ile Ala Leu Ser Arg Glu Thr Glu Arg Glu Arg
           275                 280                 285
   Glu Met Lys Glu Met Gly Tyr Ala Ala Thr Glu Gln Glu Ile Ser Leu
       290                 295                 300
   Arg Glu Lys Leu Gln Glu Glu Leu Lys Trp Arg Lys Ile Gln Tyr Met
   305                 310                 315                 320
   Ala Arg Gly Glu Lys Ser Leu Ala Tyr His Glu Trp Lys Met Ala Leu
                   325                 330                 335
   Phe Lys Pro Ala Asp Val Ile Leu Asp Pro Asp Thr Ala Asn Ala Ile
               340                 345                 350
   Leu Leu Val Ser Glu Asp Gln Arg Ser Val Gln Arg Ala Glu Glu Pro
           355                 360                 365
   Arg Asp Leu Pro Asp Asn Pro Glu Arg Phe Glu Trp Arg Tyr Cys Val
       370                 375                 380
   Leu Gly Cys Glu Asn Phe Thr Ser Gly Arg His Tyr Trp Glu Val Glu
   385                 390                 395                 400
   Val Gly Asp Arg Lys Glu Trp His Ile Gly Val Cys Ser Lys Asn Val
                   405                 410                 415
   Glu Arg Lys Lys Gly Trp Val Lys Met Thr Pro Glu Asn Gly Tyr Trp
   420 425 430
   Thr Met Gly Leu Thr Asp Gly Asn Lys Tyr Arg Ala Leu Thr Glu Pro
   435 440 445
   Arg Thr Asn Leu Lys Leu Pro Glu Pro Pro Arg Lys Val Gly Ile Phe
   450 455 460
   Leu Asp Tyr Glu Thr Gly Glu Ile Ser Phe Tyr Asn Ala Thr Asp Gly
   465 470 475 480
   Ser His Ile Tyr Thr Phe Pro His Ala Ser Phe Ser Glu Pro Leu Tyr
   485 490 495
   Phe Val Phe Arg Ile Leu Thr Leu Glu Pro Thr Ala Leu Thr Ile Cys
   500 505 510
   Pro Ile Pro Lys Glu Val Glu Ser Ser Pro Asp Pro Asp Leu Val Pro
   515 520 525
   Asp His Ser Leu Glu Thr Pro Leu Thr Pro Gly Leu Ala Asn Glu Ser
   530 535 540
   Gly Glu Pro Gln Ala Glu Val Thr Ser Leu Leu Leu Pro Ala His Pro
   545 550 555 560
   Gly Ala Glu Val Ser Pro Ser Ala Thr Thr Asn Gln Asn His Lys Leu
   565 570 575
   Gln Ala Arg Thr Glu Ala Leu Tyr
   580
<210> 6
   <211> 319
   <212> PRT
   <213> Homo sapien
<400> 6
   Met Lys Met Ala Ser Ser Leu Ala Phe Leu Leu Leu Asn Phe His Val
   1 5 10 15
   Ser Leu Leu Leu Val Gln Leu Leu Thr Pro Cys Ser Ala Gln Phe Ser
   20 25 30
   Val Leu Gly Pro Ser Gly Pro Ile Leu Ala Met Val Gly Glu Asp Ala
   35 40 45
   Asp Leu Pro Cys His Leu Phe Pro Thr Met Ser Ala Glu Thr Met Glu
   50 55 60
   Leu Lys Trp Val Ser Ser Ser Leu Arg Gln Val Val Asn Val Tyr Ala
   65 70 75 80
   Asp Gly Lys Glu Val Glu Asp Arg Gln Ser Ala Pro Tyr Arg Gly Arg
   85 90 95
   Thr Ser Ile Leu Arg Asp Gly Ile Thr Ala Gly Lys Ala Ala Leu Arg
   100 105 110
   Ile His Asn Val Thr Ala Ser Asp Ser Gly Lys Tyr Leu Cys Tyr Phe
   115 120 125
   Gln Asp Gly Asp Phe Tyr Glu Lys Ala Leu Val Glu Leu Lys Val Ala
   130 135 140
   Ala Leu Gly Ser Asn Leu His Val Glu Val Lys Gly Tyr Glu Asp Gly
   145 150 155 160
   Gly Ile His Leu Glu Cys Arg Ser Thr Gly Trp Tyr Pro Gln Pro Gln
   165 170 175
   Ile Gln Trp Ser Asn Ala Lys Gly Glu Asn Ile Pro Ala Val Glu Ala
   180 185 190
   Pro Val Val Ala Asp Gly Val Gly Leu Tyr Glu Val Ala Ala Ser Val
   195 200 205
   Ile Met Arg Gly Gly Ser Gly Glu Gly Val Ser Cys Ile Ile Arg Asn
   210 215 220
   Ser Leu Leu Gly Leu Glu Lys Thr Ala Ser Ile Ser Ile Ala Asp Pro
   225 230 235 240
   Phe Phe Arg Ser Ala Gln Pro Trp Ile Ala Ala Leu Ala Gly Thr Leu
   245 250 255
   Pro Ile Leu Leu Leu Leu Leu Ala Gly Ala Ser Tyr Phe Leu Trp Arg
   260 265 270
   Gln Gln Lys Glu Ile Thr Ala Leu Ser Ser Glu Ile Glu Ser Glu Gln
   275 280 285
   Glu Met Lys Glu Met Gly Tyr Ala Ala Thr Glu Arg Glu Ile Ser Leu
   290 295 300
   Arg Glu Ser Leu Gln Glu Glu Leu Lys Arg Lys Lys Ser Ser Thr
   305 310 315
<210> 7
   <211> 475
   <212> PRT
   <213> Homo sapien
<400> 7
<210> 8
   <211> 465
   <212> PRT
   <213> Homo sapien
<400> 8
   Met Ala Ser Thr Thr Ser Thr Lys Lys Met Met Glu Glu Ala Thr Cys
   1               5                   10                  15
   Ser Ile Cys Leu Ser Leu Met Thr Asn Pro Val Ser Ile Asn Cys Gly
               20                  25                  30
   His Ser Tyr Cys His Leu Cys Ile Thr Asp Phe Phe Lys Asn Pro Ser
           35                  40                  45
   Gln Lys Gln Leu Arg Gln Glu Thr Phe Cys Cys Pro Gln Cys Arg Ala
       50                  55                  60
   Pro Phe His Met Asp Ser Leu Arg Pro Asn Lys Gln Leu Gly Ser Leu
   65                  70                  75                  80
   Ile Glu Ala Leu Lys Glu Thr Asp Gln Glu Met Ser Cys Glu Glu His
                   85                  90                  95
   Gly Glu Gln Phe His Leu Phe Cys Glu Asp Glu Gly Gln Leu Ile Cys
               100                 105                 110
   Trp Arg Cys Glu Arg Ala Pro Gln His Lys Gly His Thr Thr Ala Leu
           115                 120                 125
   Val Glu Asp Val Cys Gln Gly Tyr Lys Glu Lys Leu Gln Lys Ala Val
       130                 135                 140
   Thr Lys Leu Lys Gln Leu Glu Asp Arg Cys Thr Glu Gln Lys Leu Ser
   145                 150                 155                 160
   Thr Ala Met Arg Ile Thr Lys Trp Lys Glu Lys Val Gln Ile Gln Arg
                   165                 170                 175
   Gln Lys Ile Arg Ser Asp Phe Lys Asn Leu Gln Cys Phe Leu His Glu
               180                 185                 190
   Glu Glu Lys Ser Tyr Leu Trp Arg Leu Glu Lys Glu Glu Gln Gln Thr
           195                 200                 205
   Leu Ser Arg Leu Arg Asp Tyr Glu Ala Gly Leu Gly Leu Lys Ser Asn
       210                 215                 220
   Glu Leu Lys Ser His Ile Leu Glu Leu Glu Glu Lys Cys Gln Gly Ser
   225                 230                 235                 240
   Ala Gln Lys Leu Leu Gln Asn Val Asn Asp Thr Leu Ser Arg Ser Trp
                   245                 250                 255
   Ala Val Lys Leu Glu Thr Ser Glu Ala Val Ser Leu Glu Leu His Thr
               260                 265                 270
   Met Cys Asn Val Ser Lys Leu Tyr Phe Asp Val Lys Lys Met Leu Arg
           275                 280                 285
   Ser His Gln Val Ser Val Thr Leu Asp Pro Asp Thr Ala His His Glu
       290                 295                 300
   Leu Ile Leu Ser Glu Asp Arg Arg Gln Val Thr Arg Gly Tyr Thr Gln
   305                 310                 315                 320
   Glu Asn Gln Asp Thr Ser Ser Arg Arg Phe Thr Ala Phe Pro Cys Val
                   325                 330                 335
   Leu Gly Cys Glu Gly Phe Thr Ser Gly Arg Arg Tyr Phe Glu Val Asp
               340                 345                 350
   Val Gly Glu Gly Thr Gly Trp Asp Leu Gly Val Cys Met Glu Asn Val
           355                 360                 365
   Gln Arg Gly Thr Gly Met Lys Gln Glu Pro Gln Ser Gly Phe Trp Thr
       370                 375                     380
   Leu Arg Leu Cys Lys Lys Lys Gly Tyr Val Ala Leu Thr Ser Pro Pro
   385                 390                 395                 400
   Thr Ser Leu His Leu His Glu Gln Pro Leu Leu Val Gly Ile Phe Leu
                   405                 410                 415
   Asp Tyr Glu Ala Gly Val Val Ser Phe Tyr Asn Gly Asn Thr Gly Cys
               420                 425                 430
   His Ile Phe Thr Phe Pro Lys Ala Ser Phe Ser Asp Thr Leu Arg Pro
           435                 440                 445
   Tyr Phe Gln Val Tyr Gln Tyr Ser Pro Leu Phe Leu Pro Pro Pro Gly
       450                 455                 460
   Asp
   465
<210> 9
   <211> 467
   <212> PRT
   <213> Homo sapien
<400> 9
   Met Gln Met Asp Asn Arg Leu Pro Pro Lys Lys Val Pro Gly Phe Cys
    1               5                  10                  15
   Ser Phe Arg Tyr Gly Leu Ser Phe Leu Val His Cys Cys Asn Val Ile
               20                  25                  30
   Ile Thr Ala Gln Arg Ala Cys Leu Asn Leu Thr Met Val Val Met Val
           35                  40                  45
   Asn Ser Thr Asp Pro His Gly Leu Pro Asn Thr Ser Thr Lys Lys Leu
       50                  55                  60
   Leu Asp Asn Ile Lys Asn Pro Met Tyr Asn Trp Ser Pro Asp Ile Gln
   65                  70                  75                  80
   Gly Ile Ile Leu Ser Ser Thr Ser Tyr Gly Val Ile Ile Ile Gln Val
                   85                  90                  95
   Pro Val Gly Tyr Phe Ser Gly Ile Tyr Ser Thr Lys Lys Met Ile Gly
               100                 105                 110
   Phe Ala Leu Cys Leu Ser Ser Val Leu Ser Leu Leu Ile Pro Pro Ala
           115                 120                 125
   Ala Gly Ile Gly Val Ala Trp Val Val Val Cys Arg Ala Val Gln Gly
       130                 135                 140
   Ala Ala Gln Gly Ile Val Ala Thr Ala Gln Phe Glu Ile Tyr Val Lys
   145                 150                 155                 160
   Trp Ala Pro Pro Leu Glu Arg Gly Arg Leu Thr Ser Met Ser Thr Ser
                   165                 170                 175
   Gly Phe Leu Leu Gly Pro Phe Ile Val Leu Leu Val Thr Gly Val Ile
               180                 185                 190
   Cys Glu Ser Leu Gly Trp Pro Met Val Phe Tyr Ile Phe Gly Ala Cys
           195                 200                 205
   Gly Cys Ala Val Cys Leu Leu Trp Phe Val Leu Phe Tyr Asp Asp Pro
       210                 215                 220
   Lys Asp His Pro Cys Ile Ser Ile Ser Glu Lys Glu Tyr Ile Thr Ser
   225                 230                 235                 240
   Ser Leu Val Gln Gln Val Ser Ser Ser Arg Gln Ser Leu Pro Ile Lys
                   245                 250                 255
   Ala Ile Leu Lys Ser Leu Pro Val Trp Ala Ile Ser Ile Gly Ser Phe
               260                 265                 270
   Thr Phe Phe Trp Ser His Asn Ile Met Thr Leu Tyr Thr Pro Met Phe
           275                 280                 285
   Ile Asn Ser Met Leu His Val Asn Ile Lys Glu Asn Gly Phe Leu Ser
       290                 295                 300
   Ser Leu Pro Tyr Leu Phe Ala Trp Ile Cys Gly Asn Leu Ala Gly Gln
   305                 310                 315                 320
   Leu Ser Asp Phe Phe Leu Thr Arg Asn Ile Leu Ser Val Ile Ala Val
                   325                 330                 335
   Arg Lys Leu Phe Thr Ala Ala Gly Phe Leu Leu Pro Ala Ile Phe Gly
   340 345 350
   Val Cys Leu Pro Tyr Leu Ser Ser Thr Phe Tyr Ser Ile Val Ile Phe
   355 360 365
   Leu Ile Leu Ala Gly Ala Thr Gly Ser Phe Cys Leu Gly Gly Val Phe
   370 375 380
   Ile Asn Gly Leu Asp Ile Ala Pro Arg Tyr Phe Gly Phe Ile Lys Ala
   385 390 395 400
   Cys Ser Thr Leu Thr Gly Met Ile Gly Gly Leu Ile Ala Ser Thr Leu
   405 410 415
   Thr Gly Leu Ile Leu Lys Gln Asp Pro Glu Ser Ala Trp Phe Lys Thr
   420 425 430
   Phe Ile Leu Met Ala Ala Ile Asn Val Thr Gly Leu Ile Phe Tyr Leu
   435 440 445
   Ile Val Ala Thr Ala Glu Ile Gln Asp Trp Ala Lys Glu Lys Gln His
   450 455 460
   Thr Arg Leu
   465
<210> 10
   <211> 436
   <212> PRT
   <213> Homo sapien
<400> 10
   Met Asp Gly Lys Pro Ala Thr Arg Lys Gly Pro Asp Phe Cys Ser Leu
   1 5 10 15
   Arg Tyr Gly Leu Ala Leu Ile Met His Phe Ser Asn Phe Thr Met Ile
   20 25 30
   Thr Gln Arg Val Ser Leu Ser Ile Ala Ile Ile Ala Met Val Asn Thr
   35 40 45
   Thr Gln Gln Gln Gly Leu Ser Asn Ala Ser Thr Glu Gly Pro Val Ala
   50 55 60
   Asp Ala Phe Asn Asn Ser Ser Ile Ser Ile Lys Glu Phe Asp Thr Lys
   65 70 75 80
   Ala Ser Val Tyr Gln Trp Ser Pro Glu Thr Gln Gly Ile Ile Phe Ser
   85 90 95
   Ser Ile Asn Tyr Gly Ile Ile Leu Thr Leu Ile Pro Ser Gly Tyr Leu
   100 105 110
   Ala Gly Ile Phe Gly Ala Lys Lys Met Leu Gly Ala Gly Leu Leu Ile
   115 120 125
   Ser Ser Leu Leu Thr Leu Phe Thr Pro Leu Ala Ala Asp Phe Gly Val
   130 135 140
   Ile Leu Val Ile Met Val Arg Thr Val Gln Gly Met Ala Gln Gly Met
   145 150 155 160
   Ala Trp Thr Gly Gln Phe Thr Ile Trp Ala Lys Trp Ala Pro Pro Leu
   165 170 175
   Glu Arg Ser Lys Leu Thr Thr Ile Ala Gly Ser Gly Ser Ala Phe Gly
   180 185 190
   Ser Phe Ile Ile Leu Cys Val Gly Gly Leu Ile Ser Gln Ala Leu Ser
   195 200 205
   Trp Pro Phe Ile Phe Tyr Ile Phe Gly Ser Thr Gly Cys Val Cys Cys
   210 215 220
   Leu Leu Trp Phe Thr Val Ile Tyr Asp Asp Pro Met His His Pro Cys
   225 230 235 240
   Ile Ser Val Arg Glu Lys Glu His Ile Leu Ser Ser Leu Ala Gln Gln
   245 250 255
   Pro Ser Ser Pro Gly Arg Ala Val Pro Ile Lys Ala Met Val Thr Cys
   260 265 270
   Leu Pro Leu Trp Ala Ile Phe Leu Gly Phe Phe Ser His Phe Trp Leu
   275 280 285
   Cys Thr Ile Ile Leu Thr Tyr Leu Pro Thr Tyr Ile Ser Thr Leu Leu
   290 295 300
   His Val Asn Ile Arg Asp Ser Gly Val Leu Ser Ser Leu Pro Phe Ile
   305                 310                 315                 320
   Ala Ala Ala Ser Cys Thr Ile Leu Gly Gly Gln Leu Ala Asp Phe Leu
                   325                 330                 335
   Leu Ser Arg Asn Leu Leu Arg Leu Ile Thr Val Arg Lys Leu Phe Ser
               340                 345                 350
   Ser Leu Asp Met Gln Val Ser Ser Trp Glu Ser Gln Gly Asp Leu Gly
           355                 360                 365
   Ser Ser Gln Glu Ser Ser Leu Pro Leu Pro Leu Asp Ser Ser Ser Val
       370                 375                 380
   Arg Ile Leu Ser Leu Val Gly Gly Met Ser Phe Ser Cys Leu Leu Gln
   385                 390                 395                 400
   Ser Thr Cys Leu Ala Trp Ser Phe Thr Ser Arg Leu Asp Lys Gln Asn
                   405                 410                 415
   Phe Lys Thr Gly Pro Lys Arg Gly Pro Leu Pro Ala Ser Glu Asp Ile
               420                 425                 430
   Lys Leu Gln Thr
           435
<210> 11
   <211> 401
   <212> PRT
   <213> Homo sapien
<400> 11
   Met Gln Val Asp Glu Thr Leu Ile Pro Arg Lys Gly Pro Ser Leu Cys
    1               5                  10                  15
   Ser Ala Arg Tyr Gly Ile Ala Leu Val Leu His Phe Cys Asn Phe Thr
               20                  25                  30
   Thr Ile Ala Gln Asn Val Ile Met Asn Ile Thr Met Val Ala Met Val
           35                  40                  45
   Asn Ser Thr Ser Pro Gln Ser Gln Leu Asn Asp Ser Ser Glu Val Leu
       50                  55                  60
   Pro Val Asp Ser Phe Gly Gly Leu Ser Lys Ala Pro Lys Ser Leu Pro
   65                  70                  75                  80
   Ala Lys Ser Ser Ile Leu Gly Gly Gln Phe Ala Ile Trp Glu Lys Trp
                   85                  90                  95
   Gly Pro Pro Gln Glu Arg Ser Arg Leu Cys Ser Ile Ala Leu Ser Gly
               100                 105                 110
   Met Leu Leu Gly Cys Phe Thr Ala Ile Leu Ile Gly Gly Phe Ile Ser
           115                 120                 125
   Glu Thr Leu Gly Trp Pro Phe Val Phe Tyr Ile Phe Gly Gly Val Gly
       130                 135                 140
   Cys Val Cys Cys Leu Leu Trp Phe Val Val Ile Tyr Asp Asp Pro Phe
   145                 150                 155                 160
   Ser Tyr Pro Trp Ile Ser Thr Ser Glu Lys Glu Tyr Ile Ile Ser Ser
                   165                 170                 175
   Leu Lys Gln Gln Val Gly Ser Ser Lys Gln Pro Leu Pro Ile Lys Ala
               180                 185                 190
   Met Leu Arg Ser Leu Pro Ile Trp Ser Ile Cys Leu Gly Cys Phe Ser
           195                 200                 205
   His Gln Trp Leu Val Ser Thr Met Val Val Tyr Ile Pro Thr Tyr Ile
       210                 215                 220
   Ser Ser Val Tyr His Val Asn Ile Arg Asp Asn Gly Leu Leu Ser Ala
   225                 230                 235                 240
   Leu Pro Phe Ile Val Ala Trp Val Ile Gly Met Val Gly Gly Tyr Leu
                   245                 250                 255
   Ala Asp Phe Leu Leu Thr Lys Lys Phe Arg Leu Ile Thr Val Arg Lys
               260                 265                 270
   Ile Ala Thr Ile Leu Gly Ser Leu Pro Ser Ser Ala Leu Ile Val Ser
           275                 280                 285
   Leu Pro Tyr Leu Asn Ser Gly Tyr Ile Thr Ala Thr Ala Leu Leu Thr
       290                 295                 300
   Leu Ser Cys Gly Leu Ser Thr Leu Cys Gln Ser Gly Ile Tyr Ile Asn
   305                 310                 315                 320
   Val Leu Asp Ile Ala Pro Arg Tyr Ser Ser Phe Leu Met Gly Ala Ser
                   325                 330                 335
   Arg Gly Phe Ser Ser Ile Ala Pro Val Ile Val Pro Thr Val Ser Gly
               340                 345                 350
   Phe Leu Leu Ser Gln Asp Pro Glu Phe Gly Trp Arg Asn Val Phe Phe
           355                 360                 365
   Leu Leu Phe Ala Val Asn Leu Leu Gly Leu Leu Phe Tyr Leu Ile Phe
       370                 375                 380
   Gly Glu Ala Asp Val Gln Glu Trp Ala Lys Glu Arg Lys Leu Thr Arg
   385                 390                 395                 400
   Leu
<210> 12
   <211> 2882
   <212> DNA
   <213> Homo sapien
<400> 12
<210> 13
   <211> 2926
   <212> DNA
   <213> Homo sapien
<400> 13
   acgcgtccgc ttcggaatga gagactcaac cataatagaa agaatggaga actattaacc      60
   accattcttc agtgggctgt gattttcaga ggggaatact aagaaatggt tttccatact     120
   ggaacccaaa ggtaaagaca ctcaaggaca gacatttttg gcagagcata gatgaaaatg     180
   gcaagttccc tggctttcct tctgctcaac tttcatgtct ccctcttctt ggtccagctg     240
   ctcactcctt gctcagctca gttttctgtg cttggaccct ctgggcccat cctggccatg     300
   gtgggtgaag acgctgatct gccctgtcac ctgttcccga ccatgagtgc agagaccatg     360
   gagctgaggt gggtgagttc cagcctaagg caggtggtga acgtgtatgc agatggaaag     420
   gaagtggaag acaggcagag tgcaccatat cgagggagaa cttcgattct gcgggatggc     480
   atcactgcag ggaaggctgc tctccgaata cacaacgtca cagcctctga cagtggaaag     540
   tacttgtgtt atttccaaga tggtgacttc tacgaaaaag ccctggtgga gctgaaggtt     600
   gcagcattgg gttctgatct tcacattgaa gtgaagggtt atgaggatgg agggatccat     660
   ctggagtgca ggtccactgg ctggtacccc caaccccaaa taaagtggag cgacaccaag     720
   ggagagaaca tcccggctgt ggaagcacct gtggttgcag atggagtggg cctgtatgca     780
   gtagcagcat ctgtgatcat gagaggcagc tctggtgggg gtgtatcctg catcatcaga     840
   aattccctcc tcggcctgga aaagacagcc agcatatcca tcgcagaccc cttcttcagg     900
   agcgcccagc cctggatcgc ggccctggca gggaccctgc ctatctcgtt gctgcttctc     960
   gcaggagcca gttacttctt gtggagacaa cagaaggaaa aaattgctct gtccagggag    1020
   acagaaagag agcgagagat gaaagaaatg ggatacgctg caacagagca agaaataagc    1080
   ctaagagaga agctccagga ggaactcaag tggaggaaaa tccagtacat ggctcgtgga    1140
   gagaagtctt tggcctatca tgaatggaaa atggccctct tcaaacctgc ggatgtgatt    1200
   ctggatccag acacggcaaa cgccatcctc cttgtttctg aggaccagag gagtgtgcag    1260
   cgtgctgaag agccgcggga tctgccagac aaccctgaga gatttgaatg gcgttactgt    1320
   gtccttggct gtgaaaactt cacatcaggg agacattact gggaggtgga agtgggggac    1380
   agaaaagagt ggcatattgg ggtatgtagt aagaacgtgg agaggaaaaa aggttgggtc    1440
   aaaatgacac cggagaacgg atactggact atgggcctga ctgatgggaa taagtatcgg    1500
   gctctcactg agcccagaac caacctgaaa cttcctgagc ctcctaggaa agtggggatc    1560
   ttcctggact atgagactgg agagatctcg ttctataatg ccacagatgg atctcatatc    1620
   tacacctttc cgcacgcctc tttctctgag cctctatatc ctgttttcag aattttgacc    1680
   ttggagccca ctgccctgac catttgccca ataccaaaag aagtagagag ttcccccgat    1740
   cctgacctag tgcctgatca ttccctggag acaccactga ccccgggctt agctaatgaa    1800
   agtggggagc ctcaggctga agtaacatct ctgcttctcc ctgcccaccc tggagctgag    1860
   gtctcccctt ctgcaacaac caatcagaac cataagctac aggcacgcac tgaagcactt    1920
   tactgatatt cattccatta ttccatatga cagttgtttt gagtttcgta ccaccttatt    1980
   gtccccttat acagataagg aaactggggt gcagaaaggt gaattaactt tacaaagtag    2040
   acatgacaag tgaacagcag agctgggatc taaacagcaa taactaacat taacagagaa    2100
   tttaaaatgt tcttagtgct gtgttataag ctttggtgga tgtcactcct ttaatcctca    2160
   caacaccctg tcgggtagtc atattttgca agtatggaag ctgaggcagg gcaacatgaa    2220
   gtaacttaca taattcatac agtaatttgt gcagttggga gatgttcagc cttagtccct    2280
   ggctaattgc ctgttctttt ccagcctgat tttttttccc acaggaagag cccacatgta    2340
   gccctgaggt ttccttccca ggacagctgc agggtagaga tcattttaag tgcttgtgga    2400
   gttgacatcc ctattgactc tttcccagct gatatcagag acttagaccc agcactcctt    2460
   ggattagctc tgcagagtgt cttggttgag agaataacct catagtacca acatgacatg    2520
   tgacttggaa agagactaga ggccacactt gataaatcat ggggcacaga tatgttccca    2580
   cccaacaaat gtgataagtg attgtgcagc cagagccagc cttccttcaa tcaaggtttc    2640
   caggcagagc aaatacccta gagattctct gtgatatagg aaatttggat caaggaagct    2700
   aaaagaatta cagggatgtt tttaatccca ctatggactc agtctcctgg aaataggtct    2760
   gtccactcct ggtcattggt ggatgttaaa cccatattcc tttcaactgc tgcctgctag    2820
   ggaaaactgc tcctcattat catcactatt attgctcacc actgtatccc ctctacttgg    2880
   caagtggttg tcaagttcta gttgttcaat aaatgtgtta ataatg                   2926
<210> 14
   <211> 1645
   <212> DNA
   <213> Homo sapien
<400> 14
   atttgctttc tctttttcct ttcttccgga tgagaggcta agccataata gaaagaatgg      60
   agaattattg attgaccgtc tttattctgt gggctctgat tctccaatgg gaataccaag     120
   ggatggtttt ccatactgga acccaaaggt aaagacactc aaggacagac atttttggca     180
   gagcatagat gaaaatggca agttccctgg ctttccttct gctcaacttt catgtctccc     240
   tcctcttggt ccagctgctc actccttgct cagctcagtt ttctgtgctt ggaccctctg     300
   ggcccatcct ggccatggtg ggtgaagacg ctgatctgcc ctgtcacctg ttcccgacca     360
   tgagtgcaga gaccatggag ctgaagtggg taagttccag cctaaggcag gtggtgaacg     420
   tgtatgcaga tggaaaggaa gtggaagaca ggcagagtgc accgtatcga gggagaactt     480
   cgattctgcg ggatggcatc actgcaggga aggctgctct ccgaatacac aacgtcacag     540
   cctctgacag tggaaagtac ttgtgttatt tccaagatgg tgacttctat gaaaaagccc     600
   tggtggagct gaaggttgca gcactgggtt ctaatcttca cgtcgaagtg aagggttatg     660
   aggatggagg gatccatctg gagtgcaggt ccaccggctg gtacccccaa ccccaaatac     720
   agtggagcaa cgccaaggga gagaacatcc cagctgtgga agcacctgtg gttgcagatg     780
   gagtgggcct atatgaagta gcagcatctg tgatcatgag aggcggctcc ggggagggtg     840
   tatcctgcat catcagaaat tccctcctcg gcctggaaaa gacagccagc atttccatcg     900
   cagacccctt cttcaggagc gcccagccct ggatcgcagc cctggcaggg accctgccta     960
   tcttgctgct gcttctcgcc ggagccagtt acttcttgtg gagacaacag aaggaaataa    1020
   ctgctctgtc cagtgagata gaaagtgagc aagagatgaa agaaatggga tatgctgcaa    1080
   cagagcggga aataagccta agagagagcc tccaggagga actcaagagg aaaaaatcca    1140
   gtacttgact cgtggagagg agtcttcgtc cgataccaat aagtcagcct gatgctctaa    1200
   tggaaaaatg gccctcttca agcctggtga ggaaatgctt cagatgaggc tccaccttgt    1260
   taaataaatt ggatgtatgg aaaaatagac tgcagaaaag gggaactcat ttagctcacg     1320
   agtggtcgag tgaagattga aaattaacct ctgagggcca gcacagcagc tcatgcctgt    1380
   aatcctagca ctttggaagg ctgaggaggg cggatcacaa ggtcaggaga tcaagaccat    1440
   cctggctaac acggtgaaac cccgtctcta ctaaaaatac aaaaaataaa aaattagccg    1500
   ggcatggtga cgggcacctg tagtcccagc tactcgggag gctgaggcag gagaatggca    1560
   tgaacccgga aggcagagct tgcagtgagc cgagatcacg ccactgcact ccagcctggg    1620
   agacagagcg agactctgtc tcaag                                          1645
<210> 15
   <211> 3416
   <212> DNA
   <213> Homo sapien
<400> 15
   ctgaagcttg catgcctgca ggtcgaccca cgcgtccgcg gacgcgtggg cggacgcgtg      60
   ggtttttcct ttcttccaga aggagattta accatagtag aaagaatgga gaactattaa     120
   ctgccttcct tctgtgggct gtgattttca gaggggaatg ctaagaggtg attttcaatg     180
   ttgggactca aaggtgaaga cactgaagga cagaattttt ggcagaggaa agatcttctt     240
   cggtcaccat acttgagtta gctctaggga agtggaggtt tccatttgga attctatagc     300
   ttcttccagg tcatagtgtc tgccccccac cttccagtat ctcctgatat gcagcatgaa     360
   tgaaaatggc aagtttcctg gccttccttc tgctcaactt tcgtgtctgc ctccttttgc    420
   ttcagctgct catgcctcac tcagctcagt tttctgtgct tggaccctct gggcccatcc     480
   tggccatggt gggtgaagac gctgatctgc cctgtcacct gttcccgacc atgagtgcag     540
   agaccatgga gctgaagtgg gtgagttcca gcctaaggca ggtggtgaac gtgtatgcag     600
   atggaaagga agtggaagac aggcagagtg caccgtatcg agggagaact tcgattctgc     660
   gggatggcat cactgcaggg aaggctgctc tccgaataca caacgtcaca gcctctgaca     720
   gtggaaagta cttgtgttat ttccaagatg gtgacttcta tgaaaaagcc ctggtggagc     780
   tgaaggttgc agcactgggt tctgatcttc acgttgatgt gaagggttac aaggatggag     840
   ggatccatct ggagtgcagg tccactggct ggtaccccca accccaaata cagtggagca    900
   acaacaaggg agagaacatc ccgactgtgg aagcacctgt ggttgcagac ggagtgggcc     960
   tgtatgcagt agcagcatct gtgatcatga gaggcagctc tggggagggt gtatcctgta    1020
   ccatcagaag ttccctcctc ggcctggaaa agacagccag catttccatc gcagacccct    1080
   tcttcaggag cgcccagagg tggatcgccg ccctggcacg gaccctgcct gtcttgctgc    1140
   tgcttcttgg gggagccggt tacttcctgt ggcaacagca ggaggaaaaa aagactcagt    1200
   tcagaaagaa aaagagagag caagagttga gagaaatggc atggagcaca atgaagcaag    1260
   aacaaagcac aagagtgaag ctcctggagg aactcagatg gagaagtatc cagtatgcat    1320
   ctcggggaga gagacattca gcctataatg aatggaaaaa ggccctcttc aagcctgcgg 1380
   atgtgattct ggatccaaaa acagcaaacc ccatcctcct tgtttctgag gaccagagga 1440
   gtgtgcagcg tgccaaggag ccccaggatc tgccagacaa ccctgagaga tttaattggc 1500
   attattgtgt tctcggctgt gagagcttca tatcagggag acattactgg gaggtggagg 1560
   taggggacag gaaagagtgg catatagggg tgtgcagtaa gaatgtgcag agaaaaggct 1620
   gggtcaaaat gacacctgag aatggattct ggactatggg gctgactgat gggaataagt 1680
   atcggactct aactgagccc agaaccaacc tgaaacttcc taagccccct aagaaagtgg 1740
   gggtcttcct ggactatgag actggagata tctcattcta caatgctgtg gatggatcgc 1800
   atattcatac tttcctggac gtctccttct ctgaggctct atatcctgtt ttcagaattt 1860
   tgaccttgga gcccacggcc ctgagtattt gtccagcgtg aaaagaagaa gagagttcct 1920
   ccaattctga ccgagtgctg atcattccct agagacacca gtaaccccgg gcttagctaa 1980
   cgaaagtggg gagcctcagg ctgaagtaac ttttctctgc ttctccctgc ccagctcaga 2040
   gctgagggcc tccccctcca cagcaaccaa tcacaaccat aaagctacaa gcacgcactg 2100
   aagcacttta ctgatactca ttcaattatt catatgacag ttgtttgagt ttggtaccat 2160
   cttattttcc ccttatacag ataaggaaac tggggtgcag aaaagtgaat tgactacaaa 2220
   gtagacatga ctagttaaca acacagctgg gatctaaaca gcaataacta acattaatgg 2280
   agaacttaaa atgctctgag tgctgtgtta tgagctttgg tggatgtcac tcctttaatc 2340
   ctcgcaacac cctgtcgggt agtctcattt agcaagtatg gaagttgagg cagggcaaca 2400
   ttaagcaact tacataactc atgcagtaat ttctgcagtt gggagatgtt cagcttcagt 2460
   ccccggccct atggccgttc ttttccaccc tgtttcttcc cccataggaa gaacccacct 2520
   gtagccctga ggttcttttc ccaggatggc tccaggataa ggatcactgt aggtggttgt 2580
   ggagttgaca cccctgttga ctccttccca gctgattgtc agagccttag acccagcacg 2640
   ccttggatta gctttgcaga gtgtcttggt tgagagaata acctcaccgt acccacatga 2700
   cacgtgattt ggaaagagac tagaggccac acttgataaa tcatggggaa cagatgtgtt 2760
   ccacccaaca aatgtgataa gtgatcatgc agccagagcc agccttcctt caatcaaggt 2820
   ttccaggcag agcaaatacc ctagagattt tctgtgatat aggaaatttg gatgaaggga 2880
   gctagaagaa atacagggat tttttttttt ttttaagatg gagtcttact ctgttgctag 2940
   gctggagtgc agtggtgcga tctcagctcc ctgcaacctc cacctcctgg gttcaaacaa 3000
   ttctcctgcc tcagcctccc gagtactggg aatataggtg cacgccacca cacccaacaa 3060
   atttttgtac ttttagtaca gatgagggtt cactatgttg gccaggatgg tctcgatctc 3120
   ttgacctcat gatccaccca cctcggtctc ccaaagtgct gggattacag gcttgagcca 3180
   ccgggtgacc ggcttacagg gatattttta atcccgttat ggactctgtc tccaggagag 3240
   gggtctatcc acccctgctc attggtggat gttaaaccaa tattcctttc aactgctgcc 3300
   tgctagggaa aaactactcc tcattatcat cattattatt gctctccact gtatcccctc 3360
   tacctggcat gtgcttgtca agttctagtt gttcaataaa tttgttaata atgctg 3416
<210> 16
   <211> 3502
   <212> DNA
   <213> Homo sapien
<400> 16
   agagaacagg tcccagatac cgagtccgca accccaaaca tcgcgattaa taggaggcct 60
   ctggtctctg cctgccctgg gtgctcatgg aaccagctgc tgctctgcac ttctccctgc 120
   cagcctccct cctcctcctc ctgctcctcc tccttctcag cctgtgtgca ctggtctcag 180
   cccagtttac tgtcgtgggg ccagctaatc ccatcctggc catggtggga gaaaacacta 240
   cattacgctg ccatctgtca cccgagaaaa atgctgagga catggaggtg cggtggttcc 300
   ggtctcagtt ctcccccgca gtgtttgtgt ataagggtgg gagagagaga acagaggagc 360
   agatggagga gtaccgggga agaatcacct ttgtgagcaa agacatcaac aggggcagcg 420
   tggccctggt catacataac gtcacagccc aggagaatgg gatctaccgc tgttacttcc 480
   aagaaggcag gtcctacgat gaggccatcc tacgcctcgt ggtggcaggc cttgggtcta 540
   agcccctcat tgaaatcaag gcccaagagg atgggagcat ctggctggag tgcatatctg 600
   gagggtggta cccagagccc ctcacagtgt ggagggaccc ctacggtgag gttgtgcccg 660
   ccctgaagga ggtttccatc gctgatgctg acggcctctt catggtcacc acagctgtga 720
   tcatcagaga caagtatgtg aggaatgtgt cctgctctgt caacaacacc ctgctcggcc 780
   aggagaagga aactgtcatt tttattccag aatcctttat gcccagcgca tctccctgga 840
   tggtggccct agctgtcatc ctgaccgcat ctccctggat ggtgtccatg actgtcatcc 900
   tggctgtttt catcatcttc atggctgtca gcatctgttg catcaagaaa cttcaaaggg 960
   aaaaaaagat tctgtcaggg gaaaagaaag ttgaacaaga ggaaaaagaa attgcacagc 1020
   aacttcaaga agaattgcga tggagaagaa cattcttaca tgctgctgat gtggtcctgg 1080
   atccagacac cgctcatccc gagctcttcc tgtcagagga ccggagaagt gtgaggcggg 1140
   gcccctacag gcagagagtg cctgacaacc cagagagatt cgacagtcag ccttgtgtcc 1200
   tgggatggga gagcttcgcc tcagggaaac attactggga ggtggaggtg gaaaacgtga 1260
   tggtgtggac tgtgggggtc tgcagacaca gtgttgagag gaaaggggag gtcctgctga 1320
   ttcctcagaa tggcttctgg accctggaga tgtttggaaa ccaataccgg gccctgtcct 1380
   cccctgagag gattctccct ttgaaggagt ccctttgccg ggtgggcgtc ttcctggact 1440
   atgaagctgg agatgtctcc ttctacaaca tgagggacag atcacacatc tacacatgtc 1500
   cccgttcagc ctttactgtg cctgtgaggc ccttcttcag gttagggtct gatgacagcc 1560
   ccatcttcat ctgccctgca ctcacaggag ccagtggggt catggtgcct gaagagggcc 1620
   tgaaacttca cagagtgggg acccaccaga gcctatagaa tcaattcctt ggactcacag 1680
   ccatgcagat aagccctggc catctcagca gccaccgcac aaccccccta atgaaagaca 1740
   cgccctcctc ccctctggtc acgtaagaga acatcttcca gctgcctttt tcacacccac 1800
   tccagccctc tgccccagtt ttctcctcct cactagtctg tggctttagt agttcctttg 1860
   cttgtaatta tgggatggga tccaggcata gggaactagt tgtttcatag ctcccagtca 1920
   aaaagaaagt gagagaagct gttgggcagt gaacctactg tttaaaatca ggataaccac 1980
   attaagccca atatgccagt tggcaccaga tgctgtggac ttggaatgag gccaacaggg 2040
   ttcaccagga tgagagagga gagaggaatc cacaggacca ccagaaggga gagggaacca 2100
   gatatgcaga tcagagatag aggaagtgga accagagagc tgggagggac caaggttgta 2160
   aggatggcta agtcccacca taagagctaa agggtcctgg gagatgatgg ctcatttcca 2220
   cccaacccca ggatttccac agcacacacc cacaggcctg gacctgggat gaagatgaat 2280
   gaagaacatg gactcatgtg gatgtggttt ggctcagatg tccctgcaat aaacaagggg 2340
   tcagtactta gtccctgagt gtggttgagg tttgaggtcc tggtcgagca gggcagtact 2400
   ggaccaggtc tacgtcagca ttcaggttca atggggacac cagtggcttc aaacttcctg 2460
   atctaattat gtttttagac acttagaagt tattgaggac tttaaagagc ttttgtttat 2520
   ttgggttaat atttatgaca tttgacattg aaacaaaaat ttaaaatgtt atcttttaat 2580
   ttatgttaaa atagcattaa taaatcagtt ataggttaat gtagatagga tgttttgtga 2640
   aaaagcaatc tattgtgtcc aaataaaaaa aacaaaaagt gtgacactgg ttaacttttt 2700
   ccagatctca tgtctggctt aataagagat atttgtatta tcatatctgc ctttgtatta 2760
   aacctattgg tatatcatag gtcatgttag ctcaaaaaaa ctttactgca cactactgag 2820
   agaatgagat gaaaaacgat taatgtttca ttattattat tgtgaaaata ttattaacac 2880
   tggggactcc ttaagagtac atcagagttc tctctaggaa tcccaaaacc acattttgaa 2940
   actagaatag tggatcctgg aagttaatcc atgtgctggt taattttaga tgtcaacctg 3000
   gtgtttccag aagagattgg caagtgagtc agtgggaaat tctctccttc tgttggctgg 3060
   gtgcccaata caacaaaaag gcagaggaaa ggcaaattct tctctcctct ggagctgaga 3120
   cactcttctt cttctgccct tggacatcag aactcctggc tctccggcct ttgaacttca 3180
   ggacttgtac caggaggccc tgggttctca ggcctttggc tttggactga gagttacaca 3240
   atcagcttcc ctggttctga ggctttcaga cttaaactga gccatgctac cagcatccca 3300
   gggtctccag cctacagatg agctgttgtg cgatttctta gcctccataa tcacatgagc 3360
   caatctcctt aataaatgcc tgctcataga tctgtatcta catctatatc tgtatgtgca 3420
   tctatatcta tgcctatatc tatatctata tcatattgat tttgtctctc tggagaaccc 3480
   tgactaataa aatgaggcat ct 3502
<210> 17
   <211> 2854
   <212> DNA
   <213> Homo sapien
<400> 17
   gacccacgcg tccgaaaagc tatggcctca accaccagca ccaagaagat gatggaggaa 60
   gccacctgct ccatctgcct gagcctgatg acgaacccag taagcatcaa ctgtggacac 120
   agctactgcc acttgtgtat aacagacttc tttaaaaacc caagccaaaa gcaactgagg 180
   caggagacat tctgctgtcc ccagtgtcgg gctccatttc atatggatag cctccgaccc 240
   aacaagcagc tgggaagcct cattgaagcc ctcaaagaga cggatcaaga aatgtcatgt 300
   gaggaacacg gagagcagtt ccacctgttc tgcgaagacg aggggcagct catctgctgg 360
   cgctgtgagc gggcaccaca gcacaaaggg cacaccacag ctcttgttga agacgtatgc 420
   cagggctaca aggaaaagct ccagaaagct gtgacaaaac tgaagcaact tgaagacaga 480
   tgtacggagc agaagctgtc cacagcaatg cgaataacta aatggaaaga gaaggtacag 540
   attcagagac aaaaaatccg gtctgacttt aagaatctcc agtgtttcct acatgaggaa 600
   gagaagtctt atctctggag gctggagaaa gaagaacaac agactctgag tagactgagg 660
   gactatgagg ctggtctggg gctgaagagc aatgaactca agagccacat cctggaactg 720
   gaggaaaaat gtcagggctc agcccagaaa ttgctgcaga atgtgaatga cactttgagc 780
   aggagttggg ctgtgaagct ggaaacatca gaggctgtct ccttggaact tcatactatg 840
   tgcaatgttt ccaagcttta cttcgatgtg aagaaaatgt taaggagtca tcaagttagt 900
   gtgactctgg atccagatac agctcatcac gaactaattc tctctgagga tcggagacaa 960
   gtgactcgtg gatacaccca ggagaatcag gacacatctt ccaggagatt tactgccttc 1020
   ccctgtgtct tgggttgtga aggcttcacc tcaggaagac gttactttga agtggatgtt 1080
   ggcgaaggaa ccggatggga tttaggagtt tgtatggaaa atgtgcagag gggcactggc 1140
   atgaagcaag agcctcagtc tggattctgg accctcaggc tgtgcaaaaa gaaaggctat 1200
   gtagcactta cttctccccc aacttccctt catctgcatg agcagcccct gcttgtggga 1260
   atttttctgg actatgaggc cggagttgta tccttttata acgggaatac tggctgccac 1320
   atctttactt tcccgaaggc ttccttctct gatactctcc ggccctattt ccaggtttat 1380
   caatattctc ctttgtttct gcctccccca ggtgactaag gaaaagagca gaagctcctt 1440
   ggtttaacca gcacagagaa aataatataa atcccataag ggcagacgtt tggtctgttt 1500
   tcttcgctgt catttcctta gtagttagac tagtgctgag attttagtgg atatataatt 1560
   gatttatgtt gaatatatgg acttagcaac taaaaatacc acagatggtt aacctggact 1620
   ggggcaaagc aagataatag tgatgatcgt atgttgctgt ctccatccgt ctttaatggg 1680
   tcagggcttt gatttccaag ggtcttcagg tgatgagtag gggtacccac aagtcagaag 1740
   gtctgcgttc tcctagtttg tttgctgcca tttgaactca tgtagggaat gaaagaaagc 1800
   tgcaattatc cgccaactgc atttaaaaca aaacaaaaca gaaaaatcaa aataacattg 1860
   actcttccaa ccactgacat gttgtttaat aatctaagcg gcagtcctgg aggctaccag 1920
   acttactgag ttctacctga gaaacagcca agcaaagtgt gagagaaggg ttaagactgg 1980
   cttacaatga gatgcttcaa atgaaaaggg aattatgagt aaaattgaac tttgatgggg 2040
   gattcagttc tggaaaagaa tttggtattt tccagtctgc taggaccaat taccttgaaa 2100
   tattttaaaa tctcagtaaa tagttattgc tgaaatggct gttggcagtt cttattatga 2160
   ttcagagaag agcaaataga ccttaacttc atttgaaaaa agaccaaatt accatacccg 2220
   agtgagtaat gacaggacta caactaaaac ataaacaaca ttaatgatga ccataaaaag 2280
   tcacaaaatt gctaaatgtt ataatttaga gttgacataa aaattgatgg ccaggcatgg 2340
   tggctcacgc ctgtaatccc agaactatgt gaggctgagg caggtggatc acttgaggtc 2400
   aggagttcaa caccagcctg gccaacatgg tgaaaccctg tctctactaa aaatacaaaa 2460
   attagccggg catggtggta ggggcctgta acccagctac tcgtgaggcc aaggcaggag 2520
   aattgcttga gcctgcagca gctgcagtaa gccaagatca tgctgtgcct caaggaaaaa 2580
   aaaaattaat gtttactgat atttgttgaa gtcctacaac atcacctctg agaataggag 2640
   aaatgaagca acagttgtgt ctagatgtca gaggcatggc tgggcctcca tctctgccta 2700
   agggagatat aaaagagttc aaactattgc ccatgttccc cagggtcaga agttctaatt 2760
   atgatgatag aggctgggtt gtaagtagta agtgaagggt agcagaatat gccatctttg 2820
   gcataagaag tattttgagt tgaagacaat tgag 2854
<210> 18
   <211> 2266
   <212> DNA
   <213> Homo sapien
<400> 18
   ggacagaaaa ctccctcctt ttccaagtta gccttatagt ctagggctta aaatactggt 60
   ttaatggtga aggtaagtgc ttttcttctt tttgggtaga aggattatta ctaacttacc 120
   aaaggtccat taaggggagg gaacagtttt aggagaagtc agagaaaaga cattaacagc 180
   aacataagga tctccatctg gtaatattgc ctaattccaa aatgaagaga ctctctgaaa 240
   aagataactg attcaatgaa gaccctaggg caaggcttga gaagccactg gtaccaatgg 300
   acactgtgga caatggtcat ttctccaagg acgctataaa agactgtcgt agtaaaagag 360
   attcagggca cagggaaact ccaccacaaa gcgtggtacc atttcccaca gaagctaaat 420
   ggacgggaag cctgccacca ggaaaggtcc agatttctgt tcattacgct atgggctggc 480
   tcttatcatg cacttctcaa acttcaccat gataacgcag cgtgtgagtc tgagcattgc 540
   gatcatcgcc atggtgaaca ccactcagca gcaaggtcta tctaatgcct ccactgaggg 600
   gcctgttgca gatgccttca ataactccag catatccatc aaggaatttg atacaaaggc 660
   ctctgtgtat caatggagcc cagaaactca gggtatcatc tttagctcca tcaactatgg 720
   gataatactg actctgatcc caagtggata tttagcaggg atatttggag caaaaaaaat 780
   gcttggtgct ggtttgctga tctcttccct tctcaccctc tttacaccac tggctgctga 840
   cttcggagtg attttggtca tcatggttcg gacagtccag ggcatggccc agggaatggc 900
   atggacaggt cagtttacta tttgggcaaa gtgggctcct ccacttgaac gaagcaagct 960
   caccaccatt gcaggatcag ggtcagcatt tggatccttc atcatcctct gtgtgggggg 1020
   actaatctca caggccttga gctggccttt tatcttctac atctttggta gcactggctg 1080
   tgtctgctgt ctcctatggt tcacagtgat ttatgatgac cccatgcatc acccgtgcat 1140
   aagtgttagg gaaaaggagc acatcctgtc ctcactggct caacagccca gttctcctgg 1200
   acgagctgtc cccataaagg cgatggtcac atgcctacca ctttgggcca ttttcctggg 1260
   ttttttcagc catttctggt tatgcaccat catcctaaca tacctaccaa cgtatatcag 1320
   tactctgctc catgttaaca tcagagatag tggagttctg tcctccctgc cttttattgc 1380
   tgctgcaagc tgtacaattt taggaggtca gctggcagat ttccttttgt ccaggaatct 1440
   tctcagattg atcactgtgc gaaagctctt ttcatctctt gatatgcaag tttcctcatg 1500
   ggaatctcaa ggggatttgg gctcatcgca ggaatcatct cttccactgc cactggattc 1560
   ctcatcagtc aggattttga gtctggttgg aggaatgtct ttttcctgtc tgctgcagtc 1620
   aacatgtttg gcctggtctt ttacctcacg tttggacaag cagaacttca agactgggcc 1680
   aaagagagga cccttacccg cctctgagga cataaagtta caaacttaaa tgtggtactg 1740
   agcatgaact ttttaaacat tttttacttc tctccatatt cctgaccata gactcagcag 1800
   ttcttaactc tggctgtgtg ttagtcttcc ctggggagcc tttataagac actgatactt 1860
   gggacccact ccagagattc tgaatgaatt ggtctggggt ggaacccaga tactactaat 1920
   ttttagatac tccttagagg tttctagcat gcgcccgggg ttgacaacag ctggacaaac 1980
   ttgaaaagtc aattcatgtg gcctttgaat tttcctcatt ggaaagtact aaataaataa 2040
   aaattcatgt gaaaatgatc actgataaat atcttcatgg tggggcaggt tattggatgc 2100
   agagaagatc tgctcggaat tgtagccata tgttacagat ctcagcaccg atcagaactg 2160
   taaagctata atccccagaa ttaaagtttt tattattttt tatacattgt aaaacataga 2220
   cgtttattta tgtgattaaa ttctattaaa atttacatgc taaaat 2266
<210> 19
   <211> 1780
   <212> DNA
   <213> Homo sapien
<400> 19
   acgcgtccgc ccacgcgtcc gcccacgcgt ccggtcgggg ccagagcgca ggtgtacctg 60
   gcggccgtgc tggagcacct gaccgccgag atcctggagc tggctggcaa cccggcccgc 120
   gacaagaaga cccgcatcat cctgcgccac ctgtagctgg ccattcgcaa cggcgaggag 180
   cttaacaagc tgctgggcga agtcaccatc gcgcagggcg gtgtcctgcc caacattcag 240
   ggcgtgcttc tgccccagaa gaccaagagc caccacaagg ccaagggtga aaaccattca 300
   ctaggagagg agaaacacaa tggccaccaa gacagagttg agtcccacag caagggagag 360
   caagaacgca caagatatgc aagtggatga gacactgatc cccaggaaag gtccaagttt 420
   atgttctgct cgctatggaa tagccctcgt cttacatttc tgcaatttca caacgatagc 480
   acaaaatgtc atcatgaaca tcaccatggt agccatggtc aacagcacaa gccctcaatc 540
   ccagctcaat gattcctctg aggtgctgcc tgttgactca tttggtggcc taagtaaagc 600
   cccaaagagt cttcctgcaa agtcctcaat acttgggggt cagtttgcaa tttgggaaaa 660
   gtggggccct ccacaagaac gaagcagact ctgcagcatt gctttatcag gaatgttact 720
   gggatgcttt actgccatcc tcataggtgg cttcattagt gaaacccttg ggtggccctt 780
   tgtcttctat atctttggag gtgttggctg tgtctgctgc cttctctggt ttgttgtgat 840
   ttatgatgac cccttttcct atccatggat aagcacctca gaaaaagaat acatcatatc 900
   ctccttgaaa caacaggtcg ggtcttctaa gcagcctctt cccatcaaag ctatgctcag 960
   atctctaccc atttggtcca tatgtttagg ctgtttcagc catcaatggt tagttagcac 1020
   aatggttgta tacataccaa cttacatcag ctctgtgtac catgttaaca tcagagacaa 1080
   tggacttcta tctgcccttc cttttattgt tgcctgggtc ataggcatgg tgggaggcta 1140
   tctggcagat ttccttctaa ccaaaaagtt tagactcatc actgtgagga aaattgccac 1200
   aattttagga agtctcccct cttcagcact cattgtgtct ctgccttacc tcaattccgg 1260
   ctatatcaca gcaactgcct tgctgacgct ctcttgcgga ttaagcacat tgtgtcagtc 1320
   agggatttat atcaatgtct tagatattgc tccaaggtat tccagttttc tcatgggagc 1380
   atcaagagga ttttcgagca tagcacctgt cattgtaccc actgtcagcg gatttcttct 1440
   tagtcaggac cctgagtttg ggtggaggaa tgtcttcttc ttgctgtttg ccgttaacct 1500
   gttaggacta ctcttctacc tcatatttgg agaagcagat gtccaagaat gggctaaaga 1560
   gagaaaactc actcgtttat gaagttatcc caccttggat ggaaaagtca ttaggcaccg 1620
   tattgcataa aatagaaggc ttccgtgatg aaaataccag tgaaaagatt tttttttcct 1680
   gtggctcttt tcaattatga gatcagttca ttattttatt cagacttttt tttgagagaa 1740
   atgtaagatg aataaaaatt caaataaaat gataactaag 1780
<210> 20
   <211> 235033
   <212> DNA
   <213> Homo sapien
<400> 20
<210> 21
   <211> 237326
   <212> DNA
   <213> Homo sapien
<400> 21
<210> 22
   <211> 15
   <212> DNA
   <213> Homo sapien
<400> 22
   tcacacaccg attgg 15
<210> 23
   <211> 16
   <212> DNA
   <213> Homo sapien
<400> 23
   ccaaactgat ctttga 16
<210> 24
   <211> 14
   <212> DNA
   <213> Homo sapien
<400> 24
   tatatatata tata 14
<210> 25
   <211> 14
   <212> DNA
   <213> Homo sapien
<400> 25
   cttttttttt tttt 14
<210> 26
   <211> 14
   <212> DNA
   <213> Homo sapien
<400> 26
   aaaaaaaaaa aaaa 14
<210> 27
   <211> 10
   <212> DNA
   <213> Homo sapien
<400> 27
   aggatggtct 10
<210> 28
   <211> 12
   <212> DNA
   <213> Homo sapien
<400> 28
   ttgttgttgt tg 12
<210> 29
   <211> 20
   <212> DNA
   <213> Homo sapien
<400> 29
   atatatatat atatatatat 20
<210> 30
   <211> 12
   <212> DNA
   <213> Homo sapien
<400> 30
   aataataata at 12
<210> 31
   <211> 11
   <212> DNA
   <213> Homo sapien
<400> 31
   tttttttttt t 11
<210> 32
   <211> 20
   <212> DNA
   <213> Artificial Sequence
   <220>
   <223> Primer
<400> 32
   cctgatgctc gagtgaattc 20
<210> 33
   <211> 20
   <212> DNA
   <213> Artificial Sequence <220> <223> Gel purified oligo
<400> 33
   cgacccagca acctggagat 20
<210> 34
   <211> 20
   <212> DNA
   <213> Artificial Sequence
<220>
   <223> Gel purified oligo
<400> 34
   agctcgagcg gccgctgcag 20
<210> 35
   <211> 20
   <212> DNA
   <213> Artificial Sequence
<220>
   <223> Gel purified oligo
<400> 35
   agaccccaac ccacaagaag 20
<210> 36
   <211> 20
   <212> DNA
   <213> Artificial Sequence
<220>
   <223> MAP1
<400> 36
   cgttagaacg cggctacaat 20
<210> 37
   <211> 20
   <212> DNA
   <213> Artificial Sequence
<220>
   <223> MAP2
<400> 37
   gccgattcat taatgcaggt 20
<210> 38
   <211> 20
   <212> DNA
   <213> Artificial Sequence
<220>
   <223> -21
<400> 38
   ctgtaaaacg acggccagtc 20
<210> 39
   <211> 19
   <212> DNA
   <213> Artificial Sequence
<220>
   <223> REV
<400> 39
   gcaggaaaca gctatgacc 19
<210> 40
   <211> 21
   <212> DNA
   <213> Artificial Sequence
<220>
   <223> Primer
<400> 40
   gcatcagcga ttaacttcta c 21
<210> 41
   <211> 22
   <212> DNA
   <213> Artificial Sequence
<220>
   <223> Primer
<400> 41
   ttgcattgtg gtgaaatcag gg 22
<210> 42
   <211> 21
   <212> DNA
   <213> Artificial Sequence
<220>
   <221> misc difference
   <222> (1) ... (1)
   <223> N = biotinylated cysteine
<223> Primer
<400> 42
   ntgagtaatt gtttaaggtg c 21
<210> 43
   <211> 21
   <212> DNA
   <213> Artificial Sequence
<220>
   <221> misc_difference
   <222> (1) ... (1)
   <223> N = biotinylated cysteine
<223> Primer
<400> 43
   ntgagtaatt gtttaaggtg t 21
<210> 44
   <211> 20
   <212> DNA
   <213> Artificial Sequence
<220>
   <221> misc difference
   <222> (1) ... (1)
   <223> N = phosphorylated digoxigenin adenine
<223> Primer
<400> 44
   ngaagagata gatatggtgg 20
<210> 45
   <211> 20
   <212> DNA
   <213> Artificial Sequence
<220>
   <223> Primer
<400> 45
   gaatgtgacc gtcccatgag 20
<210> 46
   <211> 28
   <212> DNA
   <213> Artificial Sequence
<220>
   <223> Primer
<400> 46
   caactgaata tgcagaaaaa agtacacc 28
<210> 47
   <211> 21
   <212> DNA
   <213> Artificial Sequence
<220>
   <221> misc difference
   <222> (1) ... (1)
   <223> N = biotinylated adenine
<223> Primer
<400> 47
   ngtagctggg actcacggtg t 21
<210> 48
   <211> 21
   <212> DNA
   <213> Artificial Sequence
<220>
   <221> misc difference
   <222> (1) ... (1)
   <223> N = biotinylated adenine
<223> Primer
<400> 48
   ngtagctggg actcacggtg c 21
<210> 49
   <211> 21
   <212> DNA
   <213> Artificial Sequence
<220>
   <221> misc_difference
   <222> (1) ... (1)
   <223> N = phosphorylated digoxigenin guanine
<223> Primer
<400> 49
   ncgccaccac tcccagctca t 21

## Description

### BACKGROUND OF THE INVENTION

Hereditary hemochromatosis (HH) is an inherited disorder of iron metabolism wherein the body accumulates excess iron. In symptomatic individuals, this excess iron leads to deleterious effects by being deposited in a variety of organs leading to their failure, and resulting in cirrhosis, diabetes, sterility, and other serious illnesses. The gene which is defective in this disease was disclosed in copending U.S.S.N. 08/652,265 (corresponds to US6025130, published 15.02.2000).

Fine structure mapping of the region to which the gene responsible for HH, HFE (denoted HH or HFE in some publications), was mapped makes possible the identification of candidate sequences comprising the HFE gene, along with structural elements for regulation and expression and neighboring genes.

A variety of techniques is available for fine structure mapping, including direct cDNA selection, exon-trapping, and genomic sample sequencing. The direct selection approach (Lovett et al. Proc. Natl. Acad. Sci. U.S.A. 88:9628-9623 (1991)) involves the hybridization of cDNA fragments to genomic DNA. This technique is extremely sensitive and capable of isolating portions of rare transcripts. Exon-trapping (Church et al. Nature Genetics 6:98-105 (1994)) recovers spliced introns from *in vivo* expressed genomic DNA clones and produces candidate exons without requiring any prior knowledge of the target's gene expression. High-throughput genomic DNA sequencing with comparison of the sequence data to databases of expressed sequences has also been used, such as in the positional cloning of the Werner syndrome gene (Yu et al. Science 277:258-262 (1996)) and in cloning by homology of the second Alzheimer's disease gene on chromosome 1 (Levy-Lahad et al. Science 269:973-977 (1995)).

HH is typically inherited as a recessive trait: in the current state of knowledge, homozygotes carrying two defective copies of the gene are most frequently affected by the disease. In addition, heterozygotes for the HFE gene are more susceptible to sporadic porphyria cutanea tarda and potentially other disorders (Roberts et al., Lancet 349:321-323 (1997). It is estimated that approximately 10-15% of Caucasians carry one copy of the HFE gene mutation and that there are about one million homozygotes in the United States. HH, thus, represents one of the most common genetic disease mutations in Caucasian individuals. Although ultimately HH produces debilitating symptoms, the majority of homozygotes and heterozygotes have not been diagnosed.

The need for such diagnostics is documented, for example, in Barton, J.C. et al. Nature Medicine 2:394-395 (1996); Finch, C.A. West J Med 153:323-325 (1990); McCusick. V. Mendelian Inheritance in Man pp. 1882-1887, 11th ed., (Johns Hopkins University Press, Baltimore (1994)); Report of a Joint World Health Organization/Hemochromatosis Foundation/French Hemochromatosis Association Meeting on the Prevention and Control of Hemochromatosis (1993); Edwards, C.Q. et al. New Engl J Med 328:1616-1620 (1993); Bacon, B.R. New Engl J Med 326:126-127 (1992); Balan, V. et al. Gastroenterology 107:453-459 (1994); Phatak, P.D. et al. Arch Int Med 154:769-776 (1994).

A single mutation in the HFE gene, designated 24d1 in copending U.S.S.N. 08/630,912, gave rise to the majority of disease-causing chromosomes present in the population today. This is referred to herein as the "common" or "ancestral" or "common ancestral" mutation. These terms are used interchangeably. It appears that about 80% to 90% of all HH patients carry at least one copy of the common ancestral mutation which is closely linked to specific alleles of certain genetic markers close to this ancestral HFE gene defect. These markers are, as a first approximation, in the allelic form in which they were present at the time the ancestral HFE mutation occurred. *See, for example,* Simon, M. et al. Am J Hum Genet 41:89-105 (1987); Jazwinska, E.C. et al. Am J Hum Genet 53:242-257 (1993); Jazwinska, E.C. et al. Am J Hum Genet 56:428-433 (1995); Worwood, M. et al. Brit J Hematol 86:863-866 (1994); Summers, K.M. et al. Am J Hum Genet 45:41-48 (1989).

Several polymorphic markers in the HFE region have been described and shown to have alleles that are associated with HH disease. These markers include the published microsatellite markers D6S258, D6S306 (Gyapay, G. et al. Nature Genetics 7:246-339 (1994)), D6S265 (Worwood, M. et al. Brit J Hematol 86:833-846 (1994)), D6S105 (Jazwinska, E.C. et al. Am J Hum Genet 53:242-257 (1993); Jazwinska, E.C. et al. Am J Hum Genet 56:428-433 (1995)), D6S1001 (Stone, C. et al. Hum Molec Genet 3:2043-2046 (1994)), D6S1260 (Raha-Chowdhury et al. Hum Molec Genet 4:1869-1874 (1995)) as well as additional microsatellite and single-nucleotide-polymorphism markers disclosed in co-pending PCT application WO 96/06583. Additionally, copending U.S.S.N. 08/630,912 disclosed additional markers 24d2 and 24d7.

The symptoms of HH are often similar to those of other conditions, and the severe effects of the disease often do not appear immediately. Accordingly, it would be desirable to provide a method to identify persons who may be destined to become symptomatic in order to intervene in time to prevent excessive tissue damage associated with iron overload. One reason for the lack of early diagnosis is the inadequacy of presently available diagnostic methods to ascertain which individuals are at risk, especially while such individuals are presymptomatic.

Although blood iron parameters can be used as a screening tool, a confirmed diagnosis often employs liver biopsy which is undesirably invasive, costly, and carries a risk of mortality. Thus, there is a clear need for the development of an inexpensive and noninvasive diagnostic test for detection of homozygotes and heterozygotes in order to facilitate diagnosis in symptomatic individuals, provide presymptomatic detection to guide intervention in order to prevent organ damage, and for identification of heterozygote carriers.

Furthermore, a need exists for both methods for fine structure mapping and a fine structure map of the region of the chromosome to which the HH locus maps. This and other needs are addressed by the present invention.

### SUMMARY OF THE INVENTION

The invention is defined by claims 1-4.

### BRIEF DESCRIPTION OF THE DRAWINGS

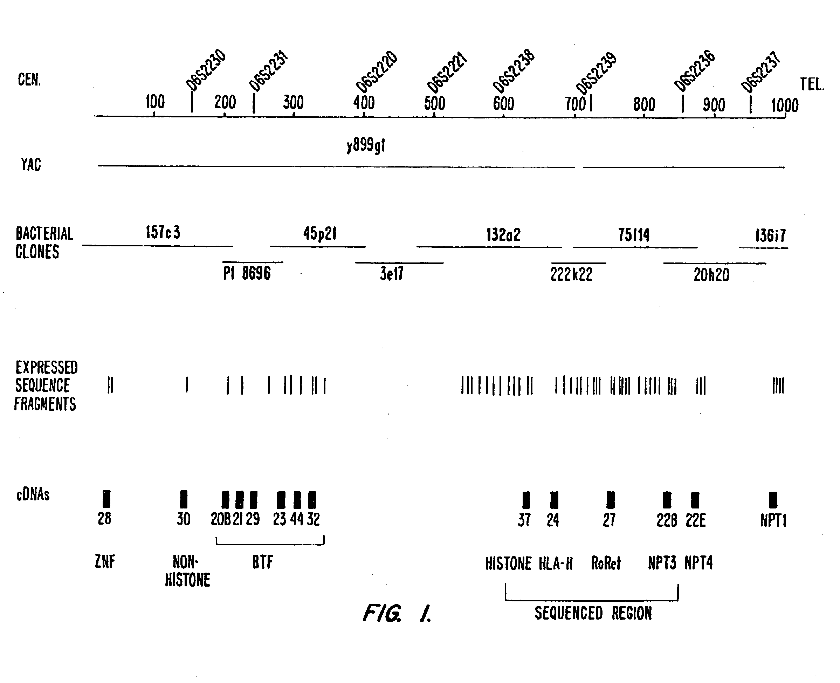

Figure 1 depicts a combination genetic, physical and transcription map of the HFE gene region. The first line shows the relative positions of selected genetic markers that define the HFE region. The heavy bar below represents the YAC clone used in the direct selection experiment. The order and positions of the bacterial clones employed in the exon-trapping and sample sequencing is indicated under the YAC. The thin bar under the bacterial clones represents the approximate locations of a subset of the expressed sequence fragments mapped to the contig. The thicker bars show the location of the cDNAs cloned. Two regions are bracketed; the butyrophilin family of genes (BTF), and the region where complete genomic sequencing was carried out.

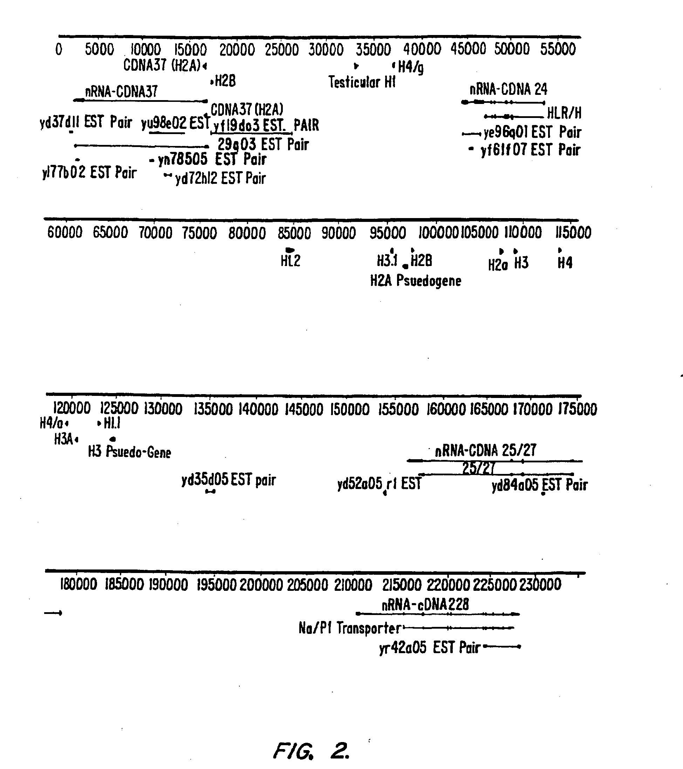

Figure 2 is a schematic of the 250 kb of genomic sequence including the HFE gene. Both the structure of the overall cDNA (top) and that corresponding to the coding regions (bottom), as well as the direction of transcription are shown. The positions of the histone genes, the zinc α-2 glycoprotein pseudogene, and the ESTs are also shown.

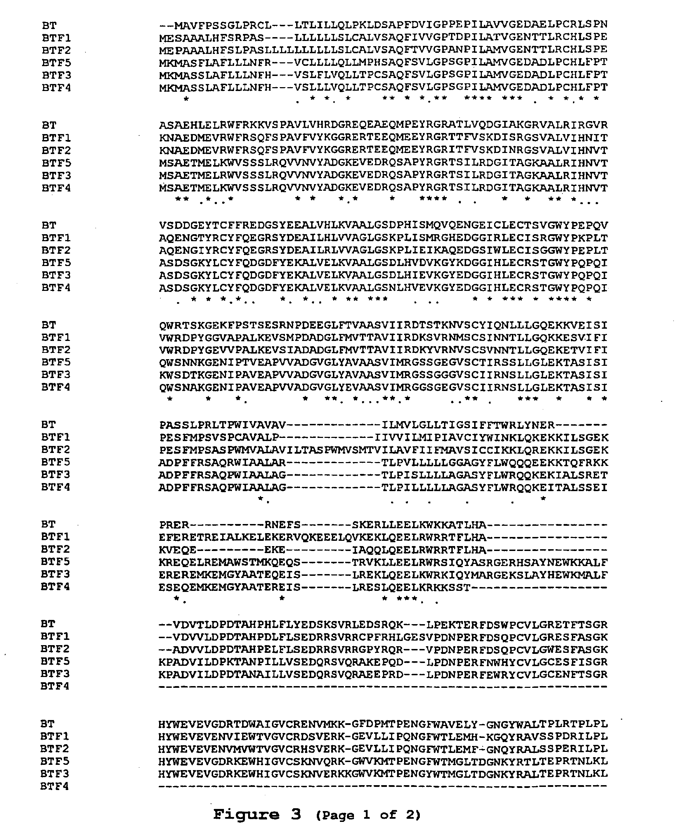

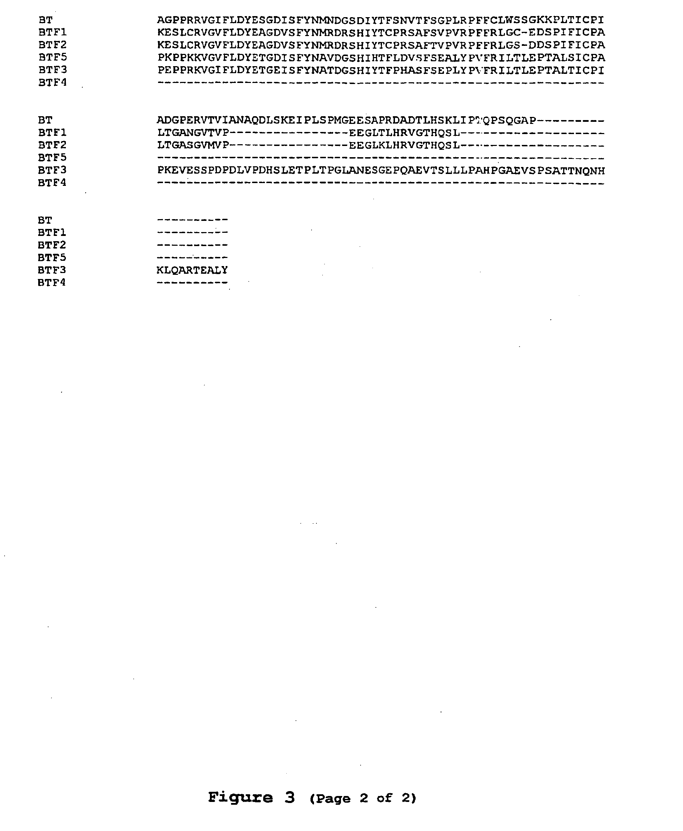

Figure 3 depicts an alignment of the predicted amino acid sequence of the BTF proteins. Sequences were aligned in a pair-wise fashion using CLUSTAL W (Thompson et al. Nucl. Acids Res. 22:4673-4680) to deduce the most parsimonious arrangement. The asterisks under the alignment represent amino acids conserved in all 6 proteins; the "dots" represent conserved amino acids substitutions. Boxed are the regions within the proteins which correspond to three conserved motifs: 1) the B-G domain, 2) the transmembrane domain (TM), and 3) the B30-2 exon domain.

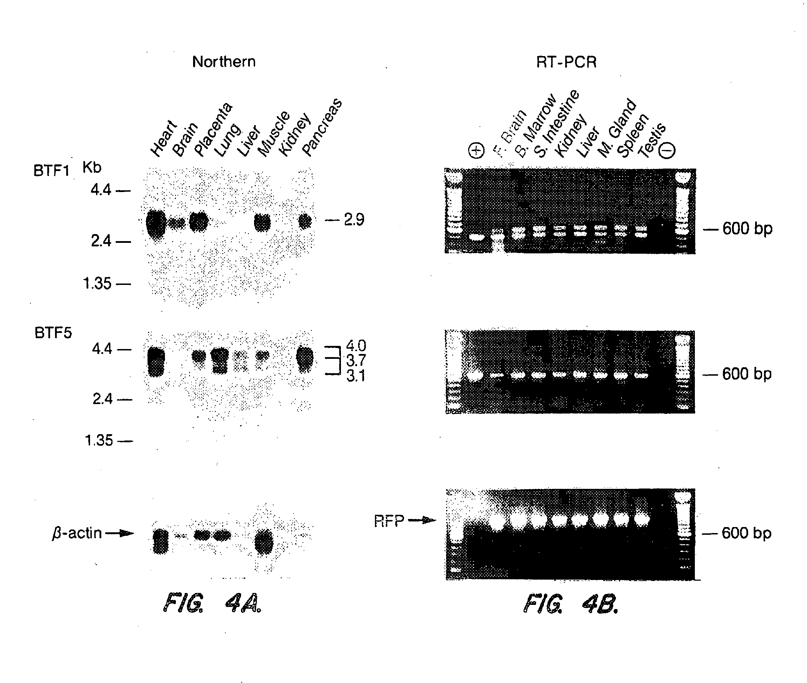

Figure 4, panel (A) depicts a Northern blot analysis of representative members of the two groups of BTF proteins, BTF1 and BTF5. BTF1 hybridized to all tissues on the blot as a major transcript at 2.9 kb and a minor one at 5.0 kb. BTF5 hybridized to several transcripts ranging between 4.0 and 3.1 kb and as a similar expression profile to BTF1. Autoradiography was for 24 hours. The β-actin hybridization demonstrated the variation in ploy (A)+ RNA between the lanes. Autoradiography was for 1 hour. In panel (B), RT-PCR analysis demonstrated that the expression of both genes was widespread. Included in the (+) lane are cDNA 21 and 44 as positive controls; the (-) lane represents the no-DNA control. Amplification using primers for the RFP gene (isomura et al. Nucleic Acid Res, 20:5305-5310 (1992)) controlled for the integrity of the cDNA. All first strand cDNAs were checked for contaminating genomic DNA amplification by carrying out an identical experiment excluding the reverse transcriptase. In all cases, no amplification was obtained (data not shown).

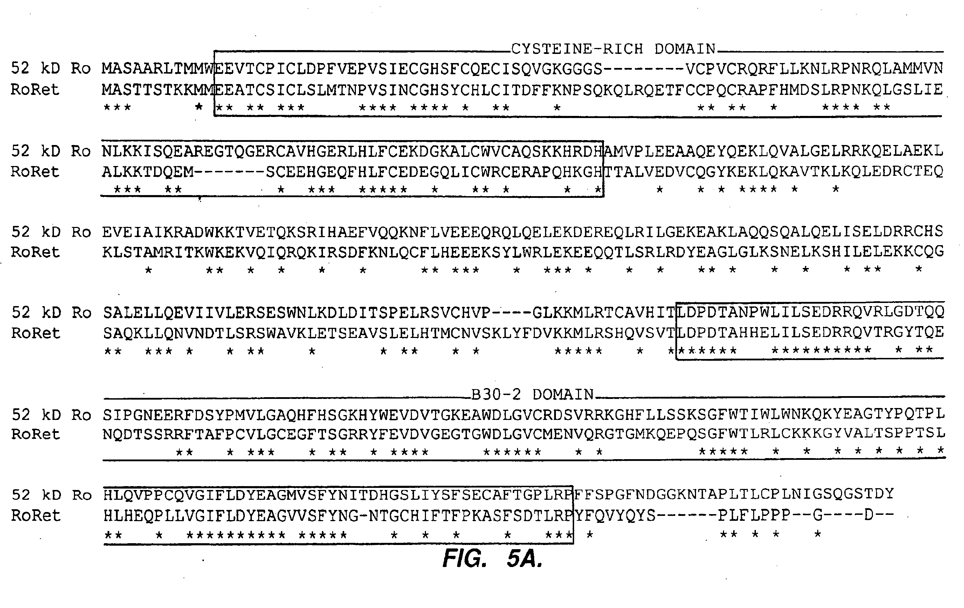

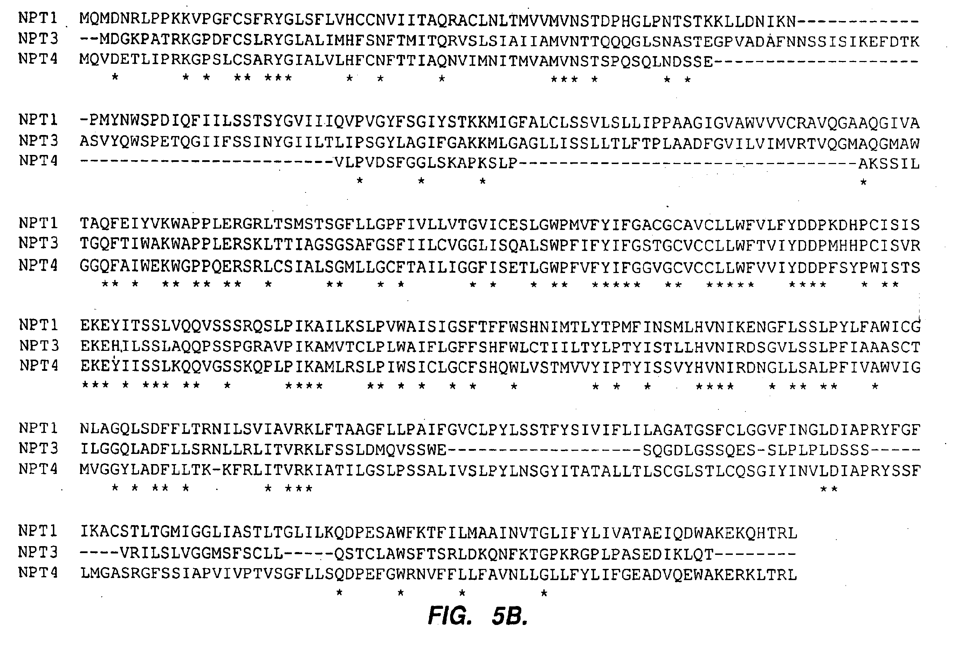

Figure 5(A) depicts an alignment of the predicted amino acid sequence of the RoRet gene to the 52 kD Ro/SSA auto-antigen protein. The asterisks under the alignment represent conserved amino acids; the "dots" represent conserved amino acids substitutions. The putative DNA binding cysteine-rich domain and the B30-2 exon domain are boxed. Figure 5(B) depicts an alignment of the predicted amino acid sequence of the two novel putative sodium phosphate transport proteins to that of the NPT1.

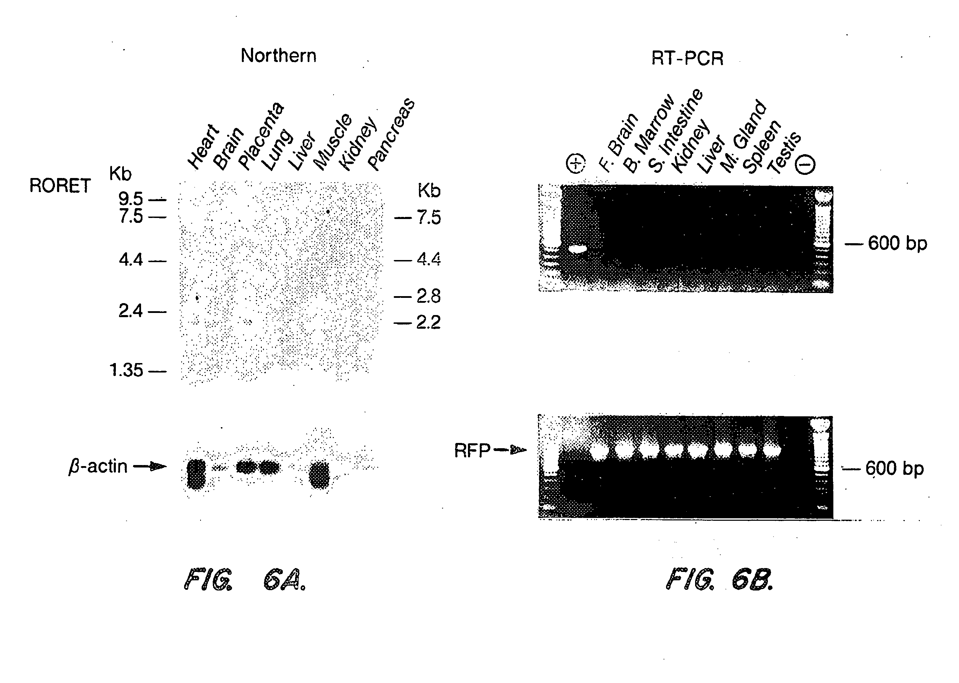

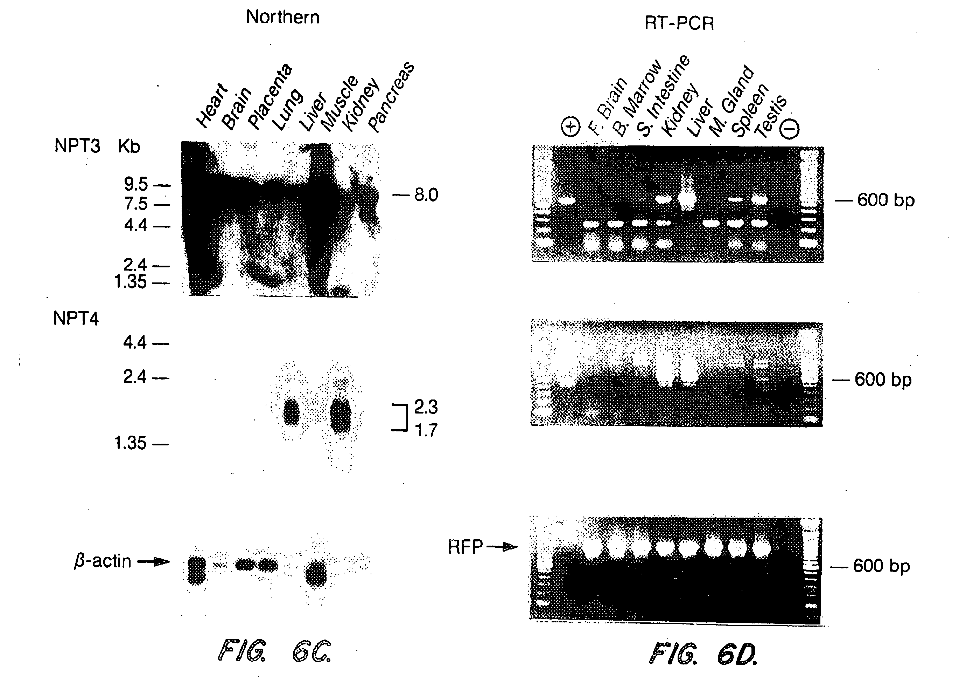

Figure 6, panel (A) depicts a Northern blot analysis of the RoRet gene. The RoRet cDNA hybridized to 4 different transcripts, ranging from 7.1 kb to 2.2 kb. Autoradiography was performed for 4 days. The re-hybridization of the blot with a β-actin probe showed the variation in poly (A)+ RNA between the lanes. Autoradiography was for 1 hour. Panel (B) depicts RT-PCR analysis of the RoRet gene. Included in the (+) lane was a cDNA 27 positive control. Weak amplification of the correct size was observed in the small intestine, kidney and liver. The other tissues were negative as was the no DNA control lane (-). The RFP primers demonstrated the integrity of the cDNA. Panel (C) depicts Northern blot analysis of NPT3 and NPT4. NPT3 was expressed at high abundance in the heart and muscle as a single 7.2 kb transcript. Lesser amounts were found in the other tissues. The expression pattern of NPT4 was more restricted, being found only in the liver and kidney as a smear of transcripts ranging from 2.6 to 1.7 kb. Panel (D) depicts RT-PCR analysis of the NPT3 and NPT4 genes. Included in the (+) lane were the respective cDNA22E and 22B positive controls. The NPT3 gene was expressed as the proper size PCR fragment in kidney, liver, spleen and testis. A smaller fragment was detected in all tissues with the exception of the liver. The no DNA control lane (-) was negative. NPT4 was expressed as the proper size fragment in the small intestine, kidney, liver and testis. Larger and smaller size fragments were found in all other tissues with the exception of the brain. For both genes these different size fragments may indicate alternative splice events. The no DNA control lane (-) was negative. The RFP primers demonstrated the integrity of the cDNA.

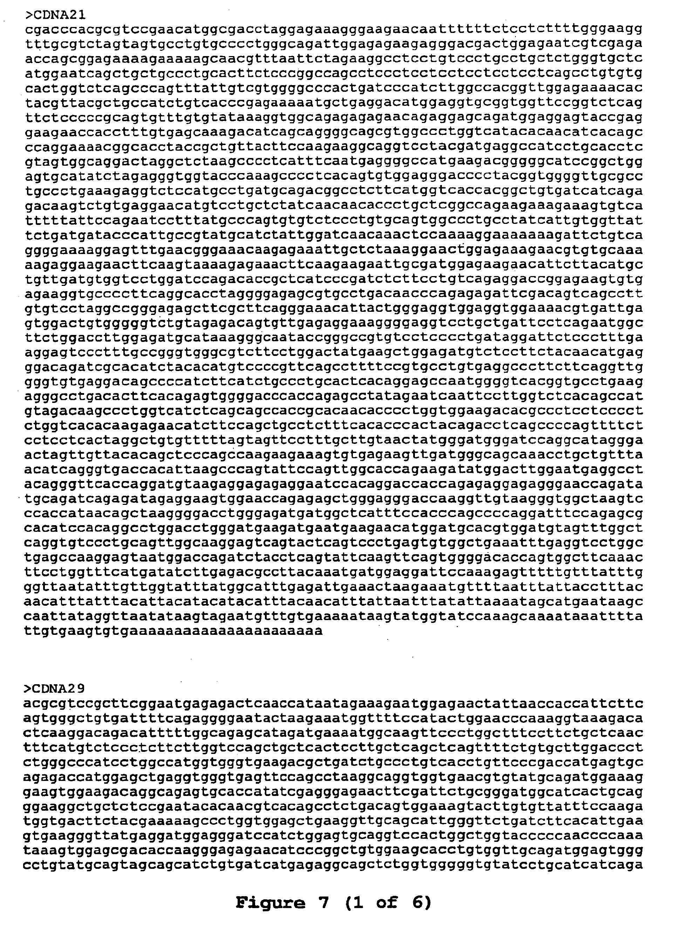

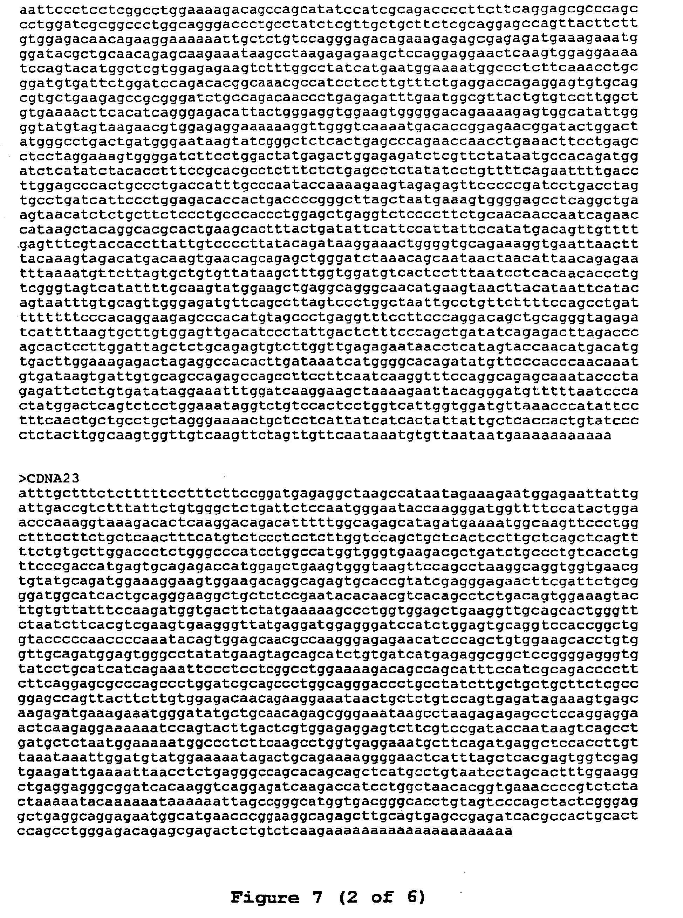

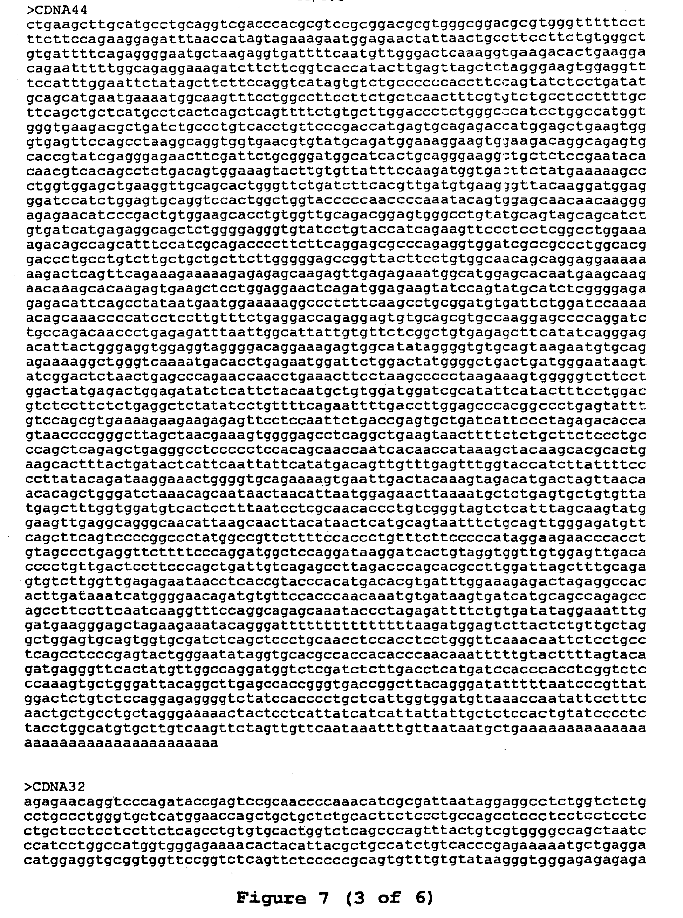

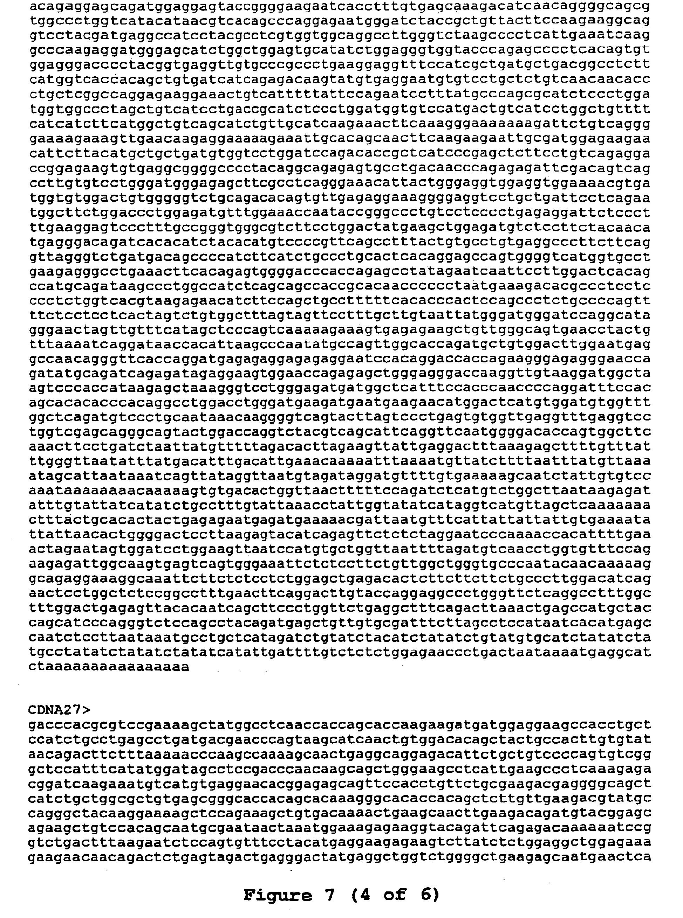

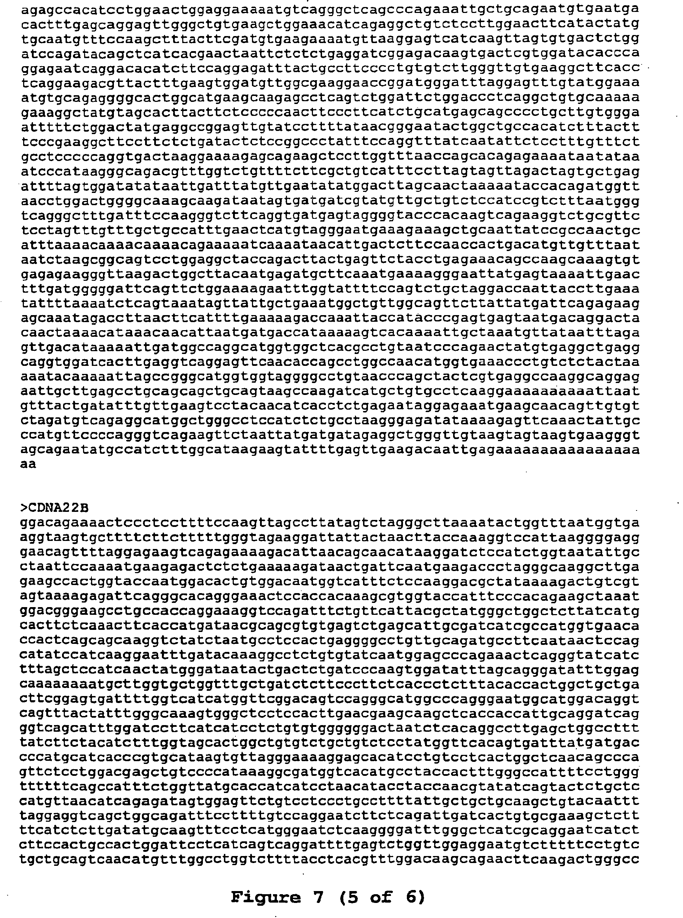

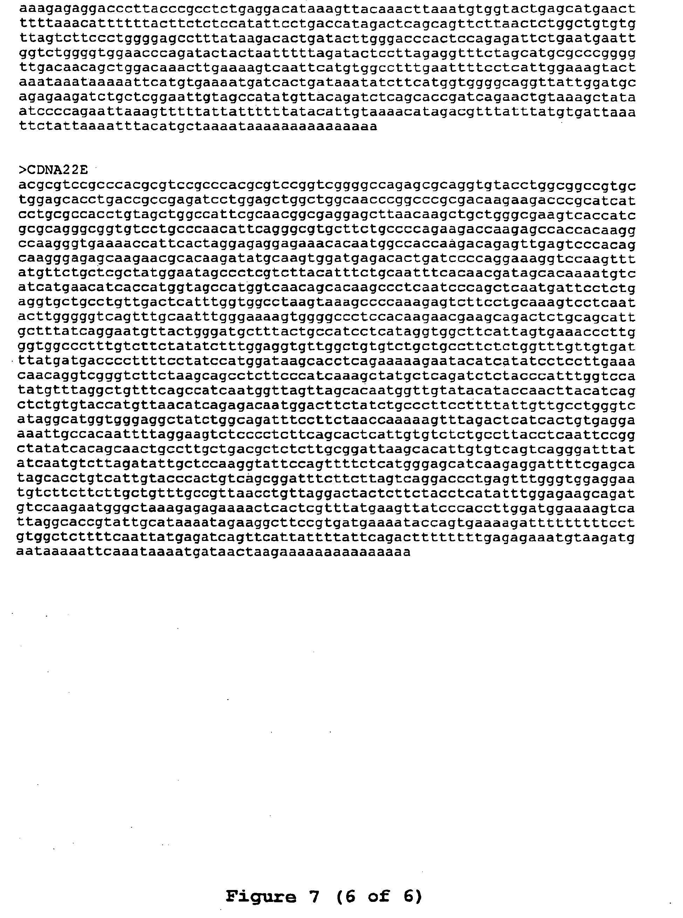

Figure 7 depicts the sequences of cDNA 21 (BTF1), cDNA 29 (BTF3), cDNA 23 (BTF4), cDNA 44 (BTF5), cDNA 32 (BTF2), cDNA 27 (RoRet), cDNA 22B (NPT3), cDNA22E (NPT4).

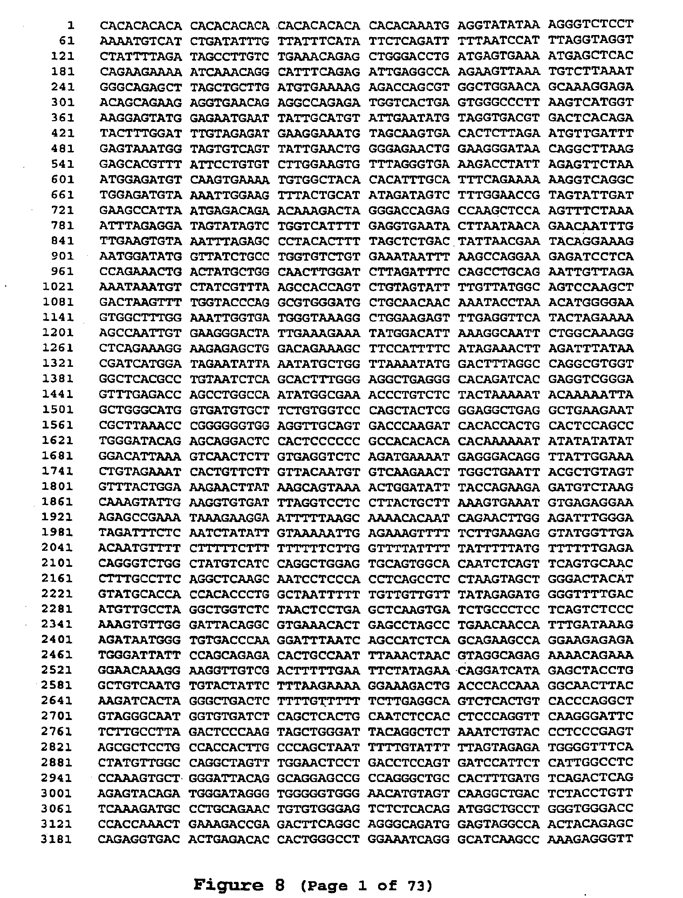

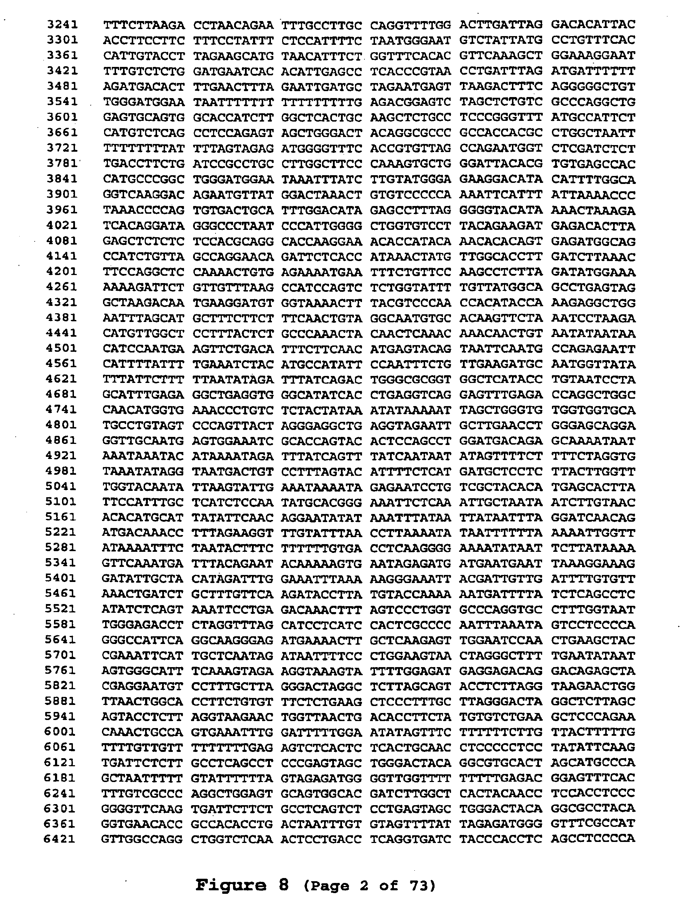

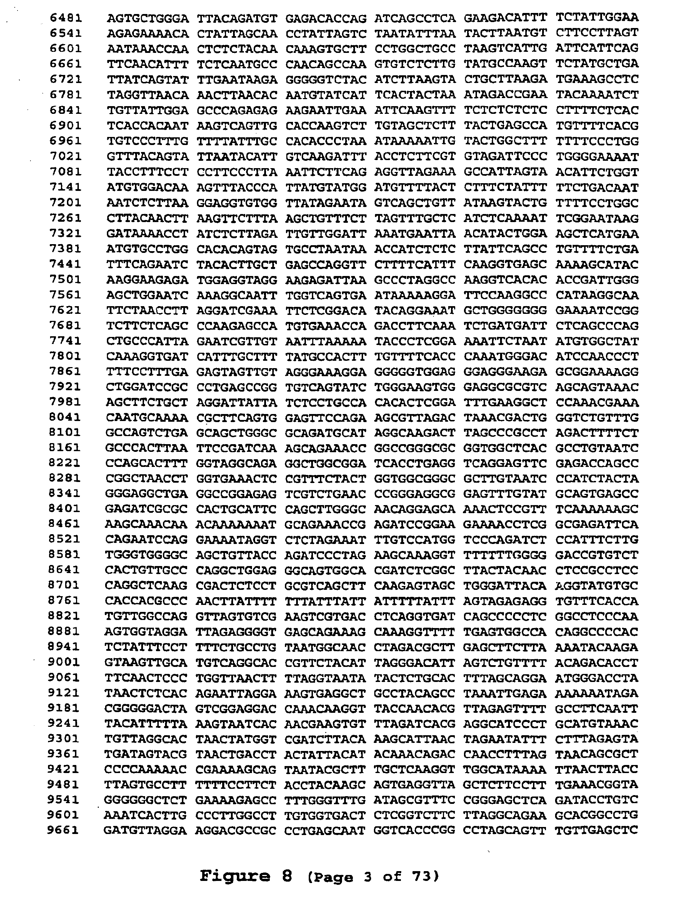

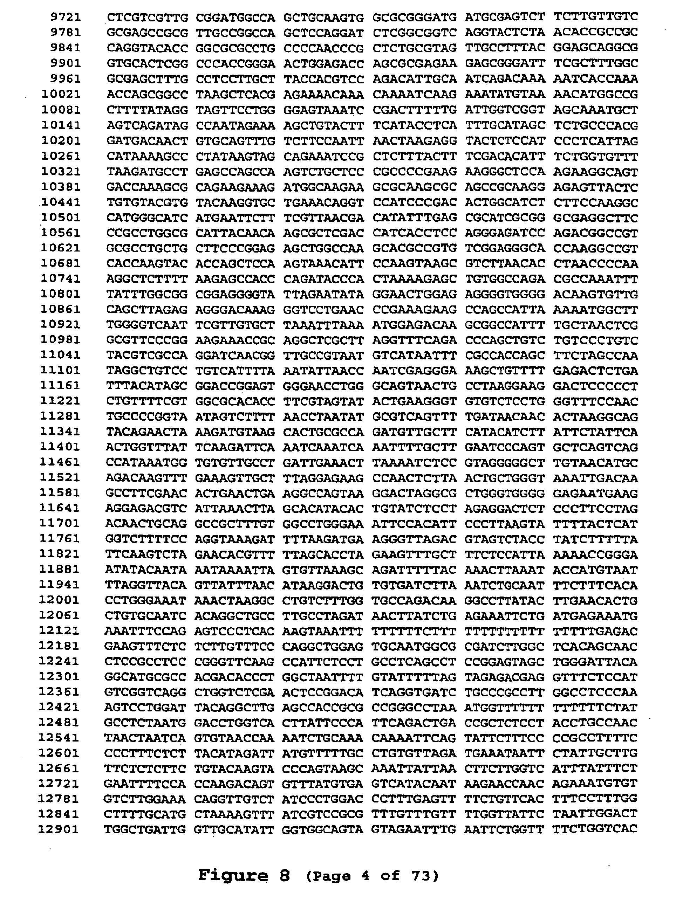

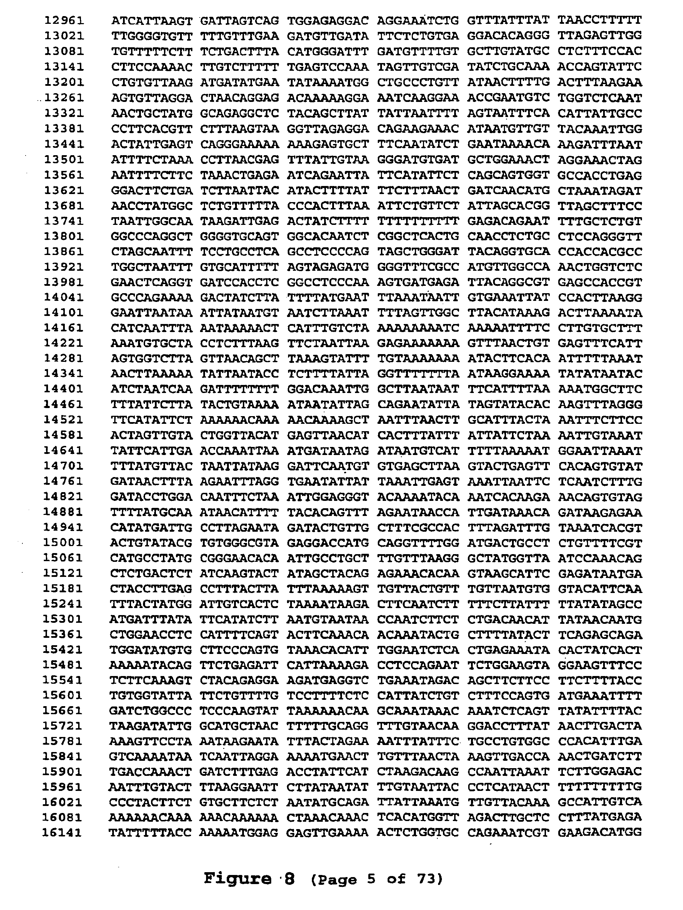

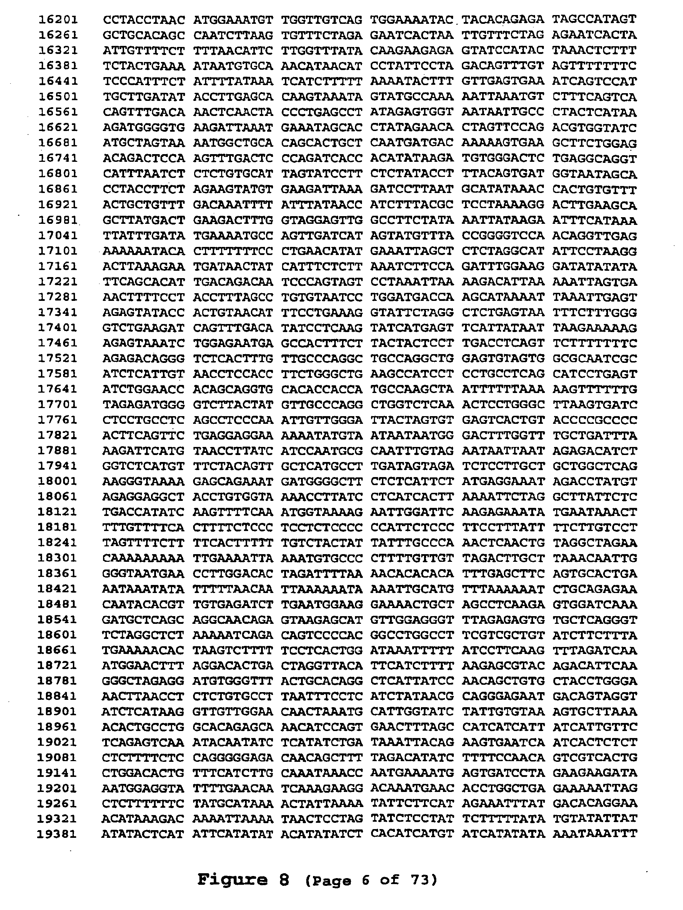

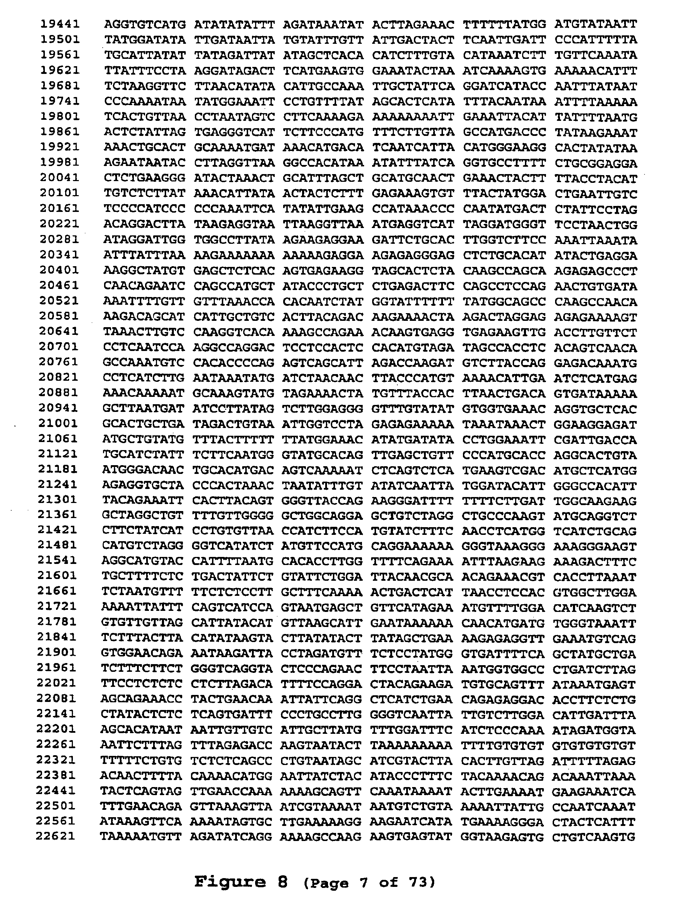

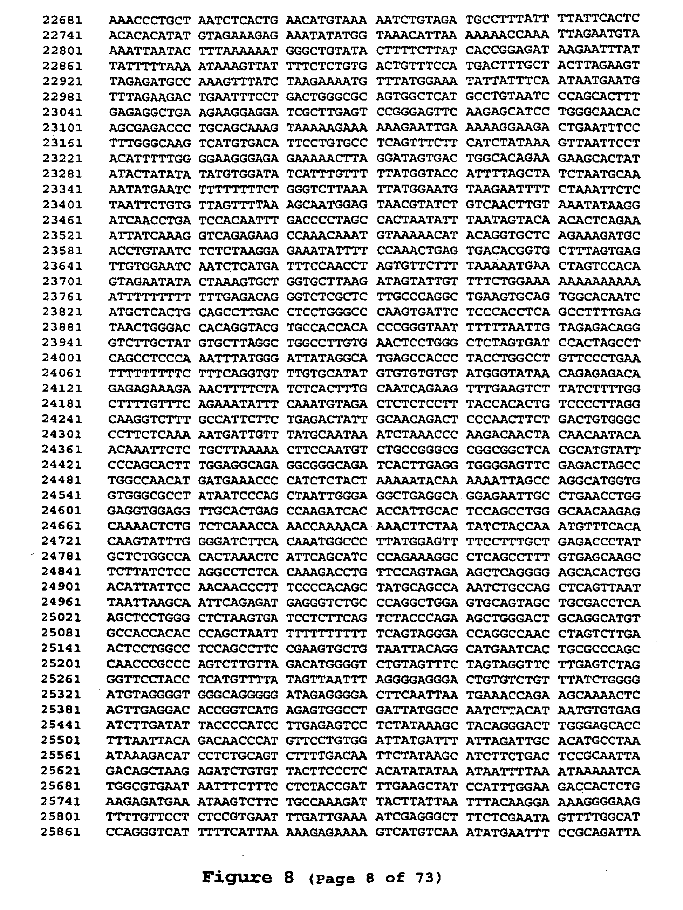

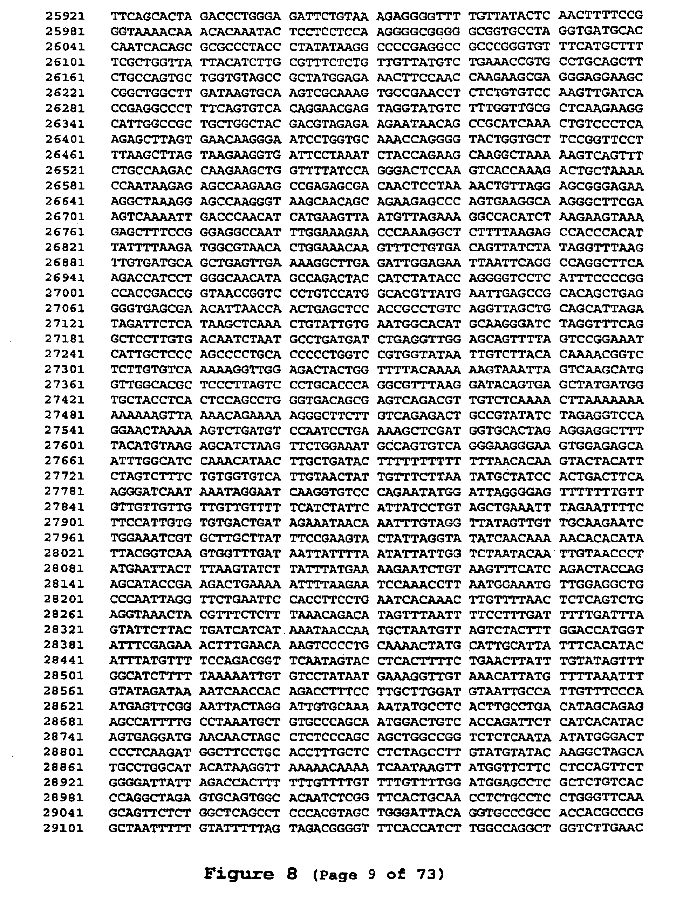

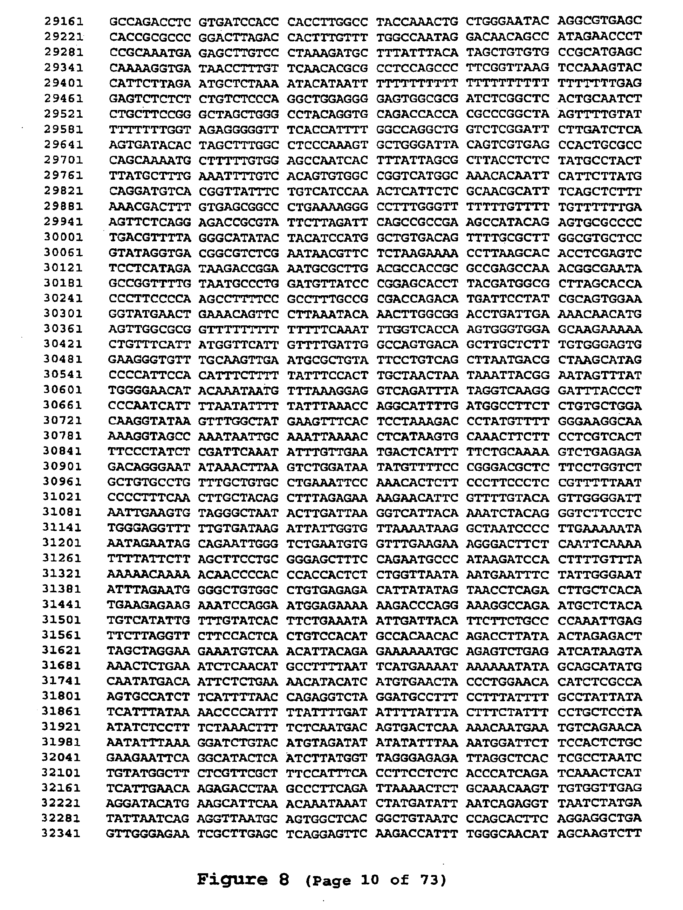

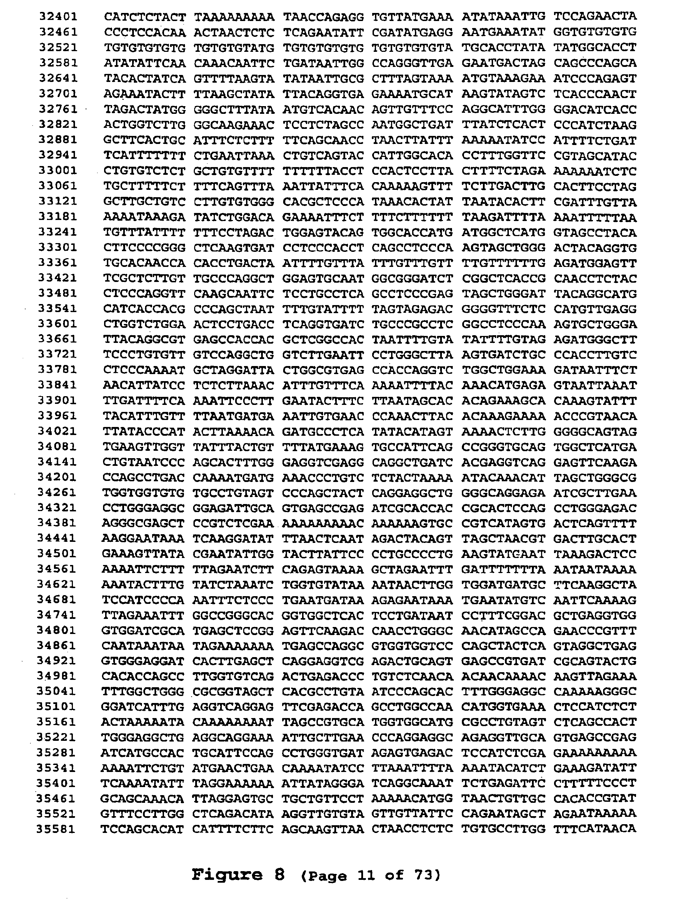

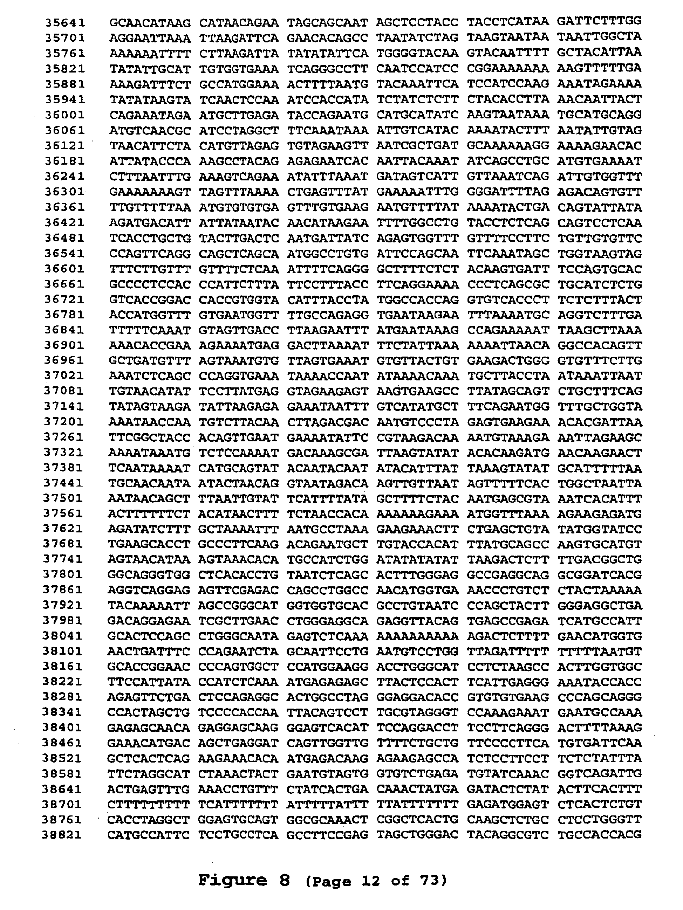

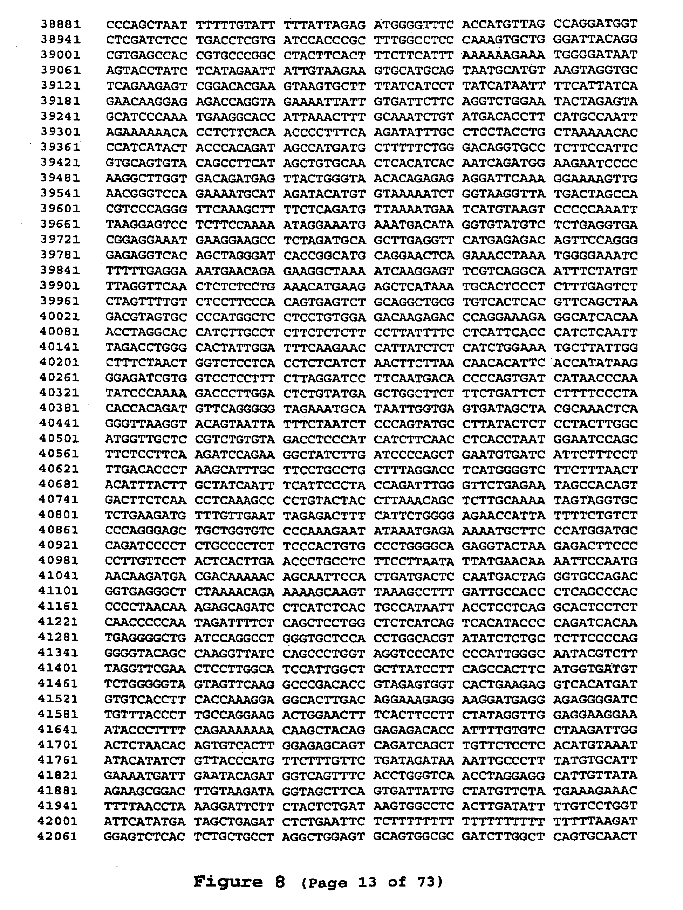

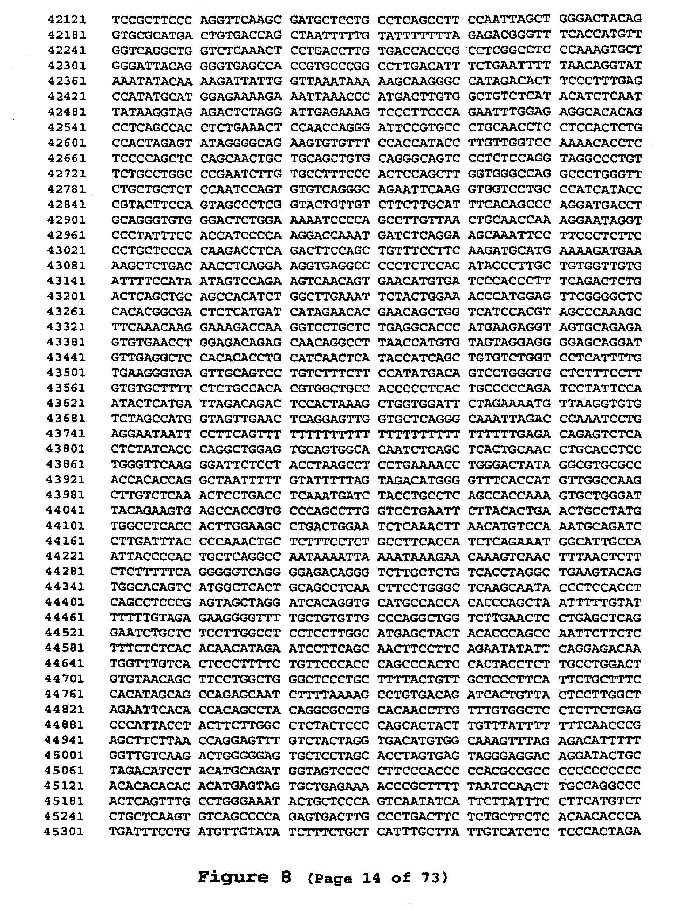

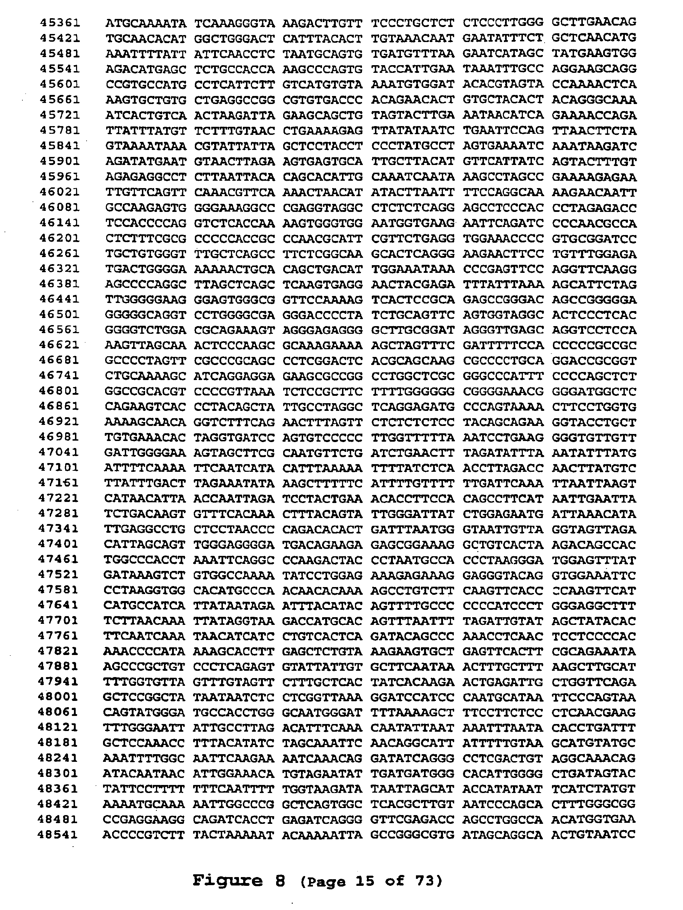

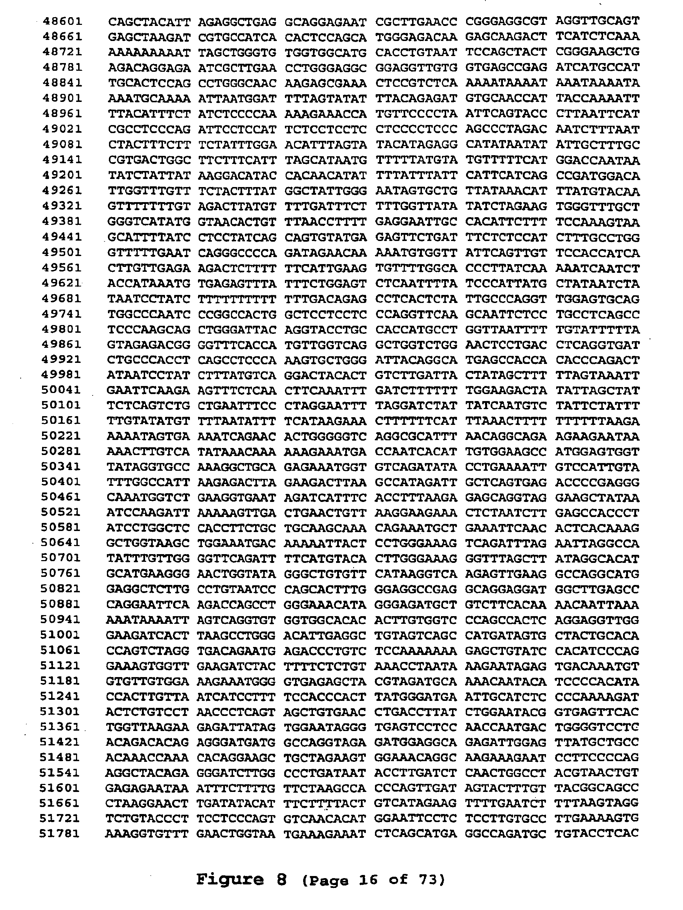

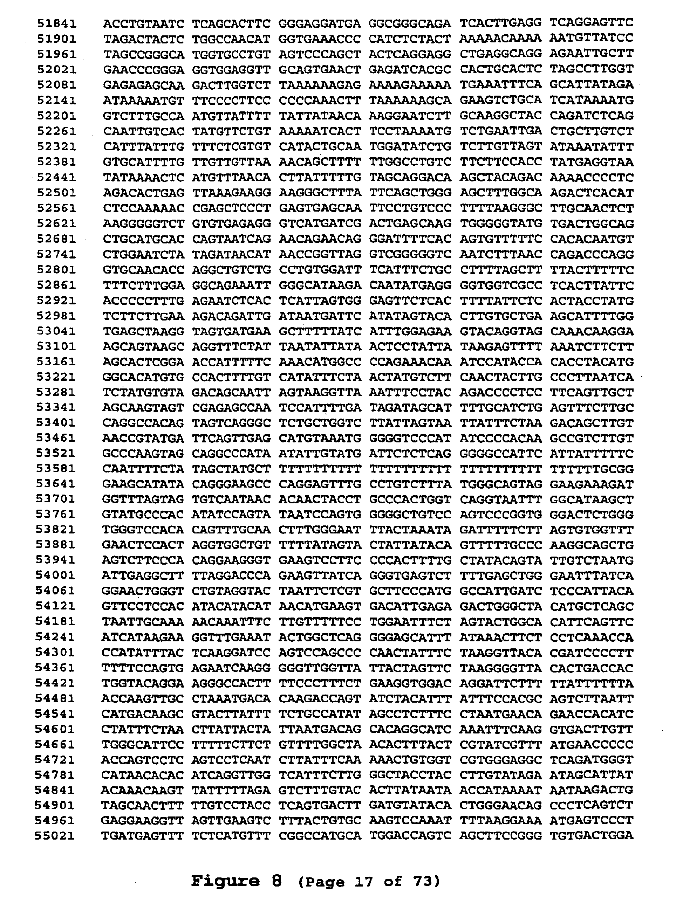

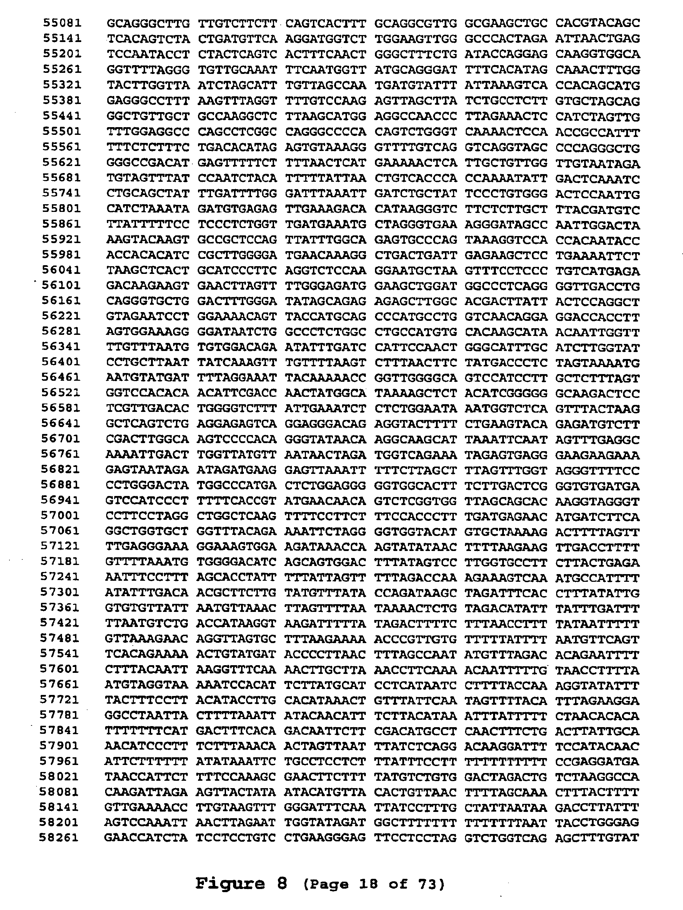

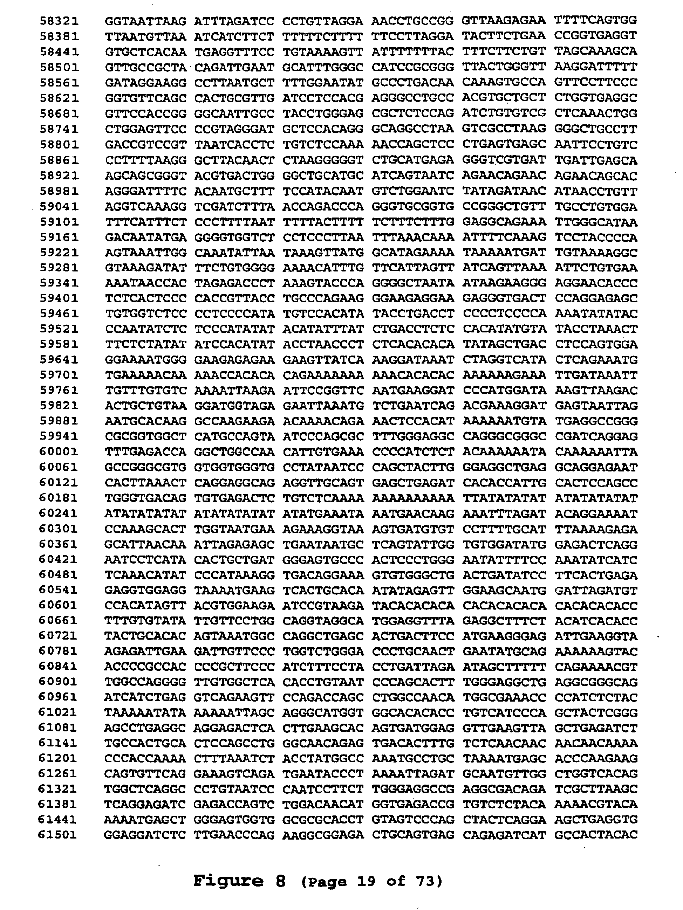

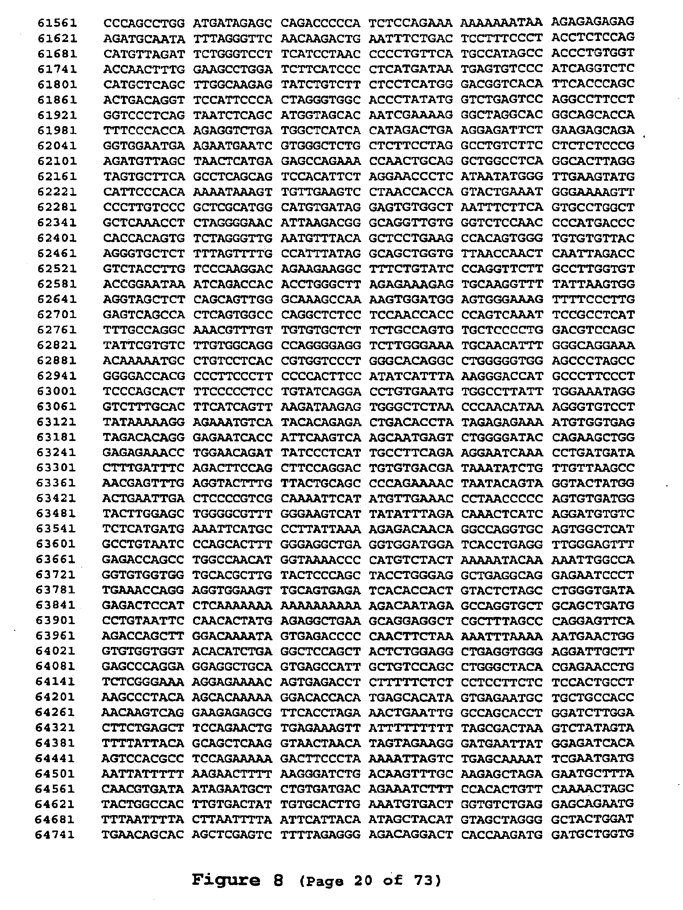

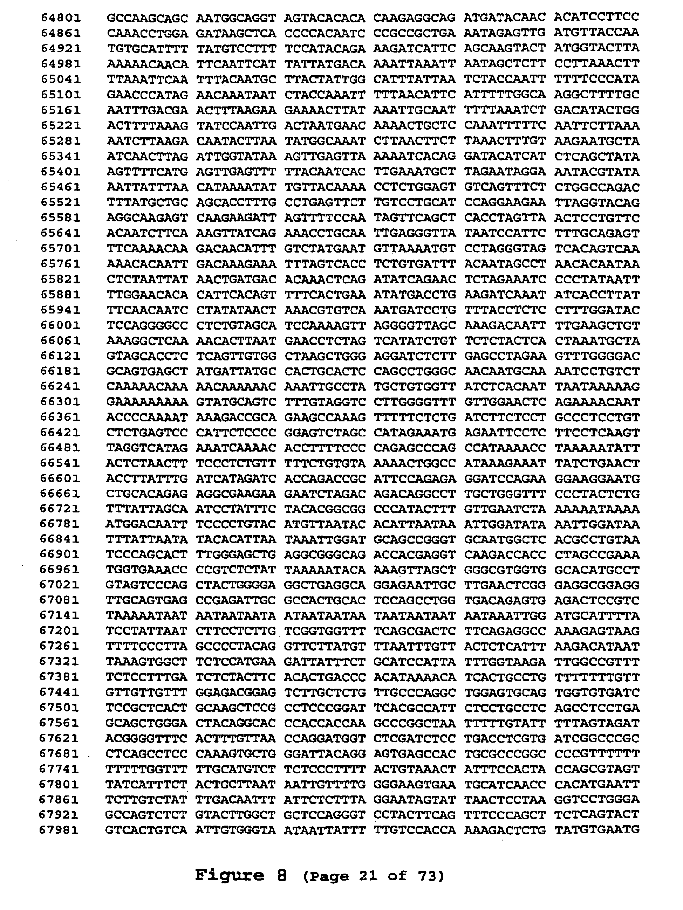

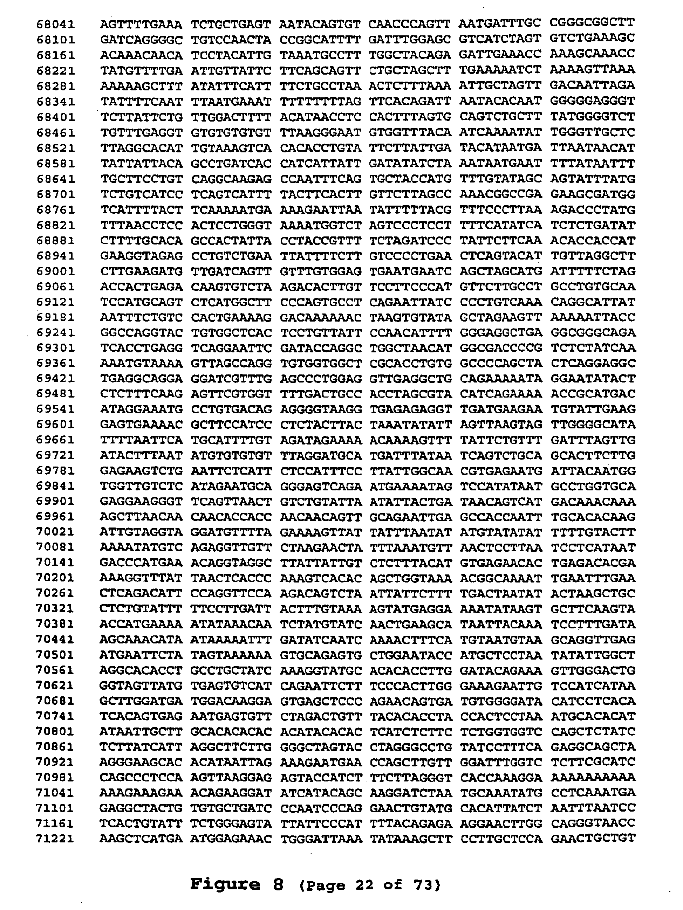

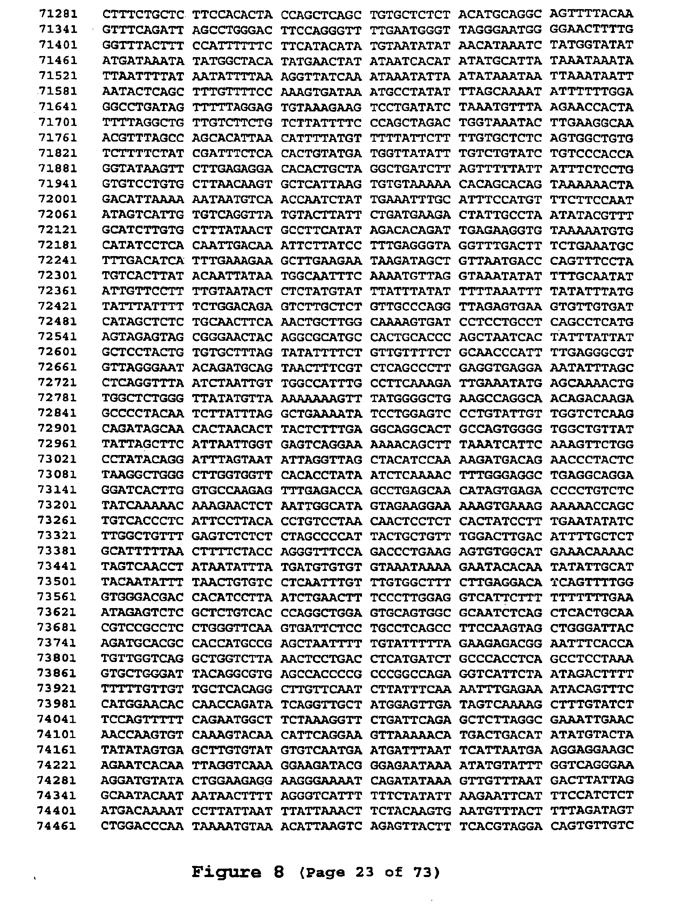

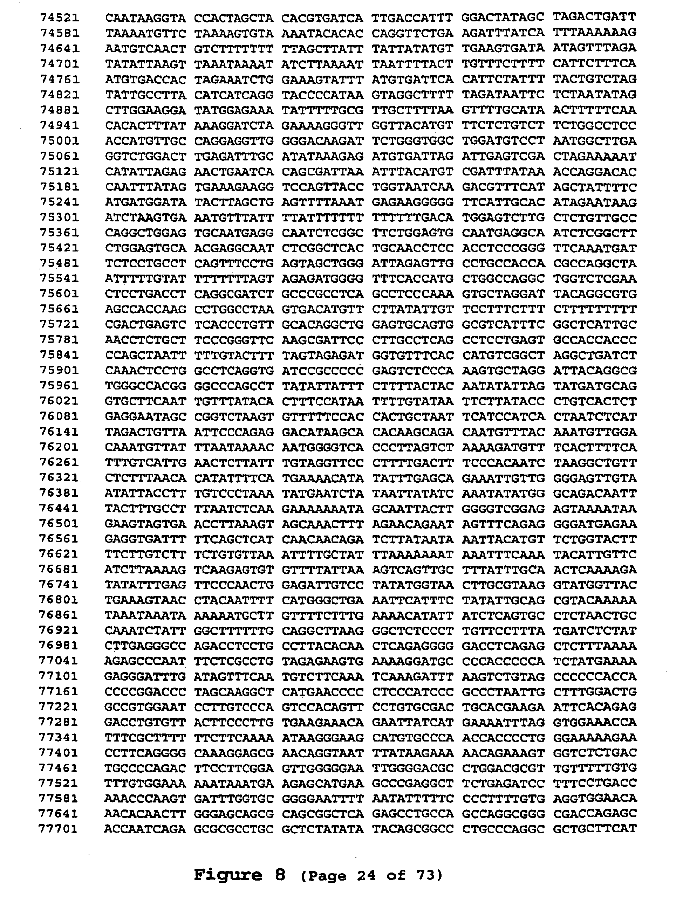

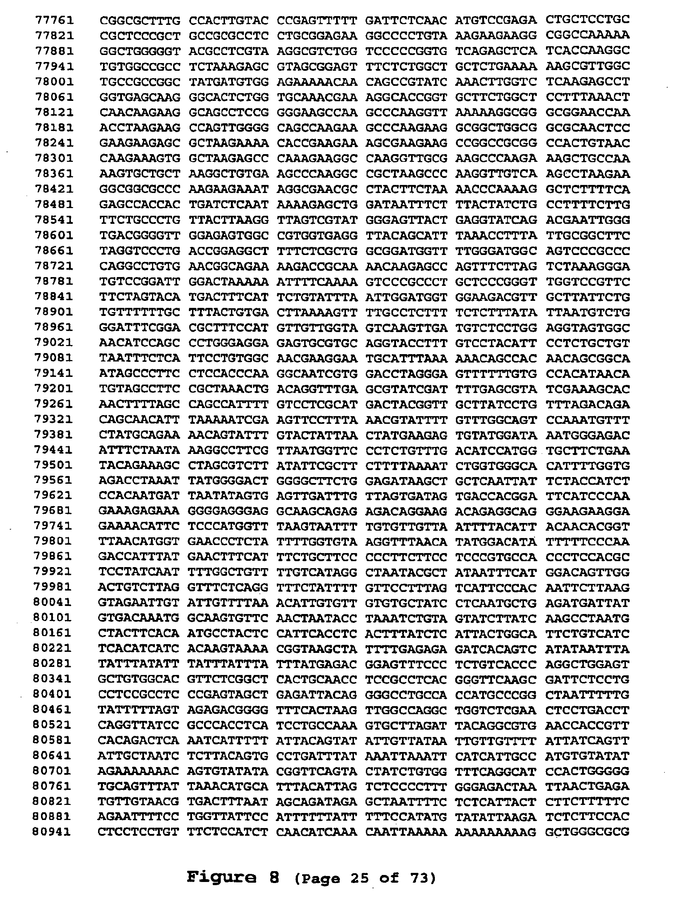

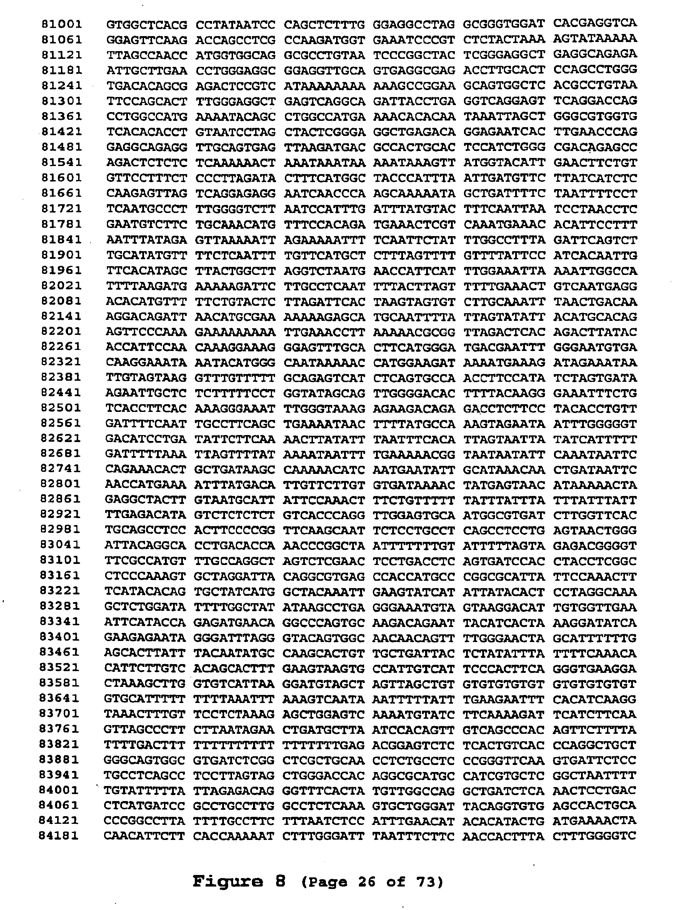

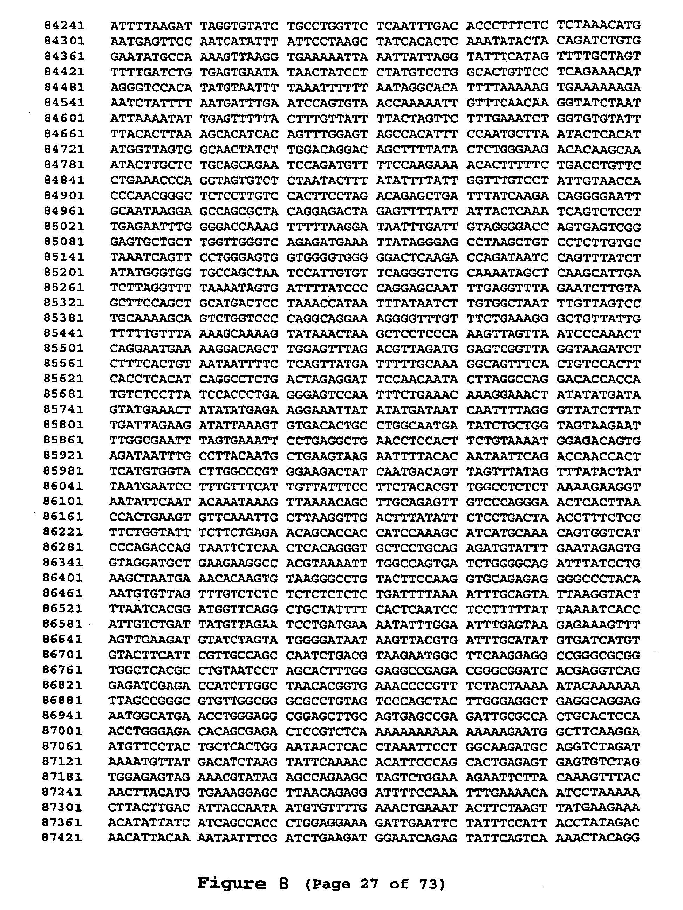

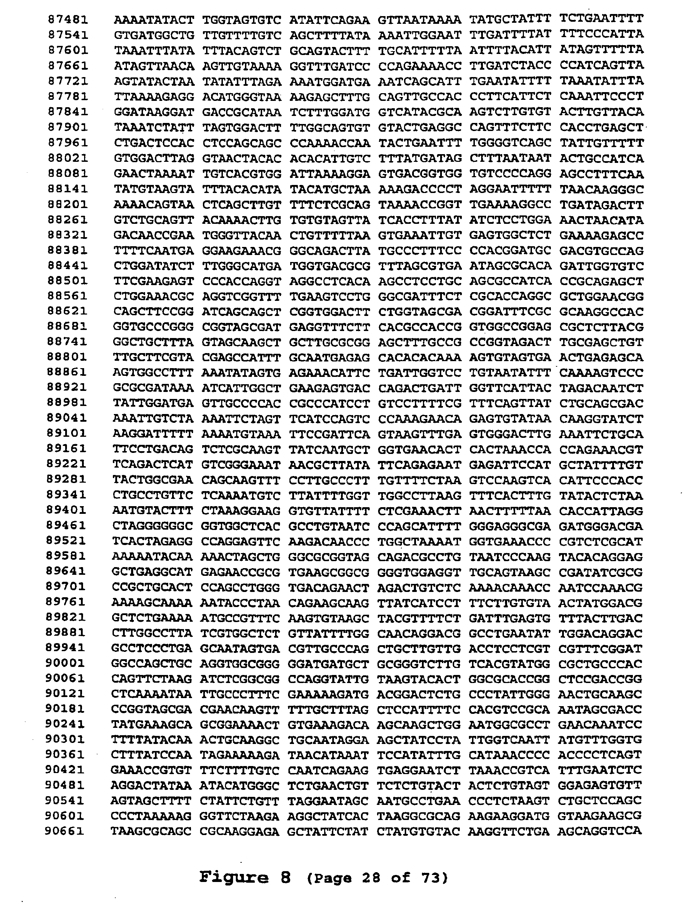

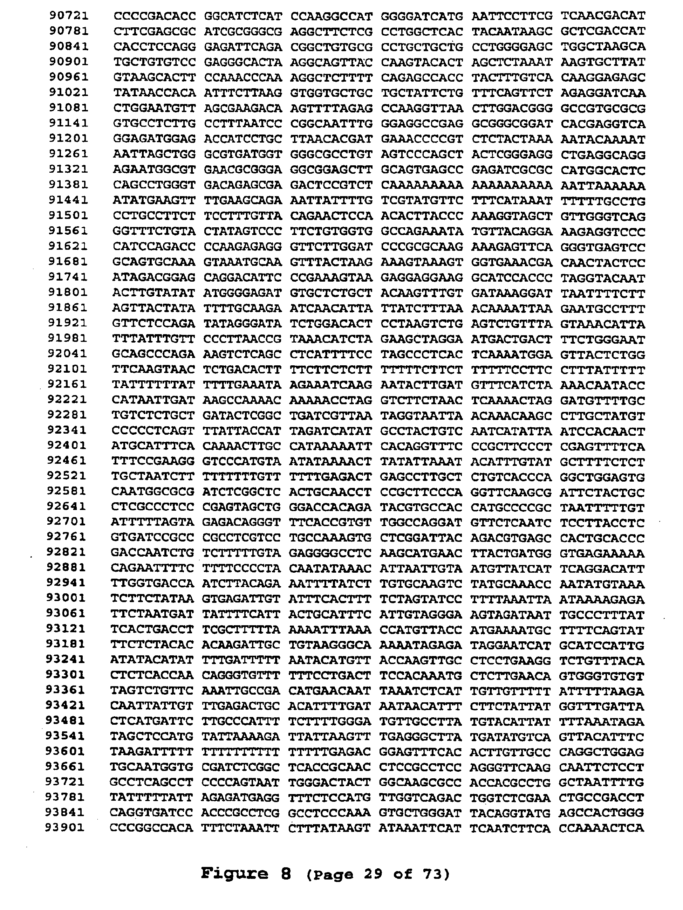

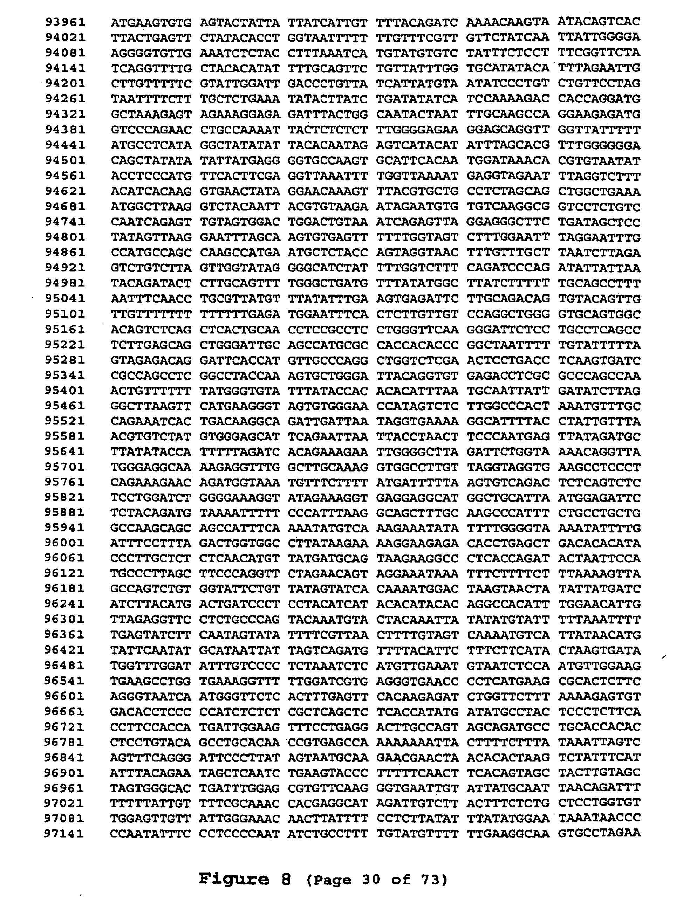

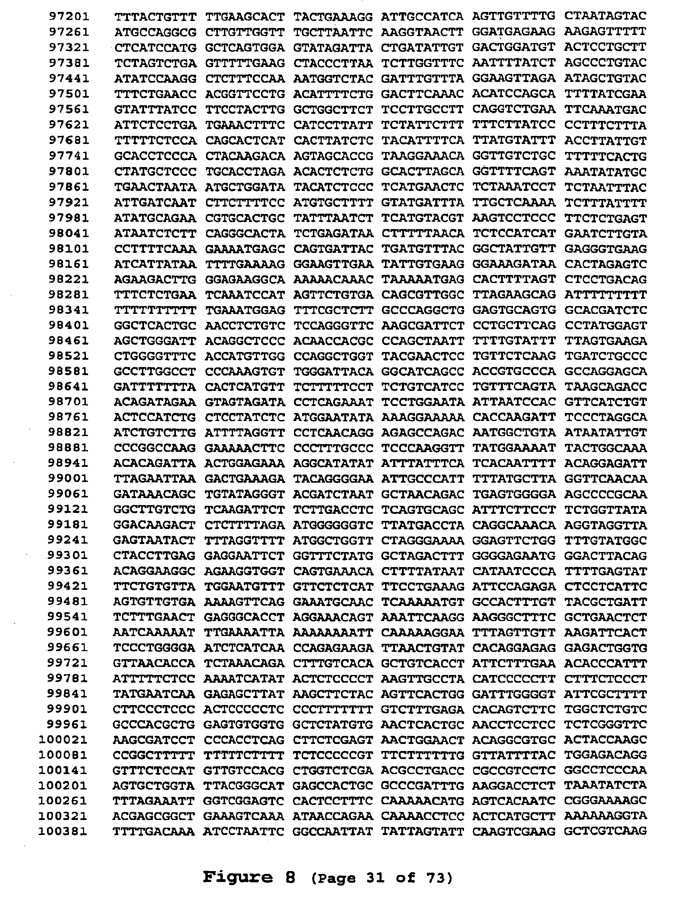

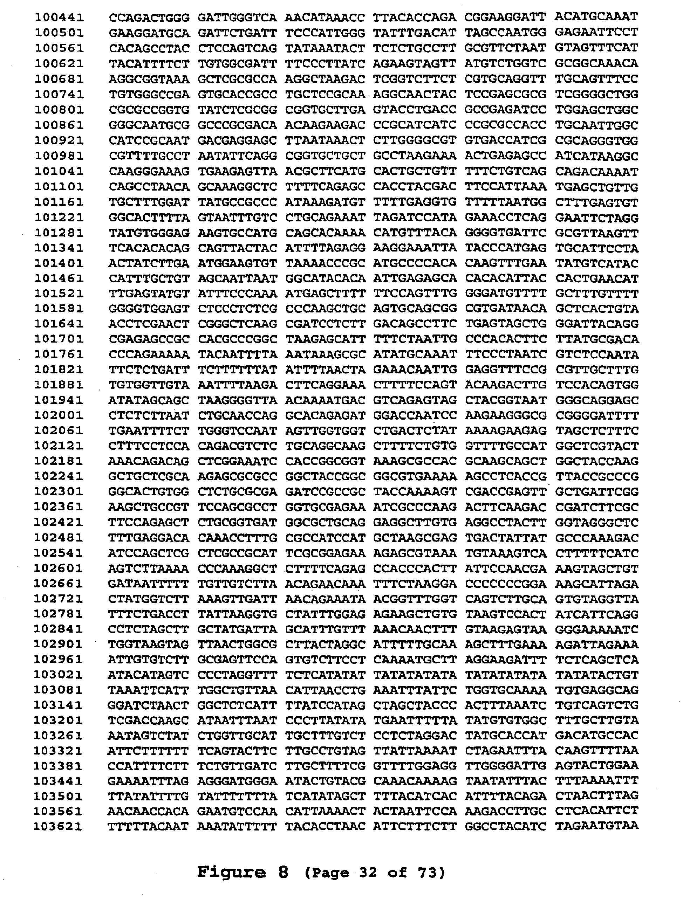

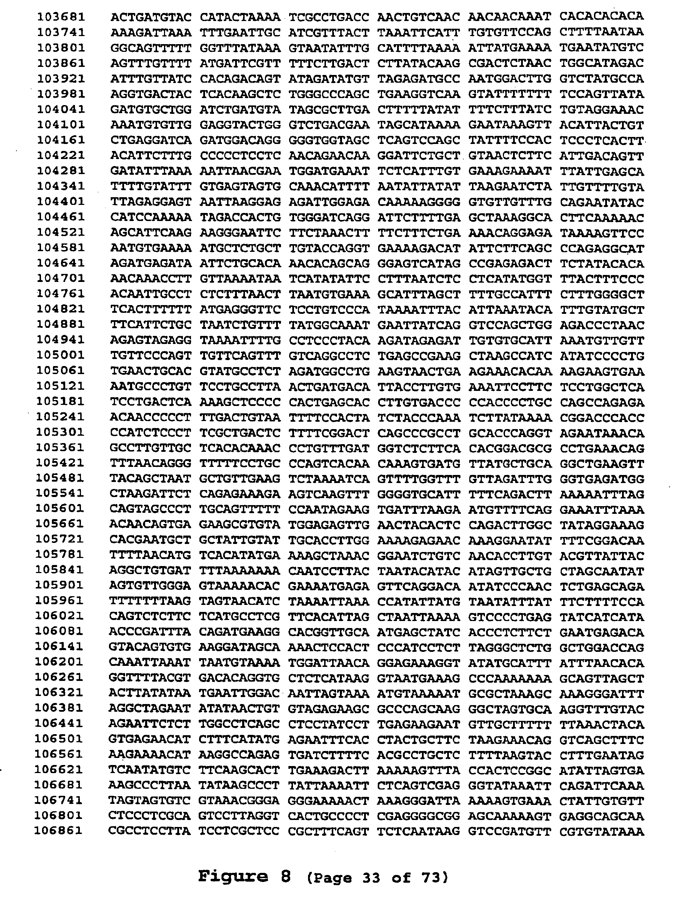

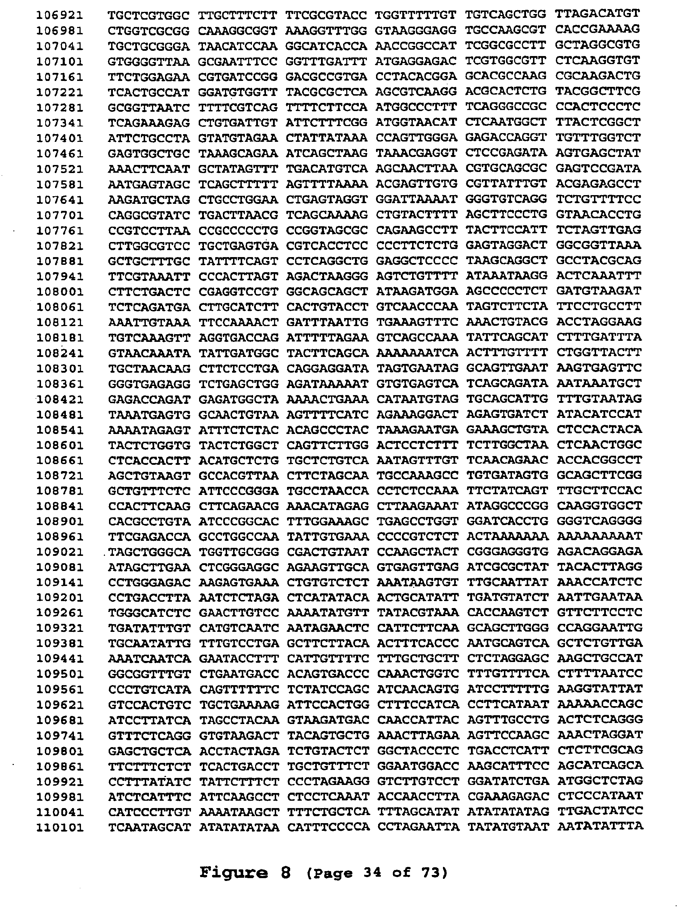

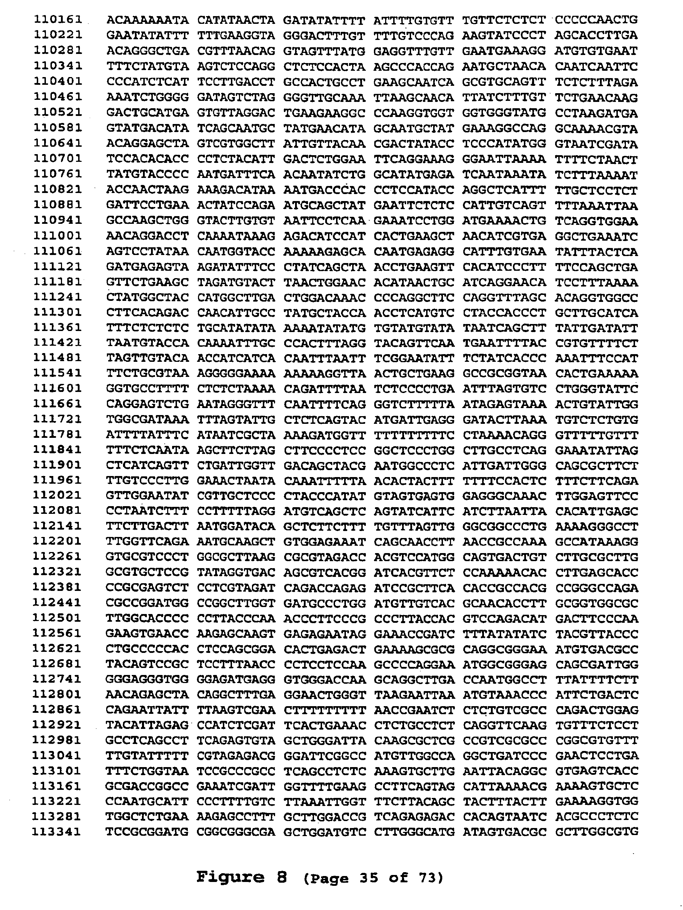

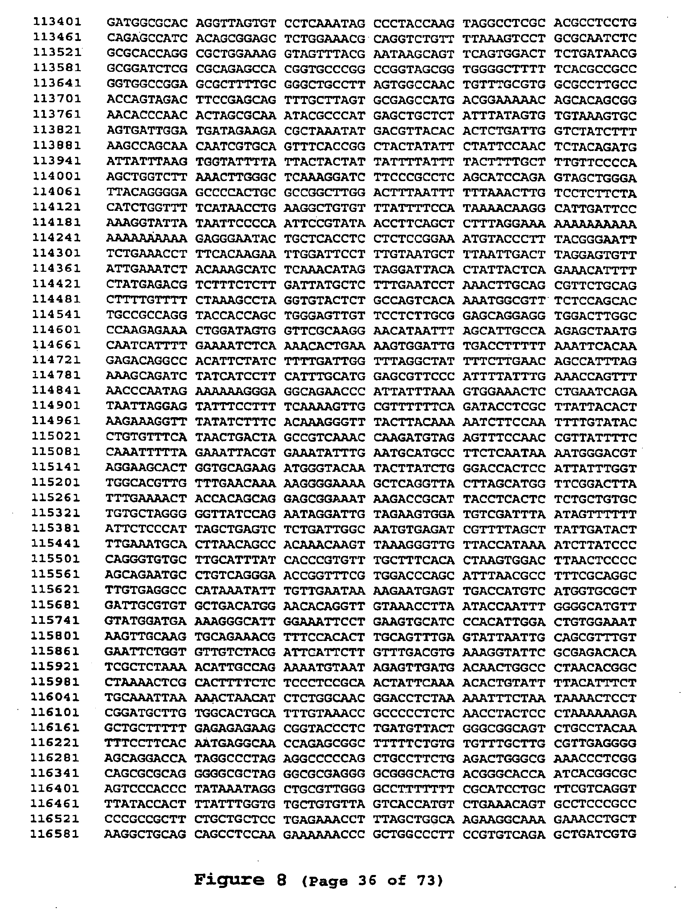

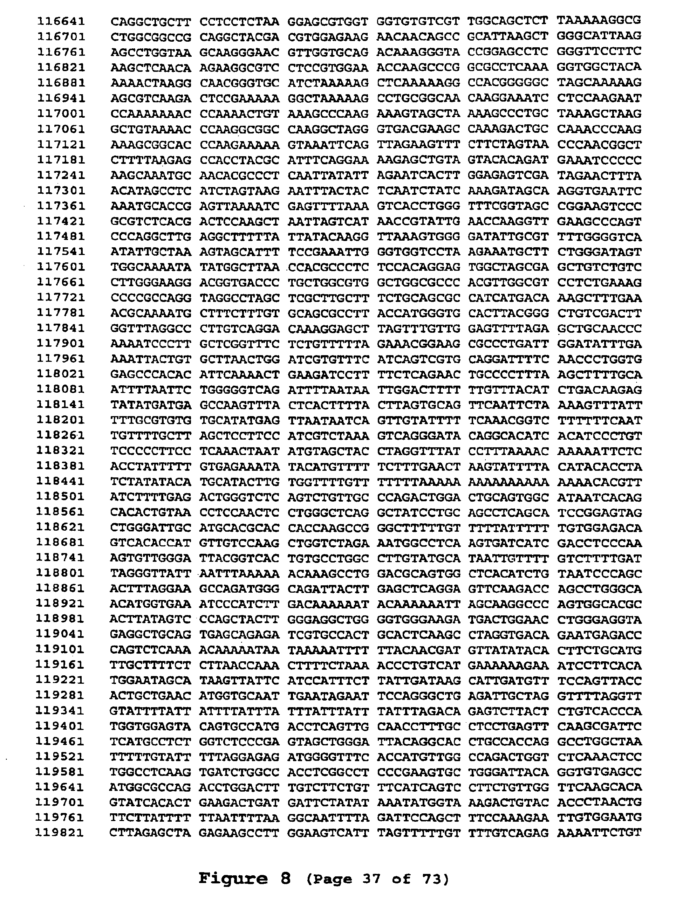

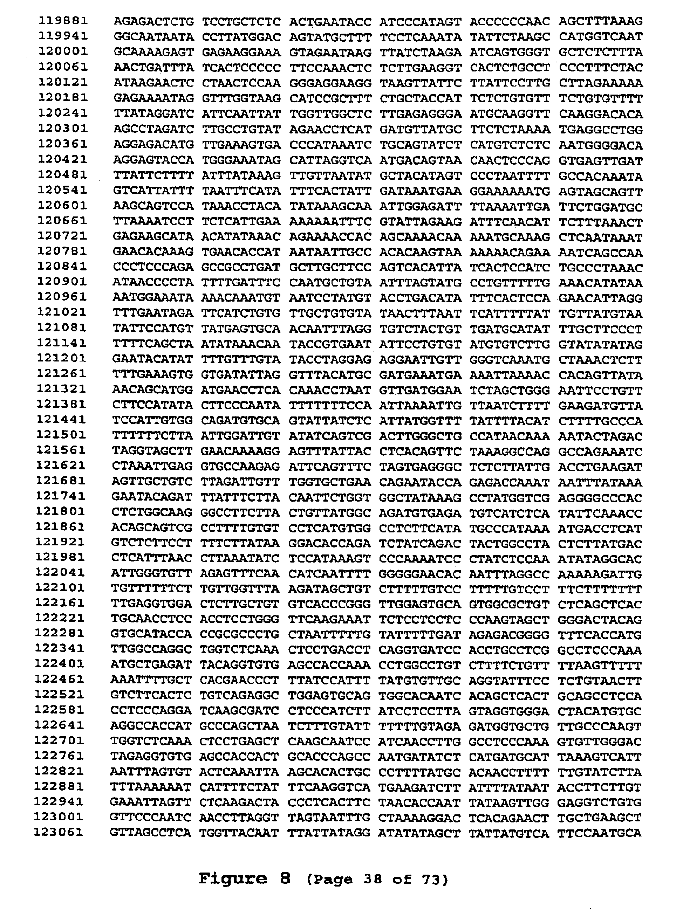

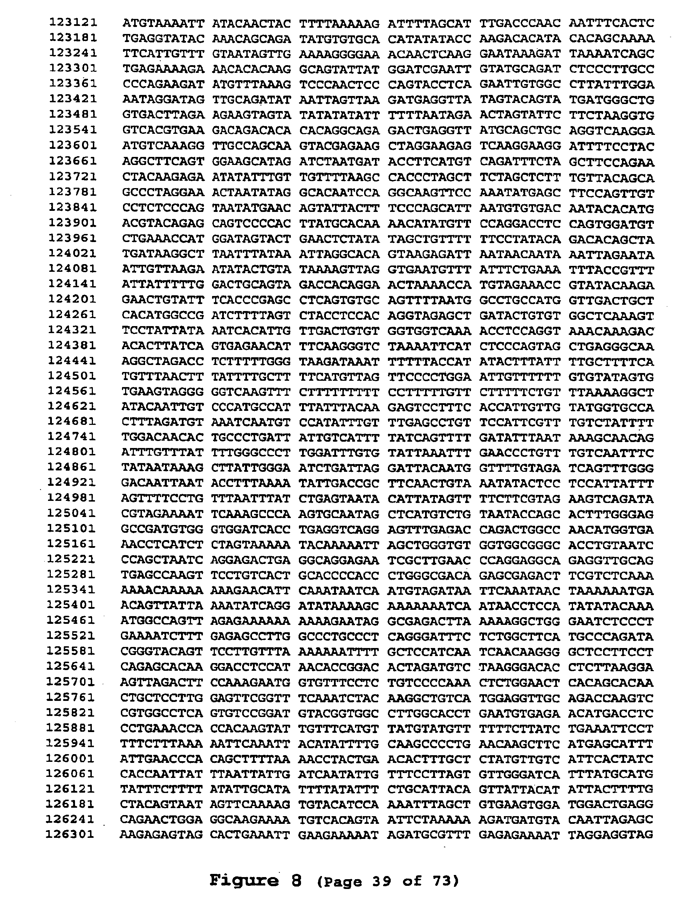

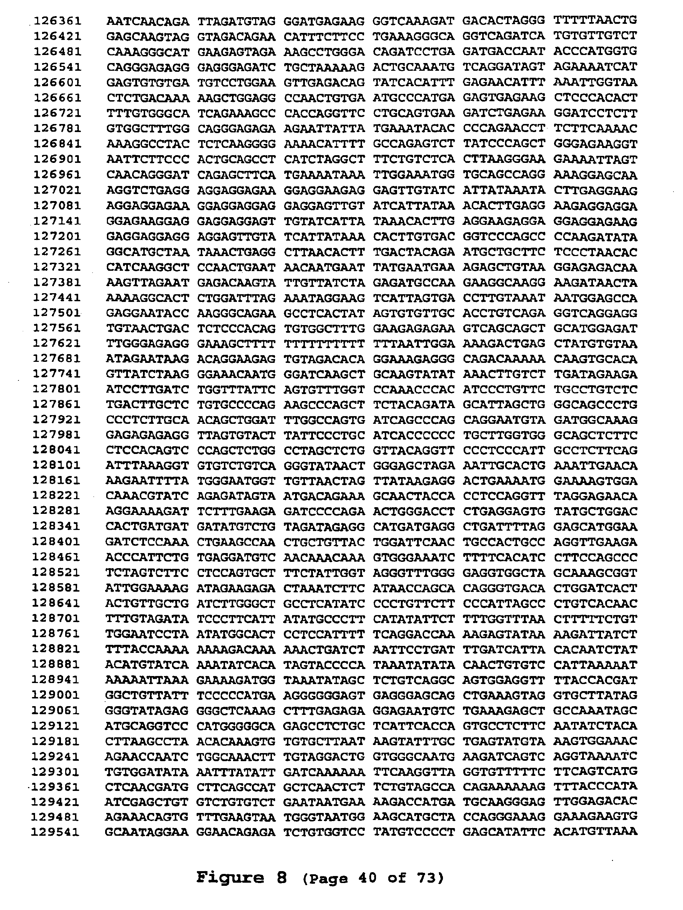

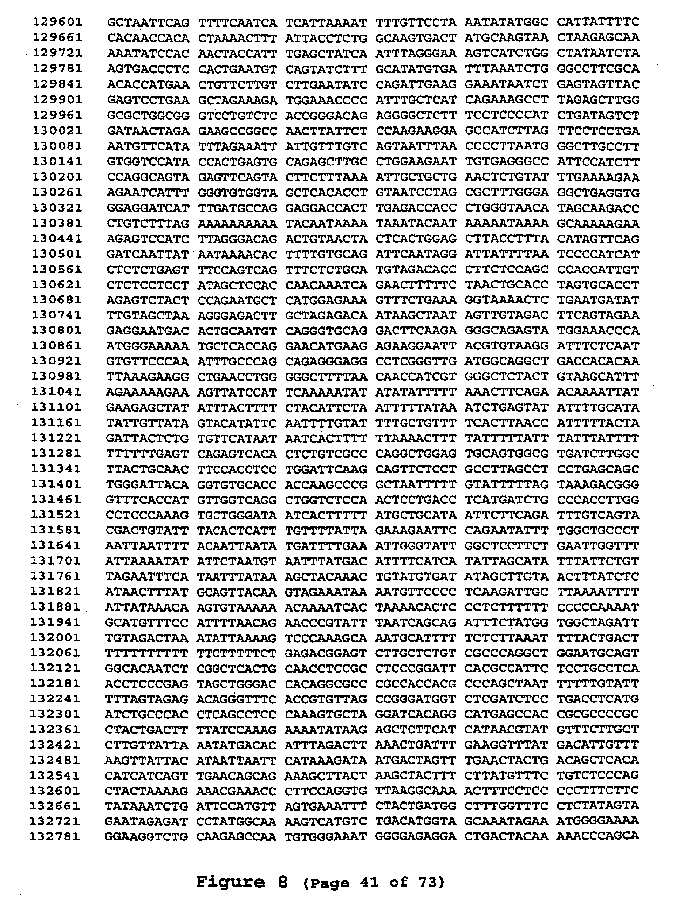

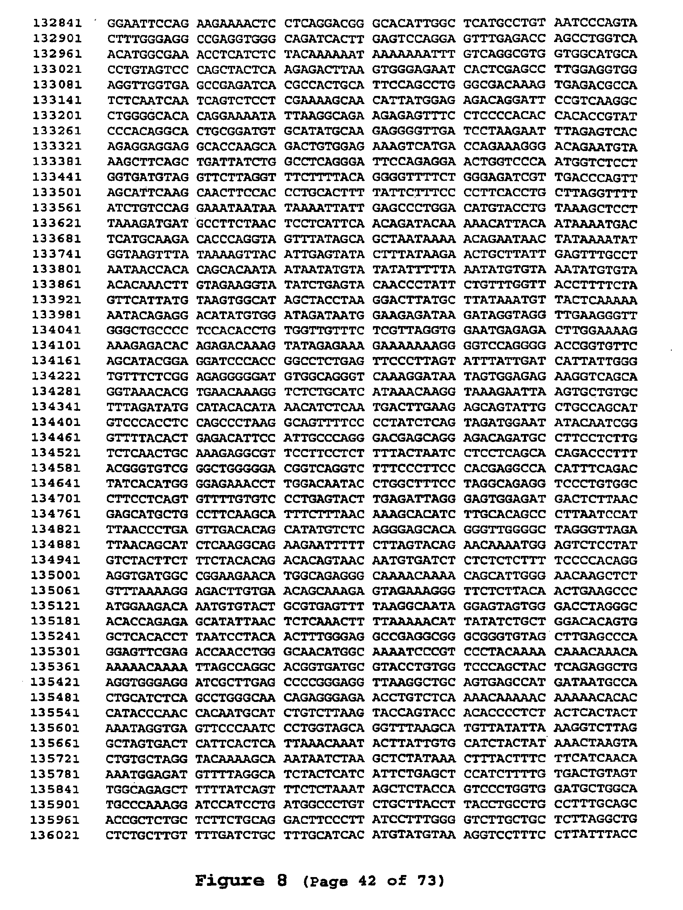

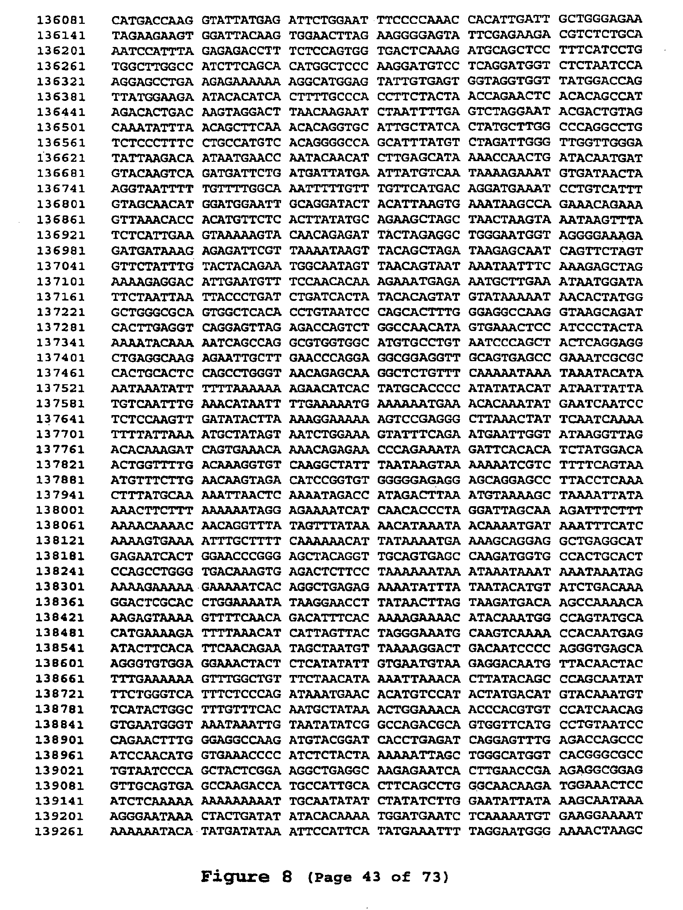

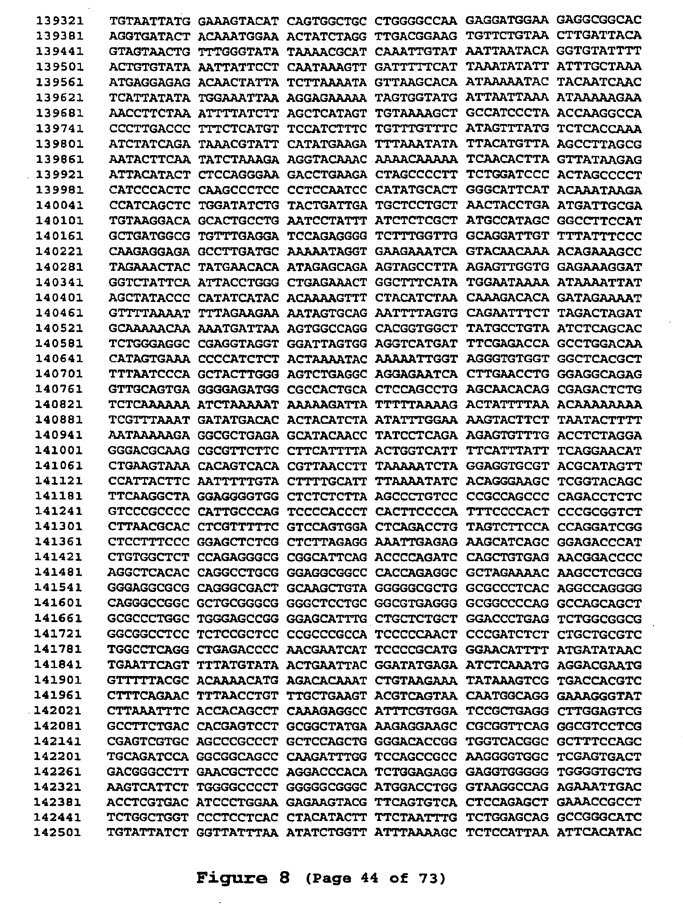

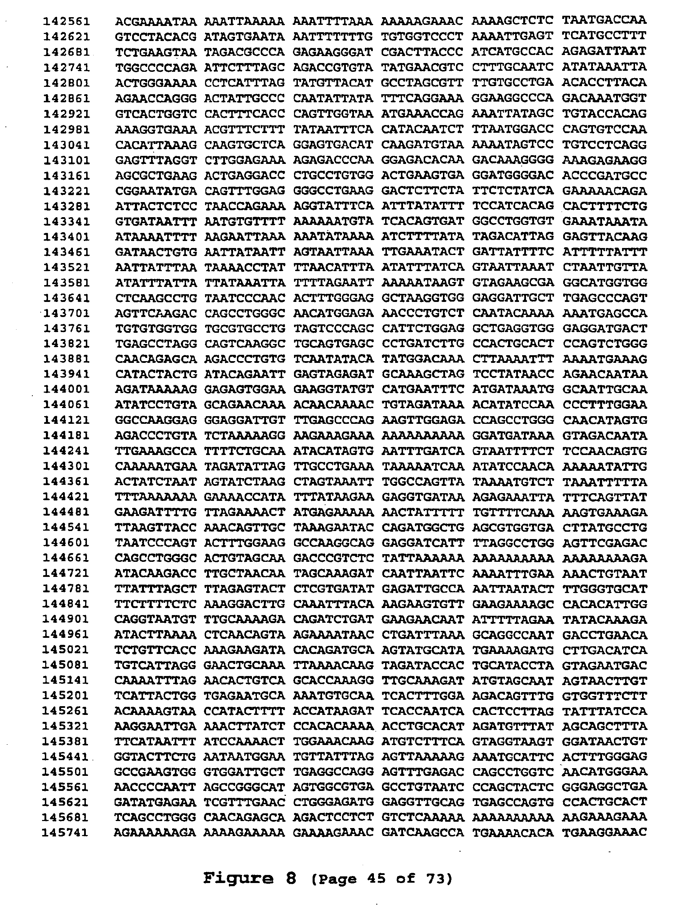

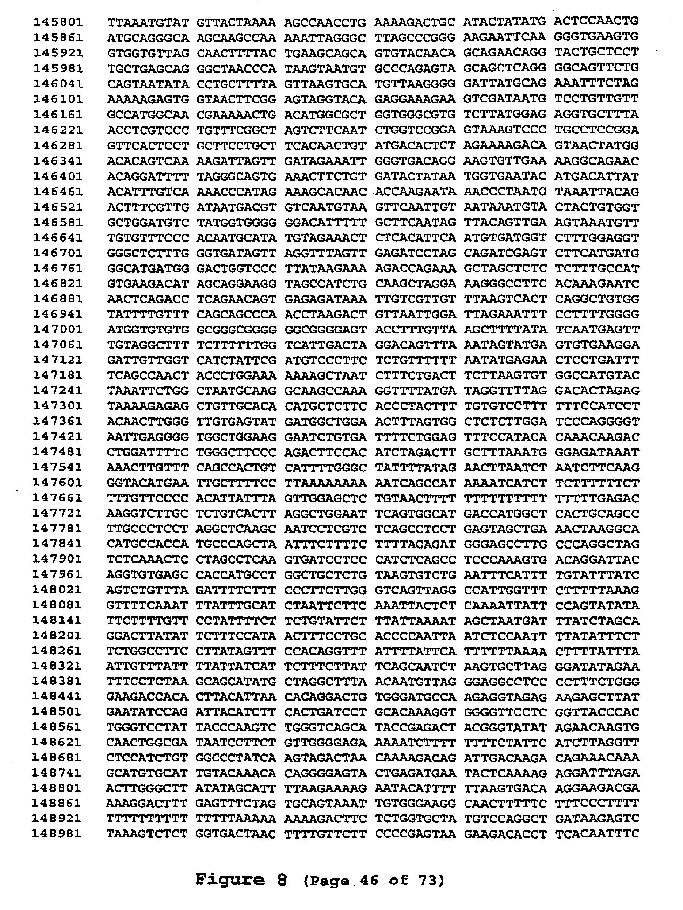

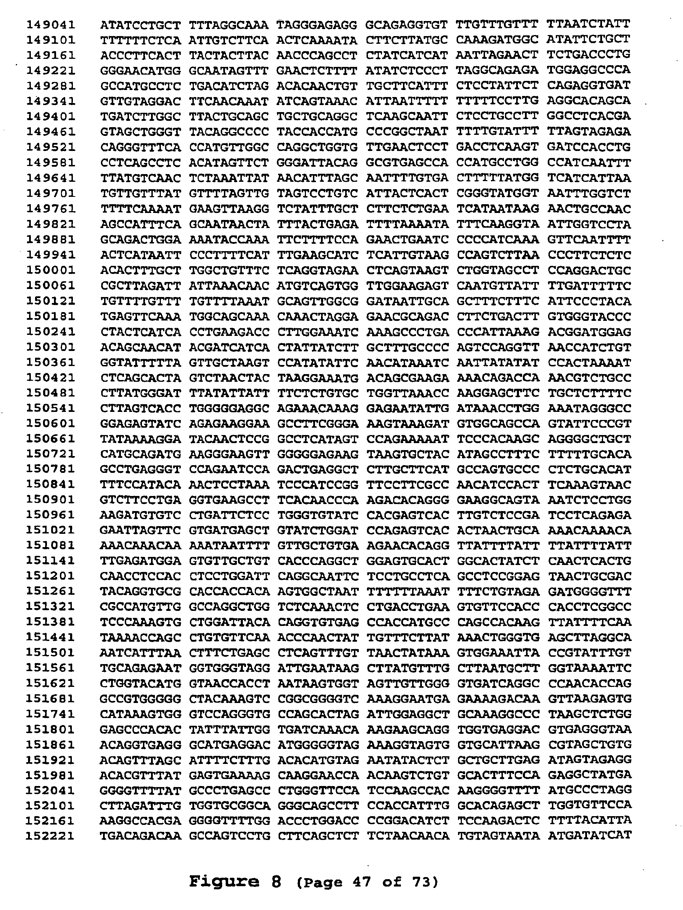

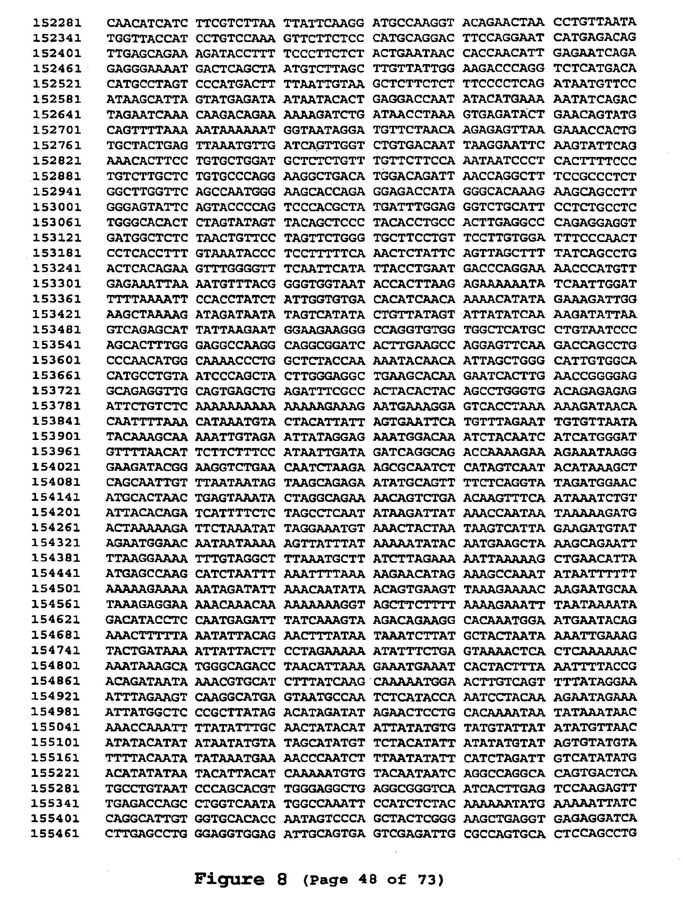

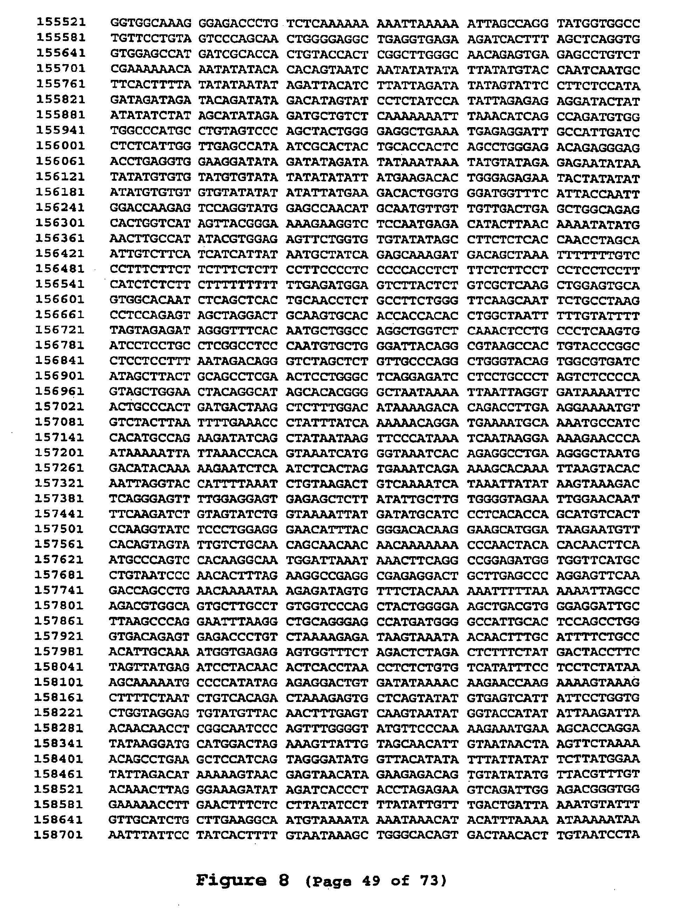

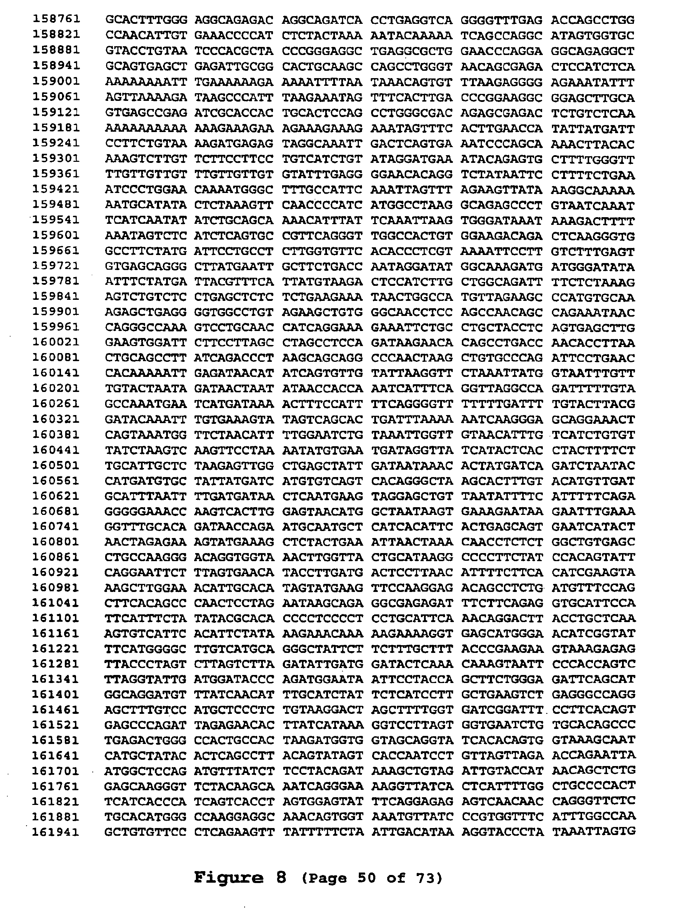

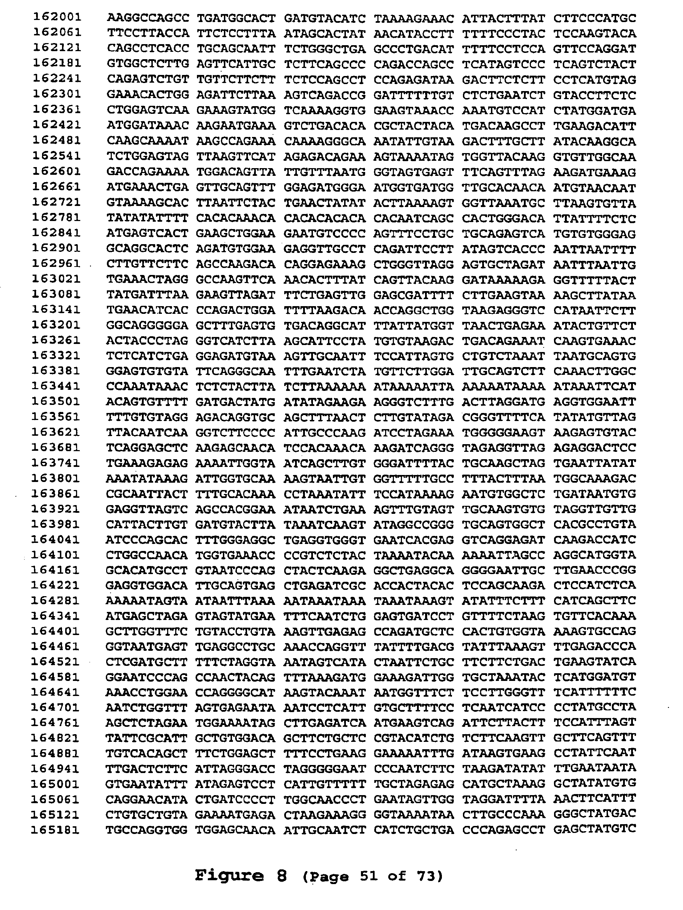

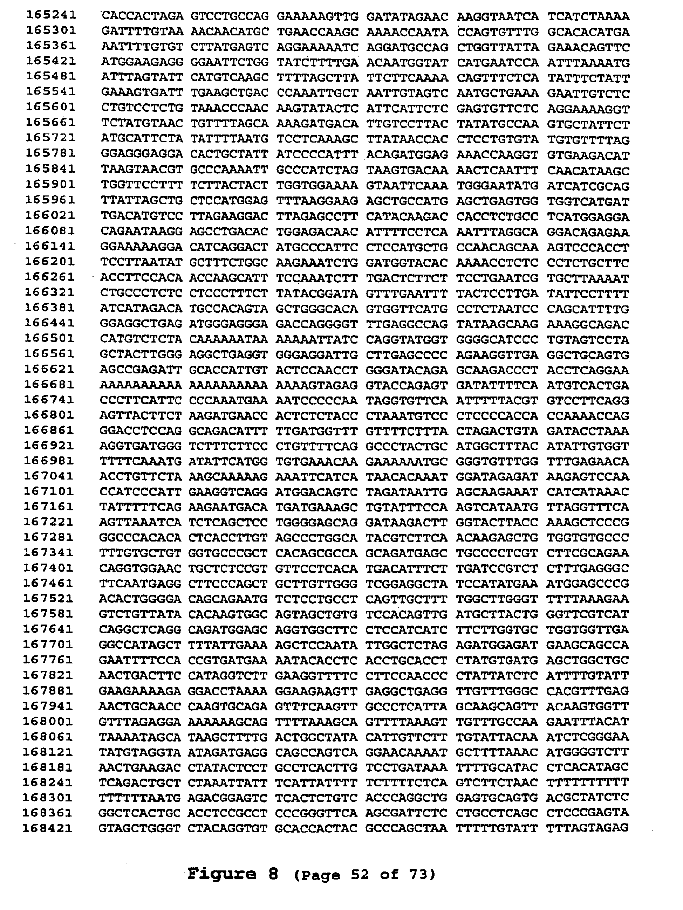

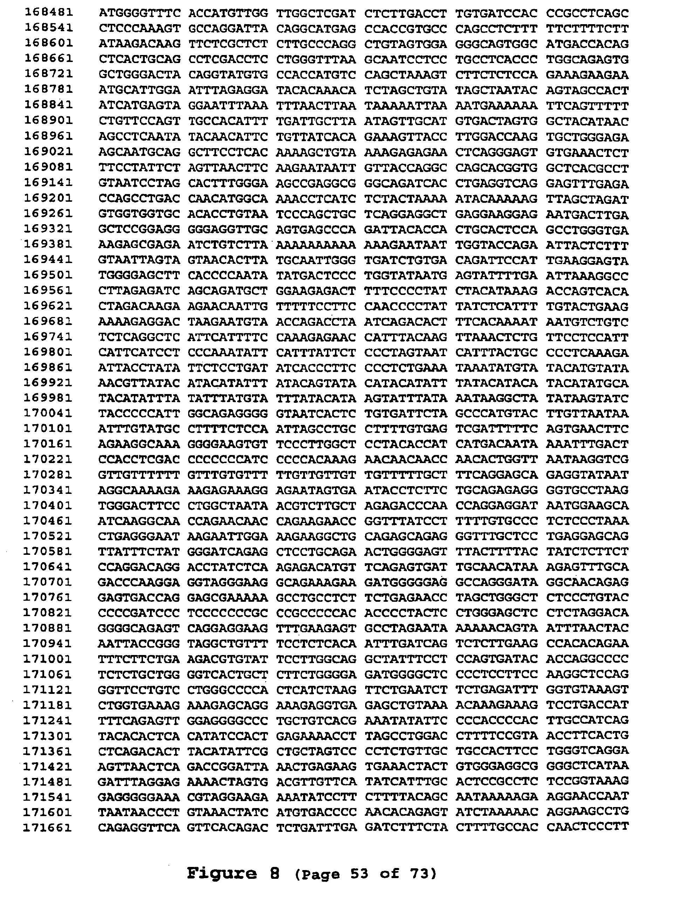

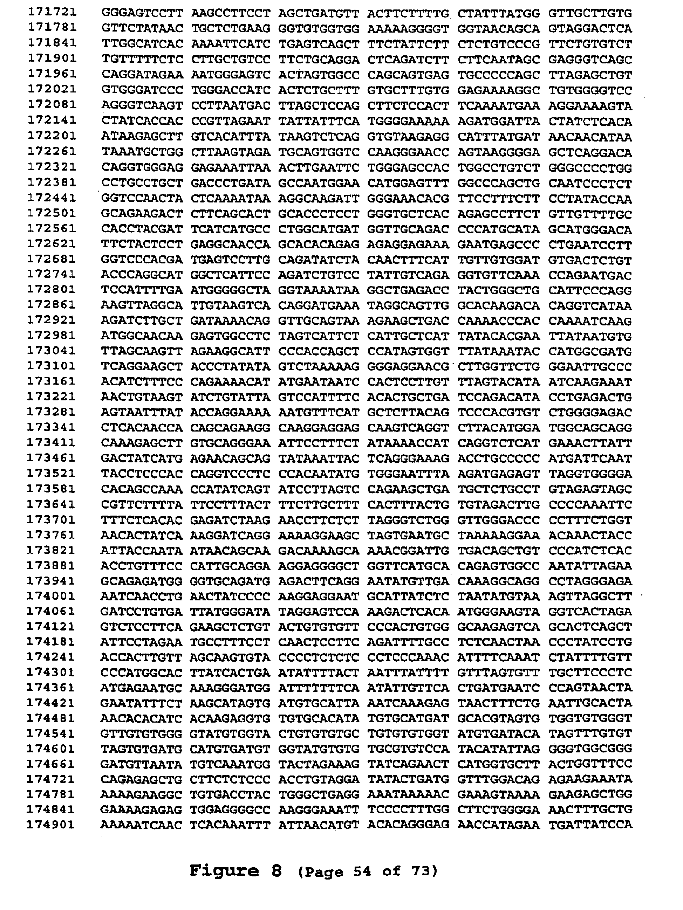

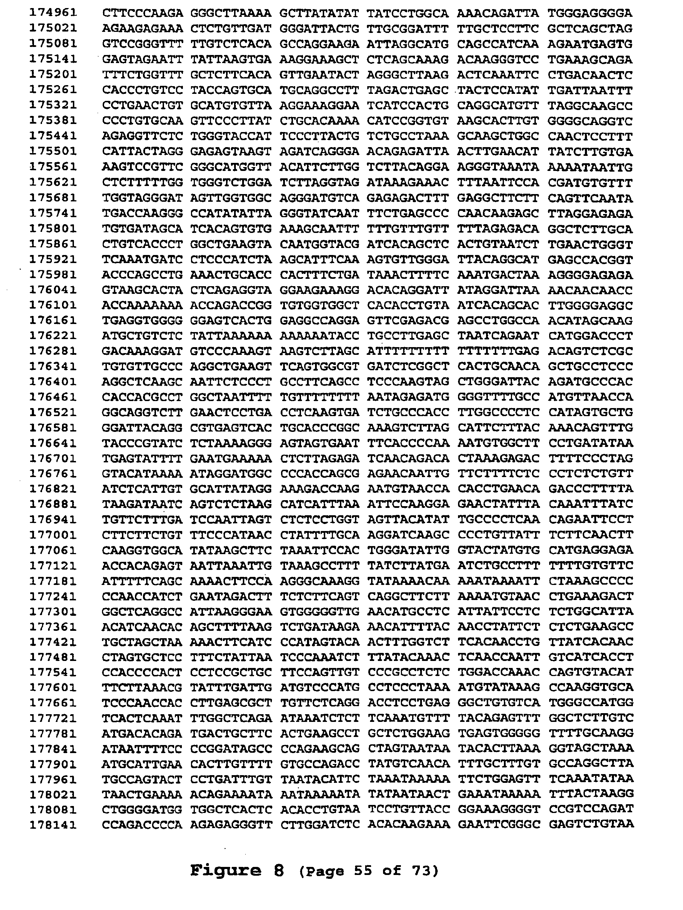

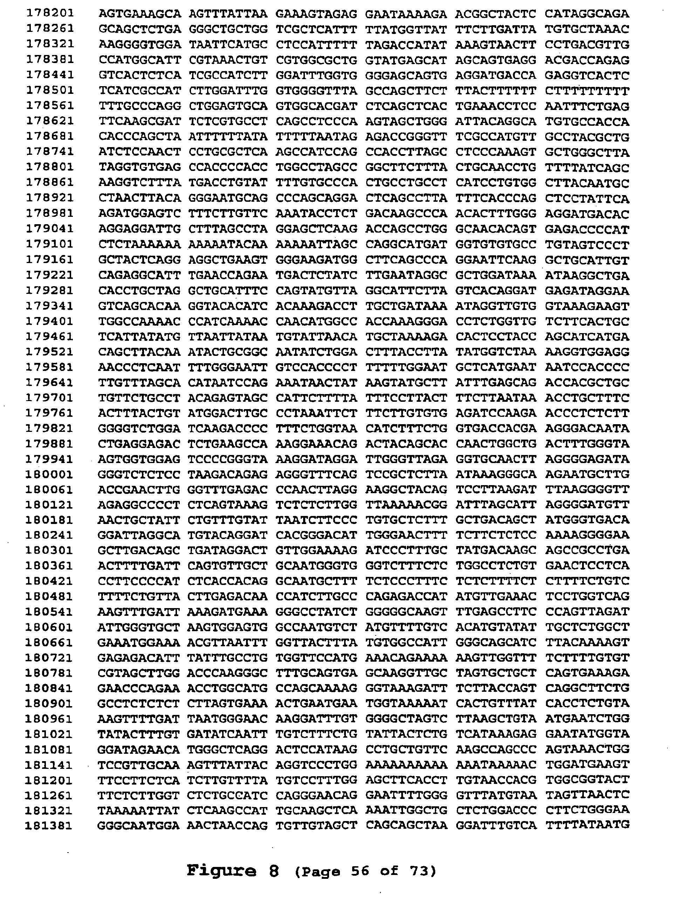

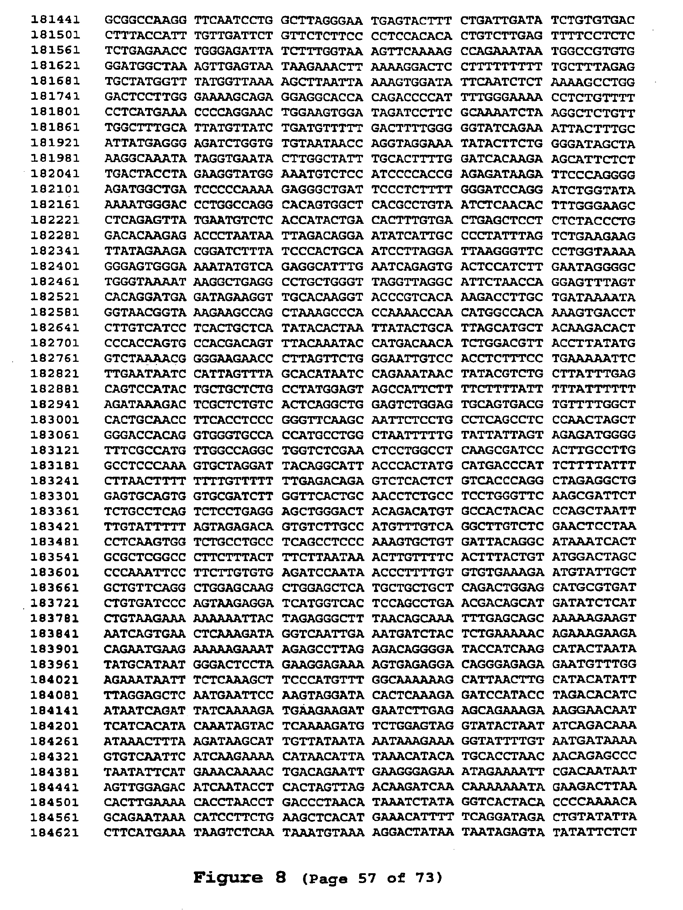

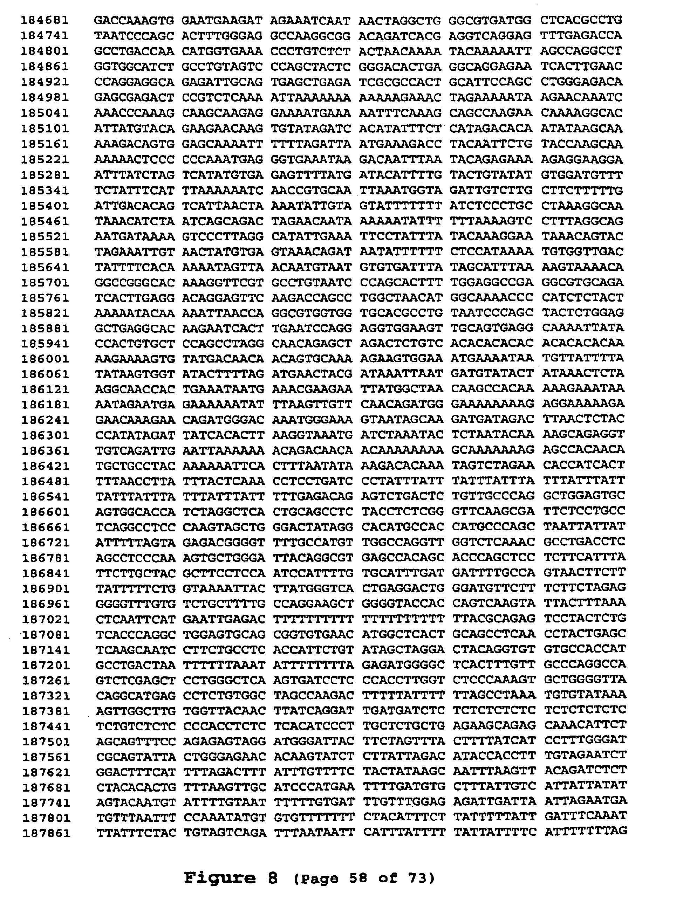

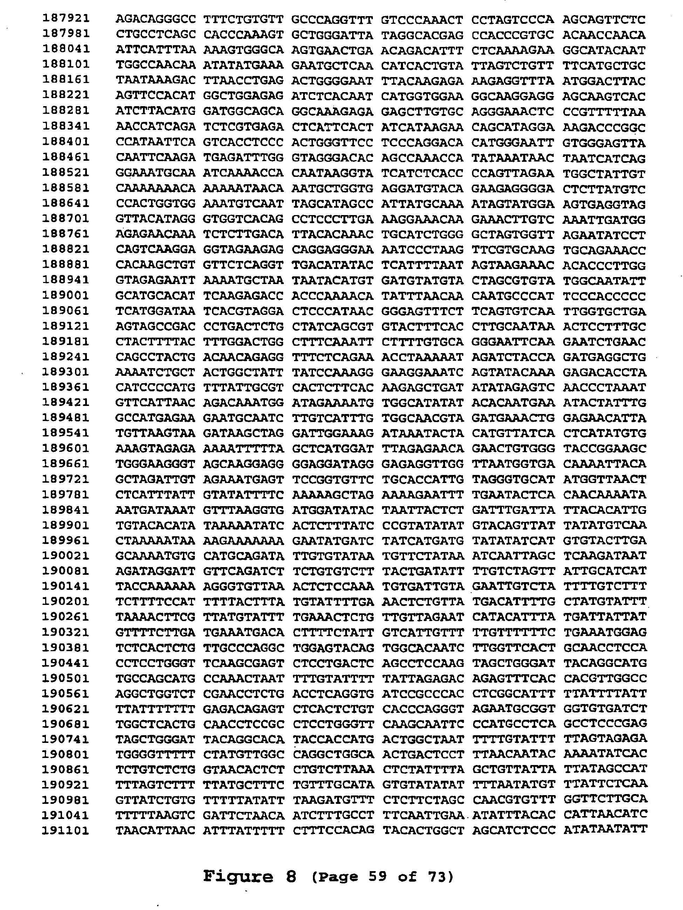

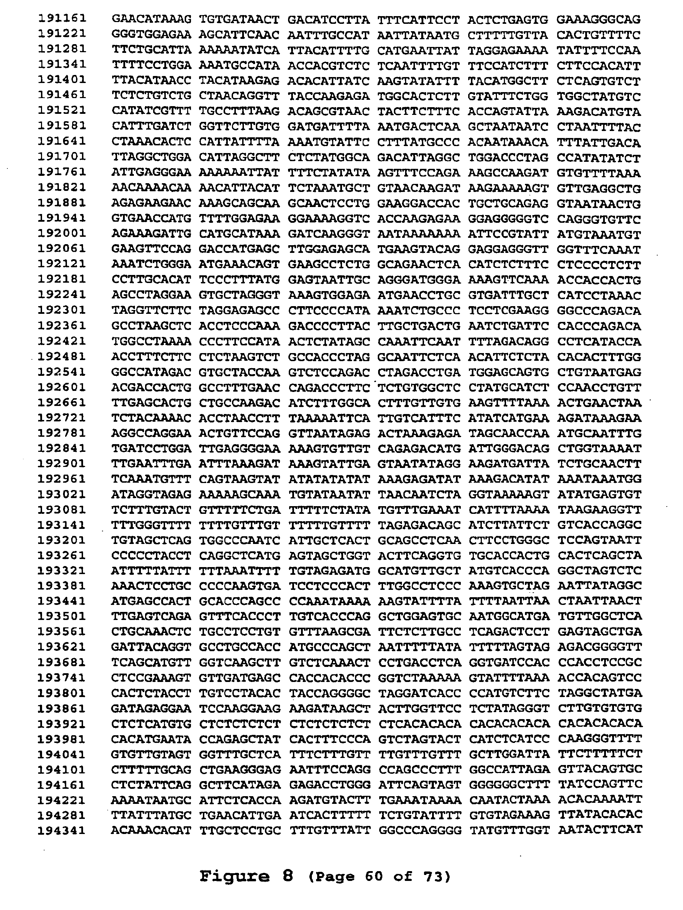

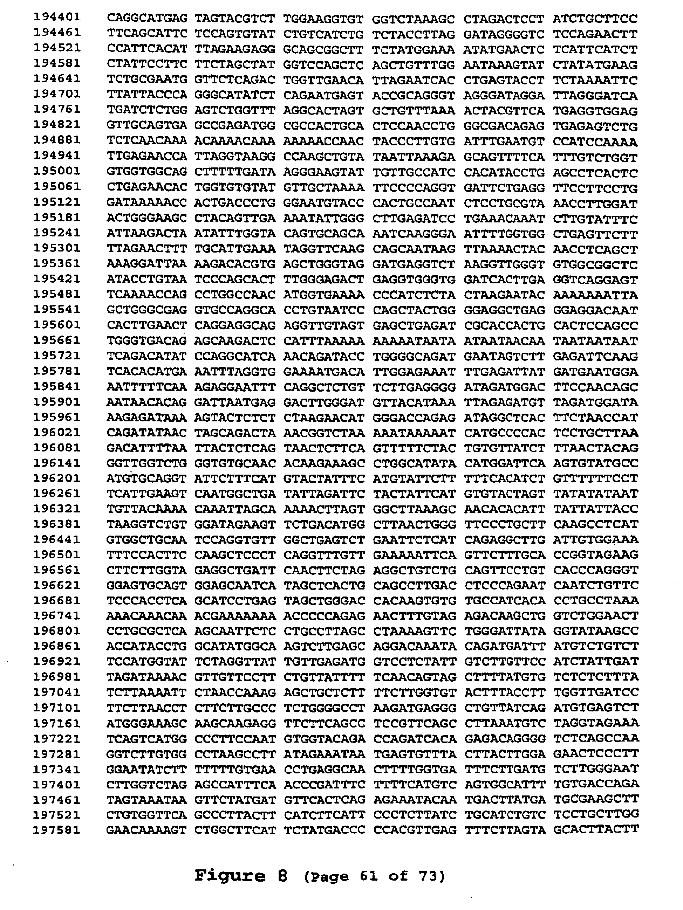

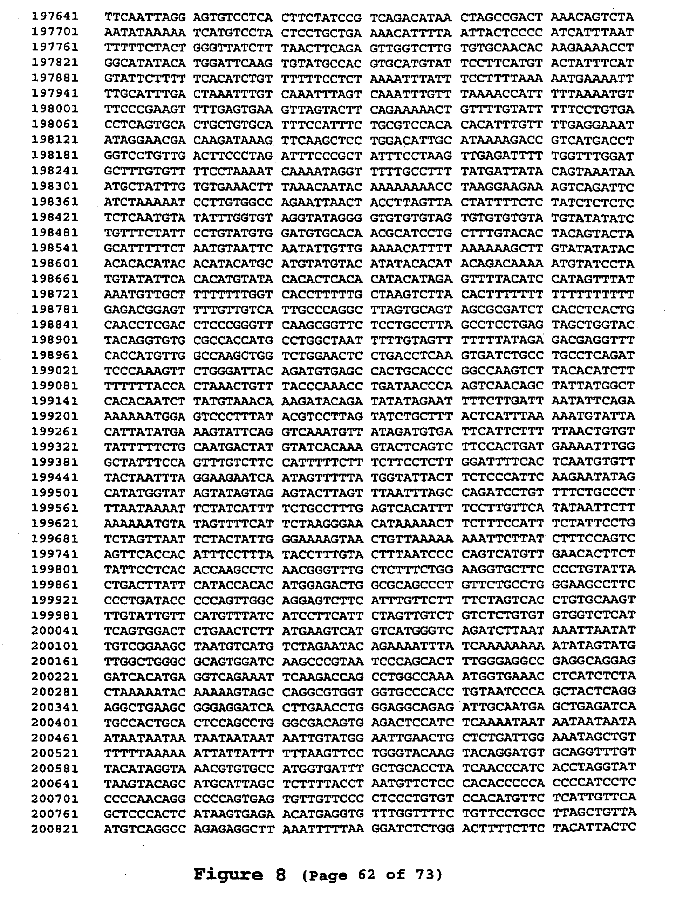

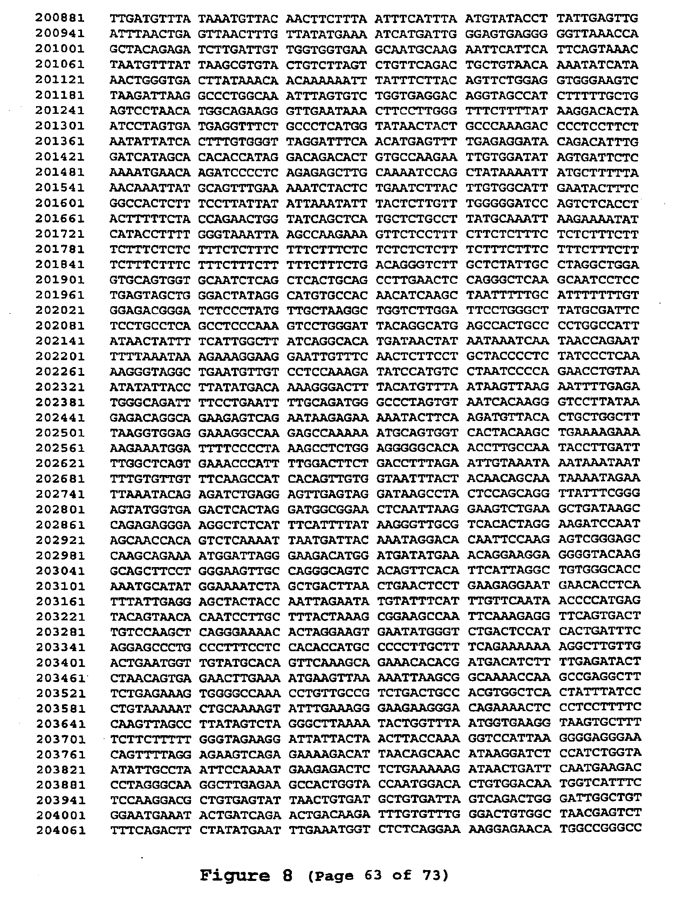

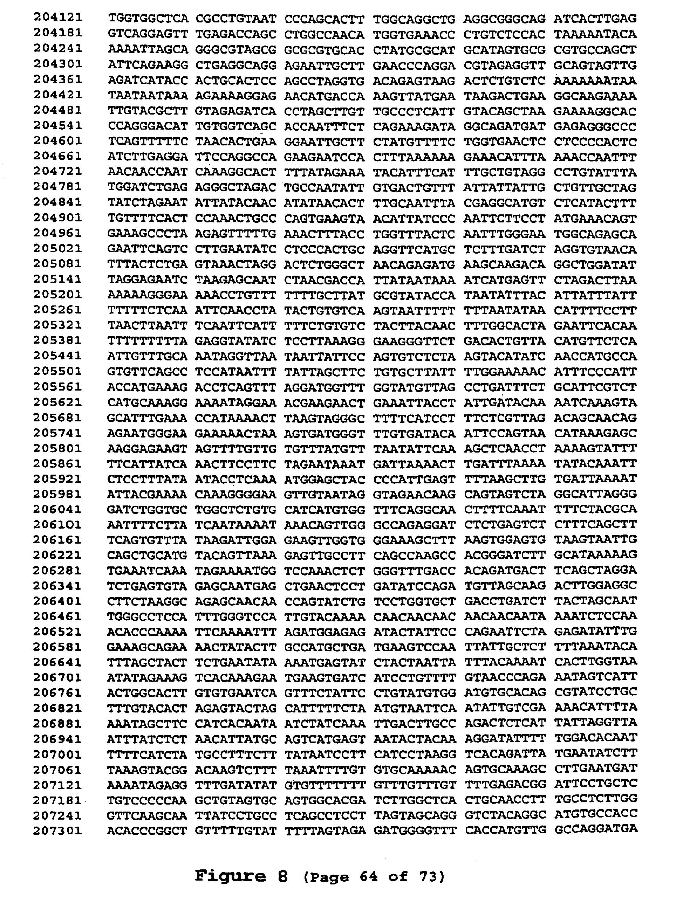

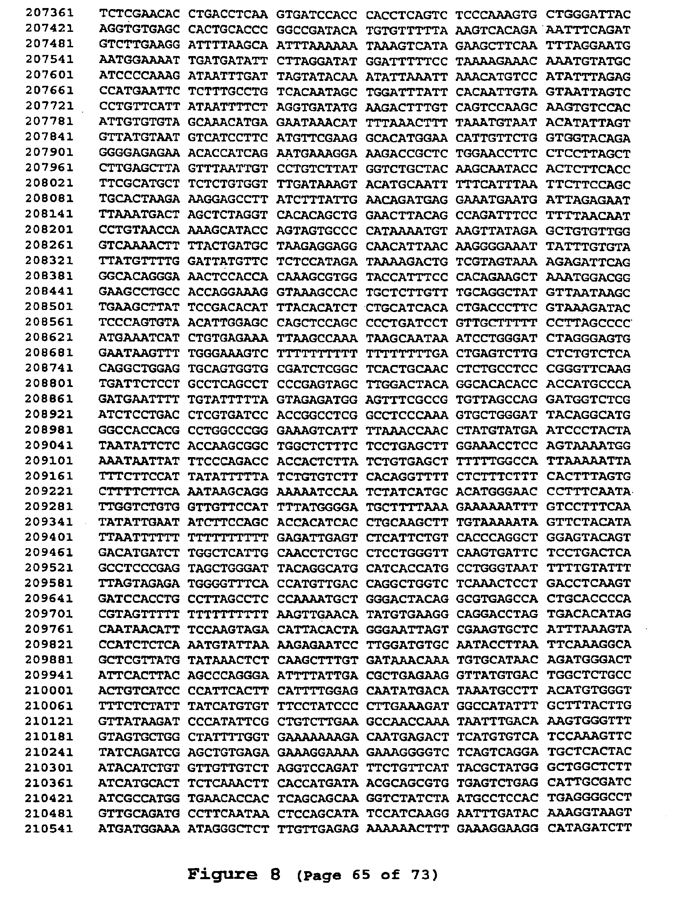

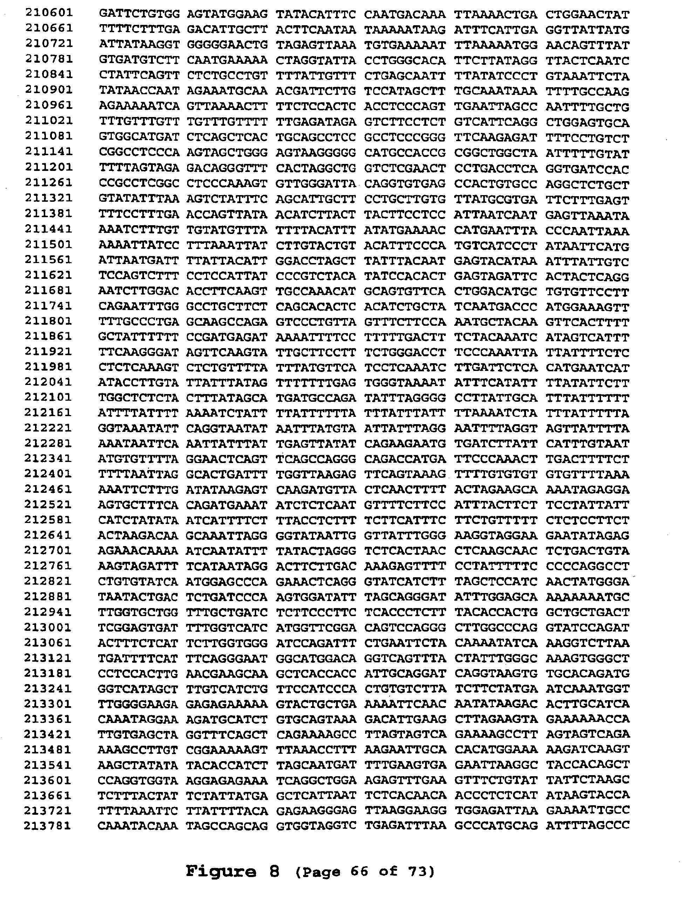

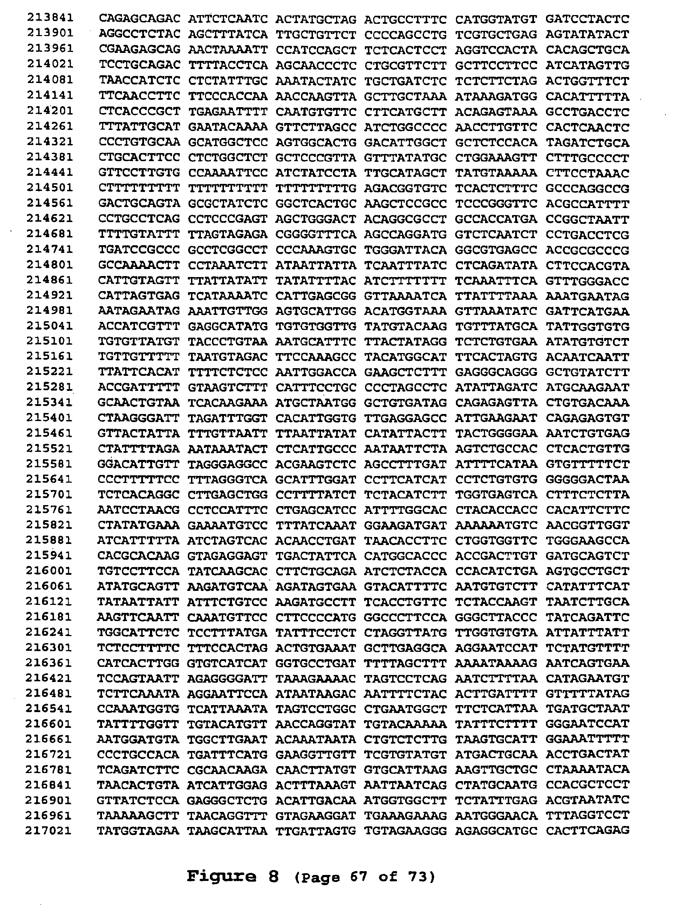

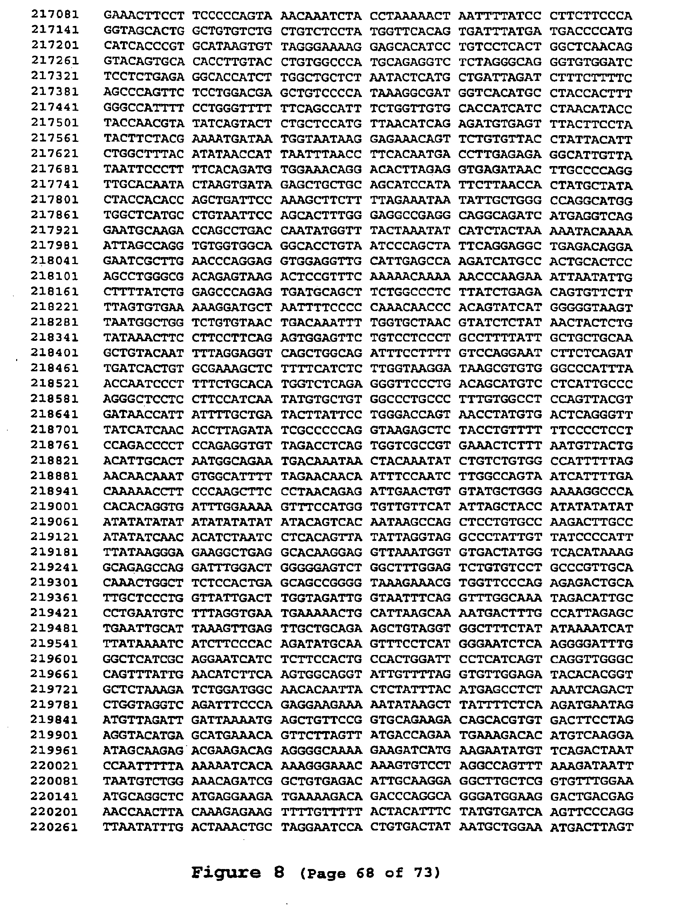

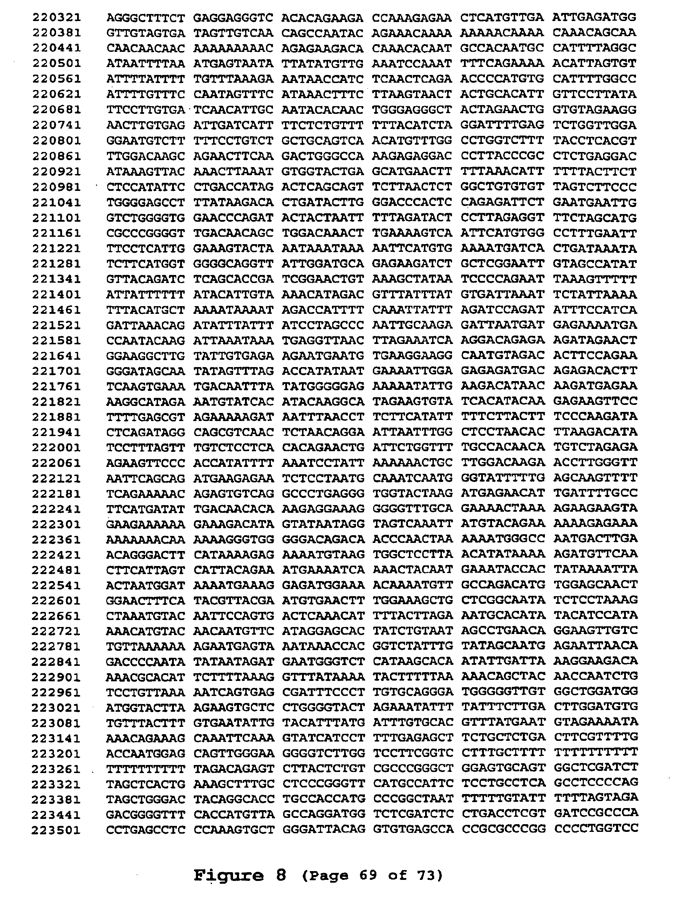

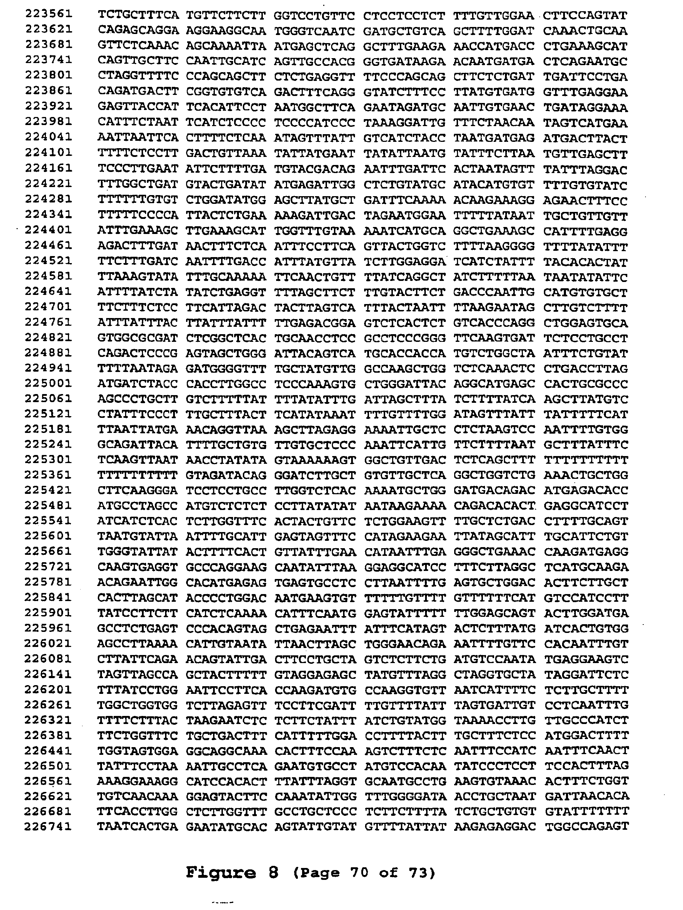

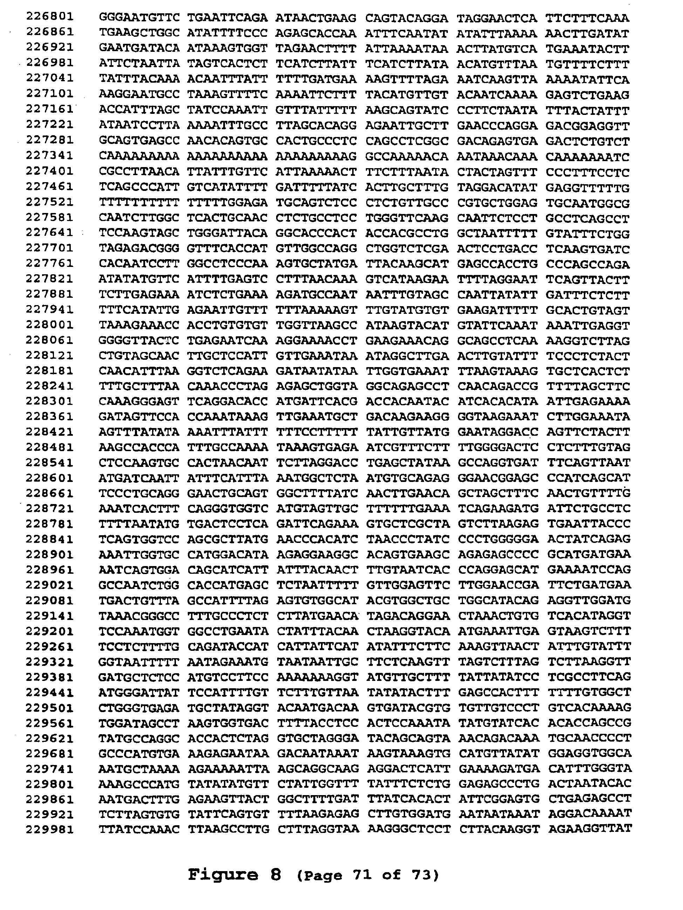

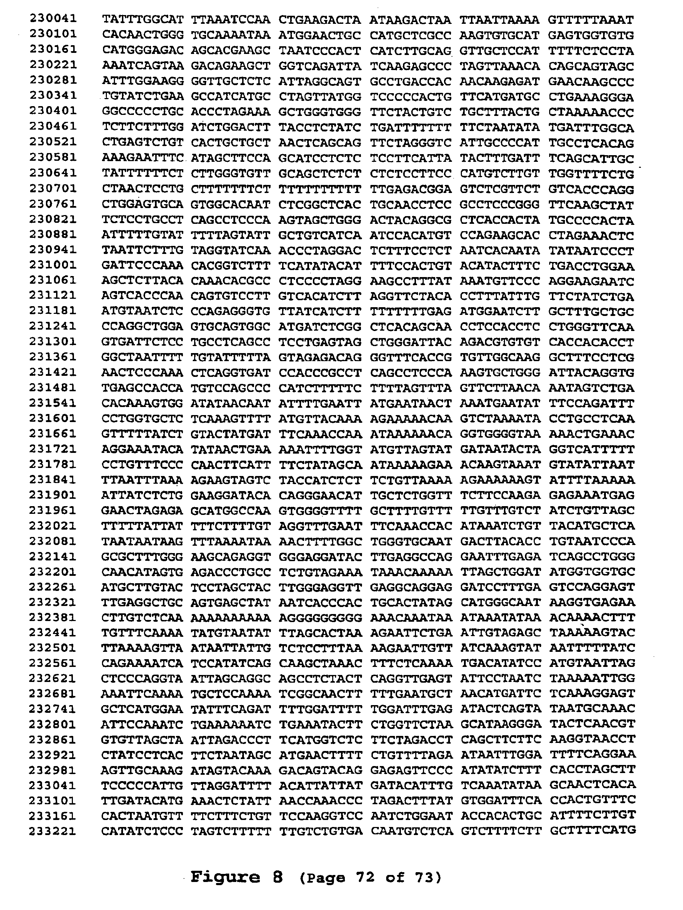

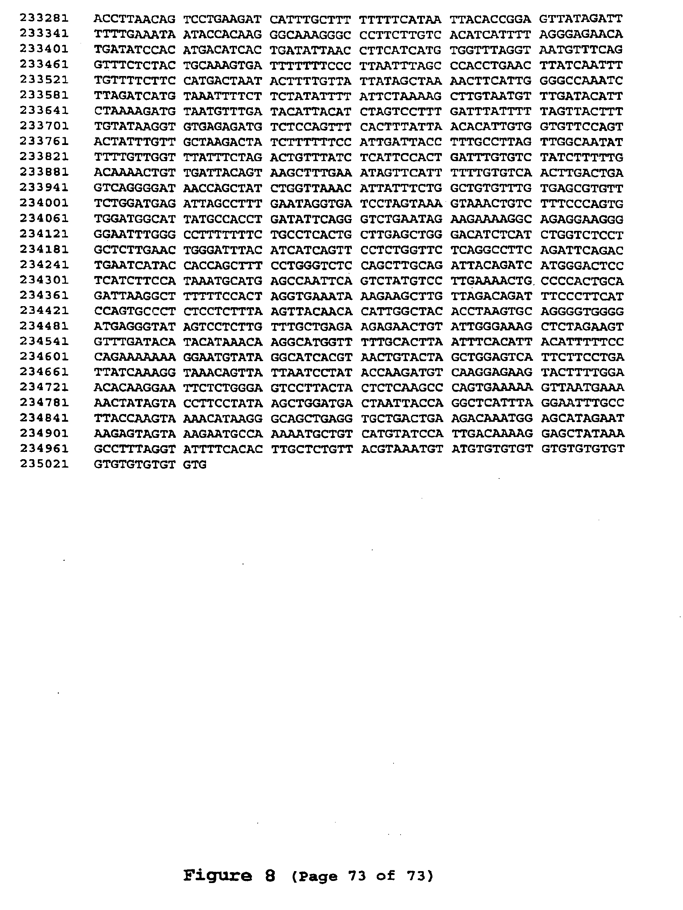

Figure 8 depicts the nucleotide sequence of approximately 235 kb in the HFE subregion from an unaffected individual.

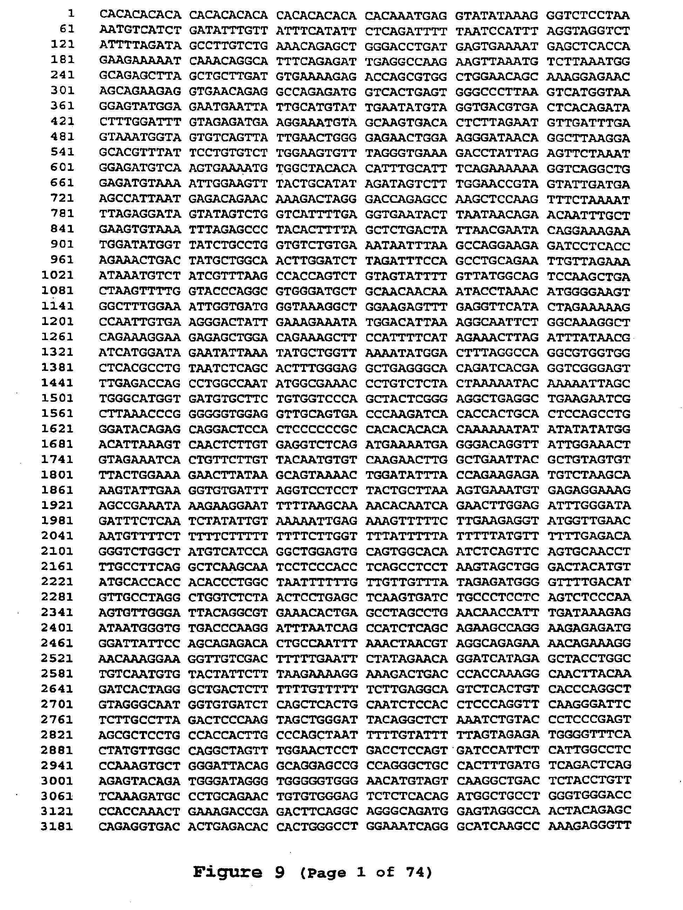

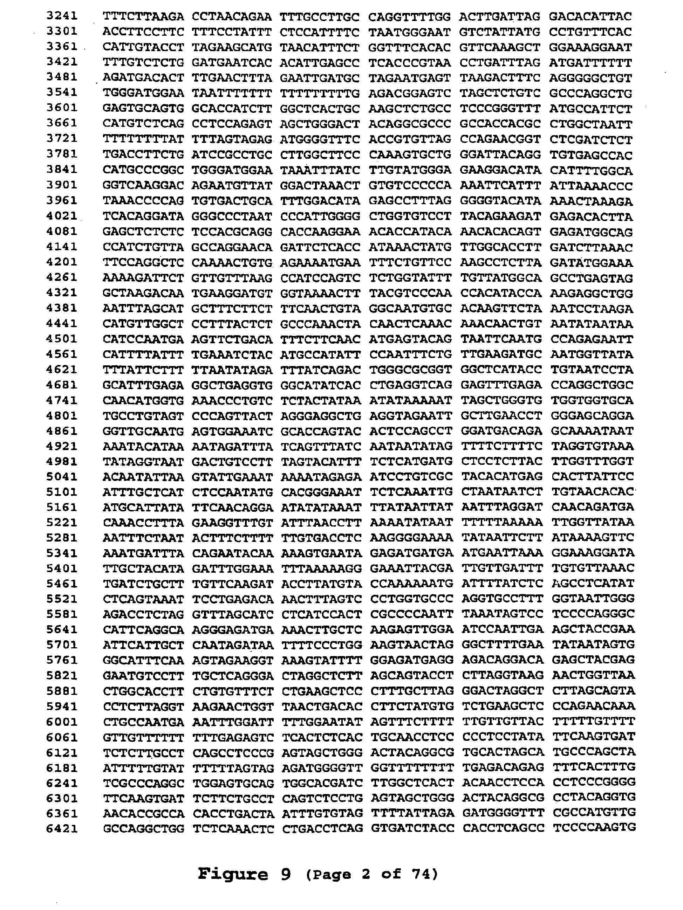

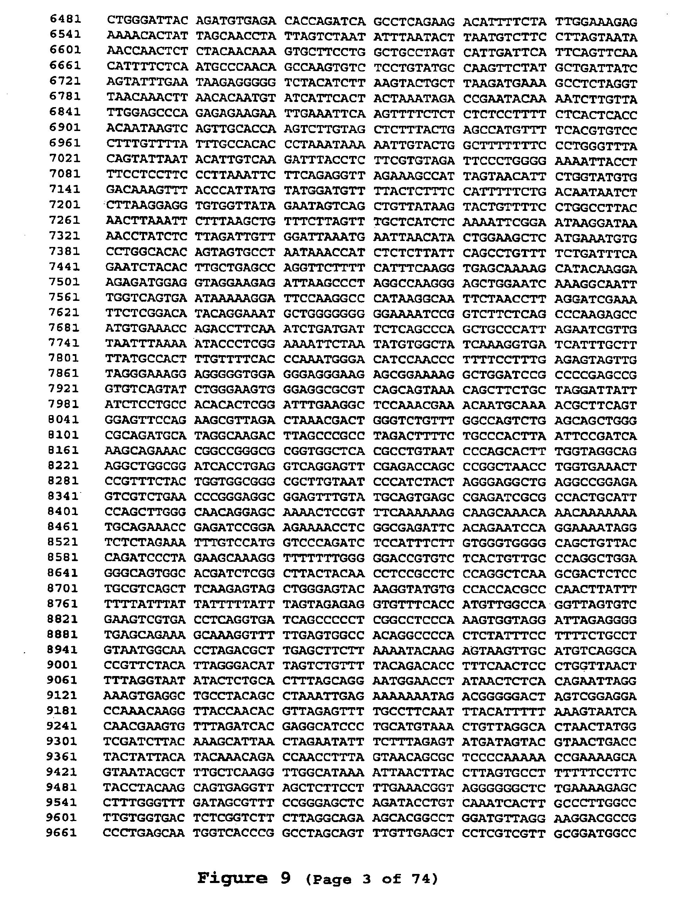

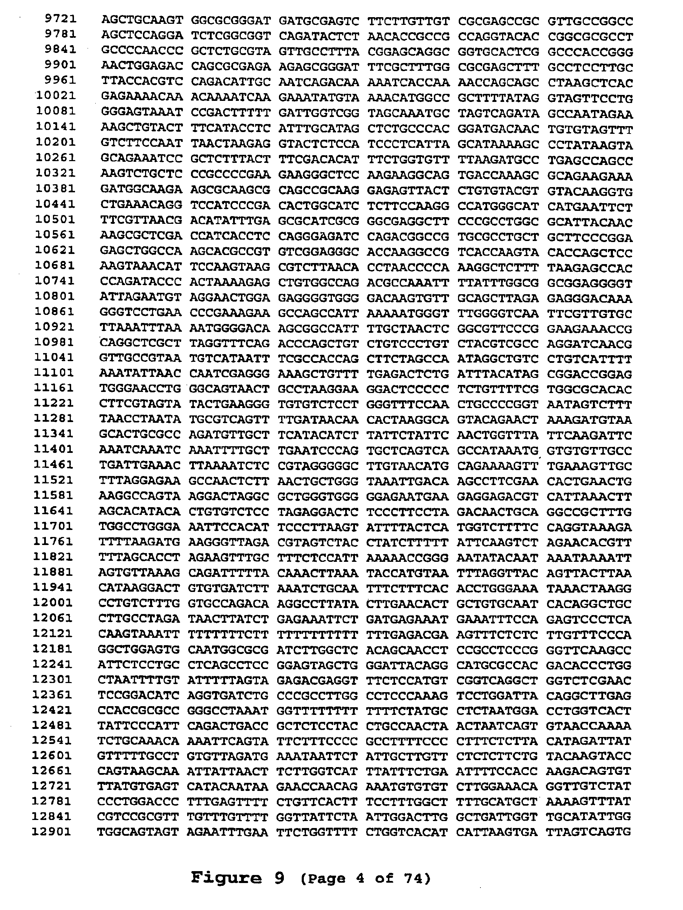

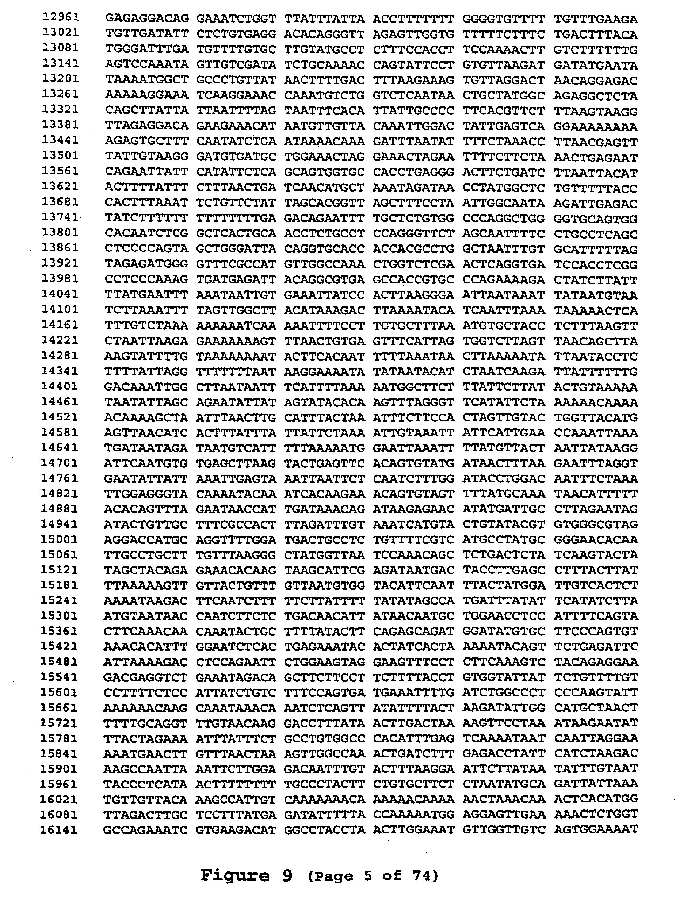

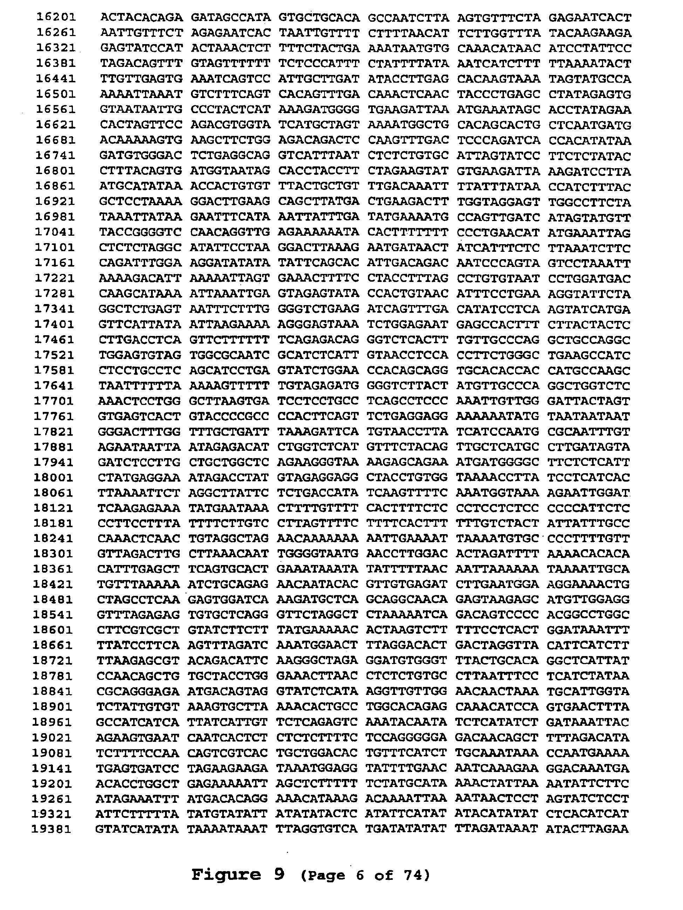

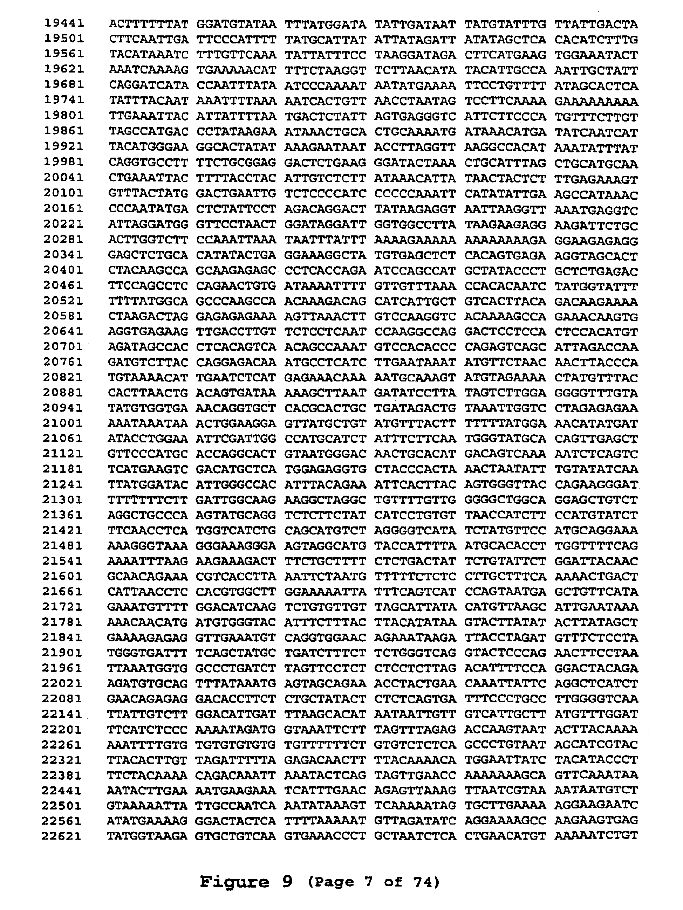

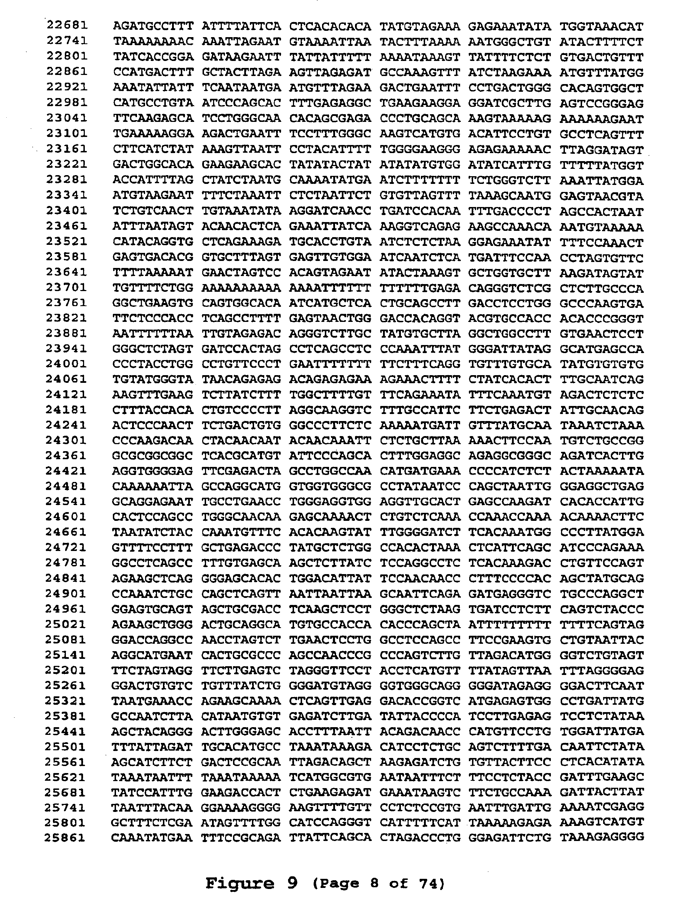

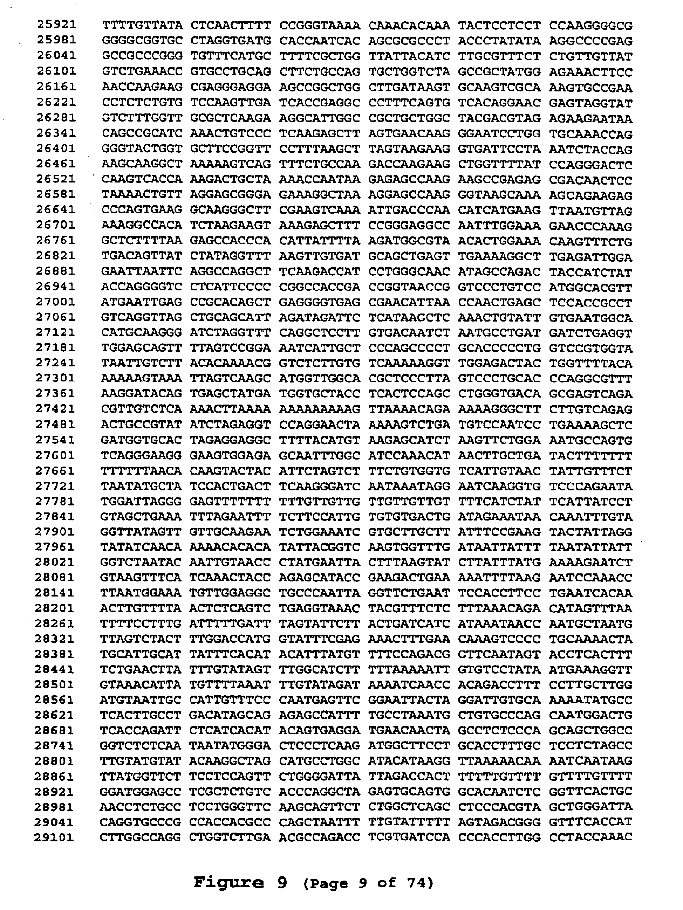

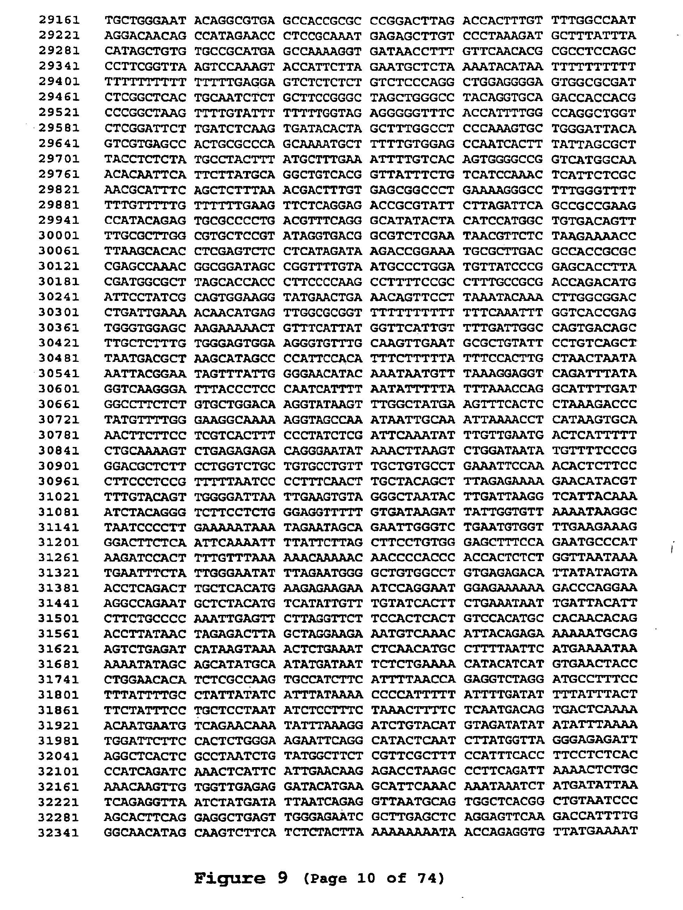

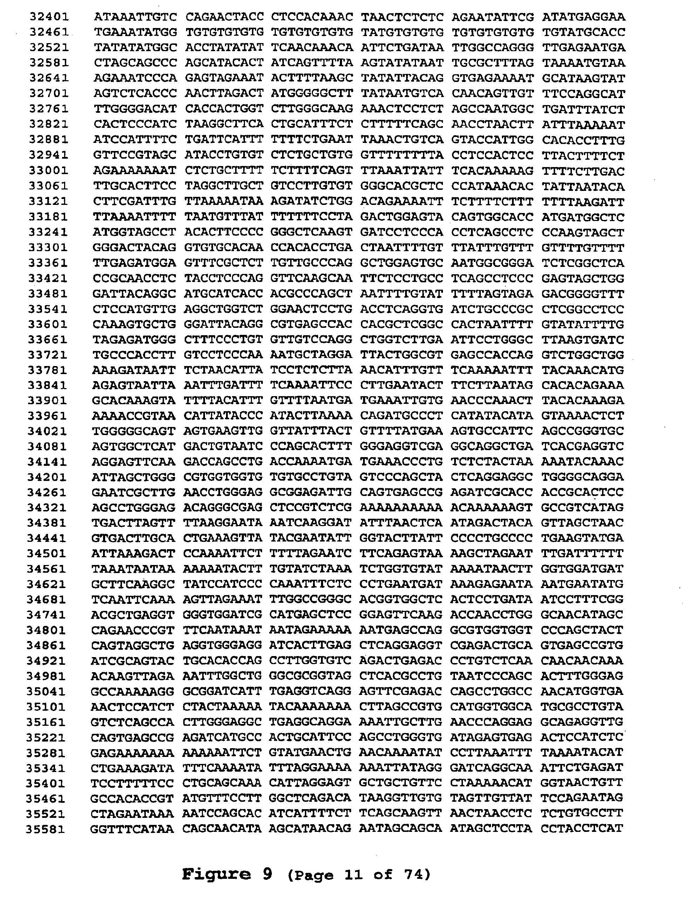

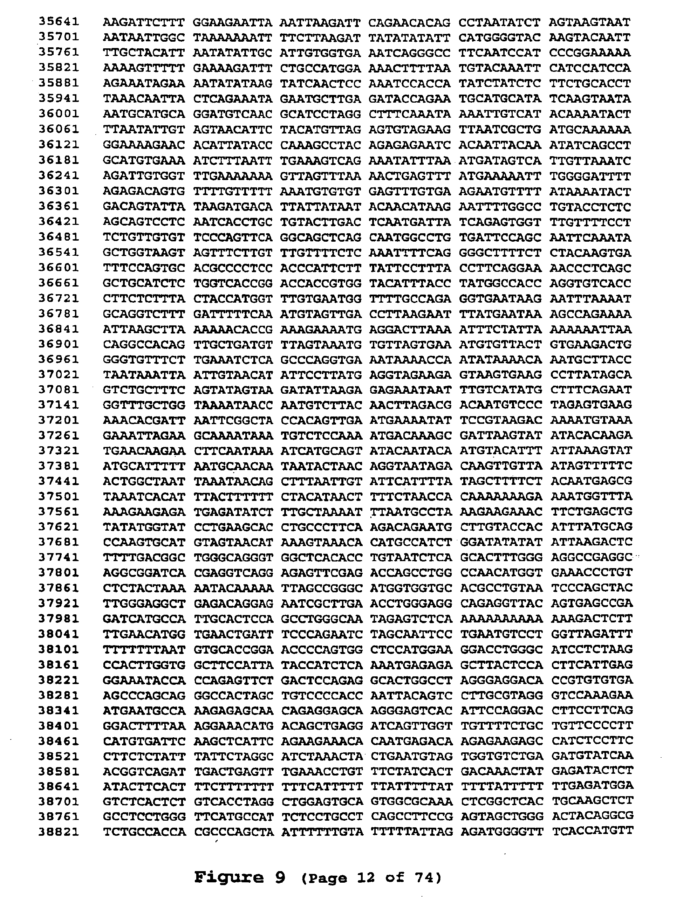

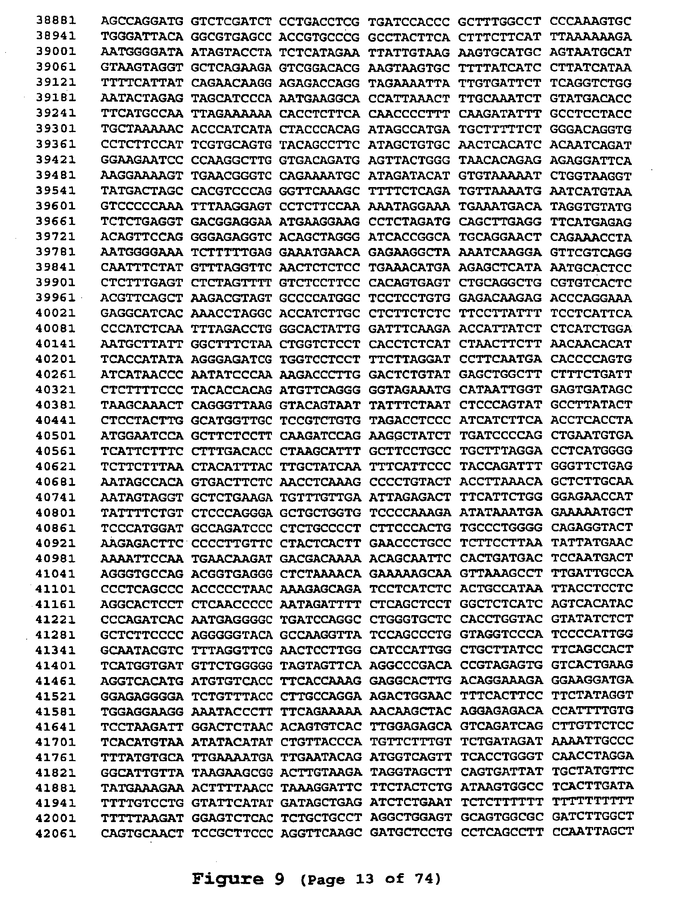

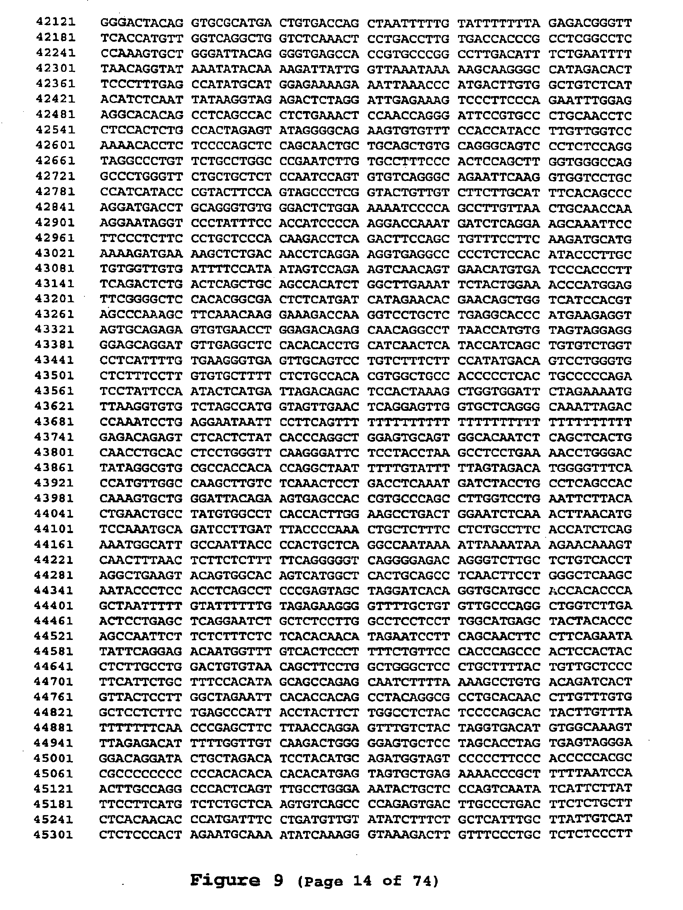

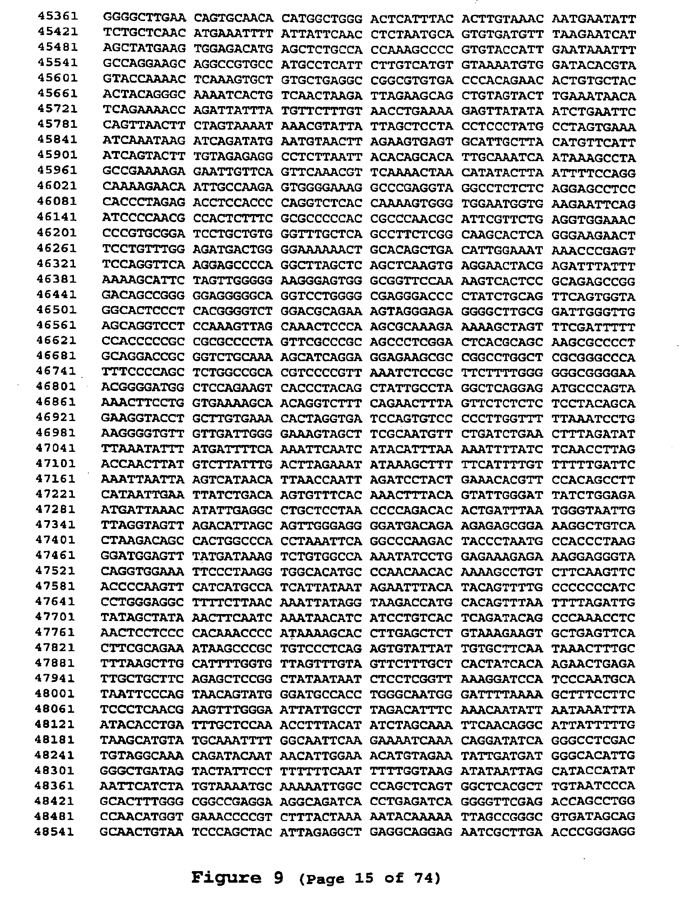

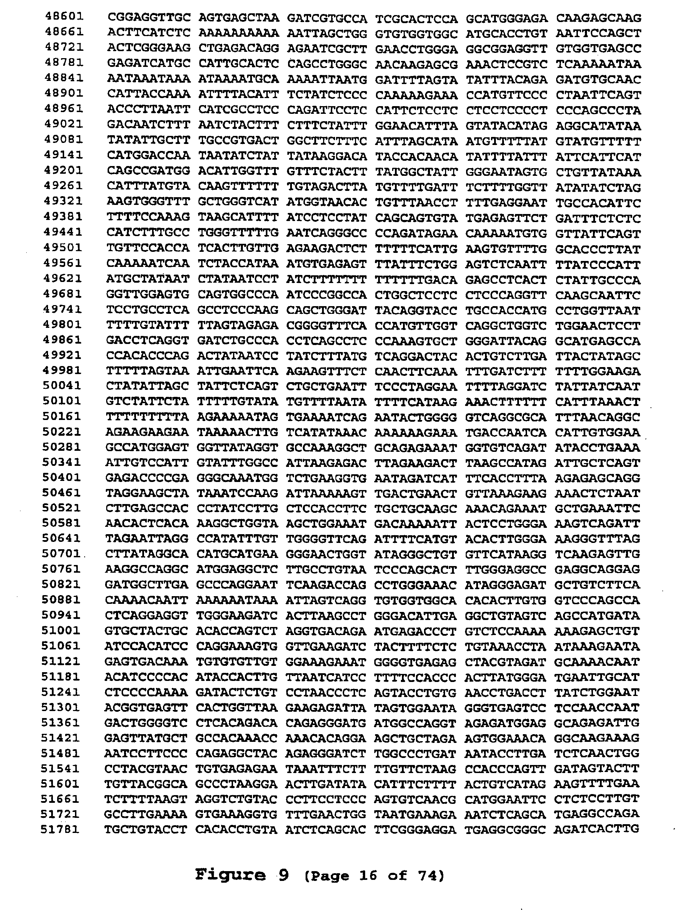

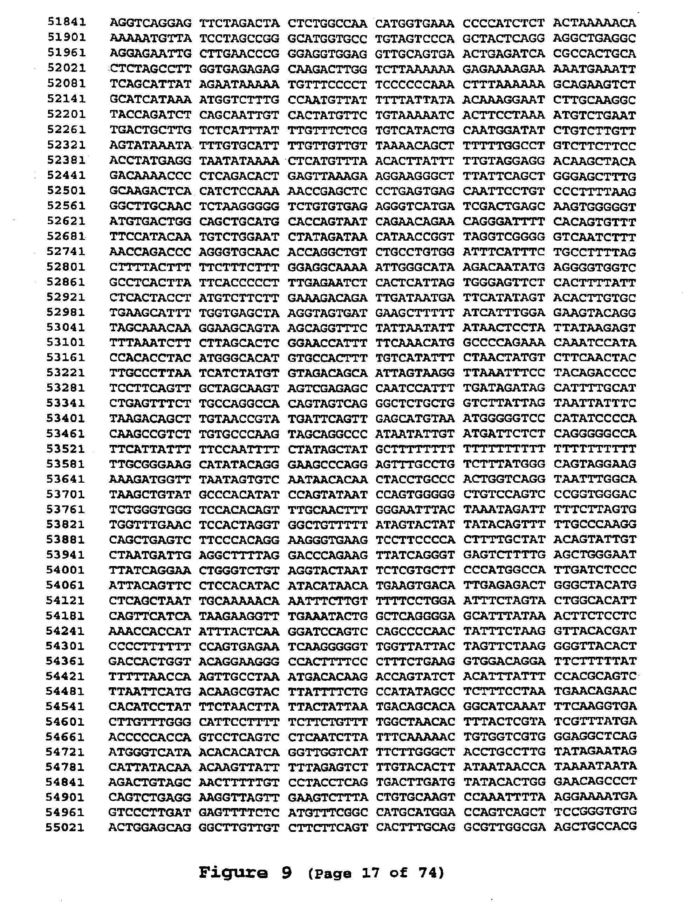

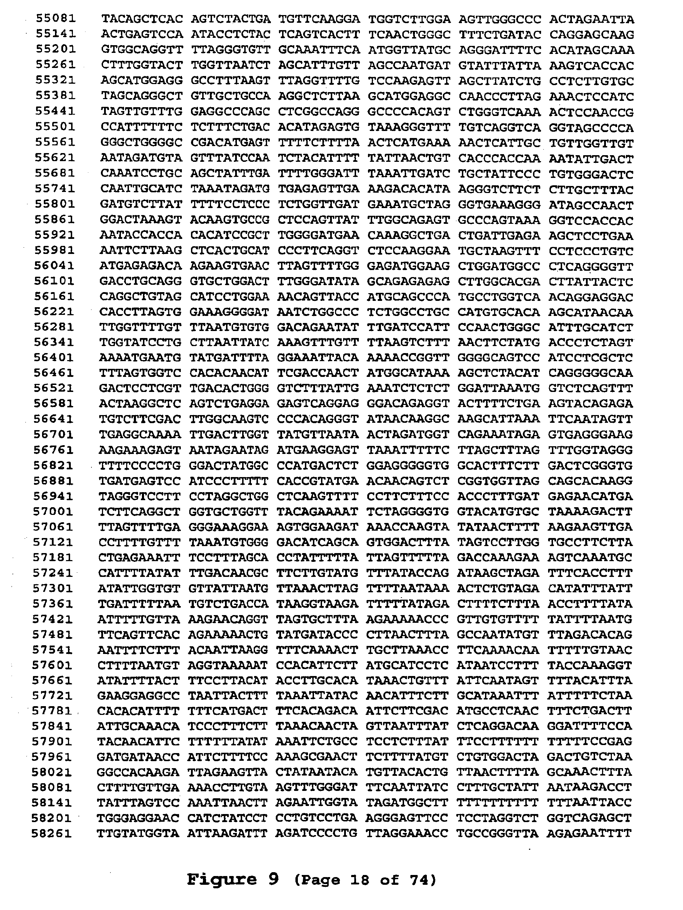

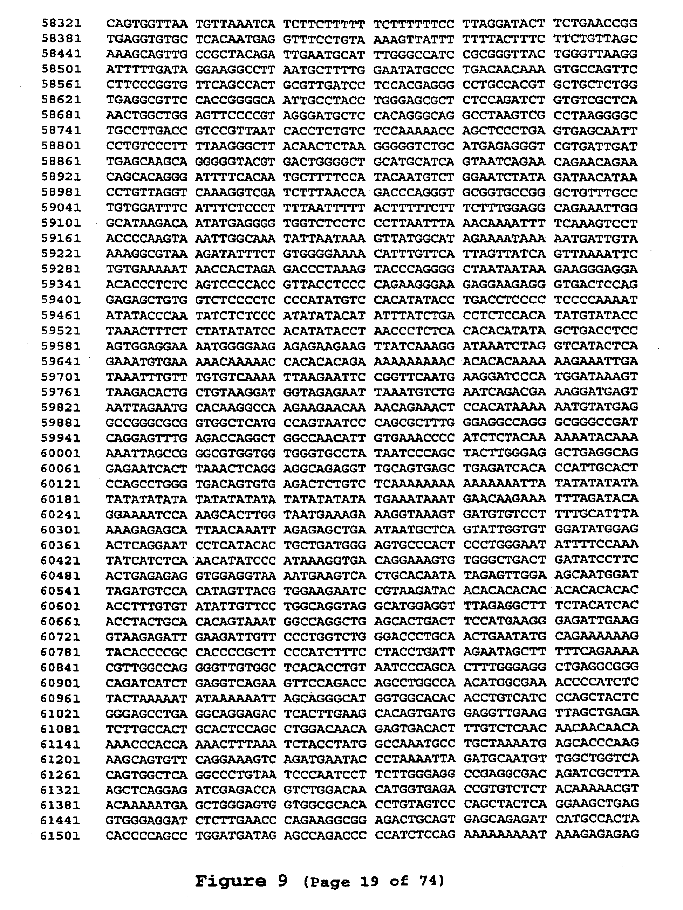

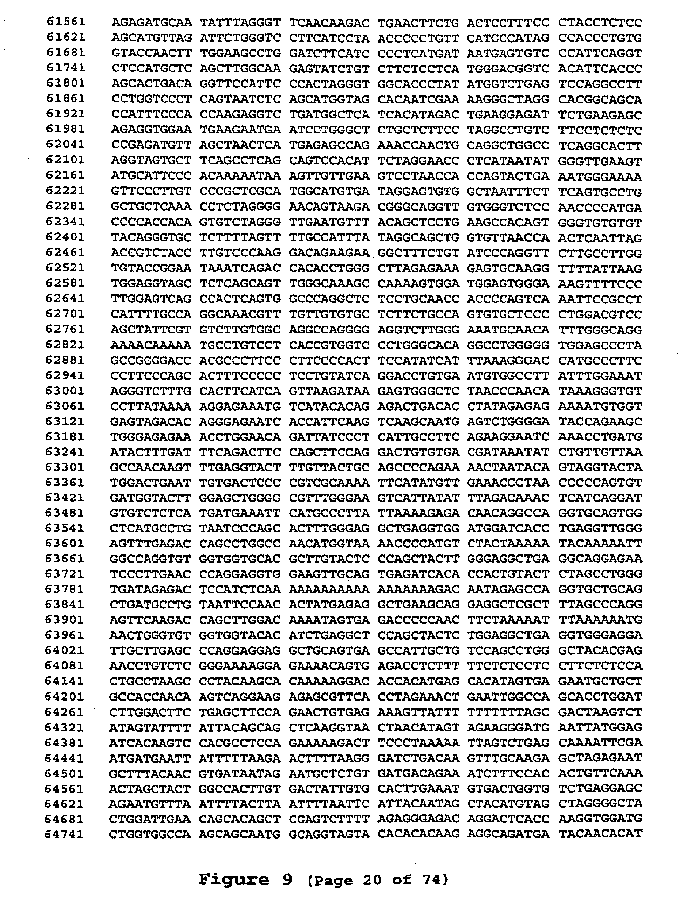

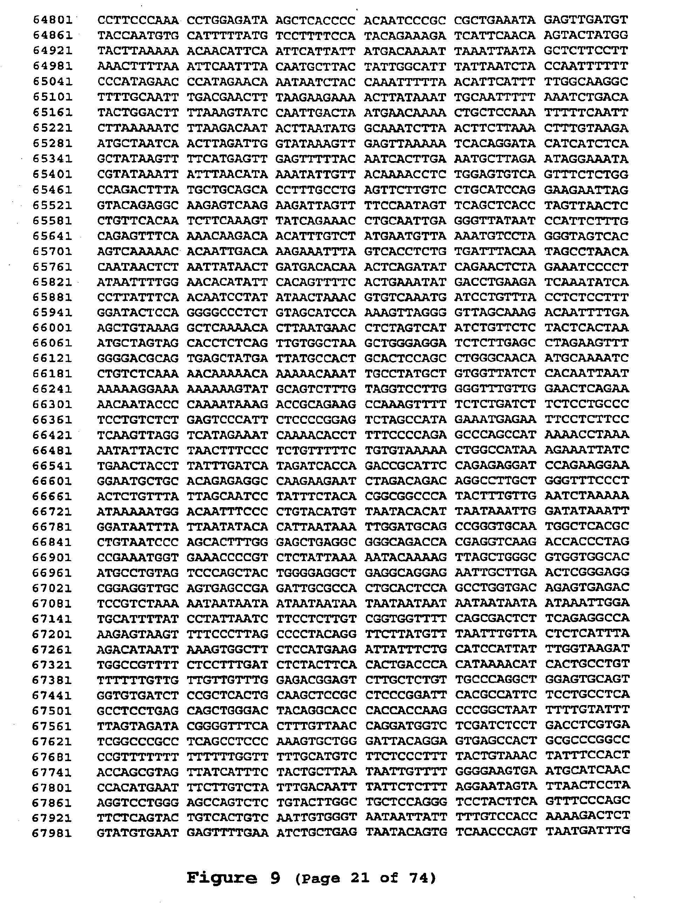

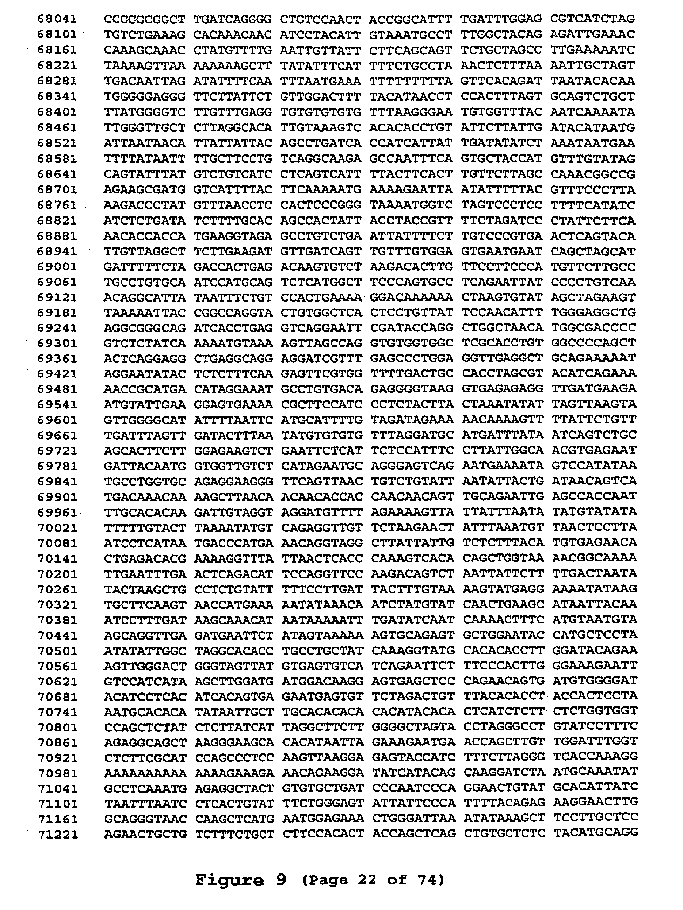

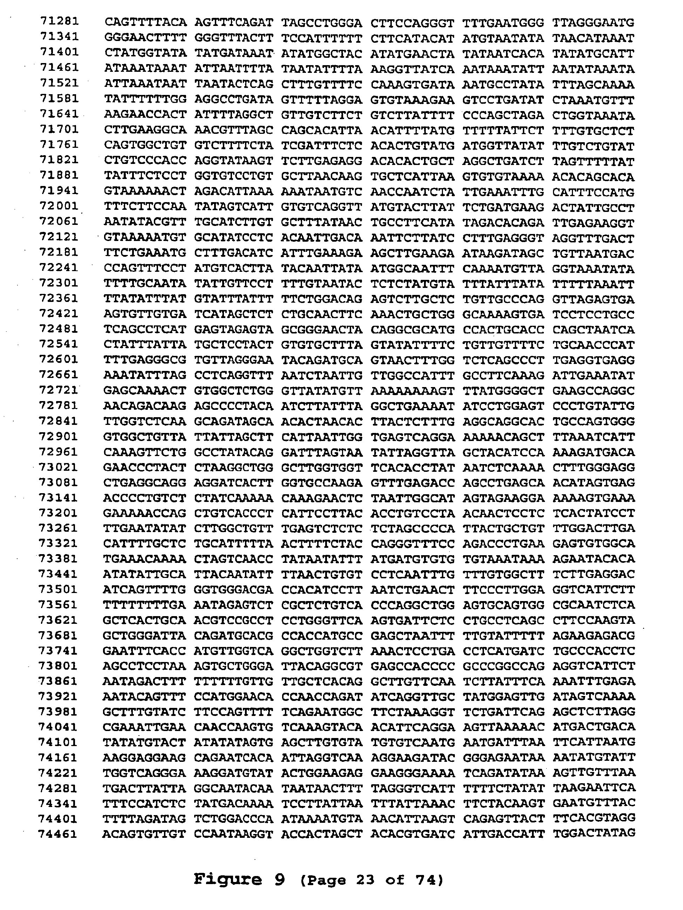

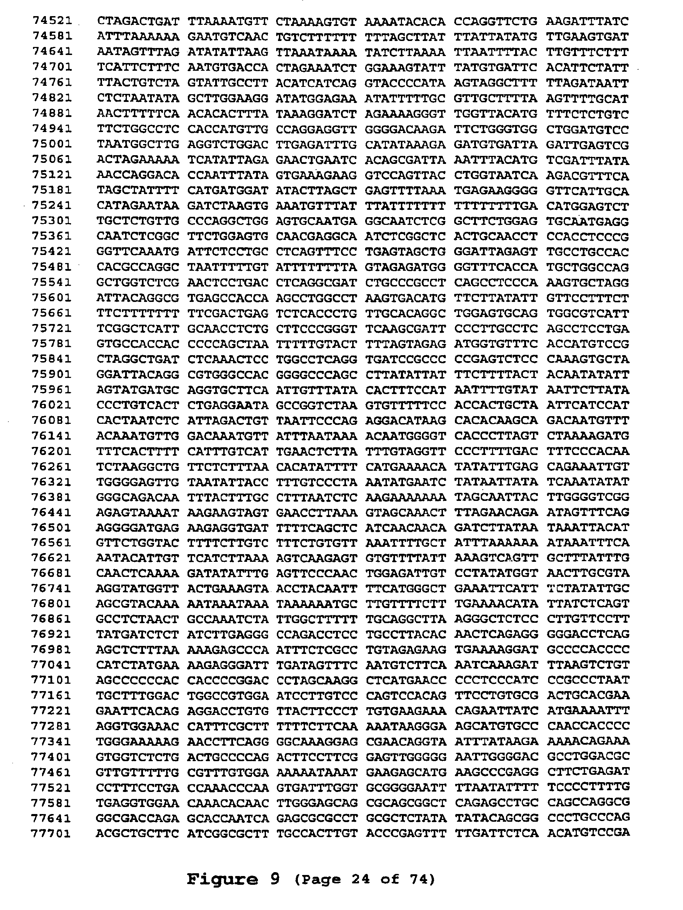

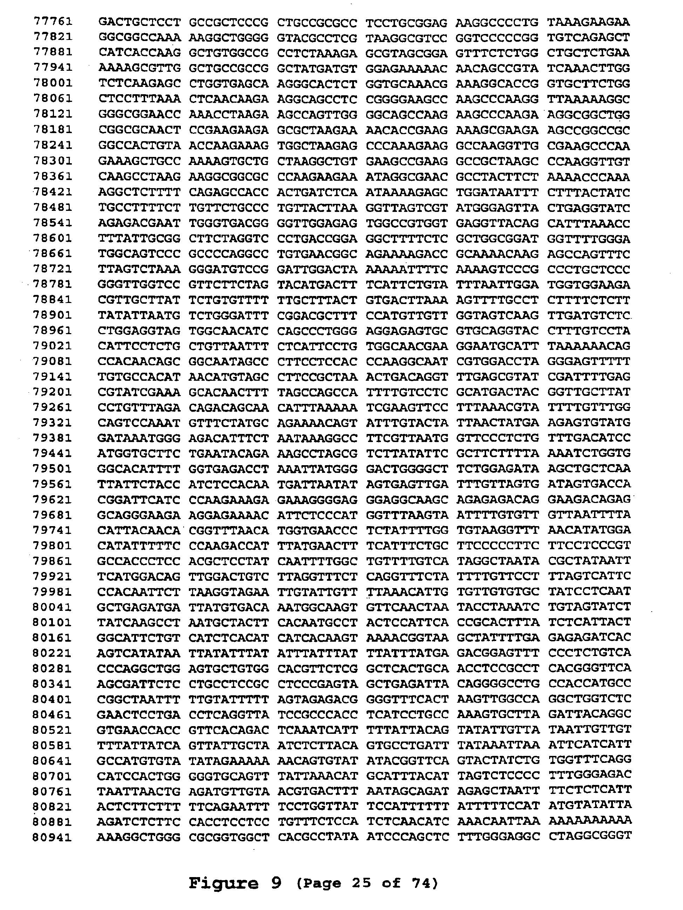

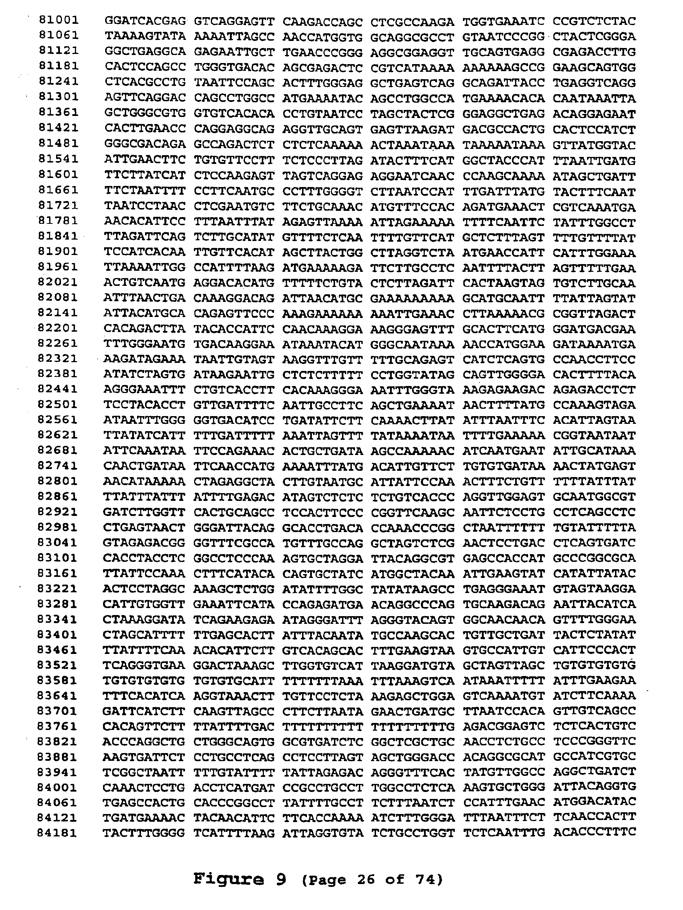

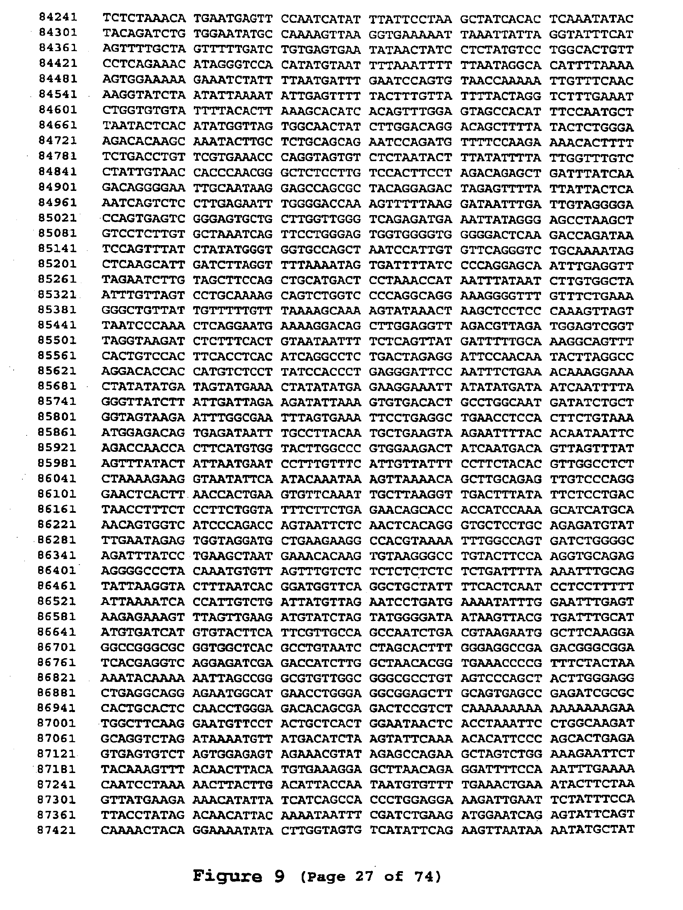

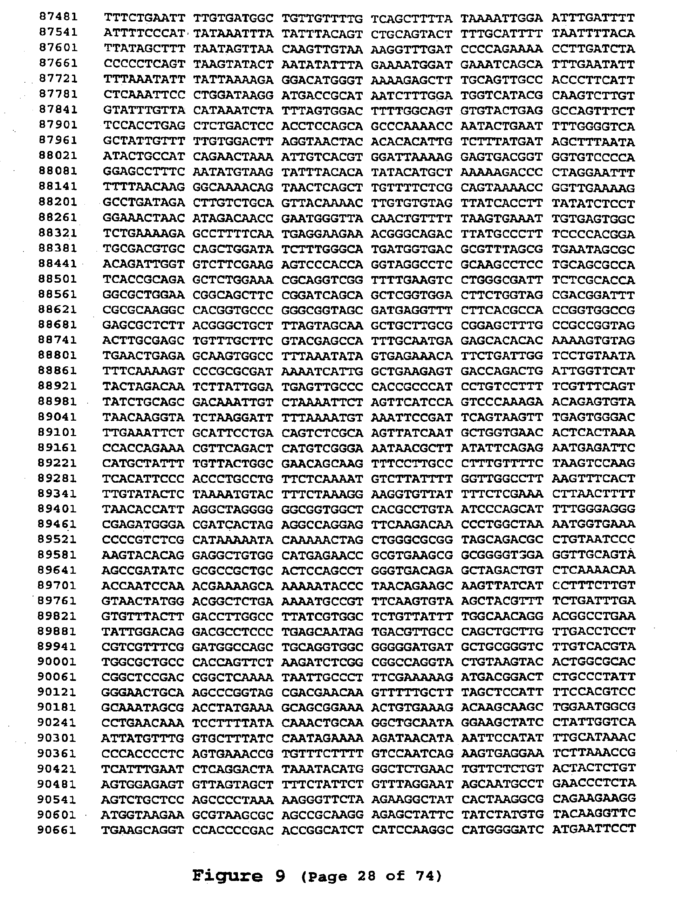

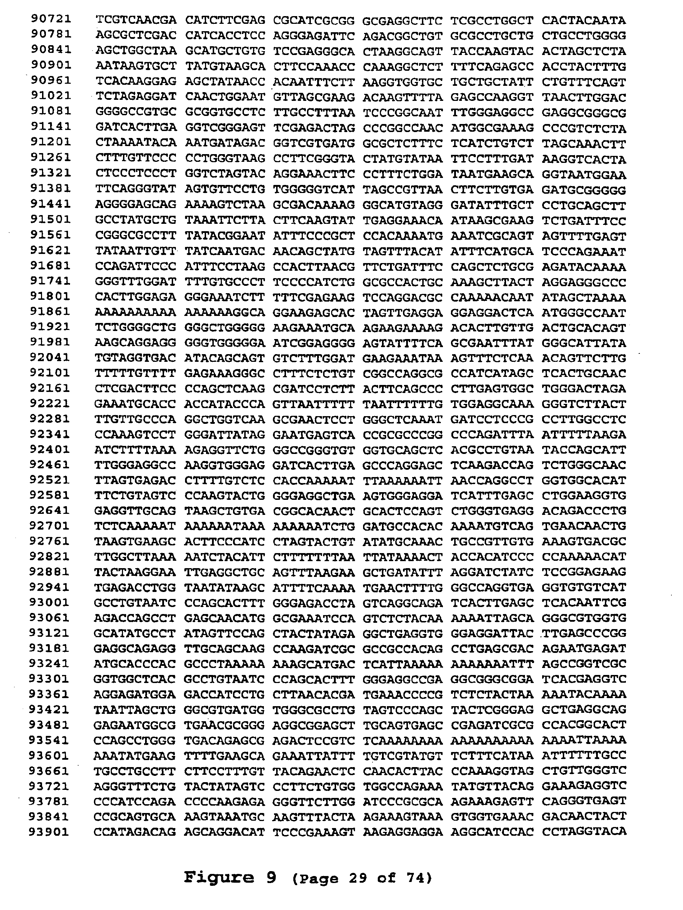

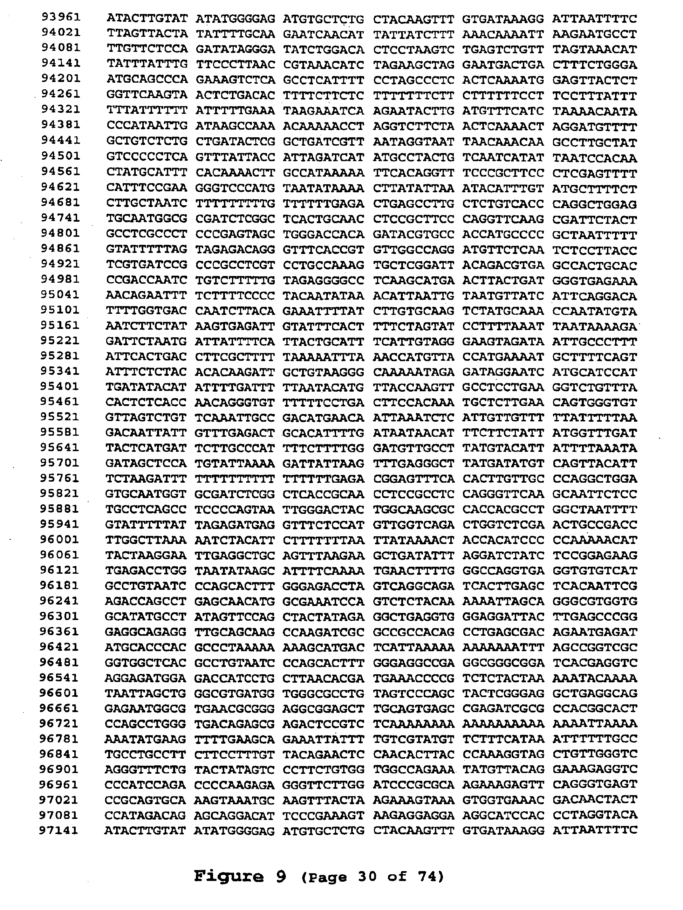

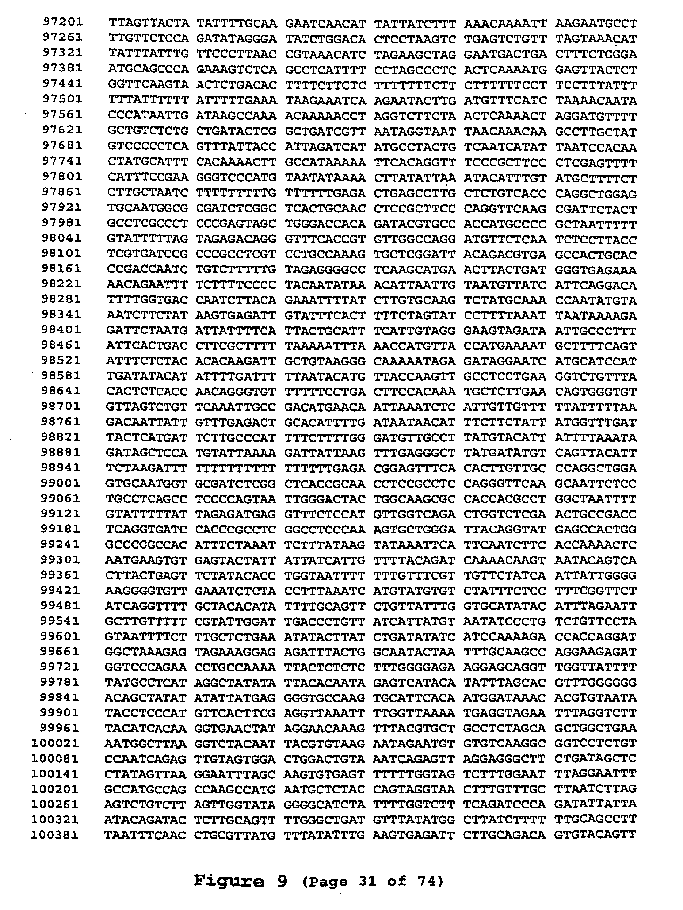

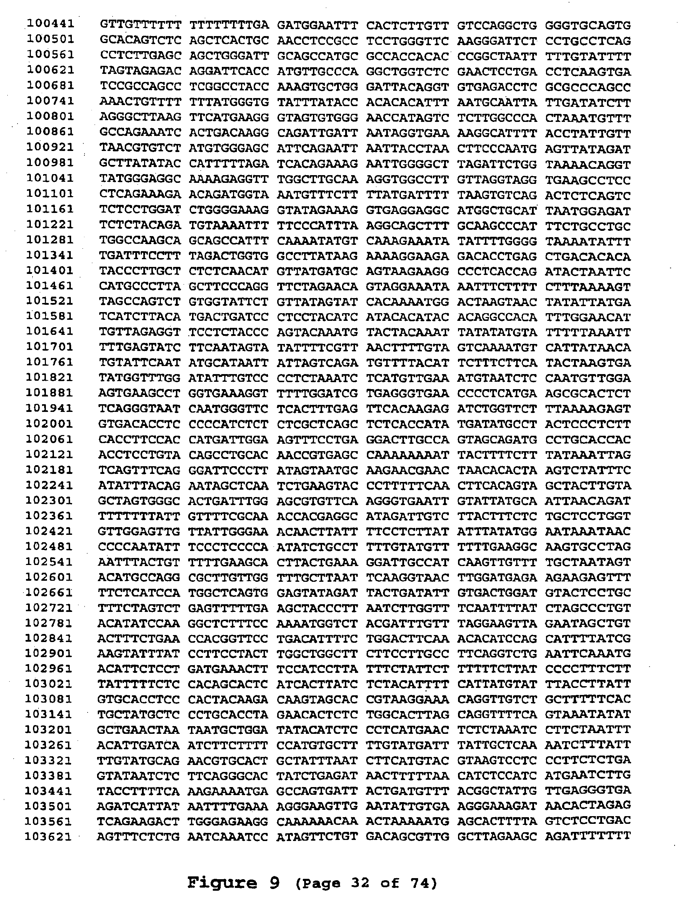

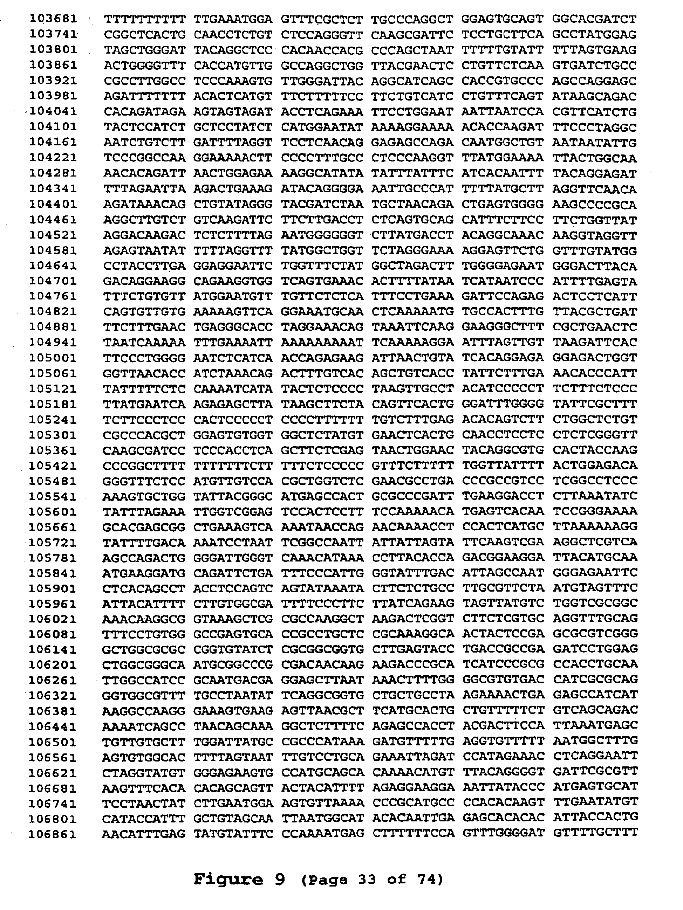

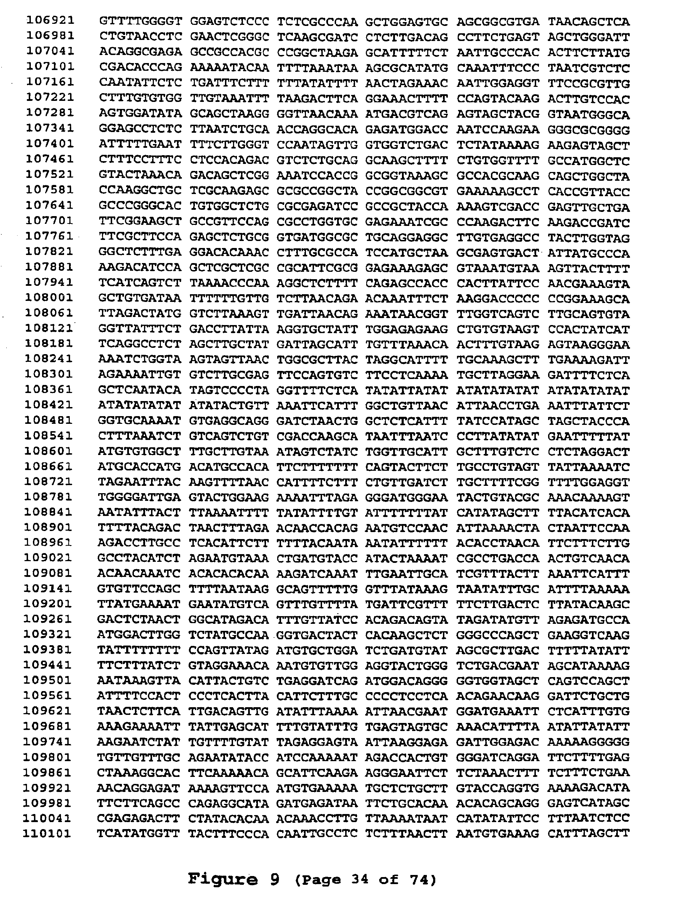

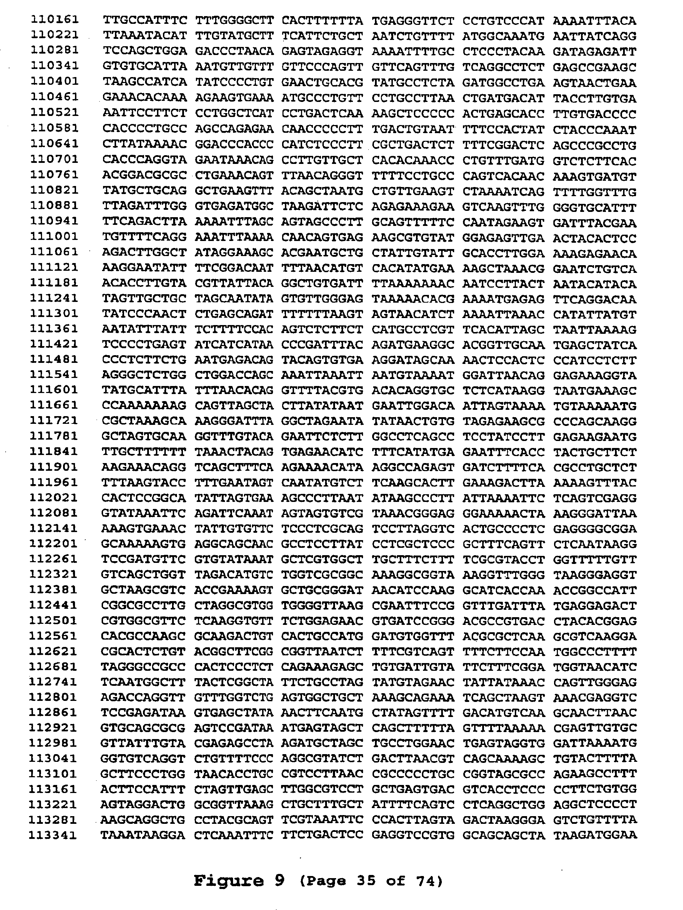

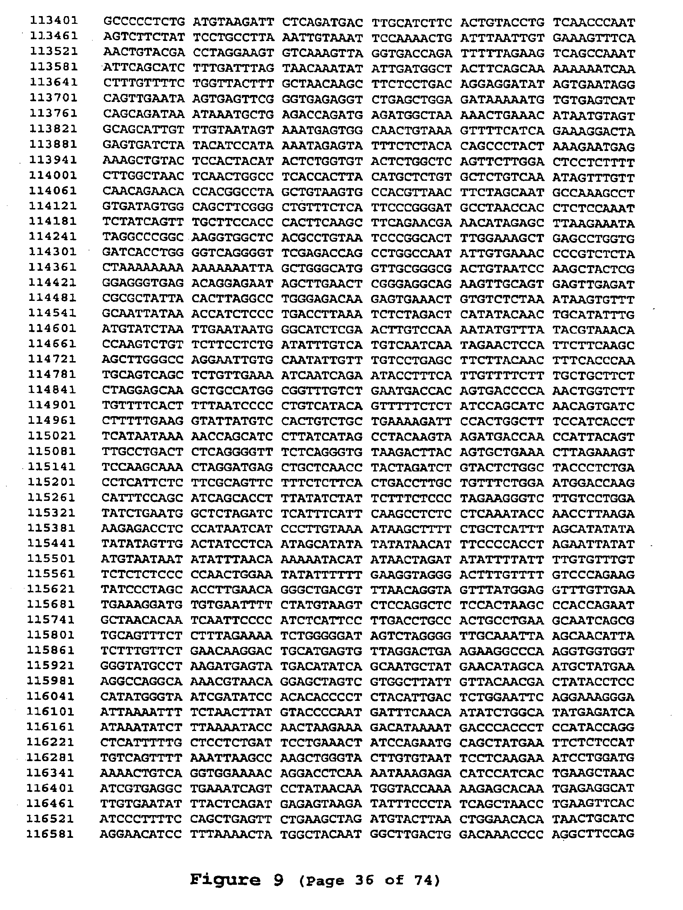

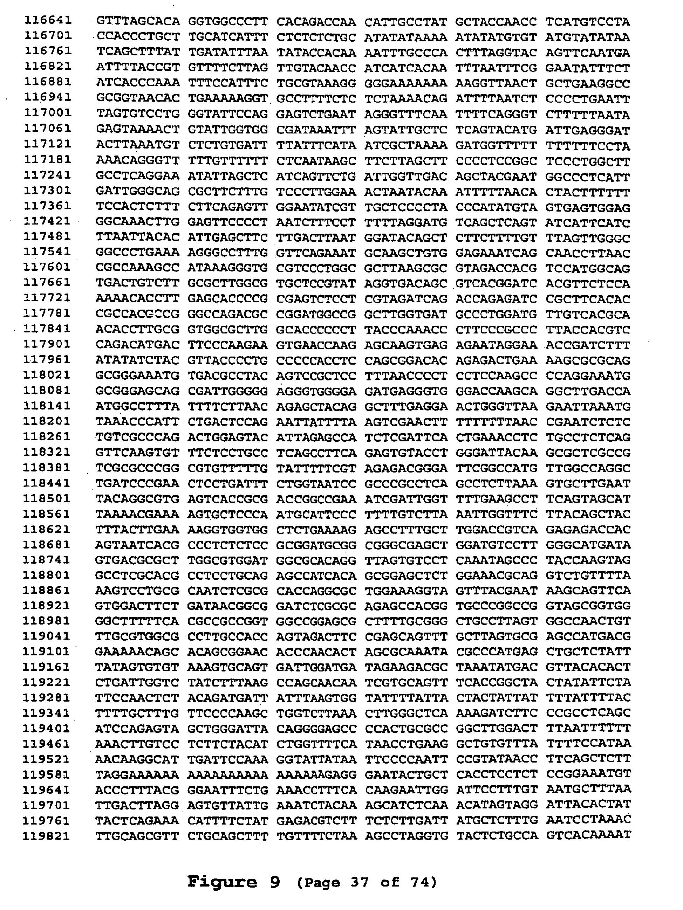

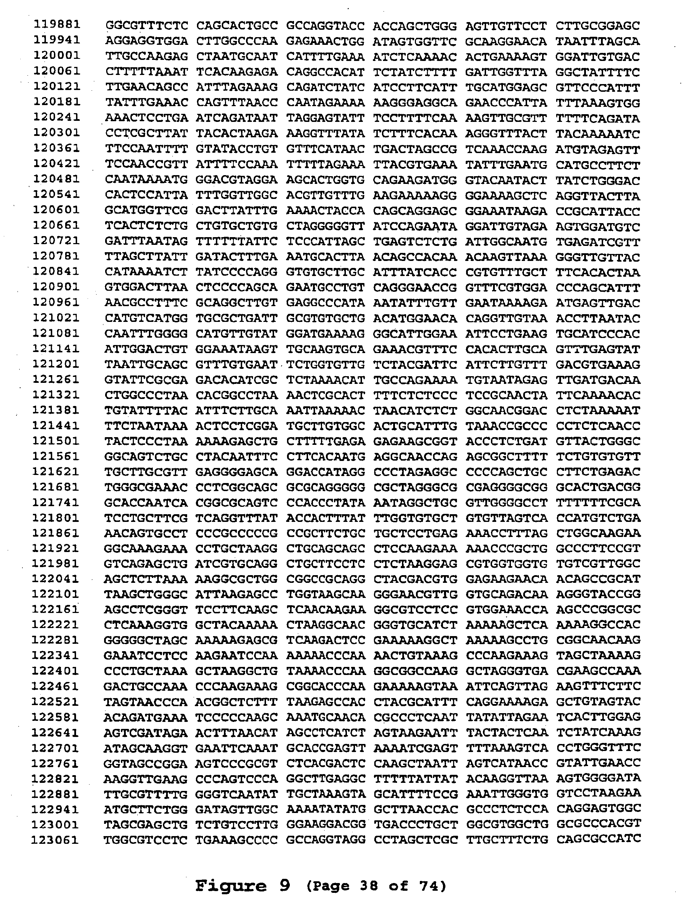

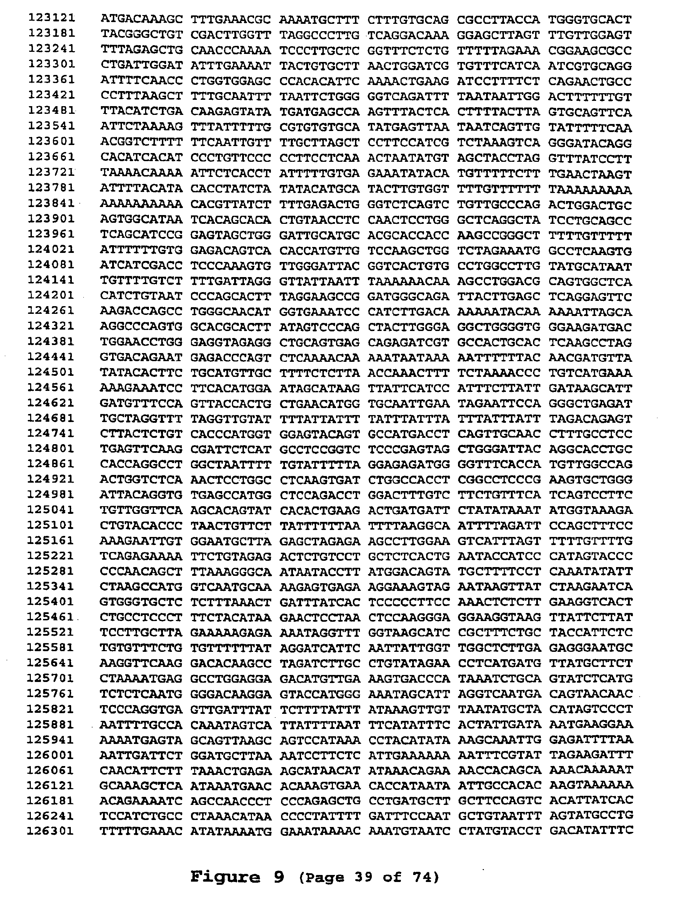

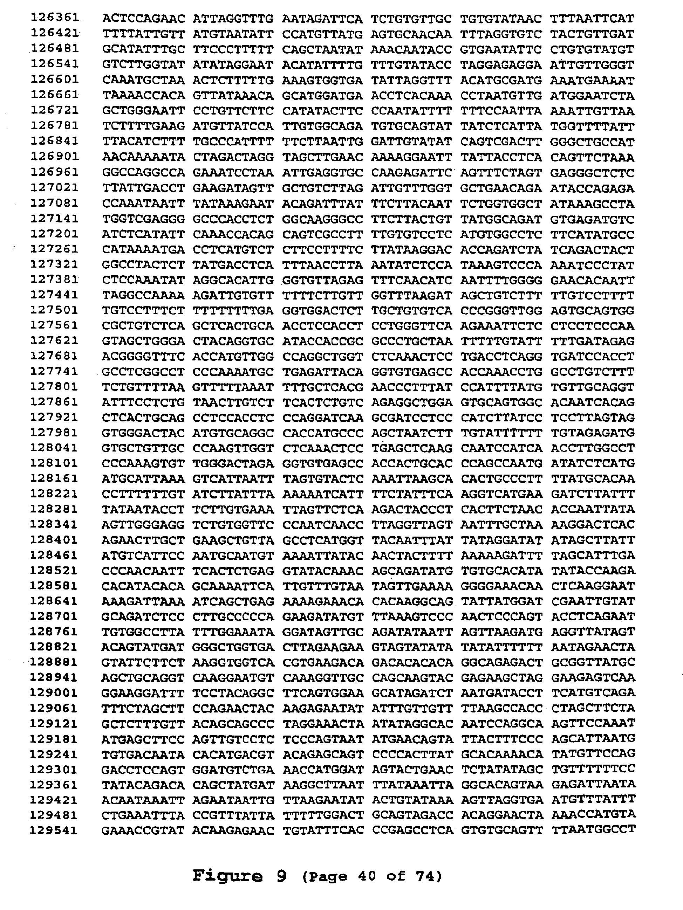

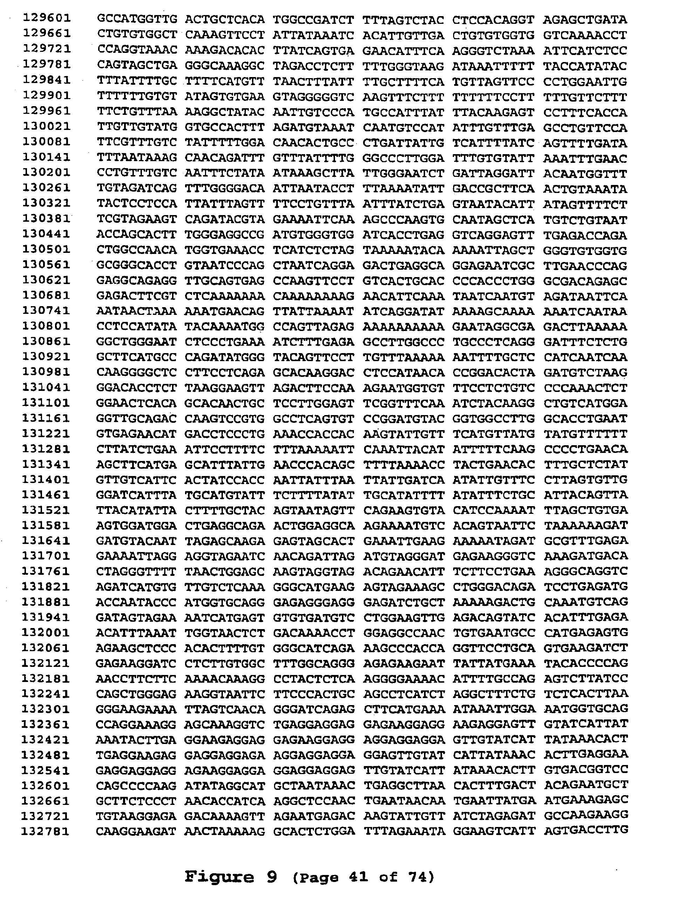

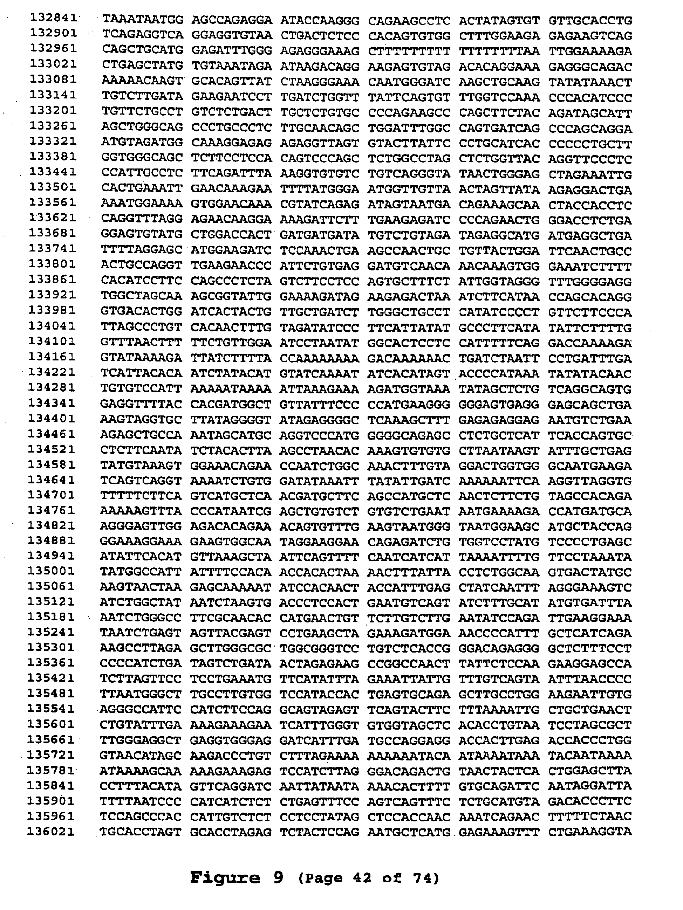

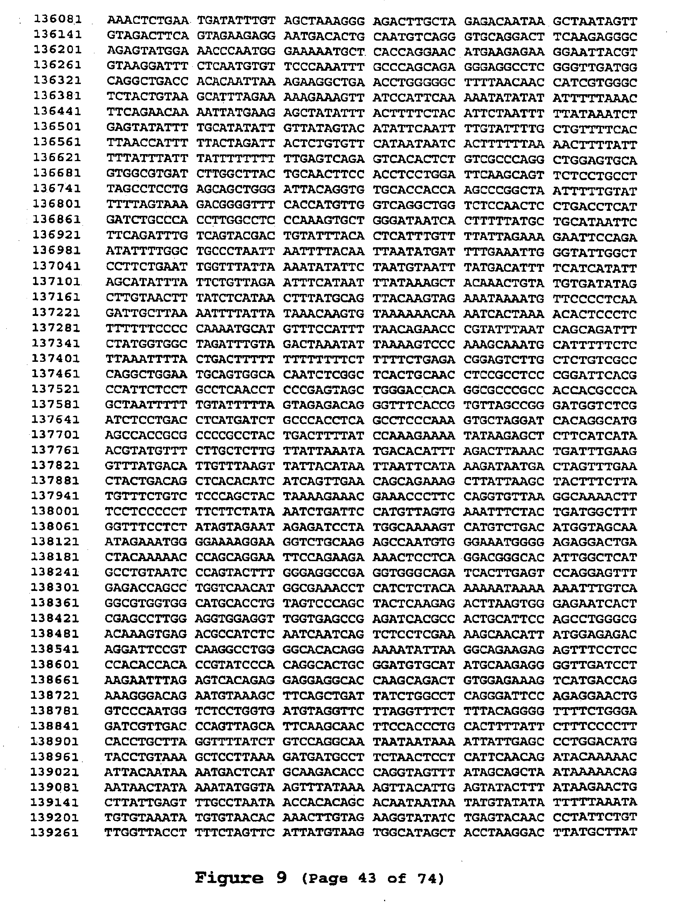

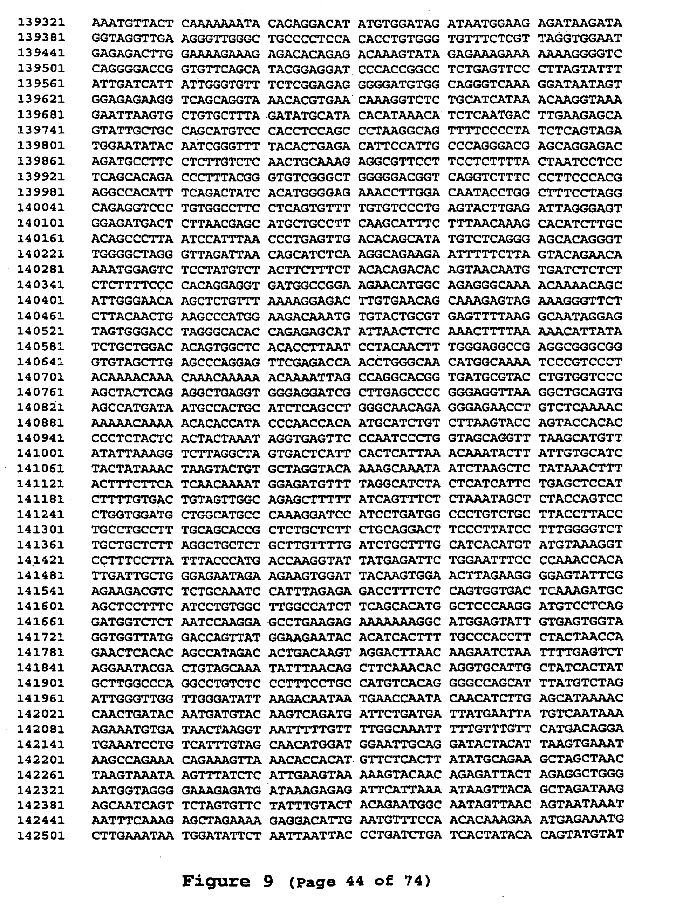

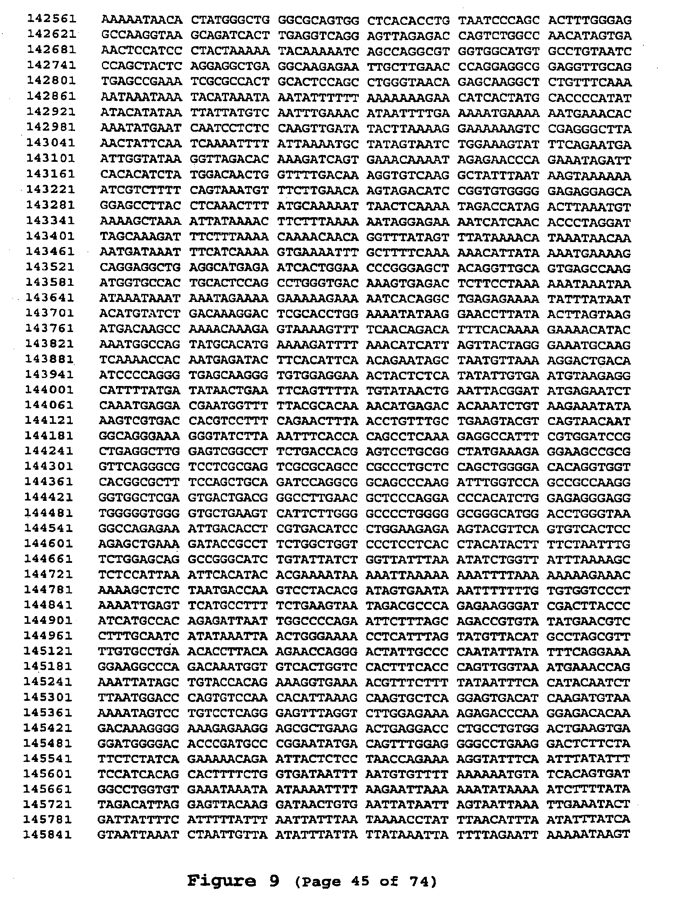

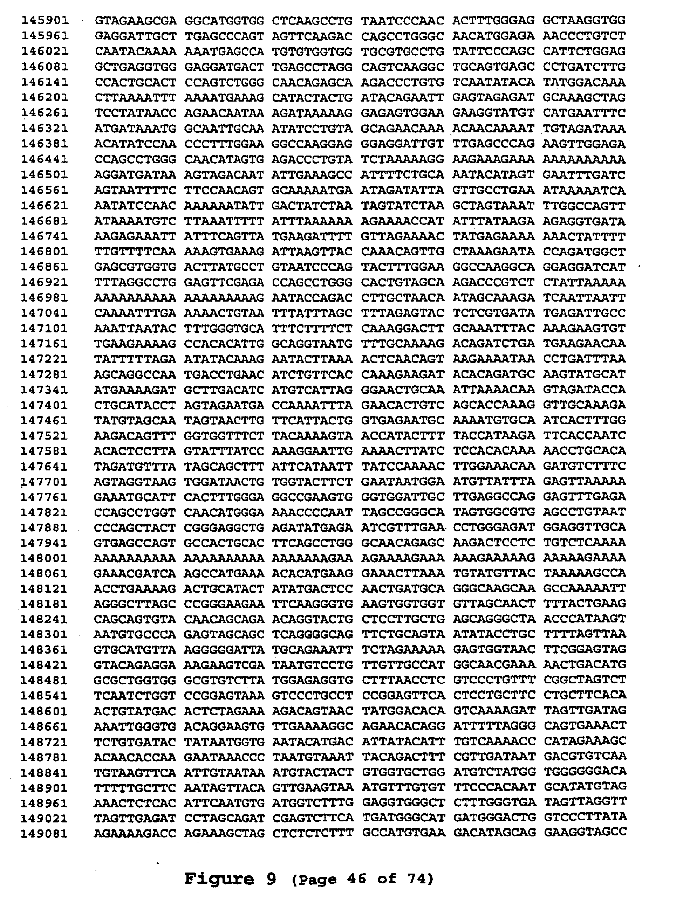

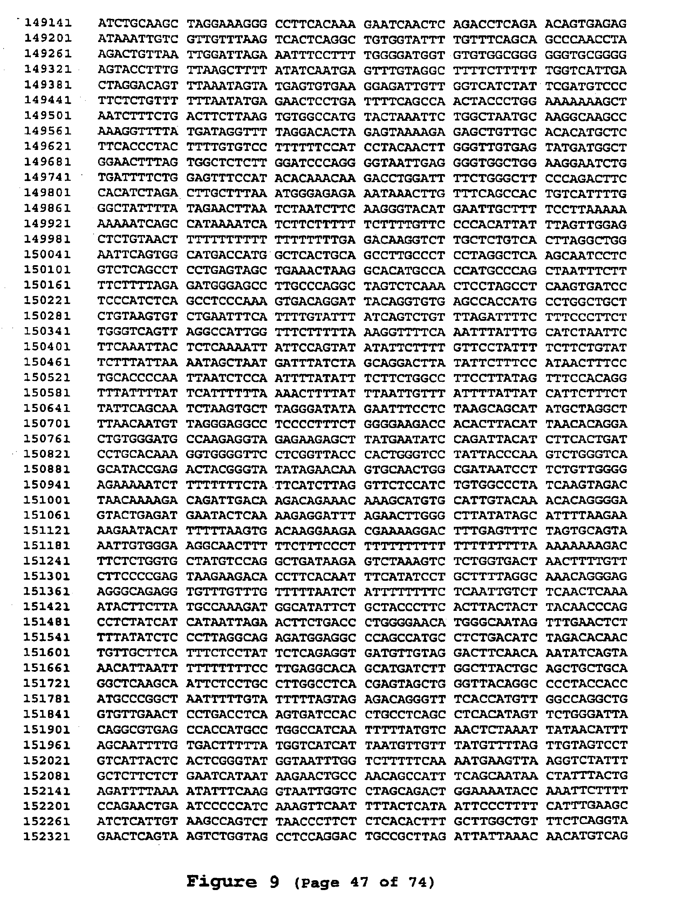

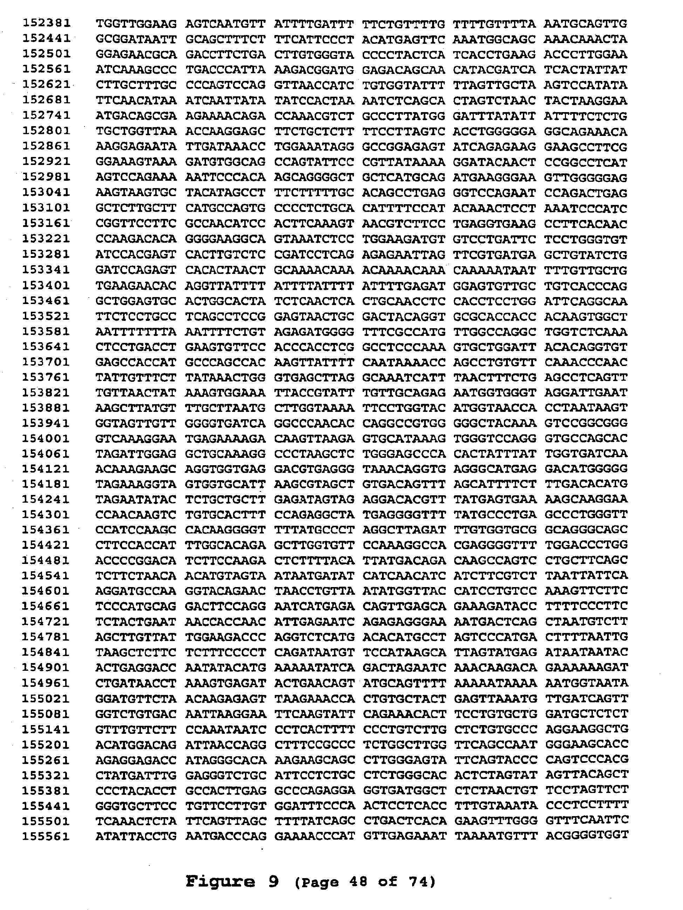

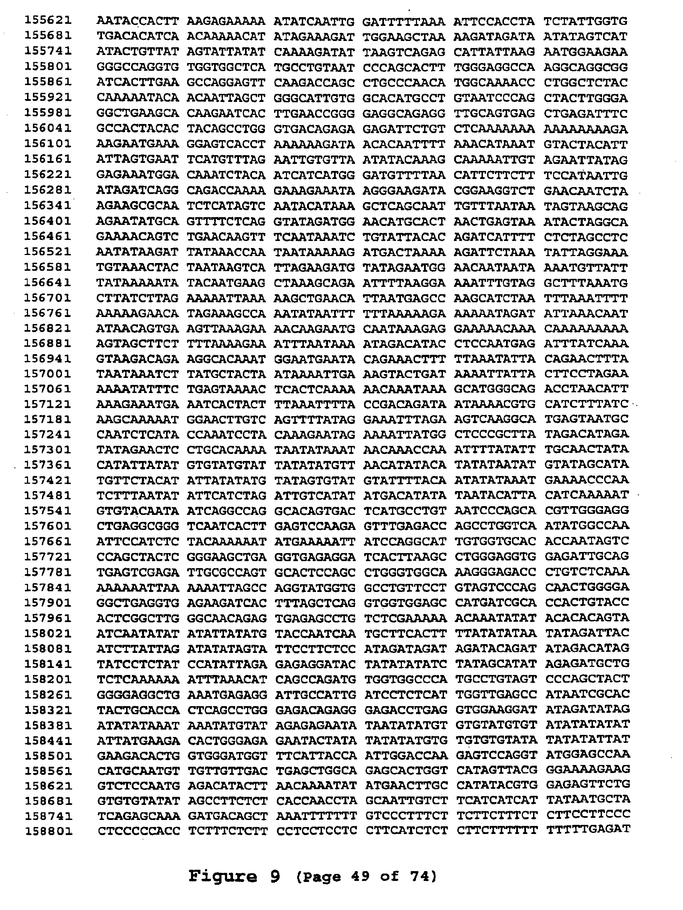

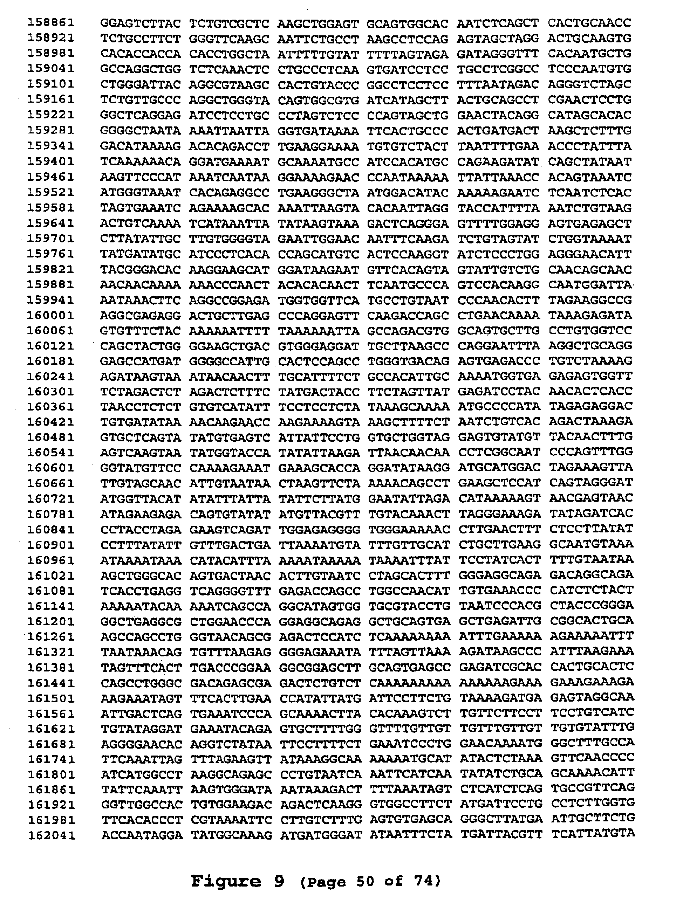

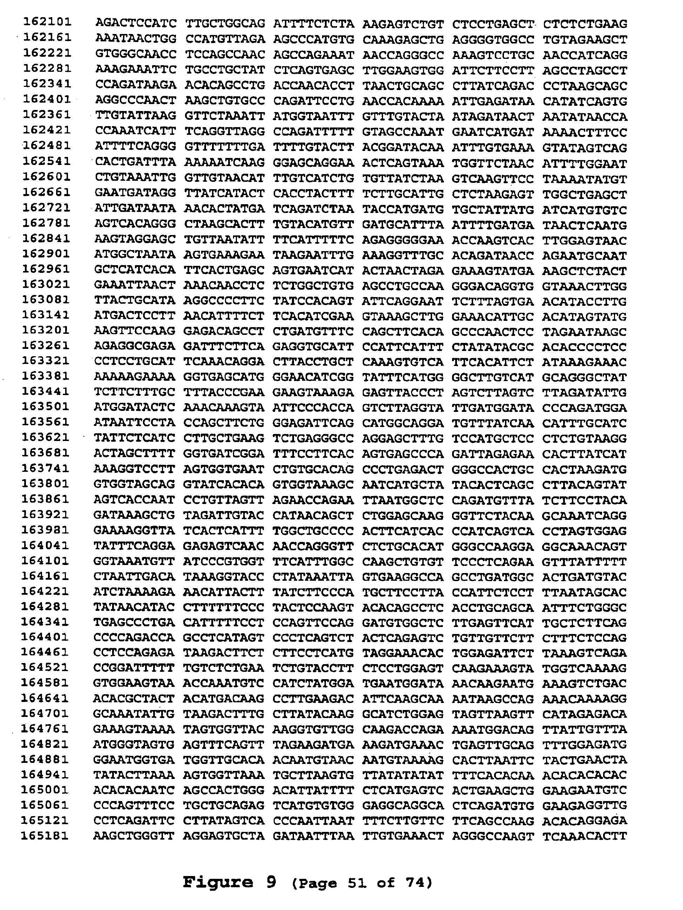

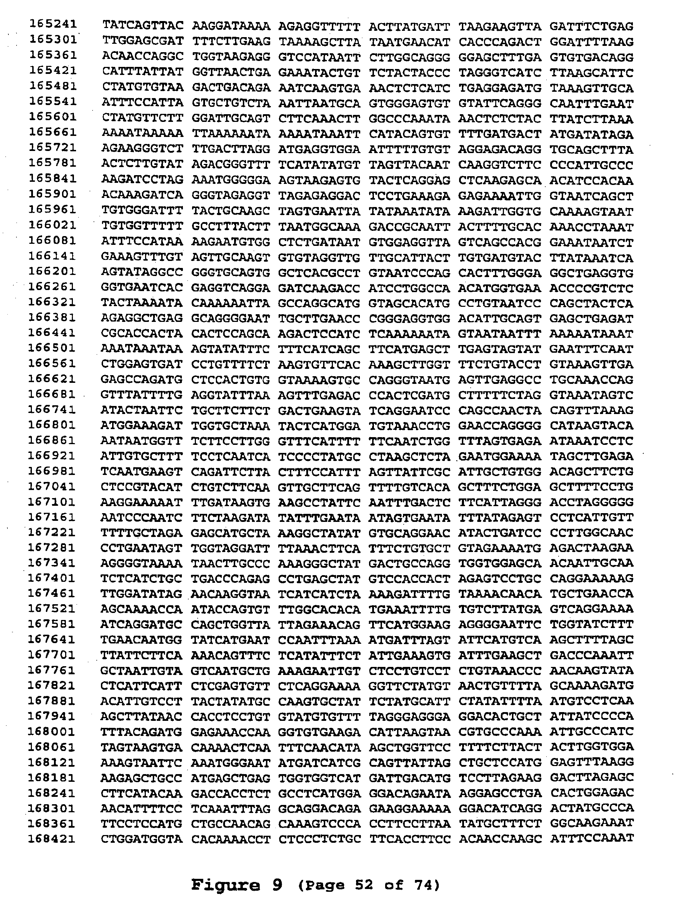

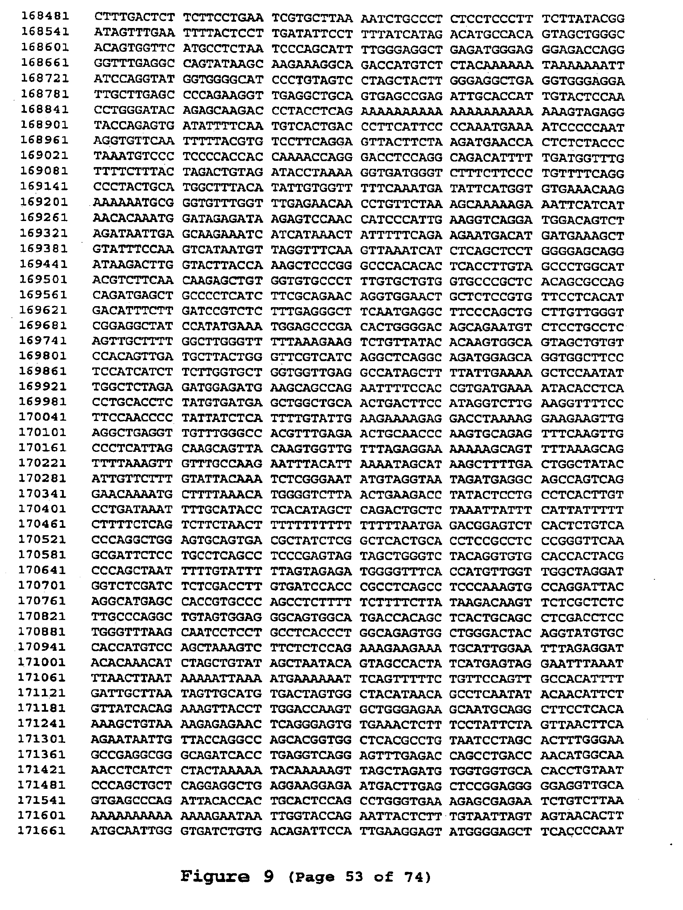

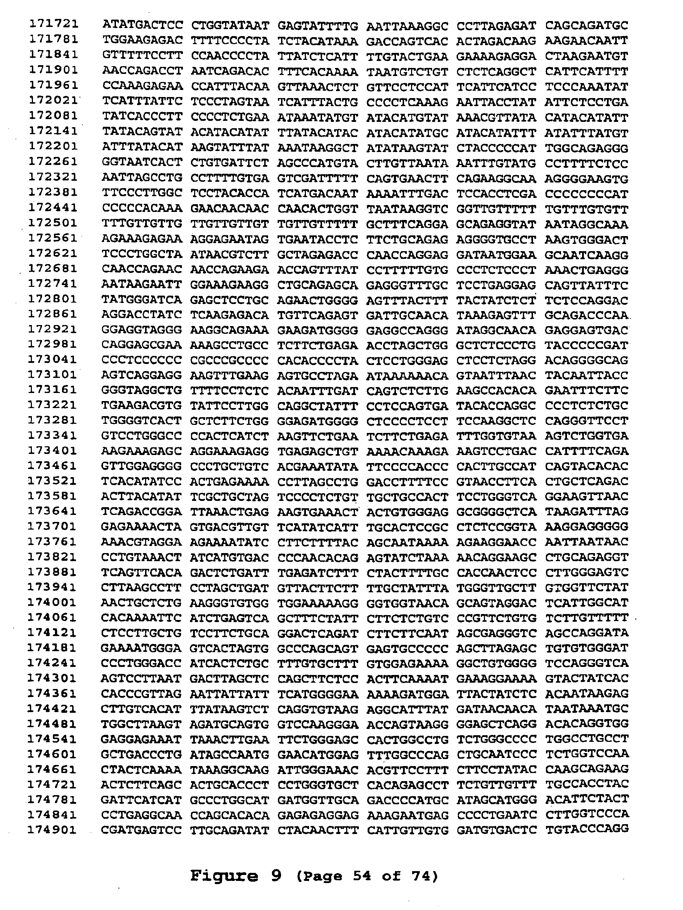

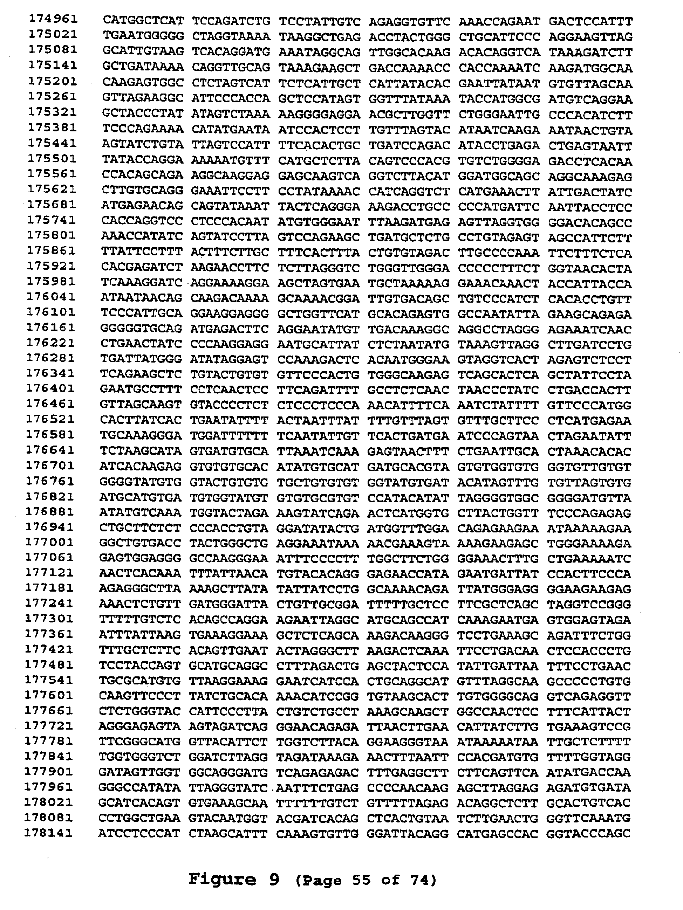

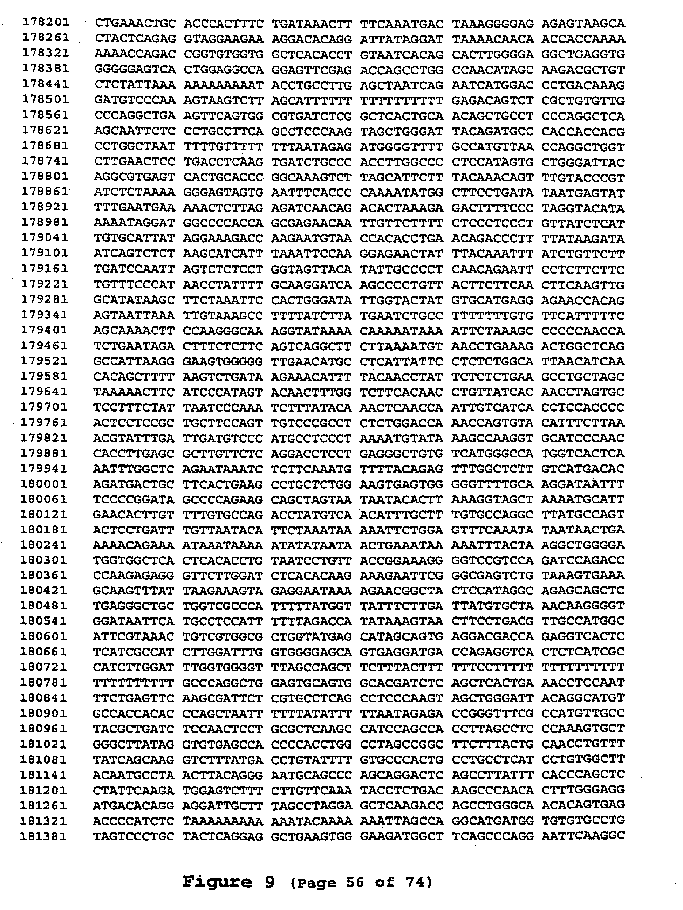

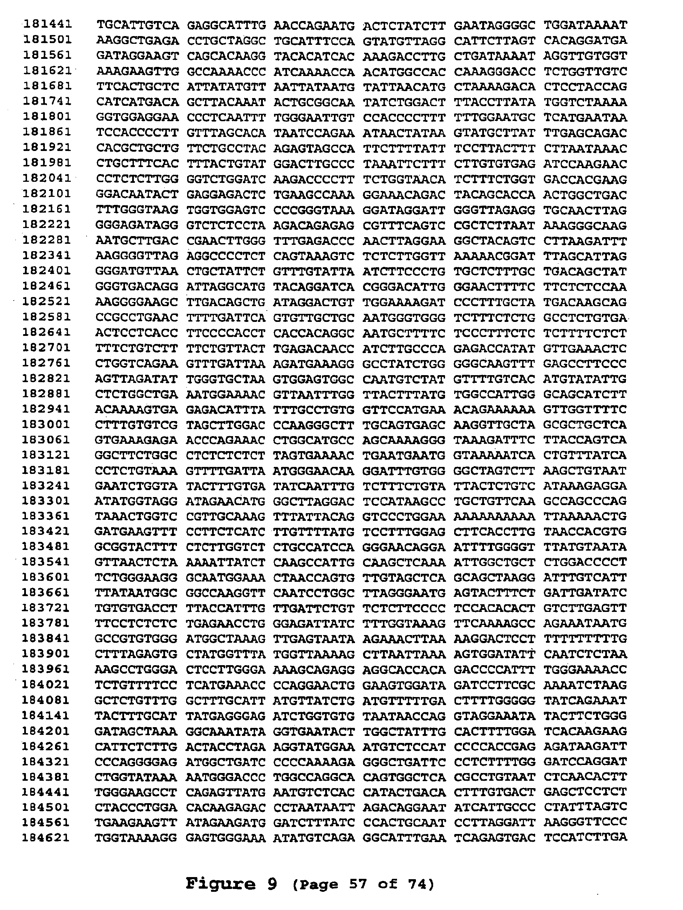

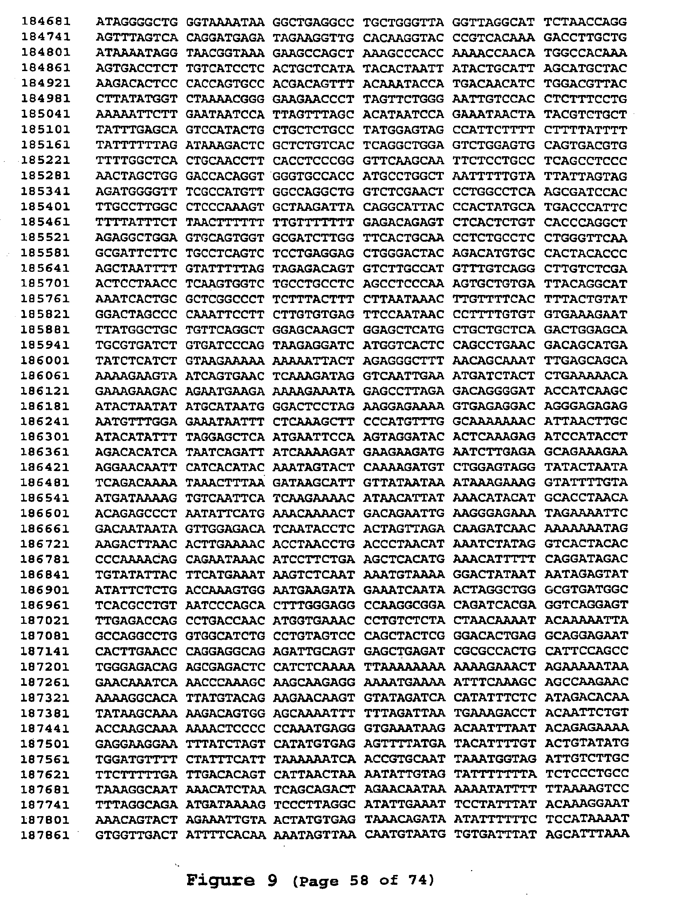

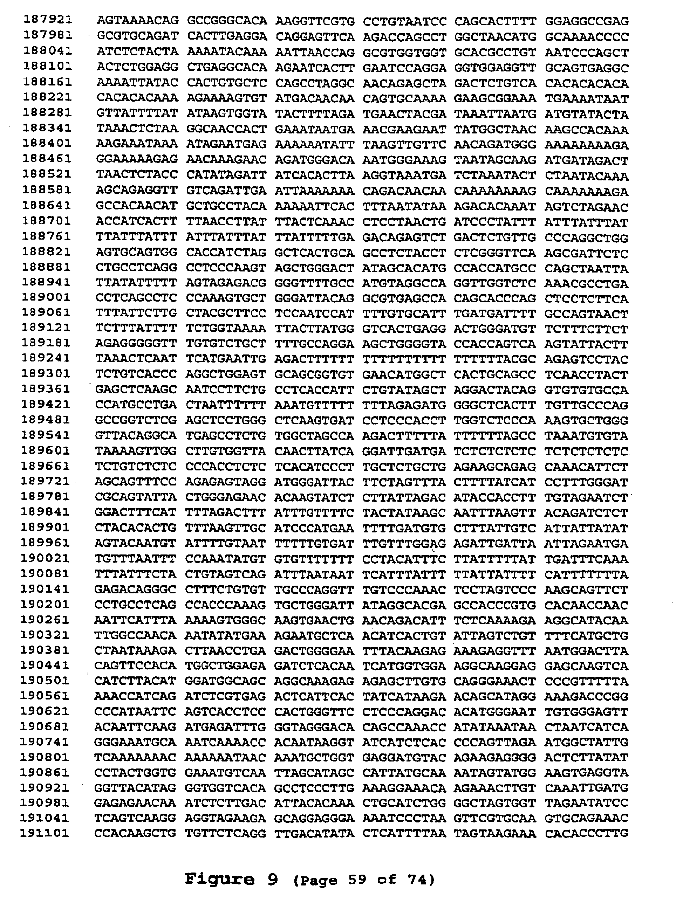

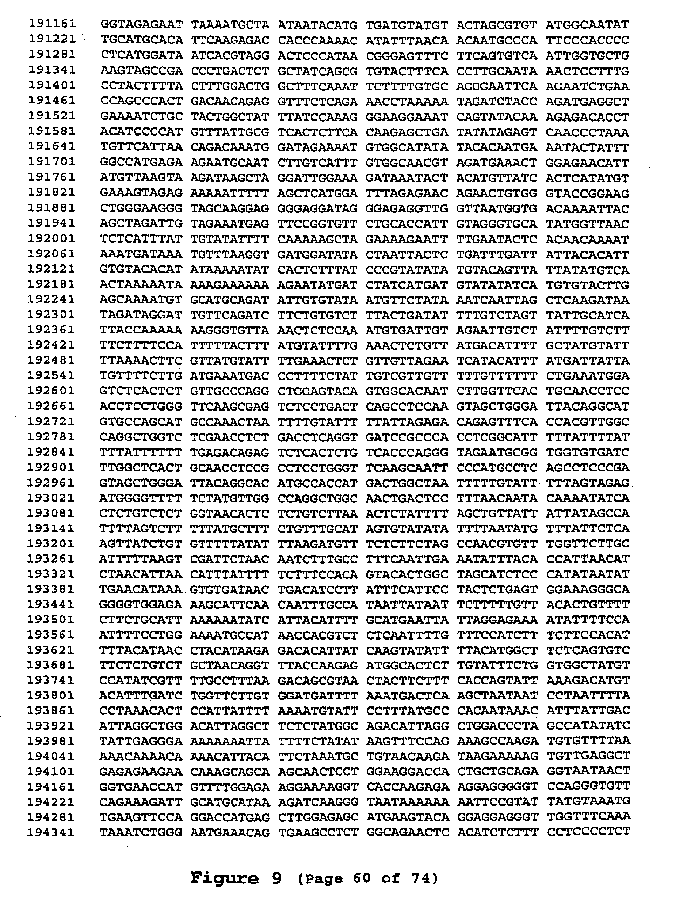

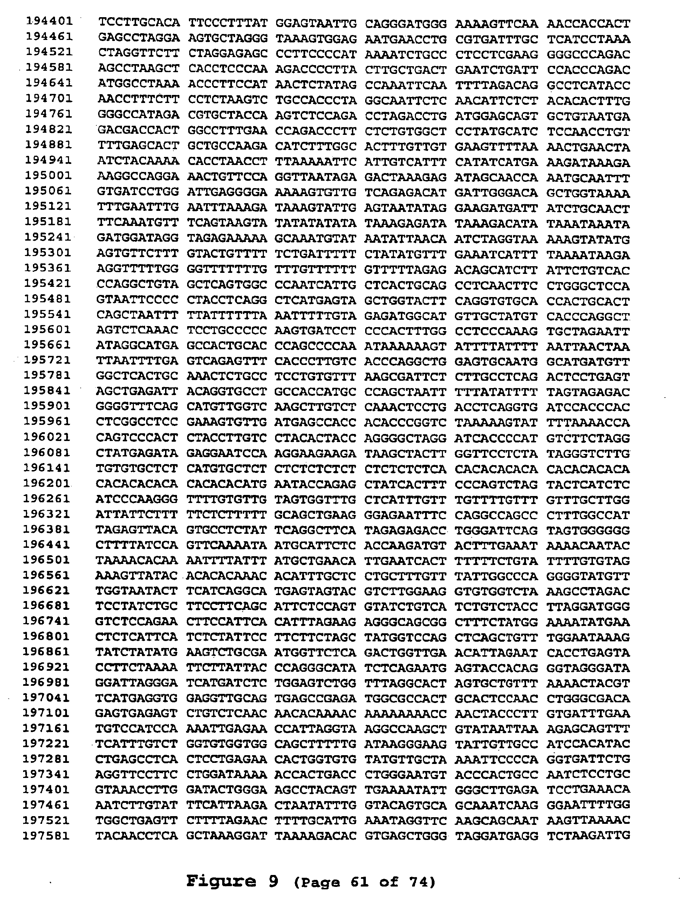

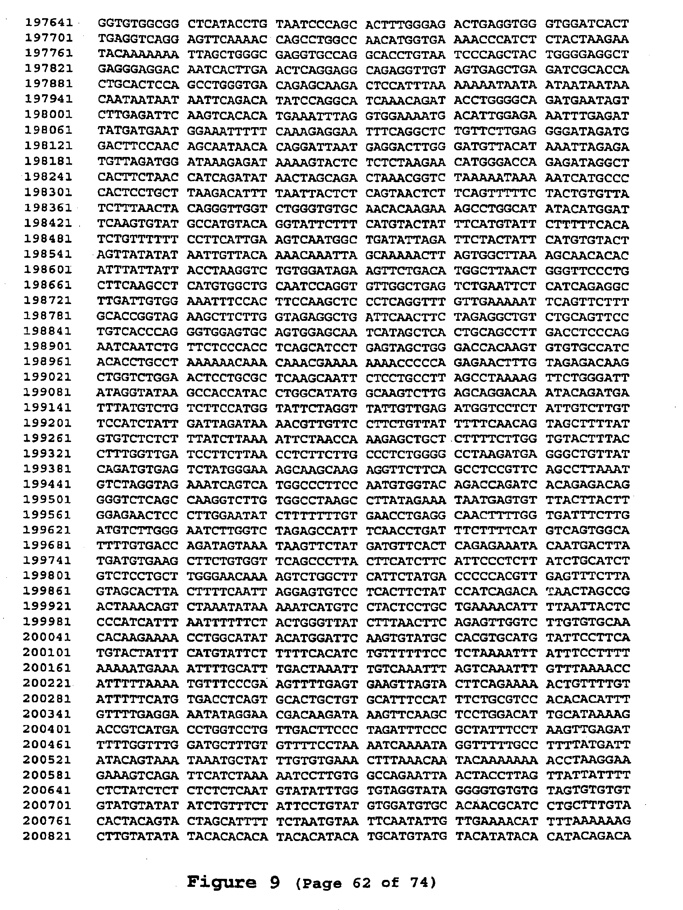

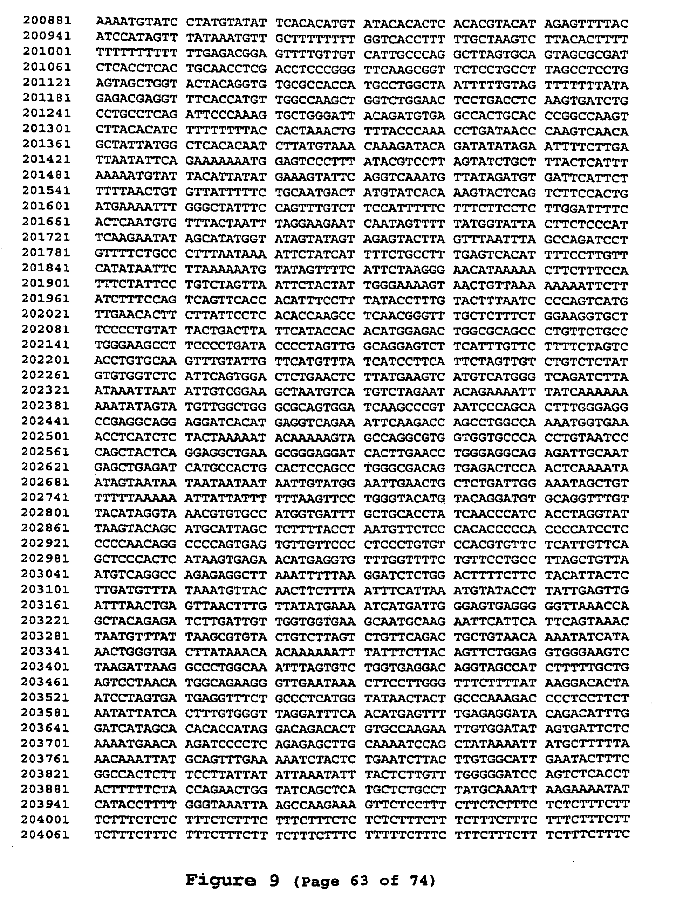

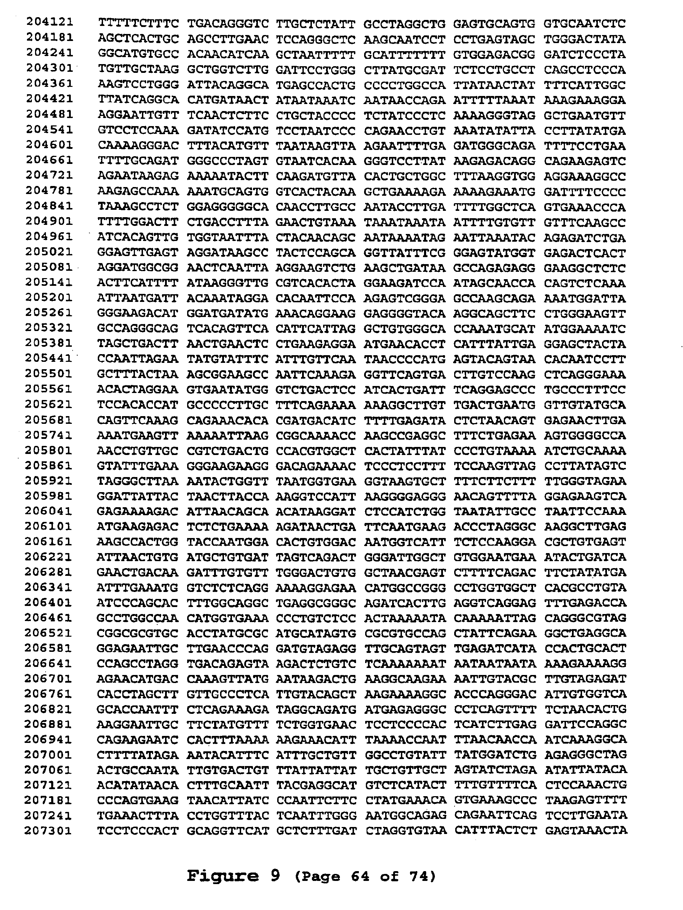

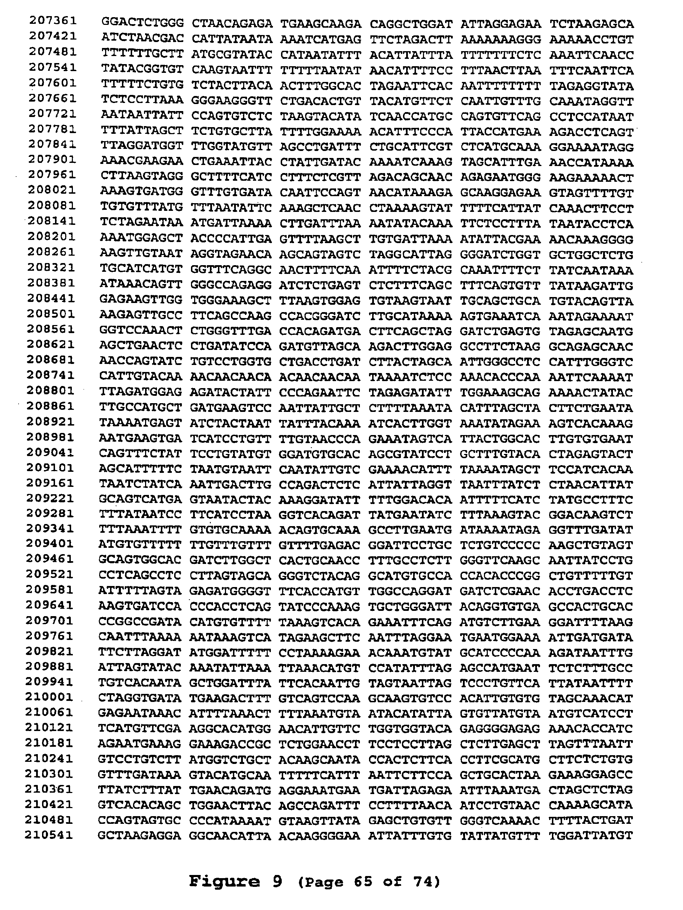

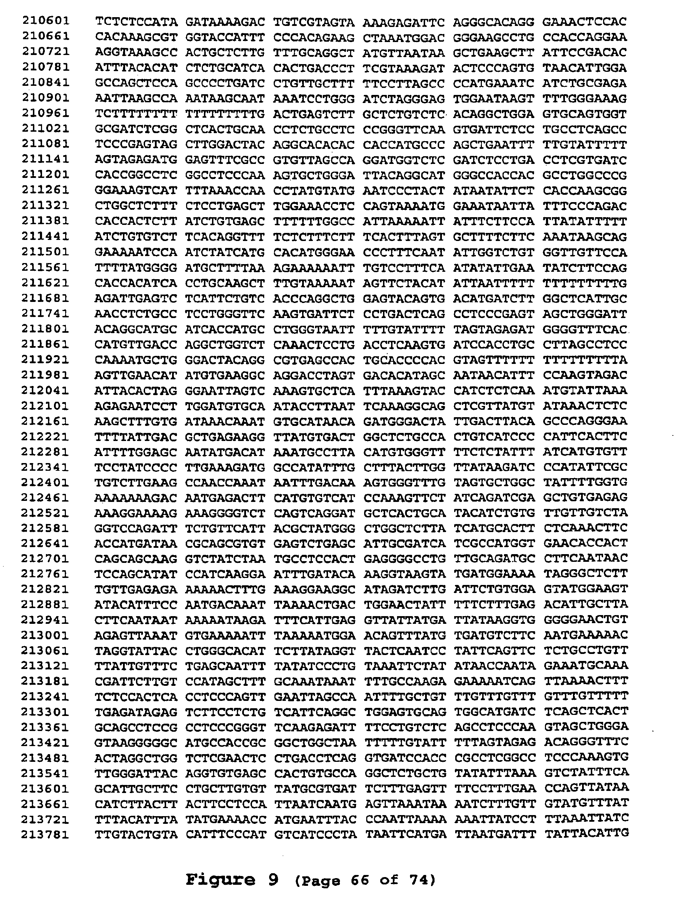

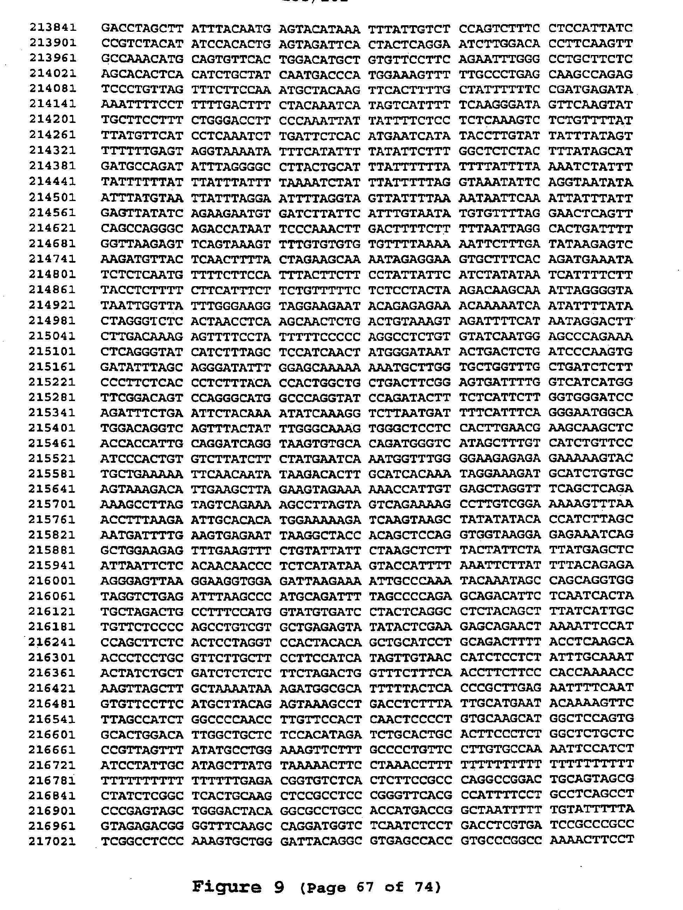

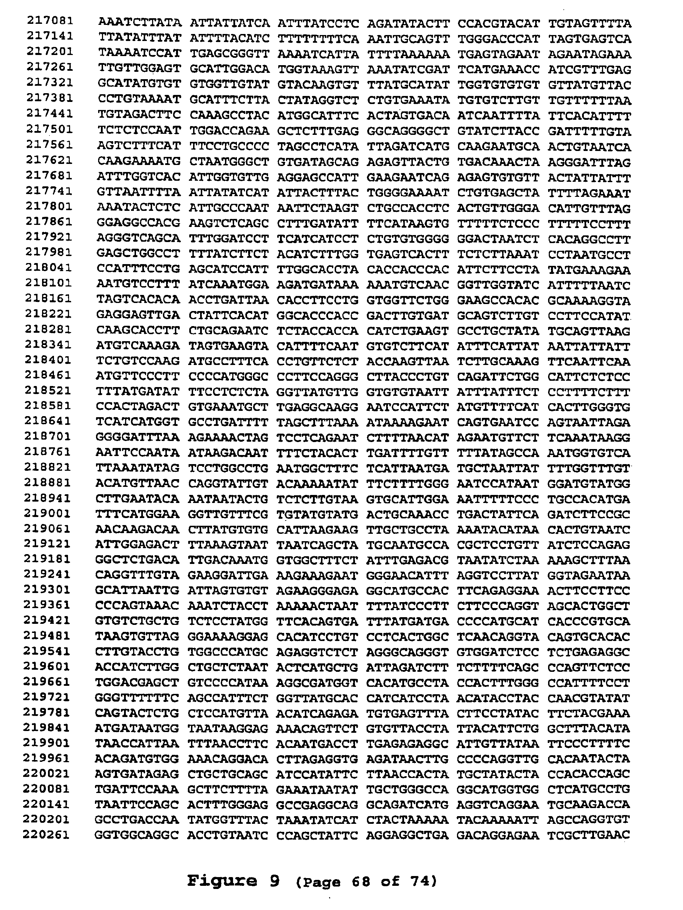

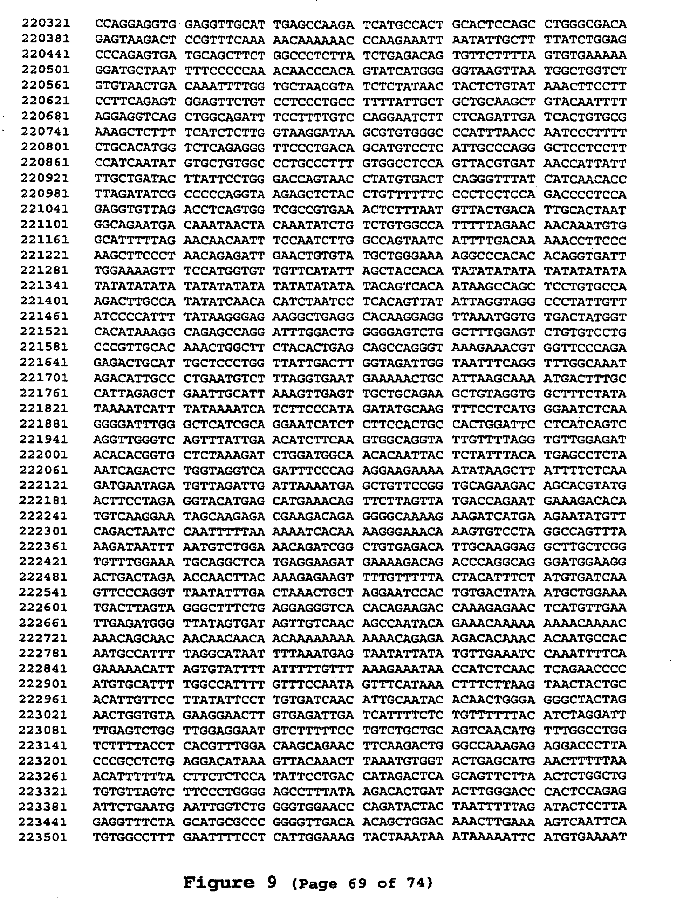

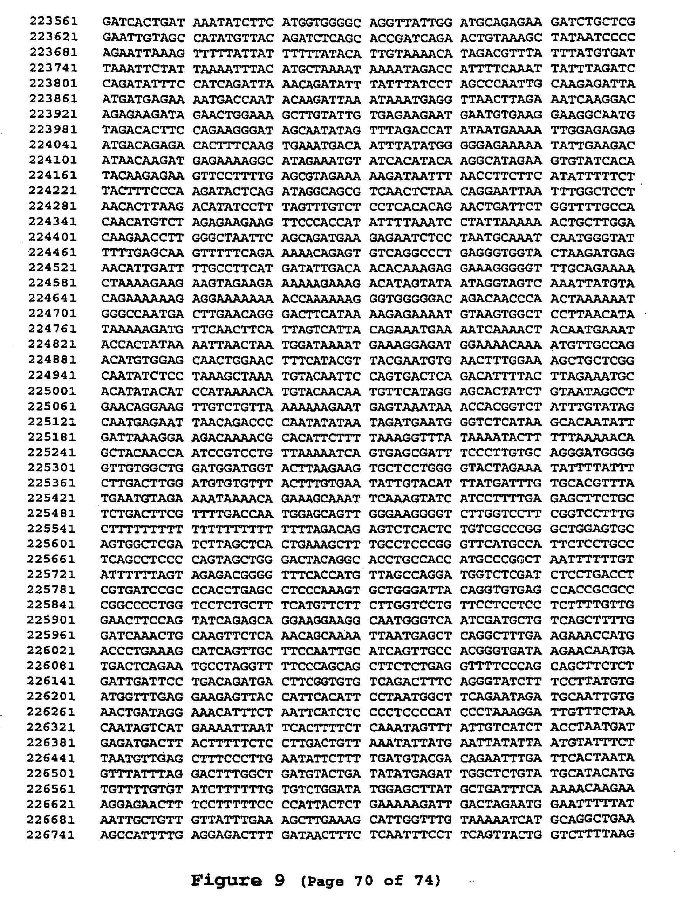

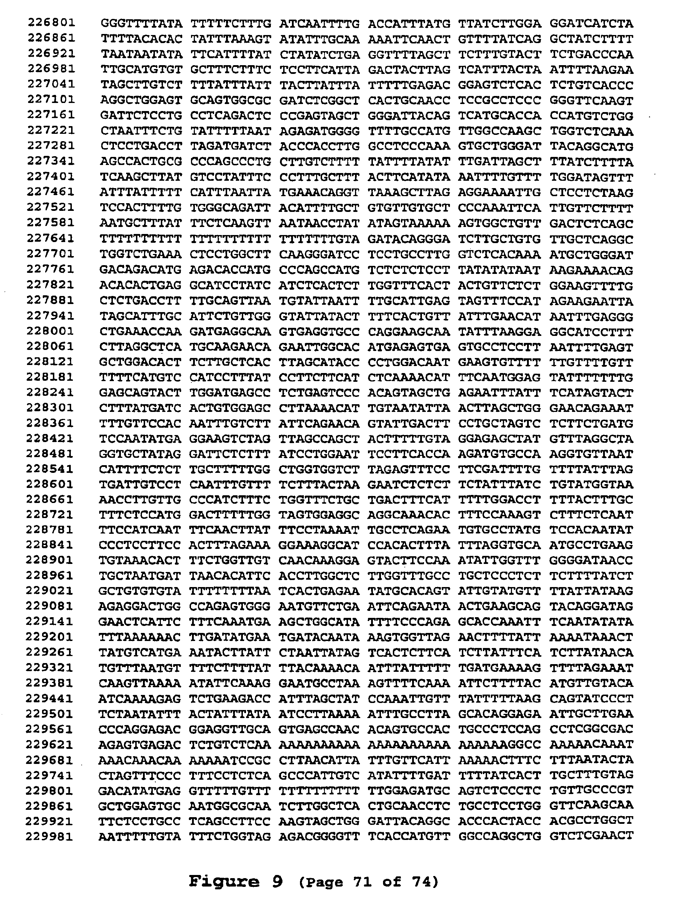

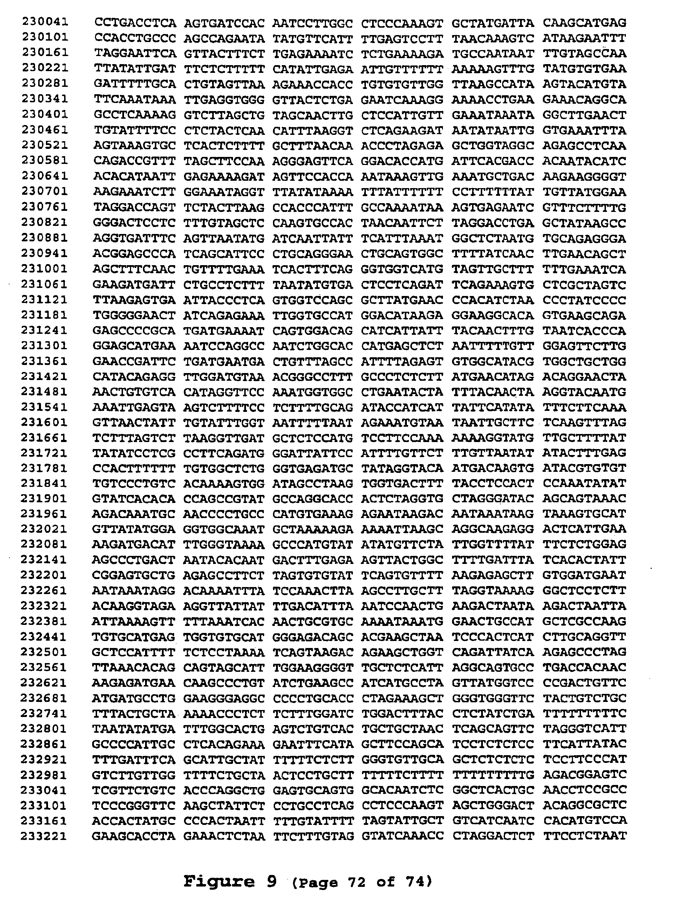

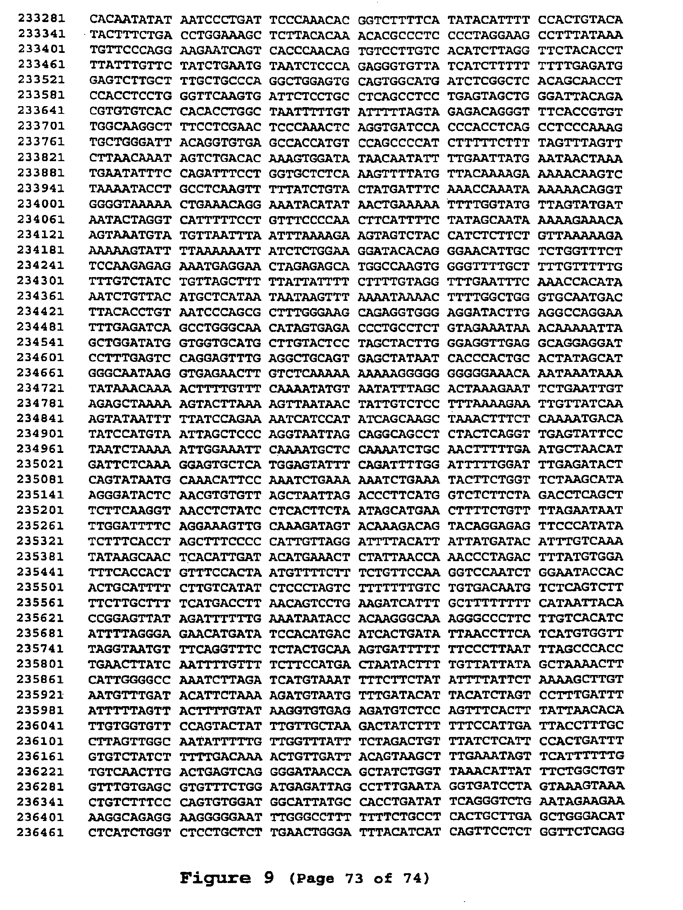

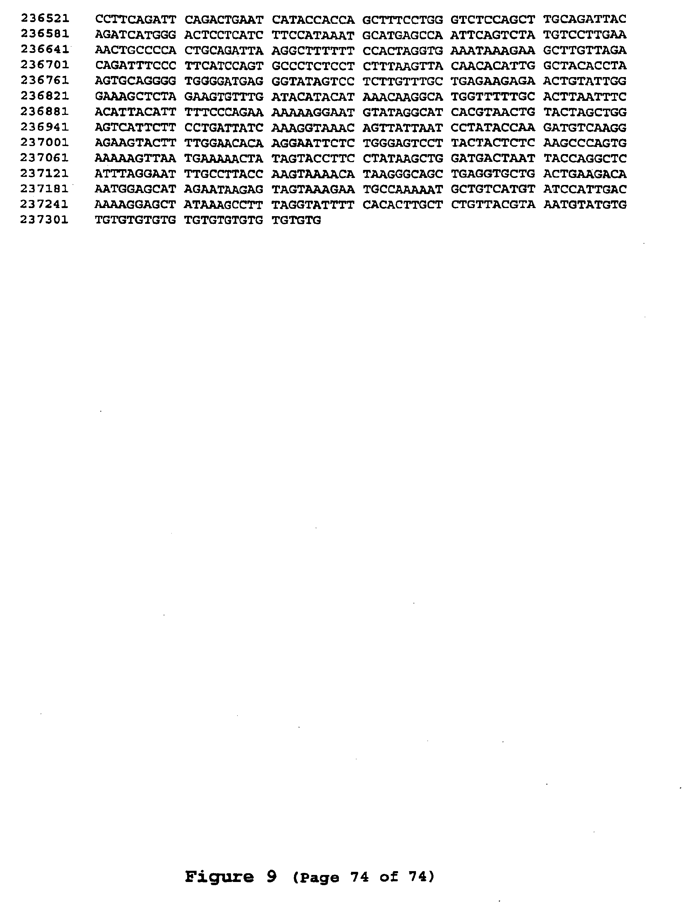

Figure 9 depicts the nucleotide sequence of approximately 235 kb in the HFE subregion from an HH affected individual. Polymorphic sites in the HH affected individual determined by comparing a sequence of the corresponding region from an HH unaffected individual are listed and described in Table I.

### DETAILED DESCRIPTION

### A. Definitions

Abbreviations for the twenty naturally occurring amino acids follow conventional usage. In the polypeptide notation used herein, the left-hand direction is the amino terminal direction and the right-hand direction is the carboxyl-terminal direction, in accordance with standard usage and convention. Similarly, unless specified otherwise, the left hand end of single-stranded polynucleotide sequences is the 5' end; the left hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

The term "nucleic acids", as used herein, refers to either DNA or RNA. "Nucleic acid sequence" or "polynucleotide sequence" refers to a single- or double-stranded polymer of deoxyribonucleotide or ribonucleotide bases read from the 5' to the 3' end. It includes both self-replicating plasmids, infectious polymers of DNA or RNA and nonfunctional DNA or RNA. The complement of any nucleic acid sequence of the invention is understood to be included in the definition of that sequence.

"Nucleic acid probes" may be DNA or RNA fragments. DNA fragments can be prepared, for example, by digesting plasmid DNA, or by use of PCR, or synthesized by either the phosphoramidite method described by Beaucage and Carruthers, Tetrahedron Lett. 22:1859-1862 (1981), or by the triester method according to Matteucci, et al., J. Am. Chem. Soc. 103:3185 (1981). A double stranded fragment may then be obtained, if desired, by annealing the chemically synthesized single strands together under appropriate conditions or by synthesizing the complementary strand using DNA polymerase with an appropriate primer sequence. Where a specific sequence for a nucleic acid probe is given, it is understood that the complementary strand is also identified and included. The complementary strand will work equally well in situations where the target is a double-stranded nucleic acid.

The phrase "selectively hybridizing to" refers to a nucleic acid probe that hybridizes, duplexes or binds only to a particular target DNA or RNA sequence when the target sequences are present in a preparation of total cellular DNA or RNA. "Complementary" or "target" nucleic acid sequences refer to those nucleic acid sequences which selectively hybridize to a nucleic acid probe. Proper annealing conditions depend, for example, upon a probe's length, base composition, and the number of mismatches and their position on the probe, and must often be determined empirically. For discussions of nucleic acid probe design and annealing conditions, see, for example, Sambrook et al., Molecular Cloning: a Laboratory Manual (2nd ed.), Vols. 1-3, Cold Spring Harbor Laboratory, (1989) or Current Protocols in Molecular Biology, F. Ausubel et al., ed. Greene Publishing and Wiley-Interscience, New York (1987).

The phrase "nucleic acid sequence encoding" refers to a nucleic acid which directs the expression of a specific protein or peptide. The nucleic acid sequences include both the DNA strand sequence that is transcribed into RNA and the RNA sequence that is translated into protein. The nucleic acid sequences include both the full length nucleic acid sequences as well as non-full length sequences derived from the full length protein. It being further understood that the sequence includes the degenerate codons of the native sequence or sequences which may be introduced to provide codon preference in a specific host cell.

The phrase "isolated" or "substantially pure" refers to nucleic acid preparations that lack at least one protein or nucleic acid normally associated with the nucleic acid in a host cell.

The phrase "expression cassette", refers to nucleotide sequences which are capable of affecting expression of a structural gene in hosts compatible with such sequences. Such cassettes include at least promoters and optionally, transcription termination signals. Additional factors necessary or helpful in effecting expression may also be used as described herein.

The term "operably linked" as used herein refers to linkage of a promoter upstream from a DNA sequence such that the promoter mediates transcription of the DNA sequence.

The term "vector", refers to viral expression systems, autonomous self-replicating circular DNA (plasmids), and includes both expression and nonexpression plasmids. Where a recombinant microorganism or cell culture is described as hosting an "expression vector," this includes both extrachromosomal circular DNA and DNA that has been incorporated into the host chromosome(s). Where a vector is being maintained by a host cell, the vector may either be stably replicated by the cells during mitosis as an autonomous structure, or is incorporated within the host's genome.

The term "gene" as used herein is intended to refer to a nucleic acid sequence which encodes a polypeptide. This definition includes various sequence polymorphisms, mutations, and/or sequence variants wherein such alterations do not affect the function of the gene product. The term "gene" is intended to include not only coding sequences but also regulatory regions such as promoters, enhancers, and termination regions. The term further includes all introns and other DNA sequences spliced from the mRNA transcript, along with variants resulting from alternative splice sites.

The term "plasmid" refers to an autonomous circular DNA molecule capable of replication in a cell, and includes both the expression and nonexpression types. Where a recombinant microorganism or cell culture is described as hosting an "expression plasmid", this includes both extrachromosomal circular DNA molecules and DNA that has been incorporated into the host chromosome(s). Where a plasmid is being maintained by a host cell, the plasmid is either being stably replicated by the cells during mitosis as an autonomous structure or is incorporated within the host's genome.

The phrase "recombinant protein" or "recombinantly produced protein" refers to a peptide or protein produced using non-native cells that do not have an endogenous copy of DNA able to express the protein. The cells produce the protein because they have been genetically altered by the introduction of the appropriate nucleic acid sequence. The recombinant protein will not be found in association with proteins and other subcellular components normally associated with the cells producing the protein. The terms "protein" and "polypeptide" are used interchangeably herein.

The following terms are used to describe the sequence relationships between two or more nucleic acids or polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing, or may comprise a complete cDNA or gene sequence.

Optimal alignment of sequences for aligning a comparison window may, for example, be conducted by the local homology algorithm of Smith and Waterman Adv, Appl. Math. 2:482 (1981), by the homology alignment algorithm of Needleman and Wunsch J. Mol. Biol. 48:443 (1970), by the search for similarity method of Pearson and Lipman Proc. Natl. Acad. Sci. U.S.A. 852444 (1988), or by computerized implementations of these algorithm (for example, GAP, BESTFIT, FASTA, and TFASTA in the Wisconsin Genetics Software Package Release 7.0, Genetics Computer Group, 575 Science Dr., Madison, WI).

The terms "substantial identity" or "substantial sequence identity" as applied to nucleic acid sequences and as used herein and denote a characteristic of a polynucleotide sequence, wherein the polynucleotide comprises a sequence that has at least 85 percent sequence identity, preferably at least 90 to 95 percent sequence identity, and more preferably at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

As applied to polypeptides, the terms "substantial identity" or "substantial sequence identity" mean that two peptide sequences, when optimally aligned, such as by the programs GAP or BESTFIT using default gap weights, share at least 80 percent sequence identity, preferably at least 90 percent sequence identity, more preferably at least 95 percent sequence identity or more. "Percentage amino acid Identity" or "percentage amino acid sequence identity" refers to a comparison of the amino acids of two polypeptides which, when optimally aligned, have approximately the designated percentage of the same amino acids. For example, "95% amino acid Identity" refers to a comparison of the amino acids of two polypeptides which when optimally aligned have 95% amino acid identity. Preferably, residue positions which are not identical differ by conservative amino acid substitutions. For example, the substitution of amino acids having similar chemical properties such as charge or polarity are not likely to effect the properties of a protein. Examples include glutamine for asparagine or glutamic acid for aspartic acid.

The phrase "substantially purified" or "isolated" when referring to a peptide or protein, means a chemical composition which is essentially free of other cellular components. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. Generally, a substantially purified or isolated protein will comprise more than 80% of all macromolecular species present in the preparation. Preferably, the protein is purified to represent greater than 90% of all macromolecular species present. More preferably the protein is purified to greater than 95%, and most preferably the protein is purified to essential homogeneity, wherein other macromolecular species are not detected by conventional techniques.

The phrase "specifically binds to an antibody" or "specifically immunoreactive with", when referring to a protein or peptide, refers to a binding reaction which is determinative of the presence of the protein in the presence of a heterogeneous population of proteins and other biologies. Thus, under designated immunoassay conditions, the specified antibodies bind to a particular protein and do not bind in a significant amount to other proteins present in the sample. Specific binding to an antibody under such conditions may require an antibody that is selected for its specificity for a particular protein. A variety of immunoassay formats may be used to select antibodies specifically immunoreactive with a particular protein. For example, solid-phase ELISA immunoassays are routinely used to select monoclonal antibodies specifically immunoreactive with a protein. See Harlow and Lane (1988) Antibodies, a Laboratory Manual, Cold Spring Harbor Publications, New York, for a description of immunoassay formats and conditions that can be used to determine specific immunoreactivity.

As used herein, "EST' or "Expressed Sequence Tag "refers to a partial DNA or cDNA sequence of about 150 to 500, more preferably about 300, sequential nucleotides of a longer sequence obtained from a genomic or cDNA library prepared from a selected cell, cell type, tissue or tissue type, or organisms which longer sequence corresponds to an mRNA or a gene found in that library. An EST is generally DNA. One or more libraries made from a single tissue type typically provide at least 3000 different (i.e. unique) EST's and potentially the full complement of all possible EST's representing all possible cDNAs, e.g., 50,000 - 100,000 in an animal such as a human. (See, for example, Adams et al. Science 252:1651-1656 (1991)).

"Stringent" as used herein refers to hybridization and wash conditions of 50% formamide at 42°C. Other stringent hybridization conditions may also be selected. Generally, stringent conditions are selected to be about 5° C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is at least about 0.02 molar at pH 7 and the temperature is at least about 60°C. As other factors may significantly affect the stringency of hybridization, including, among others, base composition and size of the complementary strands, the presence of organic solvents and the extent of base mismatching, the combination of parameters is more important than the absolute measure of any one.

### B. Transcript Map and New Genes near HH

The instant disclosure provides a fine structure map of the 1 megabase region surrounding the HFE gene. As part of that map the instant disclosure provides approximately 250 kb of DNA sequence of which about 235 kb are provided in Figure 8 and eight loci of particular interest corresponding to candidate genes within the 1 megabase region. These loci are useful as genetic and physical markers for further mapping studies. Additionally, the eight cDNA sequences corresponding to those loci are useful, for example, for the isolation of other genes in putative gene families, the identification of homologs from other species, and as probes for diagnostic assays. In particular, isolated nucleic acid sequences of at least 18 nucleotides substantially identical to contiguous nucleotides of a cDNA of the disclosure are useful as PCR primers. Typically, the PCR primer will be used as part of a pair of primers in a PCR reaction. Isolated nucleic acid sequences preferably comprising about 18-100 nucleotides, more preferably at least 18 nucleotides, substantially identical to contiguous nucleotides in a cDNA of the invention are useful in the design of PCR primers and probes for hybridization assays. Additionally, the proteins encoded by those cDNAs are useful in the generation of antibodies for analysis of gene expression and in diagnostic assays, and in the purification of related proteins.

A 235 kb sequence is provided for the HFE subregion within the 1 megabase region mapped. This sequence can serve as a reference in genetic or physical analysis of deletions, substitutions, and insertions in that region. Additionally, the sequence information provides a resource for the further identification of new genes in that region.

### C. Polymorphic Markers

397 new polymorphic sites have been identified in the region of the HFE gene. These polymorphisms are listed in Table 1. As described below, these polymorphisms were identified by comparison of the DNA sequence of an affected individual homozygous for the common ancestral HH mutation with that of an unaffected individual disclosed in copending U.S. 08/724,394.

**Table 1. Polymorphic Sites in the HH Region**

| **Base Location** | **Difference** | **Base Location** | **Difference** |
|---|---|---|---|
| 35-36 | AC DEL | 19755 | G-A |
| 841 | T-C | 19949 | C-T |
| 2662-2663 | TT DEL | 20085 | C-T |
| 3767 | T-C | 20366-20367 | A INS |
| 3829 | C-G | 20463 | C-A |
| 4925-4928 | TAAA DEL | 20841 | A-T |
| 5691 | C-T | 21059 | A-T |
| 5839 | T-C | 21117 | A-G |
| 6011 | G-A | 21837 | A-C |
| 6047 | C-G | 22293 | A-C |
| 6231 | G-A | 22786 | C-A |
| 6643 | A DEL | 23009 | G-A |
| 6698 | T-C | 24143 | T-A |
| 7186 | T-C | 26175 | G-C |
| 7273 | G-A | 26667 | C-A |
| 7545-7558 | TCACACACCGATTGG DEL | 26994 | T-C |
| 7672 | G DEL | 27838 | G-T |
| 7933 | T-C | 27861 | T DEL |
| 8746 | T-G | 28132 | G-A |
| 9115 | G-A | 29100 | G-A |
| 9823 | G-A | 29454-29457 | TTTT DEL |
| 10027 | G-A | 29787 | T-G |
| 10214 | C-T | 29825 | A-C |
| 10828 | A-G | 30009 | T-C |
| 10918 | C-G | 30177 | A-G |
| 10955 | A-G | 30400 | A-G |
| 11524 | C-A | 31059 | T-A |
| 11674 | A-G | 31280 | C-T |
| 11955 | T-C | 31749 | C-T |
| 12173-12175 | TTT DEL | 32040 | C-G |
| 13304 | G-A | 32556-32559 | TGTG DEL |
| 13455 | G-A | 33017 | T-G |
| 14416-14417 | A INS | 33026 | T DEL |
| 14998 | C-T | 34434 | C-T |
| 15564 | T-C | 35179 | A-C |
| 15887 | A-G | 35695 | G-A |
| 15904-15919 | CCAAACTGATCTTTGA DEL | 35702 | G-A |
| 16019 | T DEL | 35983 | A-G |
| 16211 | A-T | 37411 | A-G |
| 17461 | A-G | 38526 | C-T |
| 40431 | C-A | 72688 | C-G |
| 42054-42055 | TT DEL | 75323-75324 | T INS |
| 43783-43784 | TTTT INS | 75887 | G-C |
| 45120 | C DEL | 77519 | T-C |
| 45567 | A-C | 77749 | G-A |
| 46601 | A-T | 77908 | T-C |
| 47255 | C-G | 78385 | C-G |
| 47758 | C-A | 78592-78593 | AG INS |
| 47994 | G-C | 80189 | T-G |
| 48440 | G-A | 80279 | T DEL |
| 48650 | T-G | 80989-80990 | A INS |
| 48680 | A-G | 81193 | T-C |
| 50240 | C-T | 81273 | A DEL |
| 50553 | G-A | 82166 | G-A |
| 50586 | G-T | 83847 | T DEL |
| 51322 | G-C | 84161-84162 | CA-GG |
| 51747 | A-G | 84533 | A-G |
| 52474 | C-G | 84638 | T-G |
| 52733 | C-A | 85526 | T-G |
| 52875 | G-A | 85705 | G-T |
| 53631-53637 | TTTTTTT DEL | 86984 | T-C |
| 53707 | G-A | 87655 | T-C |
| 54819 | A-G | 87713 | A-C |
| 55913 | T-C | 87892 | C-T |
| 56225 | A-C | 88192 | T DEL |
| 56510 | T-C | 88528 | A-G |
| 56566 | G-A | 89645 | A-T |
| 56618 | A-T | 89728 | A-G |
| 57815 | A-G | 90088 | T-C |
| 58011 | T DEL | 91193-91194 | 2209bp INS |
| 58247-58248 | TINS | 91373 | T-C |
| 58926 | C-G | 91433-91434 | A INS |
| 59406 | C-G | 91747 | G-A |
| 59422 | G-C | 93625 | T DEL |
| 60221-60222 | A INS | 95116-95117 | T INS |
| 60656-60657 | CA DEL | 96315 | G-A |
| 61162 | G-A | 97981 | A-G |
| 61465 | G-A | 98351 | T DEL |
| 61607 | A DEL | 99249 | C-T |
| 61653 | T-C | 100094-100095 | T INS |
| 61794-61795 | T INS | 100647-100648 | TTC INS |
| 62061 | G-C | 100951 | C-T |
| 62362 | T-G | 101610 | C-G |
| 62732 | C-G | 102589 | C-T |
| 63364 | G-A | 103076-103077 | TATATATATATATA INS |
| 63430-63431 | GT INS | 103747 | T-C |
| 63754 | C-T | 105638 | A-C |
| 63785 | A-C | 107024 | C-T |
| 63870-63871 | A INS | 107322 | C-T |
| 64788 | A-G | 107858 | C-G |
| 64962 | G-A | 109019 | A DEL |
| 65891 | C-T | 109579 | T DEL |
| 66675 | G-C | 110021 | C-A |
| 67186-67187 | ATT INS | 111251 | C-A |
| 67746-67747 | TT INS | 111425 | G-A |
| 68259 | T-C | 112644 | T-A |
| 68836 | T-C | 113001 | G-C |
| 68976 | C-G | 113130 | C-T |
| 72508 | T-G | | G-A |
| 114250 | A DEL | 176222 | T-C |
| 115217 | C-G | 176524 | A-T |
| 117995 | G-A | 176684 | G-A |
| 118874 | A-G | 176815 | T-C |
| 119470 | T-C | 177049 | T-C |
| 119646 | G-T | 177065 | G-T |
| 120853 | C-T | 1178285 | T-C |
| 121582 | G-A | 178551-178552 | CTTTTTTTTTTTTT INS |
| 123576 | A-C | 179114-179115 | A INS |
| 125581 | C-T | 179260 | C-G |
| 125970 | G-T | 179281 | C-G |
| 126197 | A-G | 180023 | G-C |
| 126672 | A DEL | 180430 | T-C |
| 126672 | G-C | 180773 | T-C |
| 128220-128221 | A INS | 180824 | T-C |
| 132569 | C-T | 181097 | C-T |
| 133572 | A-C | 181183 | A-T |
| 134064 | T-G | 182351 | C-T |
| 136999 | G-A | 183197 | G-A |
| 137784 | C-T | 183623 | A-T |
| 138903 | G-A | 183653 | G-T |
| 139159-139160 | A INS | 183657 | T-G |
| 140359 | G-A | 183795-183796 | A INS |
| 140898 | C-T | 184060 | G-A |
| 141313 | C DEL | 184993 | G-A |
| 141343 | T-C | 185918 | A-G |
| 142148 | T-C | 186036 | T-C |
| 142178 | C-A | 186506-186507 | TAAC INS |
| 142433-142434 | ATAGA INS | 186561-186568 | TATTTATT DEL |
| 143783 | C-T | 186690 | G DEL |
| 144090 | C-T | 186751 | T-A |
| 144220-144221 | A INS | 187221 | A-G |
| 144725 | A-C | 187260 | A-G |
| 145732-145733 | AAAAAAAAAAAAAA INS | 187444-187447 | CTCT DEL |
| 147016-147017 | CG DEL | 187831-187832 | C INS |
| 147021 | G-T | 188638 | G-A |
| 147536 | T-G | 188642 | C-T |
| 148936 | T-A | 189246 | T-C |
| 149061 | T-C | 190340 | A-C |
| 154341 | A-T | 190354 | A-G |
| 154588 | G-A | 190762 | A-G |
| 155464 | G-A | 191260 | G-T |
| 158574 | C-G | 193018-193019 | AGAT INS |
| 160007 | C-T | 193147 | T-G |
| 164348 | A-T | 193196-193197 | C INS |
| 164499 | C-G | 193499 | C-T |
| 166677-166678 | INS | 193738 | C-G |
| 167389 | G-A | 193984-193985 | ACACACAC INS |
| 168506-168507 | AGGATGGTCT INS | 194064 | C-G |
| 168515 | T-C | 194504 | A DEL |
| 169413-169414 | AA INS | 194734 | G-A |
| 170300-170301 | TTGTTGTTGTTG INS | 194890 | A-C |
| 170491 | G-A | 195404 | G-A |
| 173428 | T-C | 195693 | A-T |
| 173642 | G-A | 196205 | G-A |
| 173948 | T-G | 197424 | C-T |
| 175330 | T-C | 197513 | C-T |
| 175836 | T-C | 197670 | G-A |
| 176200 | G-C | 198055 | C-A |
| 198401 | C-T | 215947 | C-A |
| 198692 | A-G | 216232 | A-G |
| 198780 | T DEL | 217478 | G-A |
| 199030 | T-G | 219052 | T-C |
| 199933 | C-T | 219082-219083 | ATATATATATATATATATAT INS |
| 200027 | G-A | 219314 | C-A |
| 200439 | T-A | 219327 | G-A |
| 200452 | A-G | 219560 | C-T |
| 200472-200483 | AATAATAATAAT DEL | 219660 | C-T |
| 200559 | A-T | 219889 | G-A |
| 200745 | A-G | 220198 | G-T |
| 200919 | T-A | 220384 | G-A |
| 201816 | C-T | 220451-220452 | CAAAAAINS |
| 201861-201862 | 42bp INS | 221363 | G-A |
| 202662 | T-C | 221645 | G-A |
| 202880 | T-C | 222119 | T-C |
| 204341 | C-T | 222358 | A-G |
| 204768 | A-T | 222367 | A-C |
| 205284 | T-G | 222686 | A-G |
| 207400 | C-A | 222959 | T-C |
| 208634 | T-C | 223270-223271 | TT DEL |
| 208718 | T DEL | 223283 | T-C |
| 208862 | A-C | 224964 | T-C |
| 209419-209420 | TT DEL | 225232 | A-C |
| 209802 | G-A | 225366-225367 | TTTT INS |
| 209944 | C-G | 225416 | G-C |
| 210299 | A-G | 225486 | T-C |
| 211142 | G-A | 226088 | A-G |
| 212072 | G-A | 228421 | A-G |
| 212146 | T-C | 230047 | G-A |
| 212379 | G-A | 230109 | G-C |
| 212637-212639 | TCT DEL | 230376 | C-G |
| 212696 | T-C | 230394 | A-G |
| 213042 | T-A | 231226 | A-G |
| 214192 | A-G | 231447 | G-A |
| 214529-214530 | TTTTTTTTTT INS | 231835 | A-G |
| 214549 | T-C | 232400-232402 | AAA DEL |
| 214795 | C-T | 232402-232403 | G INS |
| 214908 | T-G | 232515 | T-C |
| 214977 | A-G | 232703 | G-T |
| 215769 | C-T | 232750 | A-G |

| | | | |
|---|---|---|---|
| * D6S2238 occurs at base 1. 24d1 occurs at base 41316. D6S2239 occurs at base 84841. D6S2241 occurs at base 235032 | | | |

**Table 2. Polymorphic Allele Frequencies**

| **Location** | **Frequency of ancestral variant in random chromosomes** | **Frequency of unaffected variant in random chromosomes** |
|---|---|---|
| 232703 | 53% | 47% |
| 231835 | 53% | 47% |
| 230394 | 85% | 15% |
| 230376 | 25% | 75% |
| 230109 | 53% | 47% |
| 225486 | 45% | 55% |
| 225416 | 75% | 25% |
| 220198 | 43% | 57% |
| 219660 | 58% | 42% |
| 219560 | 53% | 47% |
| 214977 | 65% | 35% |
| 214908 | 50% | 50% |
| 214795 | 24% | 76% |
| 214549 | 53% | 47% |
| 214192 | 65% | 35% |
| 210299 | 53% | 47% |
| 208862 | 80% | 20% |
| 208634 | 48% | 52% |
| 207400 | 25% | 75% |
| 205284 | 50% | 50% |
| 204341 | 53% | 47% |
| 202880 | 58% | 42% |
| 202662 | 98% | 2% |
| 200027 | 25% | 75% |
| 199030 | 58% | 42% |
| 198692 | 55% | 45% |
| 198401 | 55% | 45% |
| 198055 | 55% | 45% |
| 195693 | 60% | 40% |
| 195404 | 25% | 75% |
| 194890 | 55% | 45% |
| 175330 | 53% | 47% |
| 173948 | 83% | 17% |
| 173642 | 55% | 45% |
| 173428 | 80% | 20% |
| 168515 | 80% | 20% |
| 160007 | 18% | 82% |
| 149061 | 58% | 42% |
| 148936 | 82% | 18% |
| 147536 | 100% | 0% |
| 147021 | 46% | 54% |
| 141343 | 55% | 45% |
| 140359 | 55% | 45% |
| 138903 | 55% | 45% |
| 132569 | 81% | 19% |
| 125581 | 18% | 82% |
| 121582 | 80% | 20% |
| 120853 | 18% | 82% |
| 118874 | 85% | 15% |
| 115217 | 50% | 50% |
| 113130 | 40% | 60% |
| 113001 | 48% | 52% |
| 107858 | 48% | 52% |
| 103747 | 50% | 50% |
| 96315 | 25% | 75% |
| 91194 | 80% | 20% |
| 90088 | 75% | 25% |
| 89728 | 50% | 50% |
| 89645 | 50% | 50% |
| 88528 | 63% | 37% |
| 87892 | 75% | 25% |
| 87713 | 60% | 40% |
| 87655 | 50% | 50% |
| 86984 | 79% | 21% |
| 85705 | 50% | 50% |
| 85526 | 50% | 50% |
| 84638 | 50% | 50% |
| 84533 | 50% | 50% |
| 82166 | 78% | 22% |
| 81193 | 58% | 42% |
| 80189 | 50% | 50% |
| 78385 | 80% | 20% |
| 77908 | 88% | 12% |
| 68976 | 50% | 50% |
| 68259 | 51% | 49% |
| 66675 | 80% | 20% |
| 62732 | 50% | 50% |
| 62362 | 40% | 60% |
| 61653 | 48% | 52% |
| 61465 | 5% | 95% |
| 61162 | 60% | 40% |
| 53707 | 100% | 0% |
| 52875 | 50% | 50% |
| 52733 | 74% | 26% |
| 52474 | 47% | 53% |
| 50586 | 50% | 50% |
| 50553 | 50% | 50% |
| 50240 | 50% | 50% |
| 48680 | 53% | 47% |
| 48650 | 63% | 37% |
| 48440 | 50% | 50% |
| 47255 | 50% | 50% |
| 46601 | 53% | 47% |
| 45567 | 49% | 51% |
| 41316 | 5% | 95% |
| 40431 | 20% | 80% |
| 38526 | 23% | 77% |
| 37411 | 70% | 30% |
| 35983 | 5% | 95% |

These polymorphisms provide surrogate markers for use in diagnostic assays to detect the likely presence of the mutations 24d1 and/or 24d2, in preferably 24d1, in homozygotes or heterozygotes. Thus, for example, DNA or RNA from an individual is assessed for the presence or absence of a genotype defined by a polymorphic allele of Table 1, wherein, as a result, the absence of a genotype defined by a polymorphic allele of Table 1 indicates the likely absence of the HFE gene mutation in the genome of the individual and the presence of the genotype indicates the likely presence of the HFE gene mutation in the genome of the individual.

These markers may be used singly, in combination with each other, or with other polymorphic markers (such as those disclosed in co-pending PCT application WO 96/06583) in diagnostic assays for the likely presence of the HFE gene mutation in an individual. For example, any of the markers defined by the polymorphic sites of Table 1 can be used in diagnostic assays in combination with 24d1 or 24d2, or at least one of polymorphisms HHP-1, HHP-19, or HHP-29, or microsatellite repeat alleles 19D9:205; 18B4:235; 1A2:239; 1 E4:271; 24E2:245; 2B8:206; 3321-1:98; 4073-1:182; 4440-1:180; 4440-2:139; 731-1:177; 5091-1:148; 3216-1:221; 4072-2:170; 950-1:142; 950-2:164; 950-3:165; 950-4:128; 950-6:151; 950-8:137; 63-1:151; 63-2:113; 63-3:169; 65-1:206; 65-2:159; 68-1:167; 241-5:108; 241-29:113; 373-8:151; and 373-29:113, D6S258:199, D6S265:122, D6S105:124; D6S306:238; D6S464:206; and D6S100.1:180.

Table 2 lists the frequency of about 100 of the alleles defined by the polymorphic sites identified. As is evident from the Table, certain of these alleles are present rarely in the general population. These polymorphisms are thus preferred as surrogate markers in diagnostic assays for the presence of a mutant HFE allele ("gene mutation") such as 24d1 or 24d2. Preferably, the frequency of the polymorphic allele used in the diagnostic assay in the general population is less than about 50%, more preferably less than about 25%, and most preferably less than about 5%. Thus, of the genotypes defined by the alleles listed in Table 2, polymorphisms occurring at base 35983 and base 61465 of Figure 1 are preferred.

It will be understood by those of skill in the art that because they were identified in an ancestral HH homozygote, the haplotypes defined by the polymorphic sites of Table 1 are predictive of the likely presence of the HFE gene mutation 24d1. Thus, for example, the likelihood of any affected individual having at least two or more of any of the polymorphic alleles defined by Table 1 is greater than that for any unaffected individual. Similarly, the likelihood of any affected individual having at least three or more of any of the polymorphic alleles defined by Table 1 is greater than that for any unaffected individual.

Thus, for example, in a diagnostic assay for the likely presence of the HFE gene mutation in the genome of the individual, DNA or RNA from the individual is assessed for the presence or absence of a haplotype of Table 1, wherein, as a result, the absence of a haplotype of Table 1 indicates the likely absence of the HFE gene mutation in the genome of the individual and the presence of the haplotype indicates the likely presence of the HFE gene mutation in the genome of the individual.

The markers defined by the polymorphic sites of Table 1 are additionally useful as markers for genetic analysis of the inheritance of certain HFE alleles and other genes which occur within the chromosomal region corresponding to the sequence of Figure 9 which include, for example, those disclosed in copending U.S.S.N. 08/724,394.

As the entire nucleotide sequence of the region is provided in Figure 9, it will be evident to those of ordinary skill in the art which sequences to use as primers or probes for detecting each polymorphism of interest. Thus, the nucleotide sequences include at least one oligonucleotide pair selected from the sequence of Figure 9 or its complement for amplification of a polymorphic site of Table 1. Furthermore, in some embodiments a preferred hybridization probe is an oligonucleotide comprising at least 8 to about 100 consecutive bases from the sequence of Figure 9, or the complement of the sequence, wherein the at least 8 to about 100 consecutive bases includes at least one polymorphic site of Table 1. In some embodiments the polymorphic site is at base 35983 or base 61465.

It will also be appreciated that the nucleic acid sequences include isolated nucleic acid molecules comprising about 100 consecutive bases to about 235 kb substantially identical to the sequence of Figure 9, wherein the DNA molecule comprises at least one polymorphic site of Table 1. Such isolated DNA sequences are useful as primers, probes, or as the component of a kit in diagnostic assays for detecting the likely presence of the HFE gene mutation in an individual.

### D. Nucleic Acid Based Screening

Individuals carrying polymorphic alleles of the invention may be detected at either the DNA, the RNA, or the protein level using a variety of techniques that are well known in the art. The genomic DNA used for the diagnosis may be obtained from body cells, such as those present in peripheral blood, urine, saliva, bucca, surgical specimen, and autopsy specimens. The DNA may be used directly or may be amplified enzymatically *in vitro* through use of PCR (Saiki et al. Science 239:487-491 (1988)) or other *in vitro* amplification methods such as the ligase chain reaction (LCR) (Wu and Wallace Genomics 4:560-569 (1989)), strand displacement amplification (SDA) (Walker et al. Proc. Natl. Acad. Sci. U.S.A. 89:392-396 (1992)), self-sustained sequence replication (3SR) (Fahy et al. PCR Methods Appl. 1:25-33 (1992)), prior to mutation analysis. The methodology for preparing nucleic acids in a form that is suitable for mutation detection is well known in the art.

The detection of polymorphisms in specific DNA sequences, such as in the region of the HFE gene, can be accomplished by a variety of methods including, but not limited to, restriction-fragment-length-polymorphism detection based on allele-specific restriction-endonuclease cleavage (Kan and Dozy Lancet ii:910-912 (1978)), hybridization with allele-specific oligonucleotide probes (Wallace et al. Nucl Acids Res 6:3543-3557 (1978)), including immobilized oligonucleotides (Saiki et al. Proc. Natl. Acad. Sci. U.S.A. 86:6230-6234 (1989)) or oligonucleotide arrays (Maskos and Southern Nucl Acids Res 21:2269-2270 (1993)), allele-specific PCR (Newton et al. Nucl Acids Res 17:2503-2516 (1989)), mismatch-repair detection (MRD) (Faham and Cox Genome Res 5:474-482 (1995)), binding of MutS protein (Wagner et al. Nucl Acids Res 23:3944-3948 (1995), denaturing-gradient gel electrophoresis (DGGE) (Fisher and Lerman et al. Proc. Natl. Acad. Sci. U.S.A. 80:1579-1583 (1983)), single-strand-conformation-polymorphism detection (Orita et al. Genomics 5:874-879 (1983)), RNAase cleavage at mismatched base-pairs (Myers et al. Science 230:1242 (1985)), chemical (Cotton et al. Proc. Natl. Acad. Sci. U.S.A. 85:4397-4401 (1988)) or enzymatic (Youil et al. Proc. Natl. Acad. Sci. U.S.A. 92:87-91 (1995)) cleavage of heteroduplex DNA, methods based on allele specific primer extension (Syvänen et al. Genomics 8:684-692 (1990)), genetic bit analysis (GBA) (Nikiforov et al. Nucl Acids Res 22:4167-4175 (1994)), the oligonucleotide-ligation assay (OLA) (Landegren et al. Science 241:1077 (1988)), the allele-specific ligation chain reaction (LCR) (Barrany Proc. Natl. Acad. Sci. U.S.A. 88:189-193 (1991)), gap-LCR (Abravaya et al. Nucl Acids Res 23:675-682 (1995)), radioactive and/or fluorescent DNA sequencing using standard procedures well known in the art, and peptide nucleic acid (PNA) assays (Orum et al., Nucl. Acids Res. 21:5332-5356 (1993); Thiede et al., Nucl. Acids Res. 24:983-984 (1996)).

In addition to the genotypes defined by the polymorphisms as described In co-pending PCT application WO 96/35802 published November 14, 1996, genotypes characterized by the presence of the alleles 19D9:205; 18B4:235; 1A2:239; 1E4:271; 24E2:245; 2B8:206; 3321-1:98 (denoted 3321-1:197 therein); 4073-1:182; 4440-1:180; 4440-2:139; 731-1:177; 5091-1:148; 3216-1:221; 4072-2:170 (denoted 4072-2:148 therein); 950-1:142; 950-2:164; 950-3:165; 950-4:128; 950-6:151; 950-8:137; 63-1:151; 63-2:113; 63-3:169; 65-1:206; 65-2:159; 68-1:167; 241-5:108; 241-29:113; 373-8:151; and 373-29:113, alleles D6S258:199, D6S265:122, D6S105:124, D6S306:238, D6S464:206; and D6S1001:180, and/or alleles associates with the HHP-1, the HHP-19 or HHP-29 single base-pair polymorphisms can also be used to assist in the identification of an individual whose genome contains 24d1 and/or 24d2. For example, the assessing step can be performed by a process which comprises subjecting the DNA or RNA to amplification using oligonucleotide primers flanking a polymorphism of Table 1, and oligonucleotides flanking 24d1 and/or 24d2, oligonucleotide primers flanking at least one of the base-pair polymorphisms HHP-1, HHP-19, and HHP-29, oligonucleotide primers flanking at least one of the microsatellite repeat alleles, or oligonucleotide primers for any combination of polymorphisms or microsatellite repeat alleles thereof.

Oligonucleotides useful in diagnostic assays are typically at least 8 consecutive nucleotides in length, and may range upwards of 18 nucleotides in length to greater than 100 or more consecutive nucleotides. Such oligonucleotides can be derived from either the genomic DNA of Figure 8 or 9, or cDNA sequences derived therefrom, or may be synthesized.

Additionally, the proteins encoded by such cDNAs are useful in the generation of antibodies for analysis of gene expression and in diagnostic assays, and in the purification of related proteins.

### E. General Methods

The nucleic acid compositions of this invention, whether RNA, cDNA, genomic DNA, or a hybrid of the various combinations, may be isolated from natural sources, including cloned DNA, or may be synthesized *in vitro*. The nucleic acids claimed may be present in transformed or transfected whole cells, in a transformed or transfected cell lysate, or in a partially purified or substantially pure form.

Techniques for nucleic acid manipulation of the nucleic acid sequences of the invention such as subcloning nucleic acid sequences encoding polypeptides into expression vectors, labeling probes, DNA hybridization, and the like are described generally in Sambrook et al., Molecular Cloning - a Laboratory Manual (2nd Ed.), Vol. 1-3, Cold Spring Harbor Laboratory, Cold Spring Harbor, New York, (1989), which is incorporated herein by reference. This manual is hereinafter referred to as "Sambrook *et al."*

There are various methods of isolating the nucleic acid sequences of the invention. For example, DNA is isolated from a genomic or cDNA library using labeled oligonucleotide probes having sequences complementary to the sequences disclosed herein. Such probes can be used directly in hybridization assays. Alternatively probes can be designed for use in amplification techniques such as PCR.

To prepare a cDNA library, mRNA is isolated from tissue such as heart or pancreas, preferably a tissue wherein expression of the gene or gene family is likely to occur. cDNA is prepared from the mRNA and ligated into a recombinant vector. The vector is transfected into a recombinant host for propagation, screening and cloning. Methods for making and screening cDNA libraries are well known. See Gubler, U. and Hoffman, B.J. Gene 25:263-269 (1983) and Sambrook *et al.*

For a genomic library, for example, the DNA is extracted from tissue and either mechanically sheared or enzymatically digested to yield fragments of about 12-20 kb. The fragments are then separated by gradient centrifugation from undesired sizes and are constructed in bacteriophage lambda vectors. These vectors and phage are packaged *in vitro,* as described in Sambrook, *et a*/*.* Recombinant phage are analyzed by plaque hybridization as described in Benton and Davis, Science 196:180-182 (1977). Colony hybridization is carried out as generally described in M. Grunstein et al. Proc. Natl. Acad. Sci. USA. 72:3961-3965 (1975).

DNA of interest is identified in either cDNA or genomic libraries by its ability to hybridize with nucleic acid probes, for example on Southern blots, and these DNA regions are isolated by standard methods familiar to those of skill in the art. *See* Sambrook, *et al.*

In PCR techniques, oligonucleotide primers complementary to the two 3' borders of the DNA region to be amplified are synthesized. The polymerase chain reaction is then carried out using the two primers. See PCR Protocols: a Guide to Methods and Applications (Innis, M, Gelfand, D., Sninsky, J. and White, T., eds.), Academic Press, San Diego (1990). Primers can be selected to amplify the entire regions encoding a full-length sequence of interest or to amplify smaller DNA segments as desired.

PCR can be used in a variety of protocols to isolate cDNA's encoding a sequence of interest. In these protocols, appropriate primers and probes for amplifying DNA encoding a sequence of interest are generated from analysis of the DNA sequences listed herein. Once such regions are PCR-amplified, they can be sequenced and oligonucleotide probes can be prepared from sequence obtained.

Oligonucleotides for use as primers or probes are chemically synthesized according to the solid phase phosphoramidite triester method first described by Beaucage, S.L. and Carruthers, M.H., Tetrahedron Lett., 22(20):1859-1862 (1981) using an automated synthesizer, as described in Needham-VanDevanter, D.R., et al., Nucleic Acids Res. 12:6159-6168 (1984). Purification of oligonucleotides is by either native acrylamide gel electrophoresis or by anion-exchange HPLC as described in Pearson, J.D. and Regnier, F.E., J. Chrom., 255:137-149 (1983). The sequence of the synthetic oligonucleotide can be verified using the chemical degradation method of Maxam, A.M. and Gilbert, W., in Grossman, L. and Moldave, D., eds. Academic Press, New York, Methods in Enzymology 65:499-560 (1980).

### 1. Expression

Once DNA encoding a sequence of interest is isolated and cloned, one can express the encoded proteins in a variety of recombinantly engineered cells. It is expected that those of skill in the art are knowledgeable in the numerous expression systems available for expression of DNA encoding a sequence of interest. No attempt to describe in detail the various methods known for the expression of proteins in prokaryotes or eukaryotes is made here.

In brief summary, the expression of natural or synthetic nucleic acids encoding a sequence of interest will typically be achieved by operably linking the DNA or cDNA to a promoter (which is either constitutive or inducible), followed by incorporation into an expression vector. The vectors can be suitable for replication and integration in either prokaryotes or eukaryotes. Typical expression vectors contain transcription and translation terminators, initiation sequences, and promoters useful for regulation of the expression of polynucleotide sequence of interest. To obtain high level expression of a cloned gene, it is desirable to construct expression plasmids which contain, at the minimum, a strong promoter to direct transcription, a ribosome binding site for translational initiation, and a transcription/translation terminator. The expression vectors may also comprise generic expression cassettes containing at least one independent terminator sequence, sequences permitting replication of the plasmid in both eukaryotes and prokaryotes, *i.e.,* shuttle vectors, and selection markers for both prokaryotic and eukaryotic systems. *See* Sambrook *et al.* Examples of expression of ATP-sensitive potassium channel proteins in both prokaryotic and eukaryotic systems are described below.

### a. Expression in Prokaryotes

A variety of procaryotic expression systems may be used to express proteins. Examples include *E. coli, Bacillus, Streptomyces,* and the like.

It is preferred to construct expression plasmids which contain, at the minimum, a strong promoter to direct transcription, a ribosome binding site for translational initiation, and a transcription/translation terminator. Examples of regulatory regions suitable for this purpose in *E. coli* are the promoter and operator region of the *E. coli* tryptophan biosynthetic pathway as described by Yanofsky, C., J. Bacteriol. 158:1018-1024 (1984) and the leftward promoter of phage lambda (Pλ) as described by Herskowitz, I. and Hagen, D., Ann. Rev. Genet. 14:399-445 (1980). The inclusion of selection markers in DNA vectors transformed in *E. coli* is also useful. Examples of such markers include genes specifying resistance to ampicillin, tetracycline, or chloramphenicol. See Sambrook *et al.* for details concerning selection markers for use in *E. coli.*

To enhance proper folding of the expressed recombinant protein, during purification from *E. coli,* the expressed protein may first be denatured and then renatured. This can be accomplished by solubilizing the bacterially produced proteins in a chaotropic agent such as guanidine HCl and reducing all the cysteine residues with a reducing agent such as beta-mercaptoethanol. The protein is then renatured, either by slow dialysis or by gel filtration. See U.S. Patent No. 4,511,503.

Detection of the expressed antigen is achieved by methods known in the art as radioimmunoassay, or Western blotting techniques or immunoprecipitation. Purification from *E. coli* can be achieved following procedures such as those described in U.S. Patent No. 4,511,503.

### b. Expression in Eukaryotes

A variety of eukaryotic expression systems such as yeast, insect cell lines, bird, fish, and mammalian cells, are known to those of skill in the art. As explained briefly below, a sequence of interest may be expressed in these eukaryotic systems.

Synthesis of heterologous proteins in yeast is well known. Methods in Yeast Genetics, Sherman, F., et al., Cold Spring Harbor Laboratory, (1982) is a well recognized work describing the various methods available to produce the protein in yeast.

Suitable vectors usually have expression control sequences, such as promoters, including 3-phosphoglycerate kinase or other glycolytic enzymes, and an origin of replication, termination sequences and the like as desired. For instance, suitable vectors are described in the literature (Botstein, et al., Gene 8:17-24 (1979); Broach, et al., Gene 8:121-133 (1979)).

Two procedures are used in transforming yeast cells. In one case, yeast cells are first converted into protoplasts using zymolyase, lyticase or glusulase, followed by addition of DNA and polyethylene glycol (PEG). The PEG-treated protoplasts are then regenerated in a 3% agar medium under selective conditions. Details of this procedure are given in the papers by J.D. Beggs, Nature (London) 275:104-109 (1978); and Hinnen, a., et al., Proc. Natl. Acad. Sci. U.S.A. 75:1929-1933 (1978). The second procedure does not involve removal of the cell wall. Instead the cells are treated with lithium chloride or acetate and PEG and put on selective plates (Ito, H., et al., J. Bact. 153:163-168 (1983)).

The proteins, once expressed, can be isolated from yeast by lysing the cells and applying standard protein isolation techniques to the lysates. The monitoring of the purification process can be accomplished by using Western blot techniques or radioimmunoassay or other standard immunoassay techniques.

The sequences encoding the proteins can also be ligated to various expression vectors for use in transforming cell cultures of, for instance, mammalian, insect, bird or fish origin. Illustrative of cell cultures useful for the production of the polypeptides are mammalian cells. Mammalian cell systems often will be in the form of monolayers of cells although mammalian cell suspensions may also be used. A number of suitable host cell lines capable of expressing intact proteins have been developed in the art, and Include the HEK293, BHK21, and CHO cell lines, and various human cells such as COS cell lines, HeLa cells, myeloma cell lines, Jurkat cells, etc. Expression vectors for these cells can include expression control sequences, such as an origin of replication, a promoter (*e.g*., the CMV promoter, a HSV *tk* promoter or *pgk* (phosphoglycerate kinase) promoter), an enhancer (Queen et al. Immunol. Rev. 89:49 (1986)), and necessary processing information sites, such as ribosome binding sites, RNA splice sites, polyadenylation sites (*e.g.,* an SV40 large T Ag poly A addition site), and transcriptional terminator sequences. Other animal cells useful for production of ATP-sensitive potassium channel proteins are available, for instance, from the American Type Culture Collection Catalogue of Cell Lines and Hybridomas (7th edition, (1992)).

Appropriate vectors for expressing proteins in insect cells are usually derived from the SF9 baculovirus. Suitable insect cell lines include mosquito larvae, silkworm, armyworm, moth and *Drosophila* cell lines such as a Schneider cell line (*See* Schneider J. Embryol. Exp. Morphol. 27:353-365 (1987).

As indicated above, the vector, *e.g.,* a plasmid, which is used to transform the host cell, preferably contains DNA sequences to initiate transcription and sequences to control the translation of the protein. These sequences are referred to as expression control sequences.

As with yeast, when higher animal host cells are employed, polyadenylation or transcription terminator sequences from known mammalian genes need to be incorporated into the vector. An example of a terminator sequence is the polyadenylation sequence from the bovine growth hormone gene. Sequences for accurate splicing of the transcript may also be included. An example of a splicing sequence is the VP1 intron from SV40 (Sprague, J. et al., J. Virol. 45: 773-781 (1983)).

Additionally, gene sequences to control replication in the host cell may be incorporated into the vector such as those found in bovine papilloma virus type-vectors. Saveria-Campo, M., 1985, "Bovine Papilloma virus DNA a Eukaryotic Cloning Vector" in DNA Cloning Vol. II a Practical Approach Ed. D.M. Glover, IRL Press, Arlington, Virginia pp. 213-238.

The host cells are competent or rendered competent for transformation by various means. There are several well-known methods of introducing DNA into animal cells. These include: calcium phosphate precipitation, fusion of the recipient cells with bacterial protoplasts containing the DNA, treatment of the recipient cells with liposomes containing the DNA, DEAE dextran, electroporation and micro-injection of the DNA directly into the cells.

The transformed cells are cultured by means well known in the art (Biochemical Methods in Cell Culture and Virology, Kuchler, R.J., Dowden, Hutchinson and Ross, Inc., (1977)). The expressed polypeptides are isolated from cells grown as suspensions or as monolayers. The latter are recovered by well known mechanical, chemical or enzymatic means.

### 2. Purification

The proteins produced by recombinant DNA technology may be purified by standard techniques well known to those of skill in the art. Recombinantly produced proteins can be directly expressed or expressed as a fusion protein. The protein is then purified by a combination of cell lysis (e.g., sonication) and affinity chromatography. For fusion products, subsequent digestion of the fusion protein with an appropriate proteolytic enzyme releases the desired polypeptide.

The polypeptides of this invention may be purified to substantial purity by standard. techniques well known in the art, including selective precipitation with such substances as ammonium sulfate, column chromatography, immunopurification methods, and others. See, for instance, R. Scopes, Protein Purification: Principles and Practice, Springer-Verlag: New York (1982), incorporated herein by reference. For example, in an embodiment, antibodies may be raised to the proteins of the invention as described herein. Cell membranes are isolated from a cell line expressing the recombinant protein, the protein is extracted from the membranes and immunoprecipitated. The proteins may then be further purified by standard protein chemistry techniques as described above.

The following examples are provided to illustrate the invention but not to limit its scope. Other variants of the invention will be readily apparent to one of ordinary skill in tie art and are encompassed by the appended claims.

### F. EXPERIMENTAL EXAMPLES

### Sequencing of 235 kb from a Homozygous Ancestral (Affected) Individual

In these studies the entire genomic sequence was determined from an HH affected individual for a region corresponding to a 235,033 bp region surrounding the HFE gene between the flanking markers D6S2238 and D6S2241. The sequence was derived from a human lymphoblastoid cell line, HC14, that is homozygous for the ancestral HH mutation and region. The sequence from the ancestral chromosome (Figure 9) was compared to the sequence of the region in an unaffected individual (Figure 8) disclosed in copending U.S.S.N. 08/724,394 to identify polymorphic sites. A subset of the polymorphic alleles so defined were further studied to determine their frequency in a collection of random individuals.

The cell line HC14 was deposited with the ATCC om June 25, 1997, and is designated ATCC CRL-12371.

### a. Cosmid Library Screening

The strategy and methodology for sequencing the genomic DNA for the affected individual was essentially as described in copending U.S.S.N. 08/724,394. Basically, a cosmid library was constructed using high molecular weight DNA from HC14 cells. The library was constructed in the supercos vector (Stratagene, La Jolla, CA). Colonies were replicated onto Biotrans nylon filters (ICN) using standard techniques. Probes from genomic subclones used in the generation of the sequence of the unaffected sequence disclosed in 08/724,394 were isolated by gel electrophoresis and electroporation. Subclones were chosen at a spacing of approximately 20 kb throughout the 235 kb region. The DNA was labeled by incorporation of 32P dCTP by the random primer labeling approach. Positively hybridizing clones were isolated to purity by a secondary screening step. Cosmid insert ends were sequenced to determine whether full coverage had been obtained, and which clones formed a minimal path of cosmids through the 235 kb region.

### b. Sample Sequencing

A minimal set of cosmid clones chosen to cover the 235 kb region were prepped with the Qiagen Maxi-Prep system. Ten micrograms of DNA from each cosmid preparation were sonicated in a Heat Systems Sonicator XL and end-repaired with Klenow (USB) and T4 DNA polymerase (USB). The sheared fragments were size selected between three to four kilobases on a 0.7% agarose gel and then ligated to BstXl linkers (Invitrogen). The ligations were gel purified on a 0.7% agarose gel and cloned into a pSP72 derivative plasmid vector. The resulting plasmids were transformed into electrocompetent DH5α cells and plated on LB-carbenicillin plates. A sufficient number of colonies was picked to achieve 15-fold clone coverage. The appropriate number of colonies was calculated by the following equation to generate a single-fold sequence coverage: Number of colonies = size of bacterial clone (in kb)/average sequence read length (0.4 kb). These colonies were prepped in the 96-well Qiagen REAL, and the 5' to 3' DNA Prep Kit, and AGCT end-sequenced with oligo MAP1 using standard ABl Dye Terminator protocols. MAP1 was CGTTAGAACGCGGCTACAAT.

### c. Genomic Sequencing

The MAP1 sequences from the cosmid clones HC182, HC187, HC189, HC195, HC199, HC200, HC201, HC206, HC207, and HC212 were assembled into contigs with the Staden package (available from Roger Staden, MRC). A minimal set of 3 kb clones was selected for sequencing with oligo labeled MAP2 that sits on the opposite end of the plasmid vector. The sequence of MAP2 was GCCGATTCATTAATGCAGGT. The MAP2 sequences were entered into the Staden database in conjunction with the MAP1 sequences to generate a tiling path of 3 kb clones across the region. The plasmid 3 kb libraries were concurrently transformed in 96 well format into pox38UR (available from C. Martin, Lawrence Berkeley Laboratories). The transformants were subsequently mated with JGM (Strathman et al. P.N.A.S. 88:1247-1250 (1991) in 96 well format. All matings of the 3 kb clones within the tiling path were streaked on LB-carbenicillin-kanamycin plates and a random selection of 12 colonies per 3 kb clone was prepped in the AGCT system. The oligos -21:
CTGTAAAACGACGGCCAGTC, and REV: GCAGGAAACAGCTATGACC were used to sequence off both ends of the transposon. Each 3 kb clone was assembled in conjunction with the end sequence information from all cosmid clones in the region.

In some regions, the coverage of the genomic sequence by cosmids was incomplete. Any gaps in the sequence were filled by using standard PCR techniques to amplify genomic DNA in those regions and standard ABI dye terminator chemistry to sequence the amplification products.

### d. Identification of Polymorphic Sites

The assembled sequence of the cosmid clones in connection with the PCR amplified genomic DNA was compared to the genomic sequence of the unaffected individual using the FASTA algorithm. Numeric values were assigned to the sequenced regions of 1 to 235,303, wherein base 1 refers to the first C in the CA repeat of D6S2238 and base 235,303 is the last T in the GT repeat of D6S2241 of the unaffected sequence (Figure 8). Table 1 lists the differences between the two compared sequences. Note that previously disclosed (Feder et al., Natute Genetics 13:399-408 (1996)) polymorphic sites D6S2238 (base 1), D6S2241 (base 235,032), 24d1 (base 41316), and D6S2239 (base 84841) are not included in the list of new polymorphisms, although they are provided for reference in a footnote to the Table and were observed in the ancestral sequence. In the Table, a single base change such as C-T refers to a C in the unaffected sequence at the indicated base position that occurred as a T in the corresponding position in the affected sequence. Similarly, an insertion of one or more bases, such as TTT in the affected sequence, is represented as "TTT INS" between the indicated bases of the unaffected sequence. A deletion of one or more bases occurring in the affected sequence, such as AAA DEL, is represented as the deletion of the indicated bases in the unaffected sequence.

### e. Characterization of Rare Polymorphisms

In this study about 100 of the polymorphisms of Table 1 were arbitrarily chosen for further characterization. Allele frequencies in the general population were estimated by OLA analysis using a population of random DNAs (the "CEPH" collection, J. Dausset et al., Genomics 6(3):575-577 (1990)). These results are provided in Table 2.

One single base pair difference, occurring at base 35983 and designated C182.1G7T/C (an A to G change on the opposite strand) was present in the ancestral chromosome and rare in the random DNAs. This change occurred in a noncoding region of the hemochromatosis gene near exon 7 approximately 5.3 kb from the 24d1 (Cys282Tyr) mutation. OLA was used to genotype 90 hemochromatosis patients for the C182.1 G7T/C base pair change. The frequency for C occurring at this position in the patients was 79.4% as compared to 5% in the random DNAs. Eighty-five of the 90 patients assayed contained identical 24d1 and C182.1G7T/C genotypes. Four of the remaining 5 patients were homozygous at 24d1 and heterozygous at C182.1G7T/C; one was heterozygous at 24d1 and homozygous at C182.1 G7T/C. The primers used for this analysis were as follows.

### PCR primers for detection:

182.1G7.F 5'-GCATCAGCGATTAACTTCTAC -3'
182.1 G7.R 5'-TTGCATTGTGGTGAAATCAGGG -3'

For the detection assay, the biotinylated primers used were as follows.
182.1G7.C 5' (b)CTGAGTAATTGTTTAAGGTGC -3'
182.1G7.T 5' (b)CTGAGTAATTGTTTAAGGTGT -3'

The phosphorylated digoxigenin-labeled primer used was:
182.1G7.D 5' (p)AGAAGAGATAGATATGGTGG -3'

The above rare allele is to be used as a marker for 24d1 and is especially useful in screening assays for the likely presence of 24d1 and/or 24d2.

## Claims

1. A pair of PCR primers for use in detecting a common hereditary hemochromatosis (HFE) gene mutation in a sample comprising a first primer having a sequence from SEQ ID NO:21 and a second primer having a sequence from a reverse complement to SEQ ID NO:21, wherein the primers amplify under PCR conditions a polynucleotide consisting of a sequence of SEQ ID NO:21 that includes a single nucleotide polymorphism (SNP) at position 35935 of SEQ ID NO:21.

2. Use of a pair of PCR primers comprising a first primer having a sequence from SEQ ID NO:21 and a second primer having a sequence from a reverse complement to SEQ ID NO:21, wherein the first sequence from SEQ ID NO:21 consists of 5'-TTGCATTGTGGTGAAATCAGGG-3' and the second sequence consists of 5'-GCATCAGCGATTAACTTCTAC-3' and wherein the amplified polynucleotide includes a SNP at base 35935 of SEQ ID NO:21, for detecting a common hereditary hemochromatosis (HFE) gene mutation in a sample.

3. A vector comprising an oligonucleotide consisting of at least 8 to about 100 consecutive nucleotide bases from the sequence of SEQ ID NO:21, or its complement, including the polymorphic site at position 35935 of SEQ ID NO:21.

4. A host cell comprising the vector of claim 3.

## Patentansprüche

1. Paar von PCR Primern für die Verwendung beim Nachweis einer allgemeinen hereditären Hämochromatose (HFE) Genmutation in einer Probe umfassend einen ersten Primer mit einer Sequenz aus SEQ ID NO:21 und einen zweiten Primer mit einer Sequenz aus einem reversen Komplement zu SEQ ID NO:21, wobei die Primer unter PCR Bedingungen ein Polynukleotid bestehend aus einer Sequenz der SEQ ID NO:21, die einen Einzelnukleotid-Polymorphismus (SNP) an Position 35935 von SEQ ID NO:21 beinhaltet, amplifizieren.

2. Verwendung eines Paares von PCR Primern umfassend einen ersten Primer mit einer Sequenz aus SEQ ID NO:21 und einen zweiten Primer mit einer Sequenz aus einem reversen Komplement zu SEQ ID NO:21, wobei die erste Sequenz von SEQ ID NO:21 aus 5'-TTGCATTGTGGTGAAATCAGGG-3' besteht und die zweite Sequenz aus 5'-GCATCAGCGATTAACTTCTAC-3' besteht und wobei das amplifizierte Polynukleotid einen SNP an der Base 35935 von SEQ ID NO:21 beinhaltet, zum Nachweis einer allgemeinen hereditären Hämochromatose (HFE) Genmutation in einer Probe.

3. Vektor umfassend ein Oligonukleotid bestehend aus mindestens 8 bis ungefähr 100 aufeinander folgenden Nukleotidbasen der Sequenz SEQ ID NO:21 oder deren Komplement, einschließlich der polymorphen Stelle an Position 35935 von SEQ ID NO:21.

4. Wirtszelle umfassend den Vektor nach Anspruch 3.

## Revendications

1. Paire d'amorces de PCR destinée à être utilisée dans la détection d'une mutation du gène courant de l'hémochromatose héréditaire (HFE) dans un échantillon comprenant une première amorce ayant une séquence provenant de la SEQ ID NO: 21 et une deuxième amorce ayant une séquence provenant d'un complément inverse de la SEQ ID NO: 21, dans laquelle, dans des conditions de PCR, les amorces amplifient un polynucléotide constitué d'une séquence de la SEQ ID NO: 21 qui comprend un polymorphisme d'un seul nucléotide (SNP) à la position 35935 de la SEQ ID NO: 21.

2. Utilisation d'une paire d'amorces de PCR comprenant une première amorce ayant une séquence provenant de la SEQ ID NO: 21 et une deuxième amorce ayant une séquence provenant d'un complément inverse de la SEQ ID NO: 21, dans laquelle la première séquence provenant de la SEQ ID NO: 21 est constituée de 5'-TTGCATTGTGGTGAAATCAGGG-3' et la deuxième séquence est constituée de 5'-GCATCAGCGATTAACTTCTAC-3' et dans laquelle le polynucléotide amplifié comprend un SNP au niveau de la base 35935 de la SEQ ID NO: 21, pour la détection d'une mutation du gène courant de l'hémochromatose héréditaire (HFE) dans un échantillon.

3. Vecteur comprenant un oligonucléotide constitué d'au moins 8 jusqu'à environ 100 bases nucléotidiques consécutives provenant de la SEQ ID NO: 21, ou de son complément, comprenant le site polymorphe à la position 35935 de la SEQ ID NO: 21.

4. Cellule hôte comprenant le vecteur de la revendication 3.
